(19) Europäisches Patentamt
European Patent Office
Office européen des brevets

(11) EP 4 741 393 A2

(12) # EUROPEAN PATENT APPLICATION

(43) Date of publication:
**13.05.2026 Bulletin 2026/20**

(21) Application number: **26168575.4**

(22) Date of filing: **08.09.2023**

(51) International Patent Classification (IPC):
***C07D 471/04*** *(2006.01)*

(52) Cooperative Patent Classification (CPC):
**A61K 47/6803; A61K 47/6849; A61K 47/6855; A61K 47/6857; A61K 47/6859; A61K 47/6861; A61K 47/6863; A61K 47/6867; A61K 47/6869; A61P 35/00; C07D 471/04;** C07K 16/2827; C07K 16/2887; C07K 16/30; C07K 16/32

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **09.09.2022 EP 22194959**
**09.09.2022 EP 22194984**
**14.04.2023 GB 202305546**
**14.04.2023 GB 202305541**

(62) Document number(s) of the earlier application(s) in accordance with Art. 76 EPC:
**23772312.7 / 4 583 919**

(71) Applicants:
- **MyricX Pharma Limited**
  **London EC2V 7AW (GB)**
- **Imperial College Innovations Limited**
  **London SW7 2AZ (GB)**

(72) Inventors:
- **CARR, Robin**
  **London EC2V 7AW (GB)**
- **SOLARI, Roberto**
  **London EC2V 7AW (GB)**
- **TATE, Edward, William**
  **London SW7 2AZ (GB)**
- **BELL, Andrew, Simon**
  **London SW7 2AZ (GB)**
- **BONNERT, Roger**
  **1215 Geneva (CH)**

(74) Representative: **Sagittarius IP**
**Marlow International**
**Parkway**
**Marlow SL7 1YL (GB)**

Remarks:
- The complete document including Reference Table(s) and the Sequence Listing(s) can be downloaded from the EPO website
- This application was filed on 27-03-2026 as a divisional application to the application mentioned under INID code 62.

(54) **NOVEL COMPOUNDS AND THEIR USE IN THERAPY**

(57) The present invention relates to ADCs comprising a NMT inhibitor conjugated to an antibody *via* a linker, and related uses.

EP 4 741 393 A2

## Description

### Field of Invention

This invention relates to novel antibody drug conjugates (ADC) comprising an inhibitor of the human N-myristoyl transferases (human NMT). The invention also *inter alia* relates to such ADCs for use as medicaments, in particular, in the treatment or prevention of hyperproliferative disorders such as cancer.

### Background to the invention

Antibody therapy has been established for the targeted treatment of patients with cancer, immunological and angiogenic disorders (Carter, P. (2006) Nature Reviews Immunology 6:343-357). The use of antibody-drug conjugates (ADC), i.e. immunoconjugates, for the local delivery of cytotoxic or cytostatic agents, i.e. drugs to kill or inhibit tumor cells in the treatment of cancer, targets delivery of the drug moiety to tumors, and intracellular accumulation therein, whereas systemic administration of these unconjugated drug agents may result in unacceptable levels of toxicity to normal cells (Xie et al (2006) Expert. Opin. Biol. Ther. 6(3):281-291; Kovtun e tal (2006) Cancer Res. 66(6):3214-3121; Law et a/ (2006) Cancer Res. 66(4):2328-2337; Wu et al (2005) Nature Biotech. 23(9):1137-1145; Lambert J. (2005) Current Opin. in Pharmacol. 5:543-549; Hamann P. (2005) Expert Opin. Ther. Patents 15(9):1087-1103; Payne, G. (2003) Cancer Cell 3:207-212; Trail etal (2003) Cancer Immunol. Immunother. 52:328-337; Syrigos and Epenetos (1999) Anticancer Research 19:605-614).

Maximal efficacy with minimal toxicity is sought thereby. Efforts to design and refine ADC have focused on the selectivity of monoclonal antibodies (mAbs) as well as drug mechanism of action, drug-linking, drug/antibody ratio (loading), and drug-releasing properties (Junutula, et al., 2008b Nature Biotech., 26(8):925-932; Dornan et al (2009) Blood 114(13):2721-2729; US 7521541; US 7723485; WO2009/052249; McDonagh (2006) Protein Eng. Design & Sei. 19(7): 299-307; Doronina etal (2006) Bioconj. Chem. 17:114-124; Erickson etal (2006) Cancer Res. 66(8):1-8; Sanderson etal (2005) Clin. Cancer Res. 11:843-852; Jeffrey etal (2005) J. Med. Chem. 48:1344-1358; Hamblett etal (2004) Clin. Cancer Res. 10:7063- 7070). Drug moieties may impart their cytotoxic and cytostatic effects by mechanisms including tubulin binding, DNA binding, proteasome and/or topoisomerase inhibition. Some cytotoxic drugs tend to be inactive or less active when conjugated to large antibodies or protein receptor ligands.

N-myristoyl transferase (NMT) is a monomeric enzyme, which is ubiquitous in eukaryotes. NMT catalyses an irreversible co-translational transfer of myristic acid (a saturated 14-carbon fatty acid) from myristoyl-Coenzyme A (myr-CoA) to a protein substrate containing an *N*-terminal glycine with formation of an amide bond (Farazi, T.A., G. Waksman, and J.I. Gordon, J. Biol. Chem., 2001. 276(43): p. 39501-39504).

There are two types of human NMT, human NMT1 (HsNMT1) and human NMT2 (HsNMT2). Inhibition of human NMT has been suggested as a target for treating or preventing various diseases or disorders, for example hyperproliferative disorders (for example cancers, e.g. human colorectal cancer, gallbladder carcinoma, brain tumors, and lymphomas such as B-cell lymphoma) (Resh MD. 1993. Biochern. Biophys.Acta 1115, 307-22; Bertiaume LG, Beuachamp E, WO2017011907), and viral infections such as HIV (Gottlinger HG, Sodroski JG, Haseltine WA. 1989. Proc. Nat. Acad. Sci. USA 86:5781-85; Bryant ML, Ratner L. 1990. Proc. Natl. Acad. Sci. USA 87:523-27) and human rhinovirus (HRV) (Davis MP, Bottley, G, Beales LP, Killington, RA, Rowlands DJ, Tuthill, TJ, 2008 Journal of Virology 82 4169-4174; Mousnier A, Bell AS, Swieboda DP, Morales-Sanfrutos J, Perez-Dorado I, Brannigan JA, Newman J, Ritzefeld M, Hutton, JA, Guedan A, Asfor AS, Robinson, SW, Hopkins-Navratilova I, Wilkinson AJ, Johnston SL, Leatherbarrow RJ, Tuthill TJ, Solari R, Tate EW 2018 Nature Chemistry 10 (6) 599-606), Corbic Ramljak I, Stanger J, Real-Hohn A. Dreier D, Wimmer L., Redlberger-Fritz M, Fischl W, Klingel K, Mihovilovic MD, Blaas D, Kowalski H, PLOS Pathogens 14(8): e1007203. As NMT plays a key role in protein trafficking, mediation of protein-protein interactions, stabilization of protein structures and signal transduction in living systems, inhibition of the HsNMT1 and/or HsNMT2 enzyme(s) has the potential to disrupt multiprotein pathways. Although it is expected that inhibitors of human NMT will inhibit both HsNMT1 and HsNMT2, their therapeutic and/or prophylactic activity is believed to primarily derive from inhibition of HsNMT1. The above characteristics are believed to be desirable to reduce the risk of the development of resistance in, for example, treatment or prevention of microbial infections and hyperproliferative disorders.

There are two known binding pockets in NMT. One is the myr-CoA binding pocket and the other is the peptide binding pocket. Most NMT inhibitors reported to date target the peptide binding pocket.

Compounds active as inhibitors of NMT have previously been disclosed, see for example WO00/37464 (Roche), WO2010/026365 (University of Dundee), WO2013/083991 (Imperial Innovations Limited), WO2017/001812 (Imperial Innovations Limited), WO2020/128473 (Imperial College Innovations Limited), WO2020/128475 (Imperial College Innovations Limited) and WO2022/058745 (Imperial College Innovations Limited et al.). Particular uses of NMT inhibitors have been disclosed, see for example WO2022/090746 (Imperial College Innovations Limited et al.) and WO2022/082306 (Pacylex Pharmaceuticals Inc.).

However, there remains a need for further ADCs which retain the efficacy of the conjugated drug moiety whilst controlling toxicity.

Surprisingly, the present inventors have now found that ADCs comprising a NMT inhibitor demonstrate potent cytotoxic activity with minimal adverse side effects such as reduction in body weight. These properties are expected to make the ADCs of the invention especially suitable for use as medicaments for the treatment or prevention of hyperproliferative diseases such as cancer.

## Summary of the invention

The invention provides an antibody drug conjugate (ADC) comprising a NMT inhibitor conjugated to an antibody *via* a linker, or a salt thereof (herein referred to as "the ADC of the invention").

The ADC of the invention may be provided in the form of a salt. Suitably, the ADC of the invention is provided in the form of a pharmaceutically acceptable salt. Suitably, the ADC of the invention is provided.

The invention also provides a pharmaceutical composition comprising an ADC of the invention, or a pharmaceutically acceptable salt thereof.

The invention also provides an ADC of the invention, or a pharmaceutically acceptable salt thereof, for use as a medicament.

The invention also provides an ADC of the invention, or a pharmaceutically acceptable salt thereof, for use in the prevention or treatment of a disease or disorder in which inhibition of N-myristoyl transferase provides a therapeutic or prophylactic effect (e.g. cancer).

The invention also provides the use of an ADC of the invention, or a pharmaceutically acceptable salt thereof, in manufacture of a medicament for the prevention or treatment of a disease or disorder in which inhibition of N-myristoyl transferase provides a therapeutic or prophylactic effect (e.g. cancer).

The invention also provides a method of preventing or treating a disease or disorder in a subject in which inhibition of N-myristoyl transferase provides a therapeutic or prophylactic effect (e.g. cancer), said method comprising administering a therapeutically effective amount of an ADC of the invention, or a pharmaceutically acceptable salt thereof.

## Description of the Figures

**Figure 1:** Shows the effect of treatment with trastuzumab (2.5 mg/kg), ADC Example 1 (2.5 mg/kg) or NMT inhibitor 1 (2mg/kg) on tumour volume in a mouse xenograft study.

**Figure 2:** Shows the effect of treatment with trastuzumab (5 mg/kg), ADC Example 1 (5 mg/kg) or NMT inhibitor 1 (2mg/kg) on tumour volume in a mouse xenograft study.

**Figure 3:** Shows the effect of treatment with trastuzumab (2.5 mg/kg), ADC Example 1 (2.5 mg/kg) or NMT inhibitor 1 (2mg/kg) on the body weight of mice in a mouse xenograft study.

**Figure 4:** Shows the effect of treatment with trastuzumab (5 mg/kg), ADC Example 1 (5 mg/kg) or NMT inhibitor 1 (2mg/kg) on the body weight of mice in a mouse xenograft study.

**Figure 5:** Shows the effect of treatment with 2.5 mg/Kg trastuzumab, 2.5mg/Kg ADC Example 1, 2.5mg/Kg trastuzumab deruxtecan, and 5mg/Kg isotype control antibody on tumour volume in a mouse Gastric Cancer Xenograft Model.

**Figure 6:** Shows the effect of treatment with 5 mg/Kg trastuzumab, 5 mg/Kg ADC Example 1, 5 mg/Kg trastuzumab deruxtecan, and 5 mg/Kg isotype control antibody on tumour volume in a mouse Gastric Cancer Xenograft Model.

**Figure 7A:** Shows the % body weight change of the mice following experiments described in Biological Example 3 (and Figure 5, 2.5mg/kg (mpk)).

**Figure 7B:** Shows the % body weight change of the mice following experiments described in Biological Example 3 (and Figure 6; 5mg/kg (mpk)).

**Figures 8-11:** Shows the results from an in vitro assessment of cytotoxicity at differing concentrations (50 nM, 3.13 nM, 0.2 nM and 0 nM) of trastuzumab conjugated to NMT inhibitor 1 (ADC Example 1; **Fig. 8),** trastuzumab **(Fig. 9),** trastuzumab deruxtecan **(Fig. 10)** and Isotype control (isotype control IgG conjugated to NMT inhibitor 1; **Fig. 11)** against a HER2 positive breast cancer cell line, BT474. Puromycin was used as a positive cytotoxic control.

**Figures 12-15:** Shows the results from an in vitro assessment of cytotoxicity at differing concentrations (50 nM, 3.13 nM, 0.2 nM and 0 nM) of trastuzumab conjugated to NMT inhibitor 1 (ADC Example 1; **Fig. 12),** trastuzumab **(Fig. 13),** trastuzumab deruxtecan **(Fig. 14)** and Isotype control (isotype control IgG conjugated to NMT inhibitor 1; **Fig. 15)** against a HER2 negative breast cancer cell line, MCF7. Puromycin was used as a positive cytotoxic control.

**Figure 16:** Shows the results from an in vitro "bystander effect" of trastuzumab conjugated to NMT inhibitor 1 (ADC Example 1), trastuzumab, Reference Example 1 (trastuzumab conjugated to monomethyl auristatin E (MMAE),

Isotype control and trastuzumab deruxtecan, at differing concentrations (12.5nM, 3.13nM, 0.78nM, 0.2nM and 0.05nM) against a HER2 negative breast cancer cell line, MCF7.

**Figure 17:** Shows the results from an in vitro "bystander effect" of trastuzumab conjugated to NMT inhibitor 1 (ADC Example 1), trastuzumab, Reference Example 1 (trastuzumab conjugated to monomethyl auristatin E (MMAE), Isotype control and trastuzumab deruxtecan, at differing concentrations (12.5nM, 3.13nM, 0.78nM, 0.2nM and 0.05nM) against a HER2 positive breast cancer cell line, BT474.

**Figures 18-27:** Show the results from an in vitro assessment of cytotoxicity of ADC Examples 1-5 against a panel of cell lines including BT474 **(Fig. 18),** JIMT 1 **(Fig. 19),** NCI N87 **(Fig. 20),** NCI H292 **(Fig. 21),** IM95-m **(Fig. 22),** ZR-75-30 **(Fig. 23),** NCI H2170 **(Fig. 24),** LNCaP **(Fig. 25),** C42 **(Fig. 26)** and VCaP **(Fig. 27)** with the various comparators provided in each Figure (such as sacituzumab, trastuzumab, ifinatamab, NMT inhibitor 1 and Isotype control).

**Figures 28-43:** Show the results of the effect of test articles Isotype control, ADC Example 1, staurosporine, trastuzumab and trastuzumab deruxtecan as single agents on cell viability of gastric cancer organoid lines using CellTiter-Glo (CTG) luminescent cell viability assay as follows: GA0429B **(Fig. 28),** GA6877B **(Fig. 29),** GA6894B **(Fig. 30),** GA2434B **(Fig. 31),** GA3102B **(Fig. 32),** GA0119B **(Fig. 33),** GA0091B **(Fig. 34),** GA6815B **(Fig. 35),** GA0098B **(Fig. 36),** GA6833B **(Fig. 37),** GA0087B **(Fig. 38),** GA2109B **(Fig. 39),** GA3055B **(Fig. 40),** GA6866B **(Fig. 41),** GA0060B **(Fig. 42)** and GA6891B **(Fig. 43).**

**Figures 44a-b:** Shows the mean (+/- SEM) level of haematologial markers, as normalised to baseline (pre-dose) values, in the 20mg/Kg group as described in Biological Example 9.

**Figure 45:** Shows body weight loss of mice in Biological Example 10.

**Figure 46:** Shows mean values left to right per dose for AST (aspartate transaminase), ALT (alanine transaminase), ALP (alkaline phosphatase), LDH (lactate dehydrogenase), CK (creatine kinase) and GGT (gamma-glutamyl transferase) in blood of animals at termination in Biological Example 10.

**Figure 47:** Shows mean values left to right per dose of LYM, MON, NEU, RBC, HGB and PLT cells in the blood of animals at termination in Biological Example 10.

**Figure 48:** Shows the effect of treatment with ifinatamab (5 mg/kg and 10 mg/kg), ifinatamab-DXd (5 mg/kg and 10 mg/kg), ADC Example 4 (5 mg/kg and 10 mg/kg) and vehicle control on tumour volume in a mouse LNCaP prostate cancer xenograft model.

**Figure 49:** Shows the effect of treatment with ifinatamab (5 mg/kg and 10 mg/kg), ifinatamab-DXd (5 mg/kg and 10 mg/kg), ADC Example 4 (5 mg/kg and 10 mg/kg) and vehicle control on body weight in a mouse LNCaP prostate cancer xenograft model.

**Figure 50:** Shows the effect of treatment with ifinatamab (5 mg/kg), ifinatamab-DXd (5 mg/kg), ADC Example 4 (2.5 mg/kg, 5 mg/kg and 10 mg/kg) and vehicle control on tumour volume in a mouse VCaP prostate cancer xenograft model.

**Figure 51:** Shows the effect of treatment with ifinatamab (5 mg/kg), ifinatamab-DXd (5 mg/kg), ADC Example 4 (2.5 mg/kg, 5 mg/kg and 10 mg/kg) and vehicle control on body weight in a mouse VCaP prostate cancer xenograft model.

**Figure 52:** Shows the effect of treatment with sacituzumab (5 mg/kg), sacituzumab govitecan (5 mg/kg) (plus ADC Example 3 5mg/kg added on Study Days 27 and 34), and ADC Example 3 (5 mg/kg) and vehicle control on tumour volume in a mouse JIMT-1 Breast cancer xenograft model.

**Figure 53:** Shows the effect of treatment with sacituzumab (2.5 mg/kg), sacituzumab govitecan (2.5 mg/kg plus ADC Example 3 5mg/kg added on Study Day 27), and ADC Example 3 (2.5 mg/kg) and vehicle control on tumour volume in a mouse JIMT-1 Breast cancer xenograft model.

**Figure 54:** Shows the effect of treatment with sacituzumab (5 mg/kg), sacituzumab govitecan (5mg/kg) (plus ADC Example 3 5mg/kg added on Study Days 27 and 34), and ADC Example 3 (5 mg/kg and 10 mg/kg) and vehicle control on body weight in a mouse JIMT-1 Breast cancer xenograft model.

**Figure 55:** Shows the effect of treatment with sacituzumab (2.5 mg/kg), sacituzumab govitecan (2.5 mg/kg plus ADC Example 3 5mg/kg added on Study Day 27) and ADC Example 3 (2.5 mg/kg) and vehicle control on body weight in a mouse JIMT-1 Breast cancer xenograft model.

**Figure 56:** Shows the results from an *in vitro* assessment of cytotoxicity at differing concentrations (50 nM, 3.13 nM, 0.2 nM and 0 nM) of trastuzumab conjugated to NMT inhibitor 1 (ADC Example 8), against a HER2 positive breast cancer cell line, BT474. Puromycin was used as a positive cytotoxic control.

**Figure 57:** Shows the results from an *in vitro* assessment of cytotoxicity at differing concentrations (50 nM, 3.13 nM, 0.2 nM and 0 nM) of trastuzumab conjugated to NMT inhibitor 1 (ADC Example 8), against a HER2 negative breast cancer cell line, MCF7. Puromycin was used as a positive cytotoxic control.

## Sequence Listings

SEQ ID NO: 1 - Amino acid sequence of the light chain of trastuzumab
SEQ ID NO: 2 - Amino acid sequence of the heavy chain of trastuzumab
SEQ ID NO: 3 - Amino acid sequence of the light chain of rituximab
SEQ ID NO: 4 - Amino acid sequence of the heavy chain of rituximab
SEQ ID NO: 5 - Amino acid sequence of the light chain of ifinatamab
SEQ ID NO: 6 - Amino acid sequence of the heavy chain of ifinatamab
SEQ ID NO: 7 - Amino acid sequence of the light chain of sacituzumab
SEQ ID NO: 8 - Amino acid sequence of the heavy chain of sacituzumab

## Detailed description of the invention

The term "hydrocarbyl" used herein will be understood to mean any compound straight or branched chain saturated, unsaturated or partially unsaturated hydrocarbon groups. Suitable examples of "hydrocarbyl" groups may include, for example, "alkyl", "alkenyl", "alkynyl" and/or "haloalkyl" groups, each of which are as defined hereinbelow.

The term "alkyl" used herein will be understood to mean straight and branched chain saturated hydrocarbon groups. Examples of "alkyl" groups include methyl, ethyl, n-propyl, iso-propyl, n-butyl, t-butyl, i-butyl, sec-butyl, pentyl and hexyl groups. Among unbranched alkyl groups, there are preferred methyl, ethyl, n-propyl, iso-propyl, n-butyl groups. Among branched alkyl groups, there may be mentioned t-butyl, i-butyl, 1-ethylpropyl and 1-ethylbutyl groups.

The term "$C_{m-n}$" or "(m-nC) group" used alone or as a prefix, refers to any group having m to n carbon atoms.

The term "alkenyl" as used herein means both straight and branched chain unsaturated hydrocarbon groups with at least one carbon carbon double bond. Examples of alkenyl groups include ethenyl, propenyl, butenyl, pentenyl and hexenyl. Preferred alkenyl groups include ethenyl, 1-propenyl, 2-propenyl and but-2-enyl.

As used herein, the term "alkynyl" means both straight and branched chain unsaturated hydrocarbon groups with at least one carbon carbon triple bond. Examples of alkynyl groups include ethynyl, propynyl, butynyl, pentynyl and hexynyl. Preferred alkynyl groups include ethynyl, 1-propynyl and 2-propynyl.

The term " carbocyclyl" (or "carbocycle") as used herein is intended to mean any 3- to 13-membered carbon ring system, which may be saturated, partially unsaturated, or aromatic. The carbon ring system may be monocyclic or contain more than one ring (e.g. the ring system may be bicyclic). Examples of monocyclic saturated carbocycles include cyclopropyl, cyclobutyl, cyclopentyl, cyclohexyl, cycloheptyl, cyclooctyl. Examples of bicyclic saturated carbocycles include bicyclooctane, bicyclononane, bicyclodecane (decalin) and bicyclooctane. A further example of a saturated carbocycle is adamantane. Examples of monocyclic non- saturated carbocycles include cyclobutene, cyclopentene, cyclopentadiene, cyclohexene. Examples of aromatic carbocycles include phenyl and naphthyl. Further examples of aromatic carbocycles include tetrahydronaphthyl (tetralin) and indane.

The term "cycloalkyl" as used herein means a saturated group in a ring system. A cycloalkyl group can be monocyclic or bicyclic. A bicyclic group may, for example, be fused or bridged. Examples of monocyclic cycloalkyl groups include cyclopropyl, cyclobutyl and cyclopentyl. Other examples of monocyclic cycloalkyl groups are cyclohexyl, cycloheptyl and cyclooctyl. Examples of bicyclic cycloalkyl groups include bicyclo [2. 2.1]hept-2-yl. Preferably, the cycloalkyl group is monocyclic. The term "halogen" or "halo" as used herein means fluorine, chlorine, bromine or iodine. Fluorine, chlorine and bromine are particularly preferred.

The term "haloalkyl" as used herein means an alkyl group having a halogen substituent, the terms "alkyl" and "halogen" being understood to have the meanings outlined above. Similarly, the term "dihaloalkyl" means an alkyl group having two halogen substituents and the term "trihaloalkyl" means an alkyl group having three halogen substituents. Examples of haloalkyl groups include fluoromethyl, chloromethyl, bromomethyl, fluoromethyl, fluoropropyl and fluorobutyl groups; examples of dihaloalkyl groups include difluoromethyl and difluoroethyl groups; examples of trihaloalkyl groups include trifluoromethyl and trifluoroethyl groups.

The term "heterocyclyl" (or heterocycle) as used herein means an aromatic or a non-aromatic cyclic group of carbon atoms wherein from one to four of the carbon atoms is/are replaced by one or more heteroatoms independently selected from nitrogen, oxygen or sulfur. A heterocyclyl (or heterocycle) group may, for example, be monocyclic or bicyclic. In a bicyclic heterocyclyl (or heterocycle) group there may be one or more heteroatoms in each ring, or only in one of the rings. A heteroatom may be S, O or N, and is preferably O or N.

Examples of monocyclic non-aromatic heterocyclyl (or heterocycle) include aziridinyl, azetidinyl, pyrrolidinyl, imidazolidinyl, pyrazolidinyl, piperidinyl, piperazinyl, tetrahydrofuranyl, tetrahydropyranyl, morpholinyl, thiomorpholinyl and azepanyl.

Examples of monocyclic aromatic heterocyclyl (or heterocycle) groups include furanyl, thienyl, pyrrolyl, oxazolyl, thiazolyl, imidazolyl, oxadiazolyl, thiadiazolyl, pyridyl, triazolyl, triazinyl, tetrazolyl, pyridazyl, isothiazolyl, isoxazolyl, pyrazinyl, pyrazolyl and pyrimidinyl.

Examples of bicyclic aromatic heterocyclyl groups (or heterocycle) include quinoxalinyl, quinazolinyl, pyridopyrazinyl, benzoxazolyl, benzothiophenyl, benzimidazolyl, naphthyridinyl, quinolinyl, benzofuranyl, indolyl, benzothiazolyl, oxazolyl[4,5-b]pyridiyl, pyridopyrimidinyl, isoquinolinyl and benzodroxazole. Further examples of bicyclic aromatic heterocyclyl groups include those in which one of the rings is aromatic and the other is non-aromatic, such as dihydrobenzofuranyl, indanyl, indolinyl, isoindolinyl, tetrahydroisoquinolinyl, tetrahydroquinolyl and benzoazepanyl.

The term "optionally substituted" refers to either groups, structures, or molecules that are substituted and those that are not substituted. The term "wherein a/any CH, $CH_2$, $CH_3$ group or heteroatom (i.e. NH) within a $R^1$ group is optionally substituted" suitably means that (any) one of the hydrogen radicals of the $R^1$ group is substituted by a relevant stipulated group.

Where optional substituents are chosen from "one or more" groups, it is to be understood that this definition includes all substituents being chosen from one of the specified groups or the substituents being chosen from two or more of the specified groups.

## NMT Inhibitors

As outlined above, the ADC of the present invention comprises an NMT inhibitor. In one embodiment, the NMT inhibitor is a compound of formula (I):

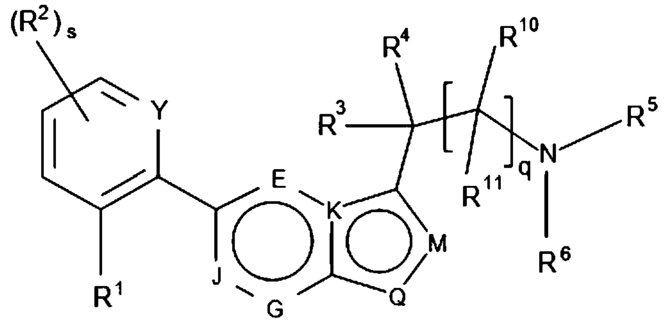

(I)

wherein:

- Y is selected from the group consisting of -CH-, -C($R^2$)- and -N-;
- $R^1$ is a group of formula -X-L-A;
- X represents -O-;
- L represents $-(CH_2)_m-$;
- m is 1, 2 or 3;
- A is a 6-10-membered aromatic carbocycle or a 5-10-membered aromatic heterocycle, said aromatic carbocycle or heterocycle being optionally substituted with 1, 2, or 3 substituents each independently selected from the group consisting of -F, -Cl, -Br, $-OCH_3$, $-OCF_3$, -CN, $-C_{1-6}$alkyl optionally substituted by up to 3 halogen, hydroxyl, or $-OC_{1-4}$alkyl groups, $-S(O)C_{1-4}$alkyl, $-S(O)_2C_{1-4}$alkyl, $-C(O)N(R^9)_2$, $-C(O)N(R^{13})C_{1-4}$alkyl$OC_{1-4}$alkyl , $-C(O)N(C_{1-4}$alkylOC$_{1-4}$alkyl$)_2$, $-CH-_2C(O)N(R^9)_2$, $-CH_2C(O)N(R^{13})C_{1-4}$alkyl$OC_{1-4}$alkyl , $-CH_2C(O)N(C_{1-4}$alkylOC$_{1-4}$alkyl$)_2$, $-S(O)_2NHC_{1-4}$alkyl, $-S(O)_2N(C_{1-4}$alkyl$)_2$, $-NHC_{1-4}$alkyl, $-N(C_{1-4}$alkyl$)_2$, $-NHC(O)C_{1-4}$alkyl,$-NHC(O)CF_3$, $-NHS(O)_2C_{1-4}$alkyl, $CH_2N(R^{13})_2$, $CH_2N(R^{13})C(O)C_{1-4}$alkyl, $CH_2N(R^{13})S(O)_2C_{1-4}$alkyl, $-CH_2S(O)_2C_{1-4}$alkyl, and $CO_2H$;
- s is 0, 1, 2, or 3;
- each $R^2$ is independently selected from the group consisting of -F, -Cl, -Br, $-OCH_3$, $-OCF_3$, -CN, - $C_{1-4}$alkyl optionally substituted by up to 3 halogen or hydroxyl groups, $-S(O)C_{1-4}$alkyl, $-S(O)_2C_{1-4}$alkyl, $-S(O)_2NHC_{1-4}$alkyl, $-S(O)_2N(C_{1-4}$alkyl$)_2$, $-NHC_{1-4}$alkyl, $-N(C_{1-4}$alkyl$)_2$, $-NHC(O)C_{1-4}$alkyl, - $NHC(O)CF_3$, and $-NHS(O)_2C_{1-4}$alkyl;
- q is 0 or 1;
- $R^3$ is hydrogen or methyl; $R^4$ is hydrogen or methyl;
- $R^5$ is hydrogen; $R^6$ is hydrogen or $C_{1-6}$alkyl optionally substituted by up to 3 -F, -Cl, -Br, -OH, - $OCH_3$, $-OCF_3$ or -CN groups;
- when present $R^{10}$ is hydrogen or methyl;
- when present $R^{11}$ is hydrogen or methyl;
- or the $R^3$ group and the $R^5$ group and the intervening atoms form a 3 to 7 membered non-aromatic heterocycle composed of the intervening atoms and bond, or the intervening atoms and $-(CHR^a)_r-$; or the $R^{10}$ group and the $R^5$ group and the intervening atoms form a 3 to 7 membered non-aromatic heterocycle composed of the intervening atoms and $-(CHR^a)_r-$;
- r is 1, 2, 3, 4 or 5; $R^a$ is hydrogen or methyl;

each $R^7$ is independently selected from the group consisting of hydrogen, halogen, $C_{1-4}$alkoxy, and $C_{1-4}$alkyl optionally substituted with 1, 2 or 3 halogens; and
$R^8$ is selected from the group selected from hydrogen and $C_{1-4}$alkyl;
each $R^9$ is independently selected from the group consisting of hydrogen and $C_{1-4}$alkyl, or two $R^9$ groups and the N they are bonded to form a 4 to 7 membered non-aromatic heterocycle, the heterocycle optionally comprising 1 or 2 further heteroatoms selected from N, O and S;
each $R^{13}$ is independently selected from the group consisting of hydrogen and $C_{1-4}$alkyl; and
wherein

i) E, J and G are each $C(R^7)$, K is carbon, Q is $N(R^8)$, and M is nitrogen;
ii) E, J and G are each $C(R^7)$, and K, Q and M are each nitrogen; or
iii) E, J, G and M are each $C(R^7)$, and K and Q are each nitrogen;

or a salt thereof.

Suitably the compound of formula (I) or a salt thereof is attached to the linker *via* the $N(R^5)(R^6)$ group.
In one embodiment, the NMT inhibitor is a compound of formula (IA^^):

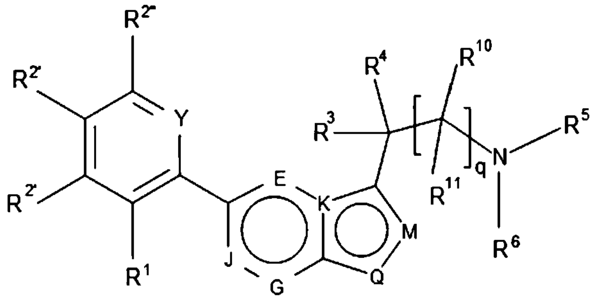

(IA^^)

wherein:

$R^1$ is a group of formula -X-L-A;
A is 4-pyrazolyl, said pyrazolyl being optionally substituted with up to 3 substituent groups selected from methyl and $-C(O)N(CH_3)_2$;
X is -O-;
L is $-(CH_2)_m-$;
m is 2;
$R^{2'}$ is selected from the group consisting of fluorine or chlorine (preferably fluorine);
$R^{2''}$ is selected from the group consisting of hydrogen, fluorine or chlorine;
q is 0;
$R^3$ is hydrogen or methyl;
$R^4$ is hydrogen or methyl;
$R^5$ is hydrogen;
$R^6$ is hydrogen or methyl; or
the $R^3$ group and the $R^6$ group and the intervening atoms form a 3 to 7 membered non-aromatic heterocycle composed of the intervening atoms and bonds;
E, J, G, K, Q and M are:

i) E, J and G are each CH, K is carbon, Q is $N(R^8)$, M is nitrogen; and $R^8$ is hydrogen or methyl; or
ii) E, J, G and M are each CH, and K and Q are each nitrogen;

with the proviso that A is substituted with no more than one $-C(O)N(CH_3)_2$ group;
or a salt thereof.

Suitably the compound of formula (IA^^) or a salt thereof is attached to the linker *via* the $N(R^5)(R^6)$ group.
Compounds of formulae (I) and (IA^^) are disclosed in WO2017/001812, the entire contents of which are incorporated by reference for the purpose of describing the synthesis and activity of NMT inhibitors.
In one embodiment, the NMT inhibitor is a compound of formula (II):

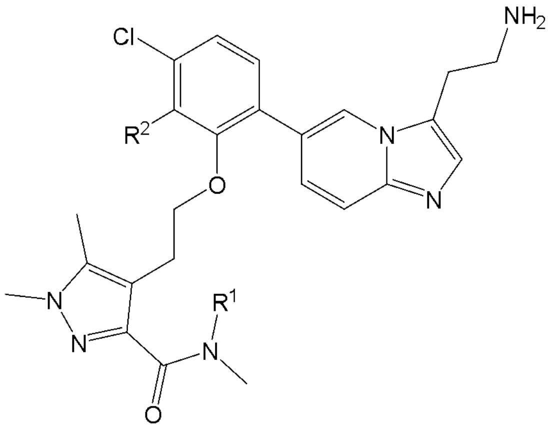

(II),

wherein:

$R^1$ is H or $-CH_3$; and
$R^2$ is H or F;
or a salt thereof.

Suitably the compound of formula (II) or a salt thereof is attached to the linker *via* the $NH_2$ group.

In one embodiment, the NMT inhibitor is 4-(2-{2-[3-(2-aminoethyl)imidazo[1,2-a]pyridin-6-yl]-5-chlorophenoxy} ethyl)-*N,N*,1,5-tetramethyl-1H-pyrazole-3-carboxamide:

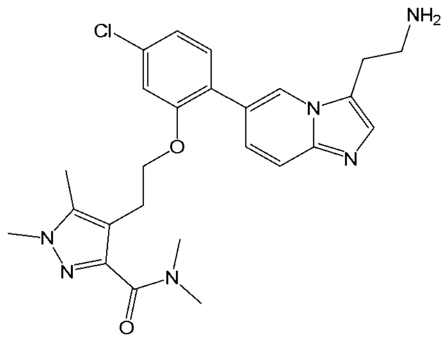

,

or a salt thereof.

Suitably the compound 4-(2-{2-[3-(2-aminoethyl)imidazo[1,2-a]pyridin-6-yl]-5-chlorophenoxy}ethyl)-*N,N*,1,5-tetramethyl-1H-pyrazole-3-carboxamide or a salt thereof is attached to the linker *via* the $NH_2$ group.

Compounds of formula (II) and 4-(2-{2-[3-(2-aminoethyl)imidazo[1,2-a]pyridin-6-yl]-5-chlorophenoxyl} ethyl)-*N,N*,1,5-tetramethyl-1H-pyrazole-3-carboxamide are disclosed in WO2020/128473, the entire contents of which are incorporated by reference for the purpose of describing the synthesis and activity of NMT inhibitors.

In one embodiment, the NMT inhibitor is is a compound of formula (III) or (IV):

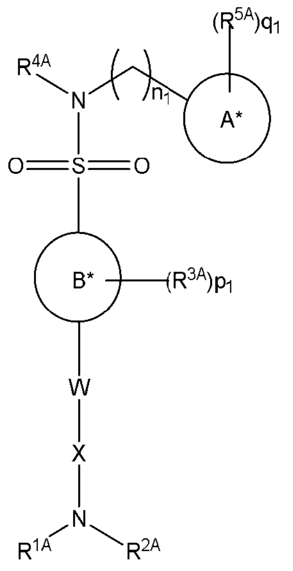

(III); or

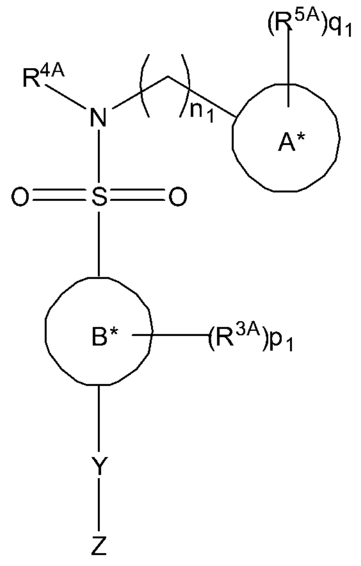

(IV)

wherein:

$n_1$ is 0, 1, 2, 3, 4, 5 or 6;
Ring A*, is an optionally substituted nitrogen containing aryl group wherein each substitutable carbon or nitrogen in Ring A* is optionally and independently substituted by one or more $R^{5A}$ and wherein if Ring A* contains an -NH- moiety that nitrogen may be optionally substituted by $C_{1-6}$alkyl (e.g. methyl); and wherein $R^{4A}$ and Ring A* together with the atoms to which they are attached may form a cyclic group,
Ring B* is an optionally substituted aryl or heteroaryl group wherein each substitutable carbon or heteroatom in Ring B* is optionally and independently substituted by one or more $R^{3A}$;
W and X, one of which may be absent, are independently selected from $R^{11A}$, hydrocarbyl (e.g. $C_{1-8}$ alkyl, alkenyl, alkynyl, or haloalkyl) optionally substituted with $R^{11A}$, and $-(CH_2)_{k1}$-heterocyclyl optionally substituted with $R^{12A}$; $k_1$ is 0, 1, 2, 3, 4, 5 or 6;
$R^{1A}$ is hydrogen;
$R^{2A}$, $R^{3A}$, $R^{4A}$ and $R^{5A}$ are independently selected from hydrogen, $R^{12A}$, hydrocarbyl (e.g. $C_{1-6}$alkyl, alkenyl, alkynyl, or haloalkyl) optionally substituted with $R^{12A}$, and a $-(CH_2)_{L1}$- heterocyclyl optionally substituted with one or more $R^{12A}$; wherein $R^{2A}$ taken together with W or X may form a heterocycle optionally substituted with one or more $R^{12A}$; and wherein one or more of $R^{3A}$ and $R^{5A}$ taken together with the atoms to which they are attached may form a carbocycle, for example heterocyclyl, optionally substituted with $R^{12A}$; $L_1$ is 0,1, 2, 3, 4, 5 or 6;
wherein:

each $R^{11A}$ and $R^{12A}$ is independently selected from halogen, trifluoromethyl, cyano, thio, nitro, oxo, $=NR^{13A}$, $-OR^{13A}$, $-SR^{13A}$, $-C(O)R^{13A}$, $-C(O)OR^{13A}$, $-OC(O)R^{13A}$, $-NR^{13A}COR^{14A}$,$-NR^{13A}CON(R^{13A})_2$, $-NR^{13a}COR^{14a}$, $-NR^{13a}CO_2R^{14A}$, $-S(O)R^{13A}$, $-S(O)_2R^{13A}$, $-SON(R^{13A})_2$, - $NR^{13A}S(O)_2R^{14A}$; $-CSR^{13A}$, $-N(R^{13A})R^{14A}$, $-C(O)N(R^{13A})R^{14A}$, $-SO_2N(R^{13A})R^{14A}$ and $R^{15A}$;
$R^{13A}$ and $R^{14A}$ are each independently selected from hydrogen or $R^{15A}$;
$R^{15A}$ is selected from hydrocarbyl (e.g. $C_{1-6}$alkyl, alkenyl, alkynyl, or haloalkyl), carbocyclyl and - $(CH_2)_{m1}$-heterocyclyl, and each $R^{15A}$ is optionally and independently substituted with one or more of halogen, cyano, amino, hydroxy, $C_{1-6}$alkyl or cycloalkyl and $C_{1-6}$alkoxy;
$m_1$ is 0, 1, 2, 3, 4, 5 or 6;
$p_1$ is 0,1, 2, 3 or 4; the values of $R^{4A}$ may be the same or different; and
$q_1$ is 0, 1, 2, 3 or 4; wherein the values of $R^{5A}$ may be the same or different;
Y and Z, one or both of which may be absent, are independently selected from hydrogen, $R^{16A}$, hydrocarbyl (e.g.$C_{1-6}$alkyl, alkenyl, alkynyl.or haloalkyl) optionally substituted with $R^{16A}$, and - $(CH_2)_{r1}$-heterocyclyl optionally substituted with $R^{16A}$, wherein each $R^{16A}$ is independently selected from halogen, trifluoromethyl, cyano, thio, nitro, oxo, $=NR^{17A}$, $-OR^{17A}$, $-SR^{17A}$, $-C(O)R^{17A}$, - $C(O)OR^{17A}$, $-OC(O)R^{17A}$, - $NR^{17A}COR^{18A}$, $-NR^{17A}CON(R^{18A})_2$, $-NR^{17A}COR^{18A}$, $-NR^{17A}CO_2R^{18A}$, - $S(O)R^{17A}$, $-S(O)_2R^{17A}$, - $SON(R^{17A})_2$, $-NR^{17A}S(O)_2R^{18A}$; $-CSR^{17A}$, $-N(R^{17A})R^{18A}$, $-C(O)N(R^{17A})R^{18A}$,$-SO_2N(R^{17A})R^{18A}$ and $R^{19A}$; $r_1$ is 0, 1,2, 3, 4, 5 or 6;

wherein:

$R^{17A}$ and $R^{18A}$ are each independently selected from hydrogen or $R^{19A}$;
$R^{19A}$ is selected from hydrocarbyl (e.g.$C_{1-6}$alkyl, alkenyl, alkynyl.or haloalkyl), carbocyclyl and - $(CH2)_{s1}$-heterocyclyl, and each $R^{19A}$ is optionally and independently substituted with one or more of halogen, cyano, amino, hydroxy, $C_{1-6}$alkyl and $C_{1-6}$alkoxy; and
$s_1$ is 0, 1, 2, 3, 4, 5 or 6;
or a salt thereof.

Suitably the compound of formula (III) or a salt thereof is attached to the linker *via* the $N(R^{1A})(R^{2A})$ group. Alternatively, suitably the compound of formula (IV) or a salt thereof is attached to the linker *via* Z when Z is $-N(R^{17A})R^{18A}$, or *via* Y when Z is absent and Y is $-N(R^{17A})R^{18A}$.

In one embodiment, the NMT inhibitor is a compound of formula (IIIa):

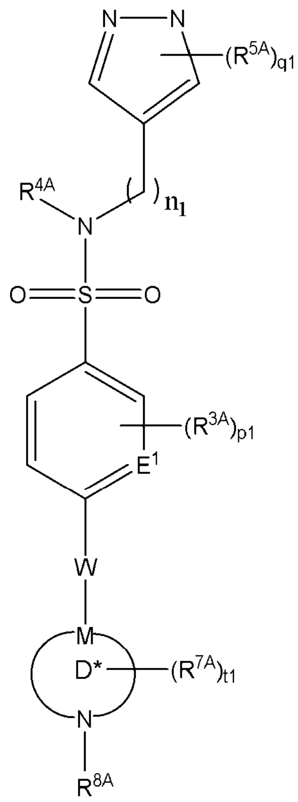

(IIIa)

wherein:

$n_1$ is 0 or 1;
$E^1$ is C;
W is a (1-4C)hydrocarbyl, an aryl (e.g. phenyl) or heteroaryl group (e.g. pyridinyl);
M is selected from C and N;
$R^{3A}$, $R^{4A}$ and $R^{5A}$ are independently selected from hydrogen, $R^{12A}$, and (1-3C)hydrocarbyl optionally substituted with $R^{12A}$;
$R^{12A}$ is independently selected from halogen, trifluoromethyl, cyano, thio, nitro, oxo, $-OR^{13A}$, - $SR^{13A}$, $-C(O)R^{13A}$, $-C(O)OR^{13A}$, $-OC(O)R^{13A}$, $-NR^{13A}COR^{14A}$ and $R^{15A}$;
$R^{13A}$ and $R^{14A}$ are each independently selected from hydrogen or a (1-4C)hydrocarbyl (e.g. methyl);
Ring D* is an optionally substituted nitrogen containing 6 or 7 membered heterocycle, wherein each substitutable carbon or nitrogen in Ring D* is optionally and independently substituted by one or more $R^{7A}$;
$R^{7A}$ is independently selected from hydrogen, (1-4C)hydrocarbyl, halogen, trifluoromethyl, cyano, thio, nitro or oxo;
$R^{8A}$ is hydrogen;
$p_1$ is 0, 1 or 2, wherein the values of $R^{4A}$ may be the same or different;
$q_1$ is 3, wherein the values of $R^{5A}$ may be the same or different; and

$t_1$ is 0, 1 or 2, wherein the values of $R^{7A}$ may be the same or different;
or a salt thereof.

Suitably the compound of formula (IIIa) or a salt thereof is attached to the linker *via* $NR^{8A}$ group.
In one embodiment, the NMT inhibitor is is (2,6-dichloro-4-(2-piperazin-1-yl-pyridin-4-yl)-N-(1,3,5-trimethyl-1H-pyraxol-4-yl)-benzenesulfonamide):

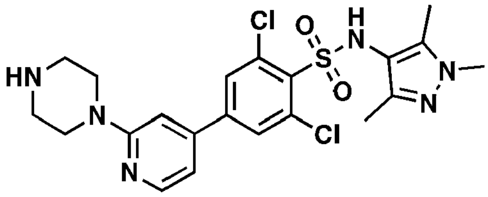

or a salt thereof.
Suitably (2,6-dichloro-4-(2-piperazin-1-yl-pyridin-4-yl)-N-(1,3,5-trimethyl-1H-pyraxol-4-yl)-benzenesulfonamide) or a salt thereof is attached to the linker *via* the NH group of the piperazinyl ring.
In one embodiment, the NMT inhibitor is 2,6-dichloro-N-(5-isobutyl-1,3-dimethyl-1H-pyrazol-4-yl(-4-(2-piperazin-1-yl-pyridin-4-yl)-benzenesulfonamide:

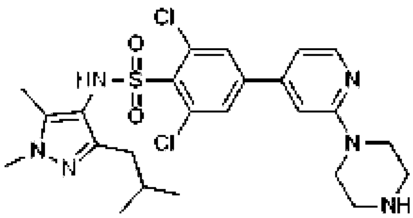,

or a salt thereof.
Suitably 2,6-dichloro-N-(5-isobutyl-1,3-dimethyl-1H-pyrazol-4-yl(-4-(2-piperazin-1-yl-pyridin-4-yl)-benzenesulfonamide or a salt thereof is attached to the linker *via* the NH group of the piperazinyl ring.
Compounds of formulae (III), (IV), (IIIa), (2,6-dichloro-4-(2-piperazin-1-yl-pyridin-4-yl)-N-(1,3,5-trimethyl-1H-pyraxol-4-yl)-benzenesulfonamide) and 2,6-dichloro-N-(5-isobutyl-1,3-dimethyl-1H-pyrazol-4-yl(-4-(2-piperazin-1-yl-pyridin-4-yl)-benzenesulfonamide are disclosed in WO2010/026365, the entire contents of which are incorporated by reference for the purpose of describing the synthesis and activity of NMT inhibitors.
In one embodiment, the NMT inhibitor is a compound of formula (V):

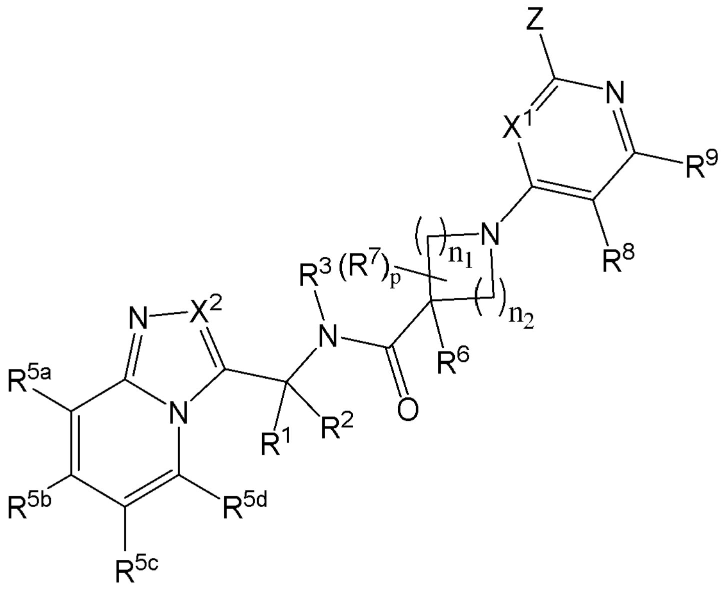

Formula (V)

wherein:

$n_1$ is 1 or 2; $n_2$ is 1 or 2;
$X^1$ is selected from the group consisting of $CR^x$ and N;
when present, $R^x$ is selected from the group consisting of hydrogen, halogen, and $-C_{1-4}$alkyl optionally substituted by 1, 2 or 3 substituents, each substituent being independently selected from the group consisting of halogen, -OH, $-OCH_3$, and $-OCF_3$;
$R^1$ is selected from the group consisting of hydrogen; $-C_{1-4}$alkyl optionally substituted by 1, 2 or 3 substituents, each substituent being independently selected from the group consisting of halogen, $-OCH_3$, and $-OCF_3$; and $-C_{3-6}$cycloalkyl optionally substituted by 1, 2 or 3 substituents, each substituent being independently selected from the group consisting of halogen, $-CH_3$, $-OCH_3$, and $-OCF_3$;
$R^2$ is selected from the group consisting of hydrogen; $-C_{1-4}$alkyl optionally substituted by 1, 2 or 3 substituents, each substituent being independently selected from the group consisting of halogen, $-OCH_3$, and $-OCF_3$; and $-C_{3-6}$cycloalkyl optionally substituted by 1, 2 or 3 substituents, each substituent being independently selected from the group consisting of halogen, $-CH_3$, $-OCH_3$, and $-OCF_3$;
or $R^1$ and $R^2$ are linked such that together with the atom to which they are attached they form a $C_{3-6}$cycloalkyl group or a 3- to 6-membered non-aromatic heterocyclyl group comprising 1 heteroatom selected from the group consisting of O and N, wherein said $C_{3-6}$cycloalkyl group or 3- to 6- membered non-aromatic heterocyclyl group is optionally substituted by 1 or 2 substituents, each substituent being independently selected from the group consisting of halogen, -OH, $-CH_3$, $-OCH_3$, and $-OCF_3$;
$R^3$ is selected from the group consisting of hydrogen; $-C_{1-4}$alkyl optionally substituted by 1, 2 or 3 substituents, each substituent being independently selected from the group consisting of halogen, -OH, $-OCH_3$, and $-OCF_3$; and $-C_{3-6}$-cycloalkyl optionally substituted by 1, 2 or 3 substituents, each substituent being independently selected from the group consisting of halogen, -OH, $-CH_3$, $-OCH_3$, and $-OCF_3$;
or $R^1$ and $R^3$ are linked such that together with the atoms to which they are attached they form a 3- to 6-membered non-aromatic heterocyclyl group comprising 1 N heteroatom, wherein said 3-to 6-membered non-aromatic heterocyclyl group is optionally substituted by 1 or 2 substituents, each substituent being independently selected from the group consisting of halogen, $-CH_3$, -OH, $-OCH_3$, and $-OCF_3$;
$X^2$ is selected from the group consisting of $CR^4$ and N;
when present, $R^4$ is selected from the group consisting of hydrogen; halogen; $-C_{1-4}$alkyl optionally substituted by 1, 2 or 3 substituents, each substituent being independently selected from the group consisting of halogen, -OH, $-OCH_3$, $-OCF_3$, and $-NR^aR^b$;
$R^{5a}$ and $R^{5d}$ are independently selected from the group consisting of hydrogen; halogen; methyl optionally substituted by 1, 2 or 3 substituents, each substituent being independently selected from the group consisting of halogen, -OH, $-OCH_3$, and $-OCF_3$; and methoxy optionally substituted by 1, 2 or 3 substituents, each substituent being independently selected from the group consisting of halogen, -OH, $-OCH_3$, and $-OCF_3$;

$R^{5b}$ and $R^{5c}$ are independently selected from the group consisting of hydrogen; halogen; -$C_{1-4}$alkyl optionally substituted by 1, 2 or 3 substituents, each substituent being independently selected from the group consisting of halogen, -OH, -$OCH_3$, and -$OCF_3$; -O-$C_{1-4}$alkyl optionally substituted by 1, 2 or 3 substituents, each substituent being independently selected from the group consisting of halogen, -OH, -$OCH_3$, and -$OCF_3$; and $C_{3-6}$cycloalkyl optionally substituted by 1, 2 or 3 substituents, each substituent being independently selected from the group consisting of halogen, -$CH_3$, -OH, -$OCH_3$, and -$OCF_3$;

or $R^{5b}$ and $R^{5c}$ are linked such that together with the atoms to which they are attached they form a 6-membered aryl group or a 5- or 6-membered aromatic heterocyclyl group comprising 1 or 2 heteroatoms selected from the group consisting of S, O and N, wherein said 6-membered aryl group or 5- or 6-membered aromatic heterocyclyl group is optionally substituted by 1 or 2 substituents, each substituent being independently selected from the group consisting of halogen, -OH, -$OCH_3$, and -$OCF_3$;

$R^6$ is selected from the group consisting of hydrogen and methyl;

when present, each $R^7$ is -$C_{1-4}$alkyl optionally substituted by 1, 2 or 3 substituents, each substituent being independently selected from the group consisting of halogen, -OH, -$OCH_3$, and -$OCF_3$;

$R^8$ is selected from the group consisting of hydrogen; halogen; -OH; -CN; -$C_{1-4}$alkyl optionally substituted by 1, 2 or 3 substituents, each substituent being independently selected from the group consisting of halogen, -OH, -CN, and methoxy optionally substituted by 1, 2 or 3 halogen; -$C_{3-6}$cycloalkyl optionally substituted by 1, 2 or 3 substituents, each substituent being independently selected from the group consisting of halogen, -$CH_3$, -OH, -CN, and methoxy optionally substituted by 1, 2 or 3 halogen; -$C_{1-4}$alkenyl optionally substituted by 1, 2 or 3 substituents, each substituent being independently selected from the group consisting of halogen, -OH, -CN, and methoxy optionally substituted by 1, 2 or 3 halogen; and -O-$C_{1-4}$alkyl optionally substituted by 1, 2 or 3 substituents, each substituent being independently selected from the group consisting of halogen, -OH, CN and methoxy optionally substituted by 1, 2 or 3 halogen; $R^9$ is selected from the group consisting of hydrogen, and -$C_{1-4}$alkyl optionally substituted by 1, 2 or 3 substituents, each substituent being independently selected from the group consisting of halogen, -OH, -$OCH_3$, and -$OCF_3$; or

$R^8$ and $R^9$ are linked such that together with the atoms to which they are attached they form a 6-membered aryl group, a $C_{5-6}$cycloalkyl group, or a 5- or 6- membered aromatic heterocyclyl group comprising 1 or 2 heteroatoms selected from N, O and S, and wherein said 6-membered aryl group, $C_{5-6}$cycloalkyl group or 5- to 6- membered aromatic heterocyclyl group is optionally substituted by 1, 2 or 3 substituents, each substituent being independently selected from the group consisting of halogen; -OH; -CN; -$C_{1-4}$alkyl optionally substituted by 1, 2 or 3 substituents, each substituent being independently selected from the group consisting of halogen, -OH, and methoxy optionally substituted by 1, 2 or 3 halogen; and -O-$C_{1-4}$alkyl optionally substituted by 1, 2 or 3 substituents, each substituent being independently selected from the group consisting of - halogen, -OH, and methoxy optionally substituted by 1, 2 or 3 halogen;

p is 0, 1, or 2;

Z is a 5- to 13-membered non-aromatic heterocyclyl group comprising 1, 2 or 3 heteroatoms selected from N, O and S, wherein at least one of the heteroatoms is N, and wherein said 5- to 13-membered non-aromatic heterocyclyl group is optionally substituted with 1, 2, 3 or 4 substituents, each substituent being independently selected from the group consisting of halogen; -$C_{1-6}$alkyl optionally substituted by 1, 2 or 3 substituents, each substituent being independently selected from the group consisting of halogen, -OH, and -O$C_{1-3}$alkyl optionally substituted by 1, 2 or 3 halogen; -O-$C_{1-6}$alkyl optionally substituted by 1, 2 or 3 substituents, each substituent being independently selected from the group consisting of halogen, -OH, and -O-$C_{1-3}$alkyl optionally substituted by 1, 2 or 3 halogen; $NR^cR^d$; and $C_{3-6}$cycloalkyl optionally substituted by 1, 2 or 3 substituents, each substituent being independently selected from the group consisting of halogen, -OH, and -O-$C_{1-3}$alkyl optionally substituted by 1, 2 or 3 halogen; or when two substituents are on adjacent ring positions they may be linked such that together with the atoms to which they are attached they form a $C_3$-$_6$cycloalkyl group or a 4- to 6-membered non-aromatic heterocyclyl group comprising 1 heteroatom selected from the group consisting of O and N, wherein said $C_3$-$_6$cycloalkyl group or 4- to 6-membered non-aromatic heterocyclyl group is optionally substituted by 1 or 2 substituents, each substituent being independently selected from the group consisting of halogen; -$C_{1-6}$alkyl optionally substituted by 1, 2 or 3 substituents, each substituent being independently selected from the group consisting of halogen, -OH, and -O$C_{1-3}$alkyl optionally substituted by 1, 2 or 3 halogen; and -O-$C_{1-6}$alkyl optionally substituted by 1, 2 or 3 substituents, each substituent being independently selected from the group consisting of halogen, -OH, and - O-$C_{1-3}$alkyl optionally substituted by 1, 2 or 3 halogen;

$R^c$ is hydrogen;

$R^d$ is selected from the group consisting of hydrogen; -$C_{1-6}$alkyl optionally substituted by 1, 2 or 3 substituents, each substituent being independently selected from the group consisting of halogen, -$OCH_3$, and -$OCF_3$; and $C_{3-6}$cycloalkyl optionally substituted by 1, 2 or 3 substituents, each substituent being independently selected from the group consisting of halogen, -$CH_3$, -OH, -$OCH_3$, and -$OCF_3$;

or Z is -$NR^{10}R^{11}$, wherein

$R^{10}$ is hydrogen; and

$R^{11}$ is a 5- to 10-membered non-aromatic heterocyclyl group comprising 1, 2 or 3 heteroatoms selected from N, O and S, wherein at least one of the heteroatoms is N, and wherein said 5- to 10-membered non-aromatic heterocyclyl group is optionally substituted with 1, 2, 3 or 4 substituents independently selected from the group consisting of halogen; -OH; -$C_{1-6}$alkyl optionally substituted by 1, 2 or 3 substituents, each substituent being independently selected from the group consisting of halogen, -OH, and -$OC_{1-3}$alkyl optionally substituted by 1, 2 or 3 halogen; and -O-$C_{1-6}$alkyl optionally substituted by 1, 2 or 3 substituents, each substituent being independently selected from the group consisting of halogen, -OH, and -O-$C_{1-3}$alkyl optionally substituted by 1, 2 or 3 halogen; and

when present, each $R^a$ and $R^b$ are independently selected from the group consisting of hydrogen and -$C_{1-4}$alkyl;

or a salt thereof.

Suitably, the compound of formula (V) is attached to the linker *via* a nitrogen atom present in group Z e.g. a nitrogen ring atom when group Z is a 5- to 13-membered non-aromatic heterocyclyl group, a $NR^cR^d$ group present in group Z when group Z is 5- to 13-membered non-aromatic heterocyclyl group, or -$NR^{10}R^{11}$. The skilled person would understand that in order for the linker to be attached to a nitrogen atom present in group Z (for example in the compounds of formula (V) and elsewhere herein), the nitrogen atom present in group Z must be attached to at least one hydrogen atom i.e. the nitrogen atom cannot be part of a tertiary amino group. An N-H covalent bond in group Z is replaced with an N-linker covalent bond in the conjugate formed between the compound (e.g. group Z) and the linker.

In one embodiment, the NMT inhibitor is a compound of formula (Vq):

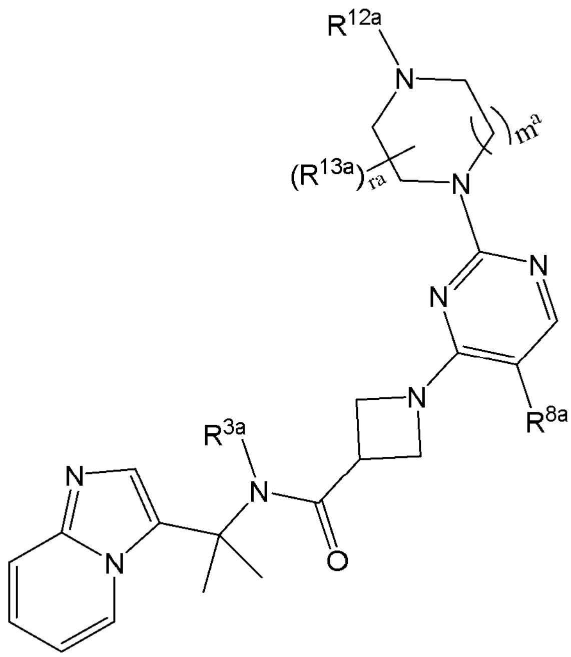

(Vq)

wherein:

$R^{3a}$ is H or $C_{1-4}$alkyl optionally substituted by 1, 2 or 3 substituents, each substituent being independently selected from the group consisting of halogen, -$OCH_3$, and -$OCF_3$;

$R^{8a}$ is halogen or $C_{1-4}$alkyl optionally substituted by 1, 2 or 3 substituents, each substituent being independently selected from the group consisting of halogen, -$OCH_3$, and -$OCF_3$;

ra is 0, 1 or 2;

$m^a$ is 1 or 2;

$R^{12a}$ is hydrogen; and

when present each $R^{13a}$ is independently selected from the group consisting of $C_{1-4}$alkyl optionally substituted by 1, 2 or 3 substituents, each substituent being independently selected from the group consisting of halogen, -$OCH_3$, and -$OCF_3$; and $C_{3-6}$cycloalkyl optionally substituted by 1, 2 or 3 substituents, each substituent being independently selected from the group consisting of halogen, -$CH_3$, -$OCH_3$, and -$OCF_3$; or

when ra is 2 and two $R^{13a}$ groups are on adjacent ring positions, said two $R^{13a}$ are linked such that together with the

atoms to which they are attached they form a $C_3$-$_6$cycloalkyl group optionally substituted by 1 or 2 substituents, each substituent being independently selected from the group consisting of halogen; $-C_{1-6}$alkyl optionally substituted by 1, 2 or 3 substituents, each substituent being independently selected from the group consisting of halogen, -OH, and $-OC_{1-3}$alkyl optionally substituted by 1, 2 or 3 halogen;
or a salt thereof.

Suitably the compound of formula (Vq) or a salt thereof is attached to the linker *via* the $NR^{12a}$ group.

In one embodiment, the NMT inhibitor is (S)-1-(5-chloro-2-(2-methylpiperazin-1-yl)pyrimidin-4-yl)-N-(2-(imidazo [1,2-a]pyridin-3-yl)propan-2-yl)azetidine-3-carboxamide (herein referred to as "NMT inihibitor 26"):

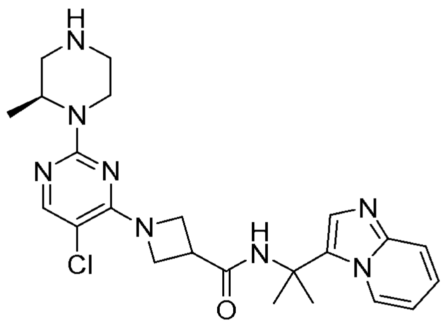

,

or a salt thereof.

Suitably (S)-1-(5-chloro-2-(2-methylpiperazin-1-yl)pyrimidin-4-yl)-N-(2-(imidazo[1,2-a]pyridin-3-yl)propan-2-yl)azetidine-3-carboxamide is attached to the linker *via* the NH group of the piperazinyl ring.

Compounds of formulae (V), (Vq) and (S)-1-(5-chloro-2-(2-methylpiperazin-1-yl)pyrimidin-4-yl)-N-(2-(imidazo [1,2-a]pyridin-3-yl)propan-2-yl)azetidine-3-carboxamide are disclosed in WO2022/058745, the entire contents of which are incorporated by reference for the purpose of describing the synthesis and activity of NMT inhibitors. In particular, NMT inhibitor 26 may be prepared as described in Example 129 in WO2022/058745.

In one embodiment, the NMT inhibitor is a compound of formula (VI):

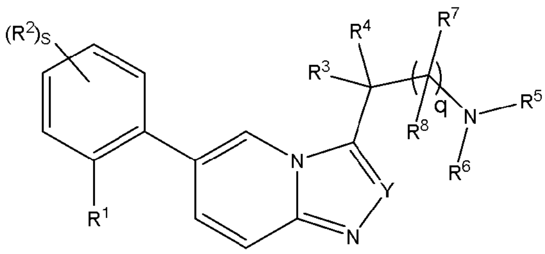

(VI)

wherein:

$R^1$ is a group of formula O-L-A;

L is $-(CHR^{12})_m-$;

each $R^{12}$ is independently H or $C_{1-4}$alkyl;
m is 1, 2 or 3;

A is:

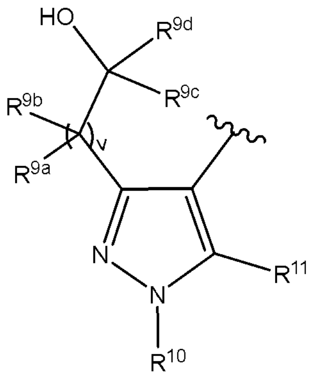

v is 0, 1 or 2;
$R^{9a}$ is H, $C_{1-4}$alkyl or $C_{1-4}$haloalkyl;
$R^{9b}$ is H, $C_{1-4}$alkyl or $C_{1-4}$haloalkyl;
$R^{9c}$ is $C_{1-4}$alkyl or $C_{1-4}$haloalkyl;
$R^{9d}$ is H, $C_{1-4}$alkyl or $C_{1-4}$haloalkyl;
$R^{10}$ is H, $C_{1-4}$alkyl or $C_{1-4}$haloalkyl;
$R^{11}$ is H, halo, CN, $C_{1-4}$alkyl, $C_{1-4}$haloalkyl, $C_{1-4}$alkoxy or $C_{1-4}$haloalkoxy;

s is 0, 1, 2 or 3;
each $R^2$ is independently F, Cl, Br, $C_{1-4}$alkyl optionally substituted by up to 3 halogen groups, $OCH_3$ or $OCF_3$;
Y is CH or $C_{1-4}$alkyl;
$R^3$ is H or $C_{1-4}$alkyl;
$R^4$ is H or $C_{1-4}$alkyl;
$R^5$ is H;
$R^6$ is H or $C_{1-4}$alkyl;
q is 0 or 1;
$R^7$ is H or methyl;
$R^8$ is H or methyl;
or $R^3$ and $R^6$ and the intervening atoms form a 3 to 7 membered non-aromatic heterocycle composed of the intervening atoms and bond, or the intervening atoms and $-(CHR^a)_r-$; or the $R^7$ group and the $R^6$ group and the intervening atoms form a 3 to 7 membered non-aromatic heterocycle composed of the intervening atoms and $-(CHR^a)_r-$;
r is 1, 2, 3, 4 or 5; and
$R^a$ is hydrogen or methyl;

or a salt thereof.

Suitably, the compounds of formula (VI) or salts thereof are attached to the linker via the $N(R^5)(R^6)$ group.

In one embodiment, the NMT inhibitor is a compound of formula (VIA):

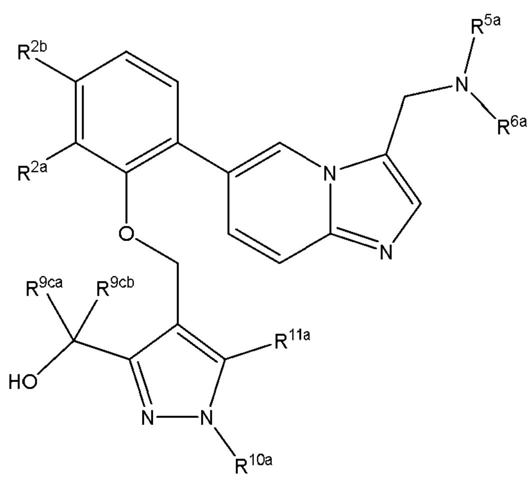

(VIA),

wherein:

$R^{2a}$ is H or F;
$R^{2b}$ is F;
$R^{5a}$ is H;
$R^{6a}$ is H or methyl;
$R^{9ca}$ is methyl, iso-propyl or tert-butyl;
$R^{9cb}$ is H or methyl;
$R^{10a}$ is methyl; and
$R^{11a}$ is methyl;
provided that when $R^{2a}$ is H, $R^{9cb}$ is H;
or a salt thereof.

Suitably, the compounds of formula (VIA) or salts thereof are attached to the linker via the N($R^{5a}$)($R^{6a}$) group.
In one embodiment, the NMT inhibitor is 1-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}-2,2-dimethylpropan-1-ol:

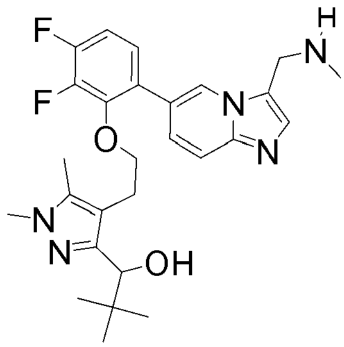

,

or a salt thereof.
Suitably, 1-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}-2,2-dimethylpropan-1-ol or a salt thereoff is attached to the linker *via* the NH(Me) group.
In one embodiment, the NMT inhibitor is 2-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}propan-2-ol:

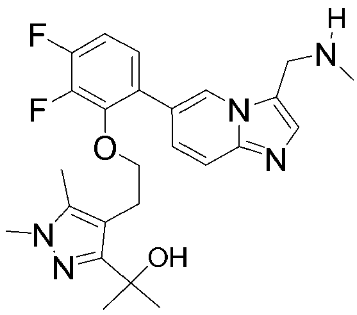

,

or a salt thereof.
Suitably, 2-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}propan-2-ol or a salt thereof is attached to the linker *via* the NH(Me) group.
Compounds of formulae (VI), (VIA), 1-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}-2,2-dimethylpropan-1-ol and is 2-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}propan-2-ol are disclosed in EP patent application no. 22194959.7, the entire contents of which are incorporated by reference for the purpose of describing the synthesis and activity of NMT inhibitors. Said compounds may also be prepared according to methods known to the skilled person, for example those disclosed herein for the synthesis of NMT inhibitor 1.
In one embodiment of the invention, the NMT inhibitor is provided in the form of a salt. In one embodiment, the NMT inhibitor is provided in the form of a pharmaceutically acceptable salt. In one embodiment, the NMT inhibitor is

provided.

**Linker**

The linker attaches an antibody to a NMT inhibitor through covalent bond(s). The linker is a bifunctional or multifunctional moiety which can be used to link one or more NMT inhibitors and an antibody to form the ADCs of the present invention. The linker may be stable outside a cell, i.e. extracellular, or it may be cleavable by enzymatic activity, hydrolysis, or other metabolic conditions. In one embodiment the linker is a cleavable linker, such as cleavable by an enzyme (in particular a lysosomal enyzyme).

ADCs of the present invention can be conveniently prepared using a linker having reactive functionality for binding to the NMT inhibitor and to the antibody. A cysteine thiol, or an amine, e.g. a chain terminus such as an N-terminus, an or amino acid side chain such as lysine, of an antibody can form a bond with a functional group of a linker e.g. maleimide.

The linkers are preferably stable extracellularly. Before transport or delivery into a cell, the ADC of the invention is preferably stable and remains intact, i.e. the antibody remains linked to the NMT inhibitor. The linkers are stable outside the target cell and may be cleaved at some efficacious rate inside the cell. An effective linker will: (i) maintain the specific binding properties of the antibody; (ii) allow intracellular delivery of the conjugate or drug moiety; (iii) remain stable and intact, i.e. not cleaved, until the conjugate has been delivered or transported to its targeted site; and (iv) maintain a cytotoxic, cell-killing effect or a cytostatic effect of the NMT inhibitor once cleaved. Stability of the ADC may be measured by standard analytical techniques such as mass spectroscopy, HPLC, and the separation/analysis technique LC/MS. Derivatisation studies (data not shown) have led the Applicants to conclude that the ADCs of the invention do not have NMT inhibitory activity and thus the NMT inhibitory activity is realised on cleavage of the linker to release the NMT inhibitor.

Covalent attachment of the antibody and the drug moiety requires the linker to have two reactive functional groups, i.e. bivalency in a reactive sense. Bivalent linker reagents which are useful to attach two or more functional or biologically active moieties, such as peptides, nucleic acids, drugs, toxins, antibodies, haptens, and reporter groups are known, and methods have been described their resulting conjugates (Hermanson, G.T. (1996) Bioconjugate Techniques; Academic Press: New York, p 234-242).

The linker may be substituted with groups which modulate aggregation, solubility or reactivity. For example, a sulfonate substituent may increase water solubility of the reagent and facilitate the coupling reaction of the linker reagent with the antibody or the drug moiety or facilitate the coupling reaction of the linker and NMT inhibitor with the antibody or the linker and antibody with the NMT inhibitor, depending on the synthetic route employed to prepare the ADC.

In one embodiment, the linker unit has the formula (VII):

$$-A_a-W_w-Y_y- \qquad \text{(formula (VII)}$$

wherein:

A is a first stretcher unit which when present forms a covalent bond with a chain terminus (such as a N-terminus) or a functional group of an amino acid side chain of the antibody;

a is 0 or 1;

each W is independently an amino acid unit or a glucuronide unit which when A and/or Y are absent forms a covalent bond with a chain terminus (such as a N-terminus) or a functional group of an amino acid side chain of the antibody and/or with a functional group of the NMT inhibitor respectively;

when W is an amino acid unit, w is 1 to 12;
when W is a glucuronide unit, w is 1 or 2;

Y is a second stretcher unit which when present forms a covalent bond with a functional group of the NMT inhibitor; and y is 0 or 1.

The NMT inhibitor forms a covalent bond with the second stretcher unit (Y), when present, or when Y is absent, with the amino acid unit or glucuronide unit (W). Suitably, the functional group on the NMT inhibitor is an amino or alcohol, for example an amino. When the NMT inhibitor forms a covalent bond via an amino group, the amino group must have an available hydrogen atom, in order to permit reaction with the corresponding functional group (e.g. carbonyl group) in the linker i.e. the amino group cannot be tertiary.

*First stretcher unit*

The stretcher unit (A), when present, is capable of linking an antibody to an amino acid unit or glucuronide unit (W). In this regard, the antibody has a functional group that can form a bond with a functional group of a stretcher unit e.g. a

functional group of an amino side chain of the antibody. Useful functional groups that can be present on the antibody, either naturally or via chemical manipulation include, but are not limited to, sulfhydryl (-SH), amino, hydroxyl, carboxy, the anomeric hydroxyl group of a carbohydrate, and carboxyl. In some embodiments, the antibody functional groups are sulfhydryl and/or amino, especially sulfhydryl. Sulfhydryl groups can be generated by reduction of an intramolecular disulfide bond of an antibody. Sulfhydryl groups also can be generated by reaction of an amino group of a lysine moiety of an antibody using 2-iminothiolane (Traut's reagent) or another sulfhydryl generating reagent.

In one embodiment, the first stretcher unit forms a bond with a sulfur atom of an antibody. The sulfur atom can be derived from a sulfhydryl group of an antibody.

In one embodiment, the stretcher unit (A) has the formula (A1):

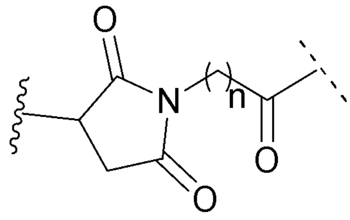

(A1)

,

wherein:

n is 1 to 6;

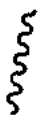 denotes the point of attachment to a chain terminus (such as a N-terminus) or a functional group of an amino acid side chain of the antibody; and 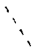 denotes the point of attachment to W.

In one embodiment, A is (A2):

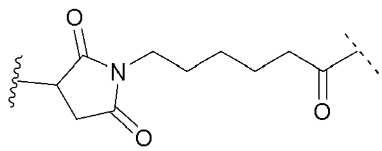

(A2)

.

In one embodiment, A is (A3):

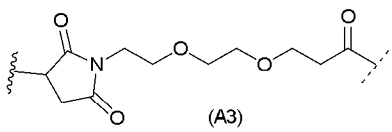

(A3)

.

*Amino acid and glucuronide units*

Each W is independently an amino acid unit or a glucuronide unit which when A and/or Y are absent forms a covalent bond with a chain terminus (such as a N-terminus) or a functional group of an amino acid side chain of the antibody and/or to a functional group of the NMT inhibitor respectively.

In some embodiments, each W is independently an amino acid unit. Within such embodiments, w is 1 to 12, such as 1, 2 or 3, especially 2.

In some embodiments the amino acid unit can be cleaved by one or more enzymes, such as a tumour-associated protease, thereby facilitating release of the NMT inhibitor from the ADC upon exposure to intracellular proteases e.g. lysosomal enzymes (Doronina et al. (2003) Nat. Biotechnol. 21:778-784).

In one embodiment, each W has the formula (WI):

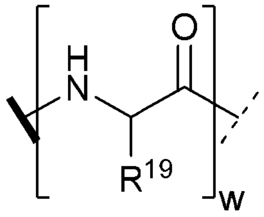

(WI) .

wherein w is as previously defined; and

$R^{19}$ is H, methyl, iso-propyl, iso-butyl, sec-butyl, benzyl, p-hydroxybenzyl, $CH_2OH$, CH(OH)Me, $CH_2CH_2SMe$, $CH_2C(O)SMe$, $CH_2C(O)NH_2$, $CH_2C(O)OH$, $CH_2CH_2C(O)NH_2$, $CH_2CH_2C(O)OH$, $(CH_2)_3NHC(=NH)NH_2$, $(CH_2)_3NH_2$, $(CH_2)_3NHC(O)Me$, $(CH_2)_3NHCHO$, $(CH_2)_4NHC(=NH)NH_2$, $(CH_2)_4NH_2$, $(CH_2)_4NHC(O)Me$, $(CH_2)_4NHCHO$, $(CH_2)_3NHC(O)NH_2$, $(CH_2)_4NHC(O)NH_2$, $CH_2CH_2CH(OH)CH_2NH_2$, 2-pyridylmethyl,3-pyridylmethyl, 4-pyridylmethyl, phenyl,

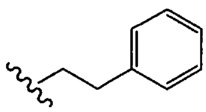 , 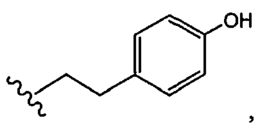 , 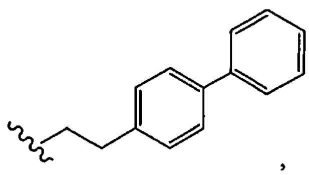 ,

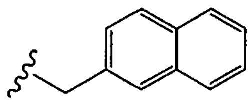 , 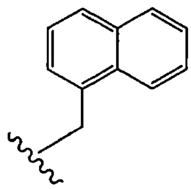 , 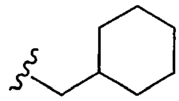 ,

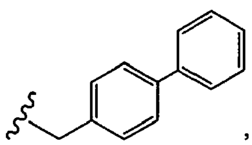 , 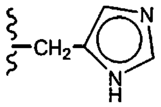 , or 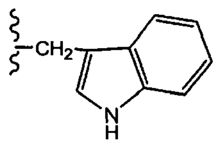 . ;

wherein 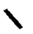 denotes the point of attachment to A (when present) or a chain terminus (such as a N-terminus) or functional group of an amino acid side chain of the antibody; and

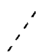 denotes the point of attachment to Y (when present) or to a functional group of the NMT inhibitor.

In one embodiment, at least one $R^{19}$ is iso-propyl. In one embodiment, at least one $R^{19}$ is $(CH_2)_3NHC(=NH)NH_2$.

In one embodiment, (W)w has the formula (WII):

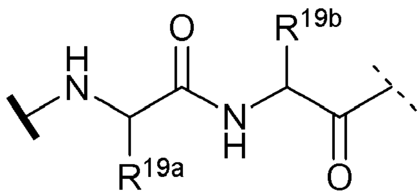

(WII),

wherein:

$R^{19a}$ is H, methyl, *iso*-propyl, *iso*-butyl, *sec*-butyl, benzyl, *p*-hydroxybenzyl, $CH_2OH$, CH(OH)Me, 2-pyridylmethyl,3-pyridylmethyl, 4-pyridylmethyl, phenyl,

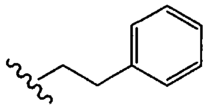 , 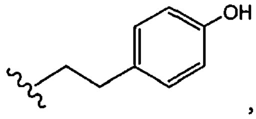 , 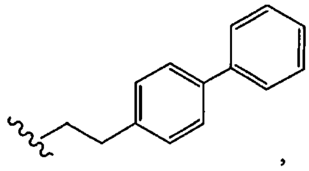 ,

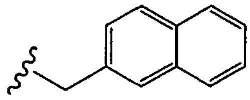 , 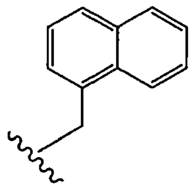 , 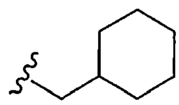 ,

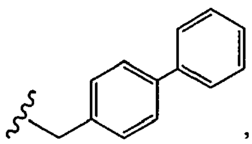 , 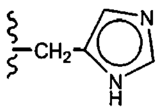 , or 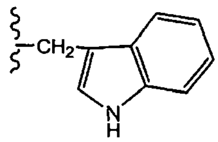 .

;

and

$R^{19b}$ is $CH_2CH_2SMe$, $CH_2C(O)SMe$, $CH_2C(O)NH_2$, $CH_2C(O)OH$, $CH_2CH_2C(O)NH_2$, $CH_2CH_2C(O)OH$, $(CH_2)_3NHC(=NH)NH_2$, $(CH_2)_3NH_2$, $(CH_2)_3NHC(O)Me$, $(CH_2)_3NHCHO$, $(CH_2)_4NHC(=NH)NH_2$, $(CH_2)_4NH_2$, $(CH_2)_4NHC(O)Me$, $(CH_2)_4NHCHO$, $(CH_2)_3NHC(O)NH_2$, $(CH_2)_4NHC(O)NH_2$ or $CH_2CH_2CH(OH)CH_2NH_2$.

In one embodiment, $R^{19a}$ is iso-propyl. In one embodiment, $R^{19b}$ is $(CH_2)_3NHC(=NH)NH_2$.

In one embodiment, each W is a glucuronide unit. Within such embodiments, w is 1 or 2, especially 1. The glucuronide unit includes a site that can be cleaved by a β-glucuronidase enzyme.

In one embodiment, W (particularly (W)w) has the formula (WIII):

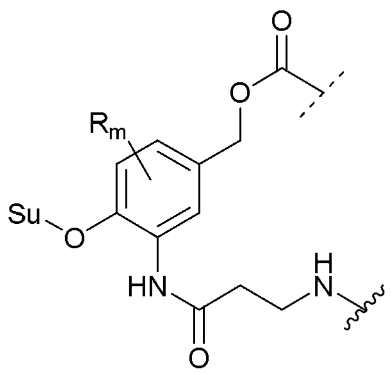

(WIII),

wherein:

R is H, halo, CN or $NO_2$;
m is 0, 1, 2 or 3;
Su is a sugar moiety;

wherein 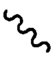 denotes the point of attachment to A (when present) or to a chain terminus (such as a N-terminus) or a functional group of an amino acid side chain of the antibody; and

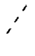 denotes the point of attachment to Y (when present) or to a functional group of the NMT inhibitor.

In one embodiment, R is H. In a second embodiment, R is halo. In a third embodiment, R is CN. In a fourth embodiment, R is $NO_2$.

In one embodiment, m is 0. In a second embodiment, m is 1. In a third embodiment, m is 2. In a fourth embodiment, m is 3.

In one embodiment, the sugar moiety (Su) is cyclic hexose, such as a pyranose, or a cyclic pentose, such as a furanose. In one embodiment, the pyranose is a glucuronide or hexose. Suitably, the sugar moiety is in the β-D conformation. In one embodiment, the pyranose is a β-D-glucuronide moiety (i.e., β-D-glucuronic acid linked to the remainder of W *via* a glycosidic bond that is cleavable by β-glucuronidase). In one embodiment, the sugar moiety is unsubstituted (e.g., a naturally occurring cyclic hexose or cyclic pentose). In one embodiment, the sugar moiety is a substituted β-D-glucuronide (e.g, glucuronic acid substituted with one or more groups, such hydrogen, hydroxyl, halogen, sulfur, nitrogen or $C_{1-6}$alkyl).

In one embodiment, Su has the following formula:

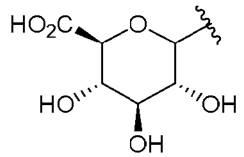

.

*Second stretcher unit*

The second stretcher unit (Y) when present, links W to the NMT inhibitor by forming a covalent bond to a functional group of the NMT inhibitor. For example, Y may comprise a carbonyl group which may form a covalent bond to an amino functional group of the NMT inhibitor. When the NMT inhibitor forms a covalent bond via an amino group, the amino group must have an available hydrogen atom, in order to permit reaction with the corresponding functional group (e.g. carbonyl group) of Y i.e. the amino group cannot be tertiary. The second stretcher unit may be a "self-immolative" or a "non-self immolative" group.

A "non-self-immolative" stretcher unit is one in which part or all of the stretcher unit remains bound to the drug moiety upon enzymatic (e.g., proteolytic) cleavage of the ADC. Examples of non-self-immolative spacer units include, but are not limited to, a glycine stretcher unit and a glycine-glycine stretcher unit. Other combinations of peptidic stretcher units susceptible to sequence specific enzymatic cleavage are also contemplated. For example, enzymatic cleavage of an ADC containing a glycine-glycine stretcher unit by a tumor-cell associated protease would result in release of a glycine-glycine-NMT inhibitor moiety from the remainder of the ADC. In one such embodiment, the glycine-glycine-NMT inhibitor moiety is then subjected to a separate hydrolysis step in the tumor cell, thus cleaving the glycine-glycine spacer unit from the NMT inhibitor moiety.

A "self-immolative" spacer unit allows for release of the NMT inhibitor without a separate hydrolysis step.

When W is an amino acid unit, suitably y is 1. When W is a glucuronide unit, suitably y is 0.

In one embodiment, the second stretcher unit (Y) has the formula (Y1):

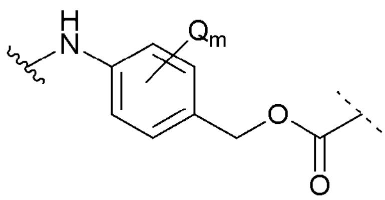

(Y1),

wherein:

each Q is independently halo, $NO_2$, CN, $C_{1-6}$alkyl, $C_{1-6}$haloalkyl, $C_{1-6}$alkoxy or $C_{1-6}$haloalkoxy;
m is 0 to 4;

wherein 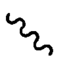 denotes the point of attachment to W; and

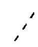 denotes the point of attachment to a functional group of the NMT inhibitor.

In one embodiment, m is not zero and at least one Q is halo. In a second embodiment, m is not zero and at least one Q is $NO_2$. In a third embodiment, m is not zero and at least one Q is CN. In a fourth embodiment, m is not zero and at least one Q is $C_{1-6}$alkyl. In a fifth embodiment, m is not zero and at least one Q is $C_{1-6}$haloalkyl. In a sixth embodiment, m is not zero and at least one Q is $C_{1-6}$alkoxy. In a seventh embodiment, m is not zero and at least one Q is $C_{1-6}$haloalkoxy.

In one embodiment, m is 0. In a second embodiment, m is 1. In a third embodiment, m is 2. In a fourth embodiment, m is 3. In a fifth embodiment, m is 4.

Suitable examples of of self-immolative stretcher units further include, but are not limited to, aromatic compounds that are electronically similar to p-aminobenzyl alcohol and its derivatives e.g. formula (Y1) (*see, e.g.,* US 2005/0256030 AI), such as 2-aminoimidazol-5-methanol derivatives (Hay et al. (1999) Bioorg. Med. Chem. Lett. 9:2237) and ortho- or para-aminobenzylacetals. Spacers that undergo cyclization upon amide bond hydrolysis, such as substituted and unsubstituted 4-aminobutyric acid amides (Rodrigues et al., Chemistry Biology, 1995, 2, 223); appropriately substituted bicyclo[2.2.1] and bicyclo[2.2.2] ring systems (Storm, et al., J. Amer. Chem. Soc., 1972, 94, 5815); and 2-aminophenylpropionic acid amides (Amsberry, et al., J. Org. Chem., 1990, 55, 5867). Elimination of amine-containing drugs that are substituted at the a-position of glycine (Kingsbury, et al., J. Med. Chem., 1984, 27, 1447) are also examples of self-immolative stretcher units useful in ADCs.

In one embodiment, the linker has the formula (LI):

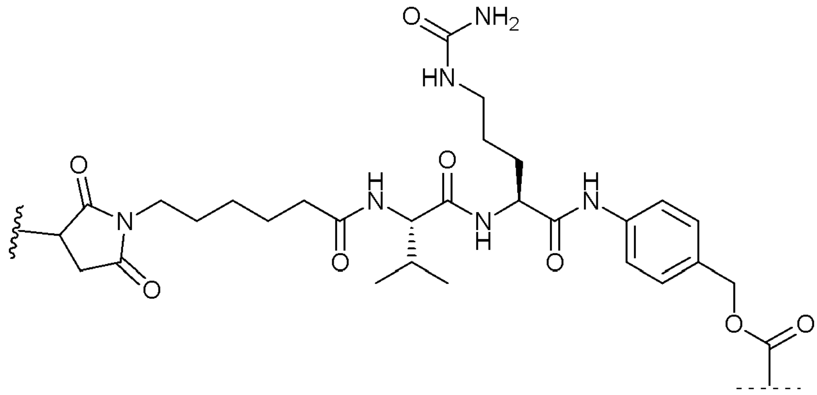
(LI)

wherein 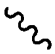 denotes the point of attachment to a chain terminus (such as a N-terminus) or a functional group on an amino acid side chain of the antibody; and

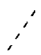 denotes the point of attachment to a functional group of the NMT inhibitor.

In one embodiment, the linker has the formula (LII):

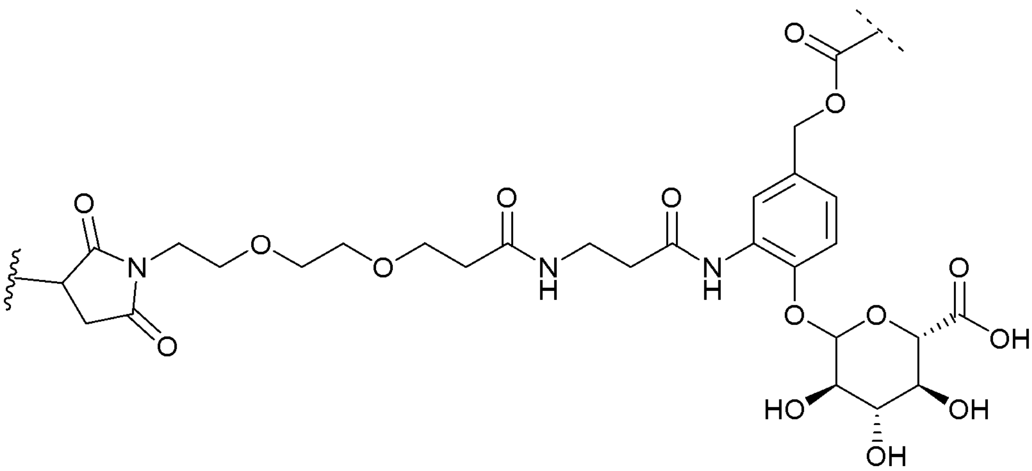

wherein 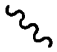 denotes the point of attachment to a chain terminus (such as a N-terminus) or a functional group on an amino acid side chain of the antibody; and

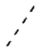 denotes the point of attachment to a functional group of the NMT inhibitor.

In one embodiment, the linker has the formula (LIII):

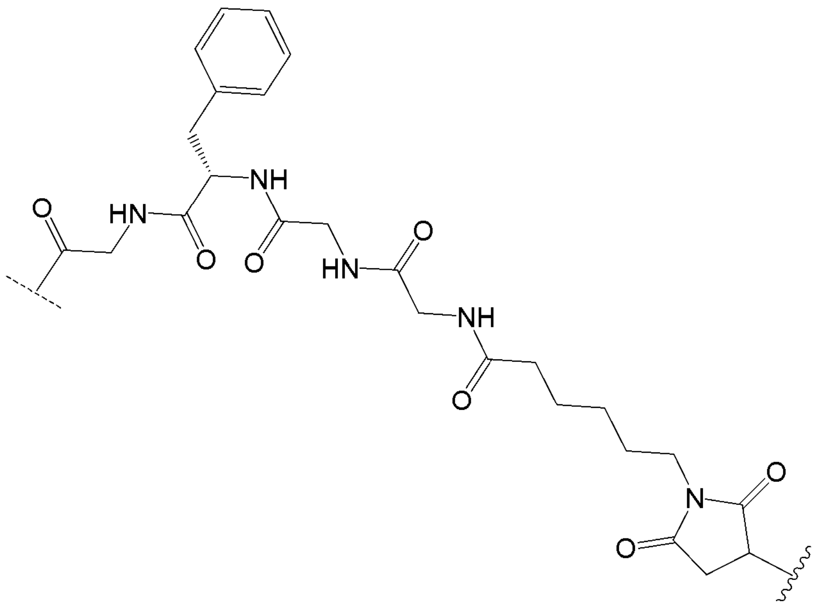

wherein 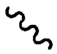 denotes the point of attachment to a chain terminus (such as a N-terminus) or a functional group on an amino acid side chain of the antibody; and

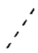 denotes the point of attachment to a functional group of the NMT inhibitor.

In one embodiment, the linker has the formula (LIV):

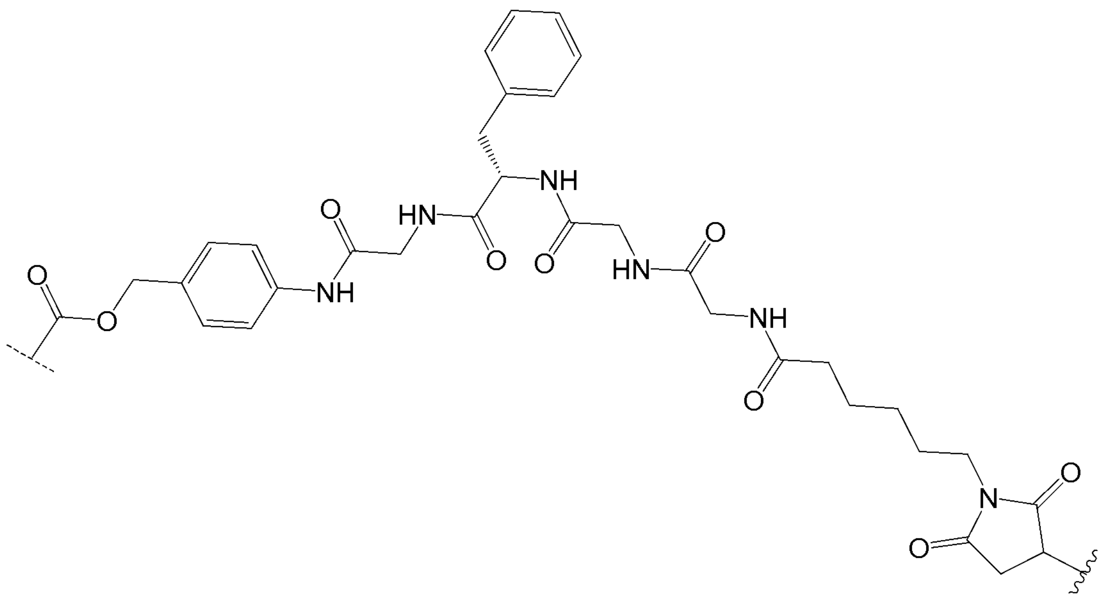

wherein 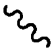 denotes the point of attachment to a chain terminus (such as a N-terminus) or a functional group on an amino acid side chain of the antibody; and

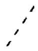 denotes the point of attachment to a functional group of the NMT inhibitor.

**Antibody**

The term "antibody" as used herein relates to monoclonal antibodies, polyclonal antibodies, dimers, multimers, multispecific antibodies (e.g., bispecific antibodies), intact antibodies and antibody fragments, so long as they exhibit the desired biological activity. Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass, or allotype (e.g. human G1m1, G1m2, G1m3, non-G1m1 [that, is any allotype other than G1m1], G1m17, G2m23, G3m21, G3m28, G3m11, G3m5, G3m13, G3m14, G3m10, G3m15, G3m16, G3m6, G3m24, G3m26, G3m27, A2m1, A2m2, Km1, Km2 and Km3) of immunoglobulin molecule. The immunoglobulins can be derived from any species, including human, murine, or rabbit origin. "Antibody fragments" comprise a portion of a full length antibody, generally the antigen binding or variable region thereof. Examples of antibody fragments include Fab, Fab', F(ab')2, and scFv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al (1975) Nature 256:495, or may be made by recombinant DNA methods (see, US 4816567). The monoclonal antibodies may also be isolated from phage antibody libraries using the techniques described in Clackson et al (1991) Nature, 352:624-628; Marks et al (1991) J. Mol. Biol., 222:581-597 or from transgenic mice carrying a fully human immunoglobulin system (Lonberg (2008) Curr. Opinion 20(4):450-459).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies include "primatized" antibodies comprising variable domain antigen-binding sequences derived from a non- human primate (e.g. Old World Monkey or Ape) and human constant region sequences.

An "intact antibody" herein is one comprising VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated

cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called $\alpha$, $\delta$, $\varepsilon$, $\gamma$, and $\mu$, respectively. The subunit structures and three-dimensional configurations of different classes of immunoglobulins are well known.

The desired biological activity of an antibody suitably comprises binding to a cancer associated antigen. Said binding is suitably specific binding i.e. the antibody binds preferentially to said cancer associated antigen and does not significantly bind to other antigens or proteins (such as BSA).

As used herein a cancer associated antigen is an antigen expressed by a cancer cell. Said cancer cell may be a tumour cell or the cell of a blood cancer e.g. a B-cell or a T-cell. Antibodies that target an antigen expressed by a cancer cell thus bind to said cancer cell and (without being limited by theory) mediate cell death through the action of the NMT inhibitor and/or antibody mediates cell cytoxocity (ADCC). Cancer associated antigens may sometimes be expressed by non-cancerous cells but preferably are preferentially expressed by cancerous cells. An example of a cancer associated antigen is HER2. HER2 is in particular expressed on some breast cancer cells. Another example of a cancer associated antigen is CD20. CD20 is expressed on some B-cells including some cells of B-cell lymphomas.

As used herein, "binds to HER2" is used to mean the antibody binds HER2 with a higher affinity than a non-specific partner such as Bovine Serum Albumin (BSA, Genbank accession no. CAA76847, version no. CAA76847.1 GI:3336842, record update date: Jan 7, 2011 02:30 PM). In some embodiments the antibody binds HER2 with an association constant (Ka) at least 2, 3, 4, 5, 10, 20, 50, 100, 200, 500, 1000, 2000, 5000, 104, 105 or 106-fold higher than the antibody's association constant for BSA, when measured at physiological conditions. The antibodies of the invention can bind HER2 with a high affinity. For example, in some embodiments the antibody can bind HER2 with a KD equal to or less than about $10^{-6}$ M, such as 1 x $10^{-6}$, $10^{-7}$, $10^{-8}$, $10^{-9}$, $10^{-10}$, $10^{-11}$, $10^{-12}$, $10^{-13}$ or $10^{-14}$.

As used herein, HER2 refers to Human Epidermal Growth Factor Receptor 2. In one embodiment, HER2 polypeptide corresponds to Genbank accession no. AAA75493, version no. AAA75493.1 GI:306840, record update date: Jun 23, 2010 08:47 AM. In one embodiment, the nucleic acid encoding HER2 polypeptide corresponds to Genbank accession no. M11730, version no. M11730.1 GL183986, record update date: Jun 23, 2010 08:47 AM.

**Modification of antibodies**

The antibodies disclosed herein may be modified, for example, to make them less immunogenic to a human subject. This may be achieved using any of a number of techniques familiar to the person skilled in the art. Some of these techniques are described in more detail below.

<u>Humanisation</u>

Techniques to reduce the *in vivo* immunogenicity of a non-human antibody or antibody fragment include those termed "humanisation". A "humanized antibody" refers to a polypeptide comprising at least a portion of a modified variable region of a human antibody wherein a portion of the variable region, preferably a portion substantially less than the intact human variable domain, has been substituted by the corresponding sequence from a non-human species and wherein the modified variable region is linked to at least another part of another protein, preferably the constant region of a human antibody. The expression "humanized antibodies" includes human antibodies in which one or more complementarity determining region ("CDR") amino acid residues and/or one or more framework region ("FW" or "FR") amino acid residues are substituted by amino acid residues from analogous sites in rodent or other non-human antibodies. The expression "humanized antibody" also includes an immunoglobulin amino acid sequence variant or fragment thereof that comprises an FR having substantially the amino acid sequence of a human immunoglobulin and a CDR having substantially the amino acid sequence of a non- human immunoglobulin.

"Humanized" forms of non-human (e.g., murine) antibodies are chimeric antibodies that contain minimal sequence derived from non-human immunoglobulin. Or, looked at another way, a humanized antibody is a human antibody that also contains selected sequences from non-human (e.g. murine) antibodies in place of the human sequences. A humanized antibody can include conservative amino acid substitutions or non-natural residues from the same or different species that do not significantly alter its binding and/or biologic activity. Such antibodies are chimeric antibodies that contain minimal sequence derived from non- human immunoglobulins.

There are a range of humanisation techniques, including 'CDR grafting', 'guided selection', 'deimmunization', 'resurfacing' (also known as 'veneering'), 'composite antibodies', 'Human String Content Optimisation' and framework shuffling.

CDR grafting

In this technique, the humanized antibodies are human immunoglobulins (recipient antibody) in which residues from a complementary-determining region (CDR) of the recipient antibody are replaced by residues from a CDR of a non-human species (donor antibody) such as mouse, rat, camel, bovine, goat, or rabbit having the desired properties (in effect, the non- human CDRs are 'grafted' onto the human framework). In some instances, framework region (FR) residues of the human immunoglobulin are replaced by corresponding non-human residues (this may happen when, for example, a particular FR residue has significant effect on antigen binding).
Furthermore, humanized antibodies can comprise residues that are found neither in the recipient antibody nor in the imported CDR or framework sequences. These modifications are made to further refine and maximize antibody performance. Thus, in general, a humanized antibody will comprise all of at least one, and in one aspect two, variable domains, in which all or all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), or that of a human immunoglobulin.

Guided selection

The method consists of combining the VH or VL domain of a given non-human antibody specific for a particular epitope with a human VH or VL library and specific human V domains are selected against the antigen of interest. This selected human VH is then combined with a VL library to generate a completely human VHxVL combination. The method is described in Nature Biotechnology (N.Y.) 12, (1994) 899-903.

Composite antibodies

In this method, two or more segments of amino acid sequence from a human antibody are combined within the final antibody molecule. They are constructed by combining multiple human VH and VL sequence segments in combinations which limit or avoid human T cell epitopes in the final composite antibody V regions. Where required, T cell epitopes are limited or avoided by, exchanging V region segments contributing to or encoding a T cell epitope with alternative segments which avoid T cell epitopes. This method is described in US 2008/0206239 A1.

Deimmunization

This method involves the removal of human (or other second species) T-cell epitopes from the V regions of the therapeutic antibody (or other molecule). The therapeutic antibodies V-region sequence is analysed for the presence of MHC class II- binding motifs by, for example, comparison with databases of MHC-binding motifs (such as the "motifs" database hosted atwww.wehi.edu.au). Alternatively, MHC class II- binding motifs may be identified using computational threading methods such as those devised by Altuvia et al. (J. Mol. Biol. 249 244-250 (1995)); in these methods, consecutive overlapping peptides from the V-region sequences are testing for their binding energies to MHC class II proteins. This data can then be combined with information on other sequence features which relate to successfully presented peptides, such as amphipathicity, Rothbard motifs, and cleavage sites for cathepsin B and other processing enzymes.
Once potential second species (e.g. human) T-cell epitopes have been identified, they are eliminated by the alteration of one or more amino acids. The modified amino acids are usually within the T-cell epitope itself, but may also be adjacent to the epitope in terms of the primary or secondary structure of the protein (and therefore, may not be adjacent in the primary structure). Most typically, the alteration is by way of substitution but, in some circumstances amino acid addition or deletion will be more appropriate.
All alterations can be accomplished by recombinant DNA technology, so that the final molecule may be prepared by expression from a recombinant host using well established methods such as Site Directed Mutagenesis. However, the use of protein chemistry or any other means of molecular alteration is also possible.

Resurfacing

This method involves:

(a) determining the conformational structure of the variable region of the non-human (e.g. rodent) antibody (or fragment thereof) by constructing a three-dimensional model of the non-human antibody variable region;
(b) generating sequence alignments using relative accessibility distributions from x-ray crystallographic structures of a sufficient number of non-human and human antibody variable region heavy and light chains to give a set of heavy

and light chain framework positions wherein the alignment positions are identical in 98% of the sufficient number of non-human antibody heavy and light chains;

(c) defining for the non-human antibody to be humanized, a set of heavy and light chain surface exposed amino acid residues using the set of framework positions generated in step (b);

(d) identifying from human antibody amino acid sequences a set of heavy and light chain surface exposed amino acid residues that is most closely identical to the set of surface exposed amino acid residues defined in step (c), wherein the heavy and light chain from the human antibody are or are not naturally paired;

(e) substituting, in the amino acid sequence of the non-human antibody to be humanized, the set of heavy and light chain surface exposed amino acid residues defined in step (c) with the set of heavy and light chain surface exposed amino acid residues identified in step (d);

(f) constructing a three-dimensional model of the variable region of the non-human antibody resulting from the substituting specified in step (e);

(g) identifying, by comparing the three-dimensional models constructed in steps (a) and (f), any amino acid residues from the sets identified in steps (c) or (d), that are within 5 Angstroms of any atom of any residue of the complementarity determining regions of the non-human antibodt to be humanized; and

(h) changing any residues identified in step (g) from the human to the original non- human amino acid residue to thereby define a non-human antibody humanizing set of surface exposed amino acid residues; with the proviso that step (a) need not be conducted first, but must be conducted prior to step (g).

Superhumanization

The method compares the non-human sequence with the functional human germline gene repertoire. Those human genes encoding canonical structures identical or closely related to the non-human sequences are selected. Those selected human genes with highest homology within the CDRs are chosen as FR donors. Finally, the non-human CDRs are grafted onto these human FRs. This method is described in patent WO 2005/079479 A2.

Human String Content Optimization

This method compares the non-human (e.g. mouse) sequence with the repertoire of human germline genes and the differences are scored as Human String Content (HSC) that quantifies a sequence at the level of potential MHC/T-cell epitopes. The target sequence is then humanized by maximizing its HSC rather than using a global identity measure to generate multiple diverse humanized variants (described in Molecular Immunology, 44, (2007) 1986-1998).

Framework Shuffling

The CDRs of the non-human antibody are fused in-frame to cDNA pools encompassing all known heavy and light chain human germline gene frameworks. Humanised antibodies are then selected by e.g. panning of the phage displayed antibody library. This is described in Methods 36, 43-60 (2005).

In one embodiment, known antibodies for the treatment or prevention of a cancer are used in accordance with the present invention. Examples of antibodies available for the treatment of cancer include, but are not limited to, trastuzumab which is a humanized anti-HER2 monoclonal antibody for the treatment of patients with metastatic breast cancer (Stebbing, J., Copson,E., and O'Reilly, S. "Herceptin (trastuzamab) in advanced breast cancer" Cancer Treat Rev (2000).26, 287-90; Miller et al (2003) Journal of Immunology 170, 4854-4861) and rituximab which is a chimeric anti-CD20 monoclonal antibody for the treatment of patients with non-Hodgkin's lymphoma. Further examples include: oregovomab (OvaRex, AltaRex Corporation, MA) which is a murine antibody for the treatment of ovarian cancer; Panorex (Glaxo Wellcome, NC) which is a murine IgG2a antibody for the treatment of colorectal cancer; BEC2 (hnClone Systems Inc., NY) which is murine IgG antibody for the treatment of lung cancer; IMC-C225 (Imclone Systems Inc., NY) which is a chimeric IgG antibody for the treatment of head and neck cancer; Vitaxin (Medhnmune, Inc., MD) which is a humanized antibody for the treatment of sarcoma; Campath 1/H (Leukosite, MA) which is a humanized IgGi antibody for the treatment of chronic lymphocytic leukemia (CLL); Smart MI95 (Protein Design Labs, Inc., CA) which is a humanized IgG antibody for the treatment of acute myeloid leukemia (AML); LymphoCide (Immunomedics, Inc., NJ) which is a humanized IgG antibody for the treatment of non-Hodgkin's lymphoma; Smart ID 10 (Protein Design Labs, Inc., CA) which is a humanized antibody for the treatment of non-Hodgkin's lymphoma; Oncolym (Techniclone, Inc., CA) which is a murine antibody for the treatment of non-Hodgkin's lymphoma; Allomune (BioTransplant, CA) which is a humanized anti-CD2 mAh for the treatment of Hodgkin's Disease or non-Hodgkin's lymphoma; anti-VEGF (Genentech, Inc., CA) which is humanized antibody for the treatment of lung and colorectal cancers; CEAcide (Immunomedics, NJ) which is a humanized anti-CEA antibody for the treatment of colorectal cancer; IMC-1C11 (ImClone Systems, NJ) which is an anti-KDR chimeric antibody for the treatment of colorectal cancer, lung cancers, and melanoma; and cetuximab (ImClone, NJ) which is an anti-EGFR chimeric antibody

for the treatment of epidermal growth factor positive cancers. Further examples of antibodies that bind to CD20 include ocrelizumab, obinutuzumab, ofatumumab, ibritumomab tiuxetan, tositumomab, and ublituximab.

Other antibodies useful in the treatment of cancer include, but are not limited to, antibodies against the following cancer associated antigens: CA125 (ovarian), CA15-3 (carcinomas), CAI9-9 (carcinomas), L6 (carcinomas), Lewis Y (carcinomas), Lewis X (carcinomas), alpha fetoprotein (carcinomas), CA 242 (colorectal), placental alkaline phosphatase (carcinomas), prostate specific antigen (prostate), prostatic acid phosphatase (prostate), epidermal growth factor (carcinomas), MAGE-1 (carcinomas), MAGE-2 (carcinomas), MAGE-3 (carcinomas), MAGE -4 (carcinomas), anti-transferrin receptor (carcinomas), p97 (melanoma), MUC1-KLH (breast cancer), CEA (colorectal), gplOO (melanoma), MARTI (melanoma), PSA (prostate), IL-2 receptor (T-cell leukemia and lymphomas), CD19 (B-cell lymphoma), CD20 (non-Hodgkin's lymphoma), CD52 (leukemia), CD33 (leukemia), CD22 (lymphoma), human chorionic gonadotropin (carcinoma), CD38 (multiple myeloma), CD40 (lymphoma), mucin (carcinomas), P21 (carcinomas), MPG (melanoma), and Neu oncogene product (carcinomas). Some specific useful antibodies include, but are not limited to, BR96 mAb (Trail, P. A., Willner, D., Lasch, S. J., Henderson, A. J., Hofstead, S. J., Casazza, A. M., Firestone, R. A., Hellstrom, I., Hellstrom, K. E., "Cure of Xenografited Human Carcinomas by BR96-Doxorubicin Immunoconjugates" Science 1993,261,212-215), BR64 (Trail, PA, Willner, D, Knipe, J., Henderson, A. J., Lasch, S. J., Zoeckler, M. E., Trailsmith, M. D., Doyle, T. W., King, H. D., Casazza, A. M., Braslawsky, G. R., Brown, J. P., Hofstead, S. J., (Greenfield, R. S., Firestone, R. A., Mosure, K., Kadow, D. F., Yang, M. B., Hellstrom, K. E., and Hellstrom, I. "Effect of Linker Variation on the Stability, Potency, and Efficacy of Carcinoma-reactive BR64-Doxorubicin Immunoconjugates" Cancer Research 1997, 57, 100-105, mAbs against the CD40 antigen, such as S2C6 mAb (Francisco, J. A., Donaldson, K. L., Chace, D., Siegall, C. B., and Wahl, A. F. "Agonistic properties and in vivo antitumor activity of the anti-CD-40 antibody, SGN-14" Cancer Res. 2000, 60, 3225-3231), mAbs against the CD70 antigen, such as 1F6 mAb, and mAbs against the CD30 antigen, such as AGIO (Bowen, M. A., Olsen, K. J., Cheng, L., Avila, D., and Podack, E. R. "Functional effects of CD30 on a large granular lymphoma cell line YT" J. Immunol., 151, 5896-5906, 1993). Many other internalizing antibodies that bind to tumor associated antigens can be used in this invention, and have been reviewed (Franke, A. E., Sievers, E. L., and Scheinberg, D. A., "Cell surface receptor-targeted therapy of acute myeloid leukemia: a review" Cancer Biother Radiopharm. 2000,15, 459-76; Murray, J. L., "Monoclonal antibody treatment of solid tumors: a coming of age" Semin Oncol. 2000, 27, 64-70; Breitling, F., and Dubel, S., Recombinant Antibodies, John Wiley, and Sons, New York, 1998).

Other antibodies which may be useful in the treatment of cancer include those disclosed in Tong et al, Molecules 2021, 26, 5847, https://doi.org/10.3390/molecules26195847, and Coats et al., Clin Cancer Res 2019;25:5441-8, the entire contents of which are incorporated by reference for the purpose of defining the antibody.

In one embodiment the antibody is selected from the group consisting of gemtuzumab brentuximab, ado-trastuzumab, fam-trastuzumab, inotuzumab, polatuzumab, acituzumabab, sacituzumab, belantamab, ioncastuximab, tisotumab, indatuximab, naratuximab and depatuxizumab.

In another embodiment, the antibody is sacituzumab. Alternatively, the antibody is ifinatamab.

Suitably, the antibody is selected from the group consisting of trastuzumab, rituximab, sacituzumab and ifinatamab.

In one embodiment, the antibody is a humanised, chimeric, human antibody or an antibody fragment.

In one embodiment, the antibody binds to HER2. In one embodiment, the antibody is trastuzumab, pertuzumab, margetuximab, ertumaxomab, MM-111, HER2Bi-aATCs, MCLA-128, ZW25, MDX-210 ado-trastuzumab and fam-trastuzumab. Suitably, the antibody is trastuzumab. In one embodiment, the antibody is an antibody that has the 6 CDRs of trastuzumab. Trastuzumab comprises the heavy chain of SEQ ID NO: 2 and light chain of SEQ ID NO: 1.

In one embodiment, the antibody binds to CD20. Suitably, the antibody is rituximab. In one embodiment, the antibody is an antibody that has the 6 CDRs of rituximab. Rituximab comprises the heavy chain of SEQ ID NO: 4 and light chain of SEQ ID NO: 3.

In one embodiment, the antibody binds to an antigen selected from the group consisting of: CLDN18, FOLR1, EGFR, Nectin-4, CD22, c-MET, CD19, CEACAM5, Mesothelin, PSMA, ROR1, TF, TNF-BCAM, TROP2, VTCN1, 5T4 oncofetal antigen, AXL, CD276, CD30, CD38, IL3RA, NaPi2b, BRAF, Cadherin-6, CD37, CD70, DLK1, ENPP3, EpCAM, HER3, LRRC15, PD-L1, PTK7, STING, TEM1, TOP1 and VEGF.

In one embodiment, the antibody binds to members of the Claudin 18 specifically Claudin 18.2 and for example an ADC comprising said antibody may be for use in the treatment of gastric cancer. In one embodiment, the cancer expresses one or more members of the Claudin 18 specifically Claudin 18.2.

In one embodiment, the antibody binds to the Folate receptor and for example an ADC comprising said antibody may be for use in the treatment of ovarian and endometrial cancer. In this embodiment, suitably the antibody is mirvetuximab. In one embodiment, the cancer expresses a Folate receptor.

In one embodiment, the antibody binds to the EGF receptor or a member of the EGF Receptor family and for example an ADC comprising said antibody may be for use in the treatment of multiple types of epithelial cell cancer. In one embodiment, the cancer expresses a EGF receptor.

In one embodiment, the antibody binds to Nectin 4 and for example an ADC comprising said antibody may be for

use in the treatment of urothelial carcinoma, bladder cancer, pancreatic cancer, triple-negative breast cancer, non-small cell lung cancer, gastric cancer, esophageal cancer or ovarian cancer. In this embodiment, suitably the antibody is enfortumab. In one embodiment, the cancer expresses Nectin 4.

In one embodiment, the antibody binds to CD22 and for example an ADC comprising said antibodymay be for use in the treatment of Non-Hodgkin Lymphoma. In one embodiment, the cancer expresses CD22.

In one embodiment, the antibody binds to c-MET and for example an ADC comprising said antibody may be for use in the treatment of non-small cell lung cancer (NSCLC). In one embodiment, the cancer expresses c-MET.

In one embodiment, the antibody binds to Trop-2 and for example an ADC comprising said antibody may be for use in the treatment of Metastatic triple-negative breast cancer and metastatic urothelial cancer. In this embodiment, suitably the antibody is sacituzumab. In one embodiment, the cancer expresses Trop-2. Sacituzumab comprises the heavy chain of SEQ ID NO: 8 and light chain of SEQ ID NO: 7.

In one embodiment, the antibody binds to Tissue factor and for example an ADC comprising said antibody may be for use in the treatment of previously treated recurrent or metastatic cervical cancer. In this embodiment, suitably the antibody is tisotumab. In one embodiment, the cancer expresses Tissue factor.

In one embodiment, the antibody binds to CD276 (B7-H3) and for example an ADC comprising said antibody may be for use in the treatment of prostate cancer. In this embodiment, suitably the antibody is ifinatamab. In one embodiment, the cancer expresses CD276 (B7-H3). Ifinatamab comprises the heavy chain of SEQ ID NO: 6 and light chain of SEQ ID NO: 5.

In one embodiment, the antibody binds to receptor tyrosine kinase like orphan receptor 1 (ROR1) and for example an ADC comprising said antibody may be for use in the treatment of various hematological and solid malignancies. In one embodiment, the cancer expresses tyrosine kinase like orphan receptor 1 (ROR1).

In one embodiment, the antibody binds to BCMA and for example an ADC comprising said antibody may be for use in the treatment of multiple myeloma. In one embodiment, the cancer expresses BCMA.

In one embodiment, the antibody binds to PSMA and for example an ADC comprising said antibody may be for use in the treatment of prostate cancer. In one embodiment, the cancer expresses PSMA.

In one embodiment, the antibody binds to CEACAM5 and for example an ADC comprising said antibody may be for use in the treatment of Advanced Non squamous non-small cell lung cancer (NSCLC). In one embodiment, the cancer expresses CEACAM5.

In one embodiment, the antibody binds to Mesothelin and for example an ADC comprising said antibody may be for use in the treatment of mesothelioma, ovarian, pancreatic, gastric, and non-small cell lung tumors. In one embodiment, the cancer expresses Mesothelin.

In one embodiment, the antibody binds to AXL and for example an ADC comprising said antibody may be for use in the treatment of multiple solid tumours including non-small cell lung cancer (NSCLC) and triple negative breast cancer (TNBC). In this embodiment, suitably the antibody is enapotamab. In one embodiment, the cancer expresses AXL.

**Drug Loading**

The drug loading (referred to as variable "p") is the average number of NMT inhibitors per antibody. Where the compounds of the invention are bound to cysteine residues, drug loading may range from 1 to 10 NMT inhibitors per antibody, i.e. where 1, 2 ,3, 4, 5, 6, 7, 8, 9 or 10 NMT inhibitors are covalently attached to the antibody. Compositions of conjugates include collections of antibodies, conjugated with a range of NMT inhibitors from 1 to 10.

The average number of NMT inhibitors per antibody in the preparation of ADC from conjugation reactions may be characterized by conventional means such as UV, reverse phase HPLC, HIC, mass spectroscopy, ELISA assay, and electrophoresis. The quantitative distribution of ADC in terms of p may also be determined. By ELISA, the averaged value of p in a particular preparation of ADC may be determined (Hamblett et al (2004) Clin. Cancer Res. 10:7063-7070; Sanderson et al (2005) Clin. Cancer Res. 11:843-852). However, the distribution of p (NMT inhibitor) values is not discernible by the antibody-antigen binding and detection limitation of ELISA. Also, ELISA assay for detection of ADCs does not determine where the drug moieties are attached to the antibody, such as the heavy chain or light chain fragments, or the particular amino acid residues. In some instances, separation, purification, and characterization of homogeneous ADC where p is a certain value from ADCs with other NMT inhibitor loadings may be achieved by means such as reverse phase HPLC or electrophoresis. Such techniques are also applicable to other types of conjugates.

For some ADCs, p may be limited by the number of attachment sites on the antibody. For example, an antibody may have only one or several cysteine thiol groups, or may have only one or several sufficiently reactive thiol groups through which a linker may be attached. Higher drug loading, e.g. p >5, may cause aggregation, insolubility, toxicity, or loss of cellular permeability of certain ADCs.

Typically, fewer than the theoretical maximum of NMT inhibitors are conjugated to an antibody during a conjugation reaction. An antibody may contain, for example, many lysine residues that do not react with the NMT inhibitor-linker intermediate or linker reagent. Only the most reactive lysine groups may react with an amine-reactive linker

reagent. Also, only the most reactive cysteine thiol groups (e.g. sulfhydryl) may react with a thiol-reactive linker reagent. Generally, antibodies do not contain many, if any, free and reactive cysteine thiol groups which may be linked to a NMT inhibitor. Most cysteine thiol residues in the antibodies of the compounds exist as disulfide bridges and must be reduced with a reducing agent such as dithiothreitol (DTT) or TCEP, under partial or total reducing conditions. The loading of an ADC may be controlled in several different manners, including: (i) limiting the molar excess of NMT inhibitor-linker intermediate or linker reagent relative to antibody, (ii) limiting the conjugation reaction time or temperature, and (iii) partial or limiting reductive conditions for cysteine thiol modification.

Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by engineering one, two, three, four, or more cysteine residues (e.g., preparing mutant antibodies comprising one or more non-native cysteine amino acid residues). US 7521541 teaches engineering antibodies by introduction of reactive cysteine amino acids. Cysteine amino acids may be engineered at reactive sites in an antibody and which do not form intrachain or intermolecular disulfide linkages (Junutula, et al., 2008, Nature Biotech., 26(8):925-932; Dornan etal (2009) Blood 114(13):2721-2729; US 7521541; US 7723485; WO2009/052249). The engineered cysteine thiols may react with linker reagents or the drug-linker reagents of the present invention which have thiol-reactive, electrophilic groups such as maleimide or alpha-halo amides to form ADC with cysteine engineered antibodies and the NMT inhibitor moieties. The location of the NMT inhibitor moiety can thus be designed, controlled, and known. The NMT inhibitor loading can be controlled since the engineered cysteine thiol groups typically react with thiol-reactive linker reagents or drug-linker reagents in high yield. Engineering an IgG antibody to introduce a cysteine amino acid by substitution at a single site on the heavy or light chain gives two new cysteines on the symmetrical antibody. A NMT inhibitor loading near 2 can be achieved with near homogeneity of the conjugation product ADC.

Alternatively, site-specific conjugation can be achieved by engineering antibodies to contain unnatural amino acids in their heavy and/or light chains as described by Axup et al. ((2012), Proc Natl Acad Sci USA. 109(40):16101-16116). The unnatural amino acids provide the additional advantage that orthogonal chemistry can be designed to attach the linker reagent and NMT inhibitor.

Where more than one nucleophilic or electrophilic group of the antibody reacts with a NMT inhibitor-linker intermediate, or linker reagent followed by NMT inhibitor, then the resulting product is a mixture of ADC compounds with a distribution of NMT inhibitors attached to an antibody, e.g. 1, 2, 3, etc. Liquid chromatography methods such as polymeric reverse phase (PLRP) and hydrophobic interaction (HIC) may separate compounds in the mixture by NMT inhibitor loading value. Preparations of ADC with a single NMT inhibitor loading value (p) may be isolated, however, these single loading value ADCs may still be heterogeneous mixtures because the NMT inhibitors may be attached, via the linker, at different sites on the antibody.

Thus the ADCs of the invention include mixtures of ADC compounds where the antibody has one or more NMT inhibitor moieties and where the NMT inhibitor moieties may be attached to the antibody at various amino acid residues.

In one embodiment, the drug loading (p) of NMT inhibitor to antibody is between 1 to 10 NMT inhibitor(s) to antibody. Suitably, the drug loading (p) is between 2 and 6, 4 and 6, 8 and 10, or 6 and 8 NMT inhibitors to antibody.

In one embodiment, the ADC of the invention comprises the following formula:

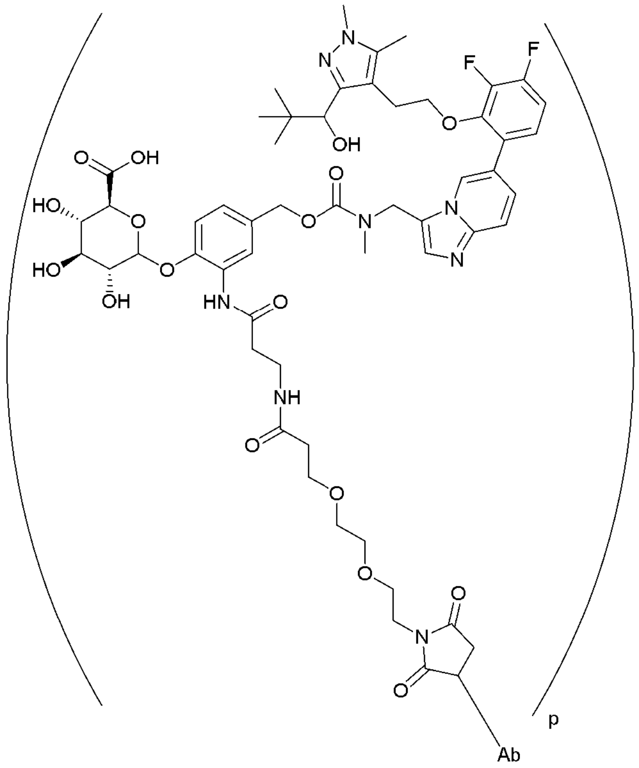

,

wherein Ab is an antibody as defined herein and p is as defined herein. Suitably, the ADC of the invention, or a salt thereof is bound to the antibody via a sulfhydryl group on the side chain of a cysteine amino acid on the antibody. Suitably, the antibody is trastuzumab or rituximab, especially trastuzumab. Alternatively, the antibody is sacituzumab. Alternatively, the antibody is ifinatamab. Suitably p is between 1 to 10, for example p is between 2 and 6, 4 and 6, 8 and 10, or 6 and 8. Most suitably, p is around e.g. is 5.

In one embodiment, the ADC of the invention comprises the following formula:

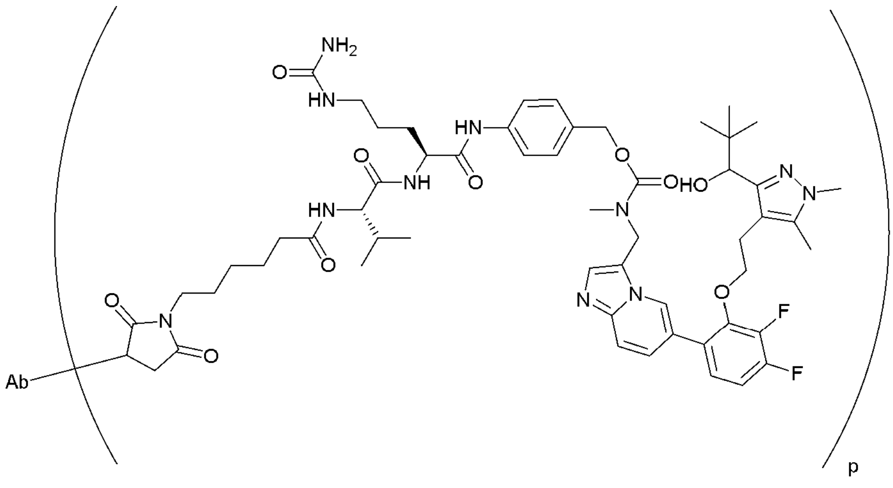

,

wherein Ab is an antibody as defined herein and p is as defined herein. Suitably, the ADC of the invention, or a salt thereof is bound to the antibody via a sulfhydryl group on the side chain of a cysteine amino acid on the antibody. Suitably, the antibody is trastuzumab or rituximab, especially trastuzumab. Alternatively, the antibody is sacituzumab. Alternatively, the antibody is ifinatamab.

Suitably p is between 1 to 10, for example p is between 2 and 6, 4 and 6, 8 and 10, or 6 and 8. Most suitably, p is around e.g. is 5.

In one embodiment, the ADC of the invention comprises the following formula:

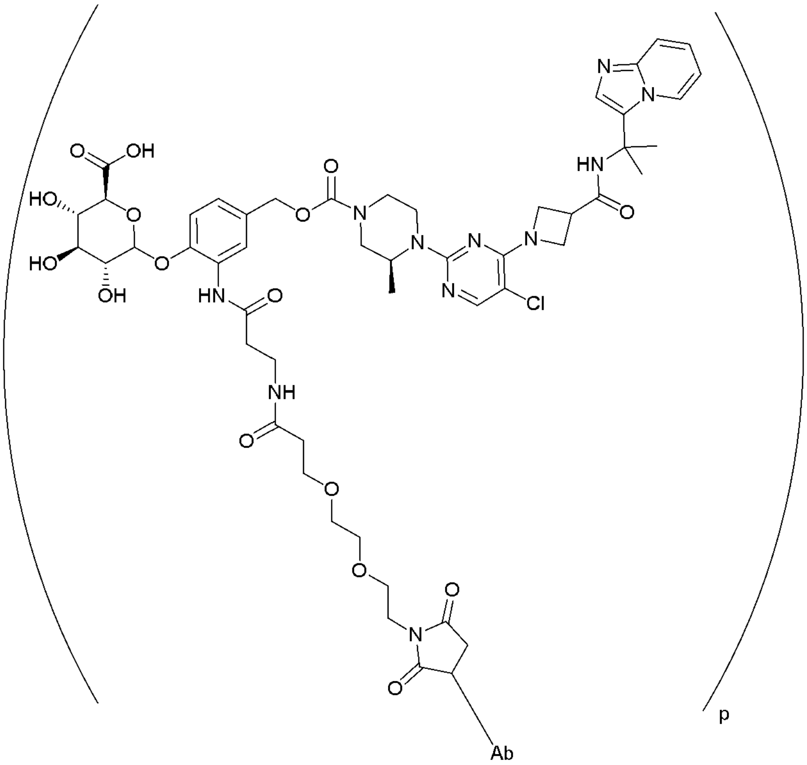

,

wherein Ab is an antibody as defined herein and p is as defined herein. Suitably, the ADC of the invention, or a salt thereof is bound to the antibody via a sulfhydryl group on the side chain of a cysteine amino acid on the antibody. Suitably, the antibody is trastuzumab or rituximab, especially trastuzumab. Suitably p is between 1 to 10, for example p is between 2 and 6, 4 and 6, 8 and 10, or 6 and 8. Most suitably, p is around e.g. is 5.

In one embodiment, the ADC of the invention comprises the following formula:

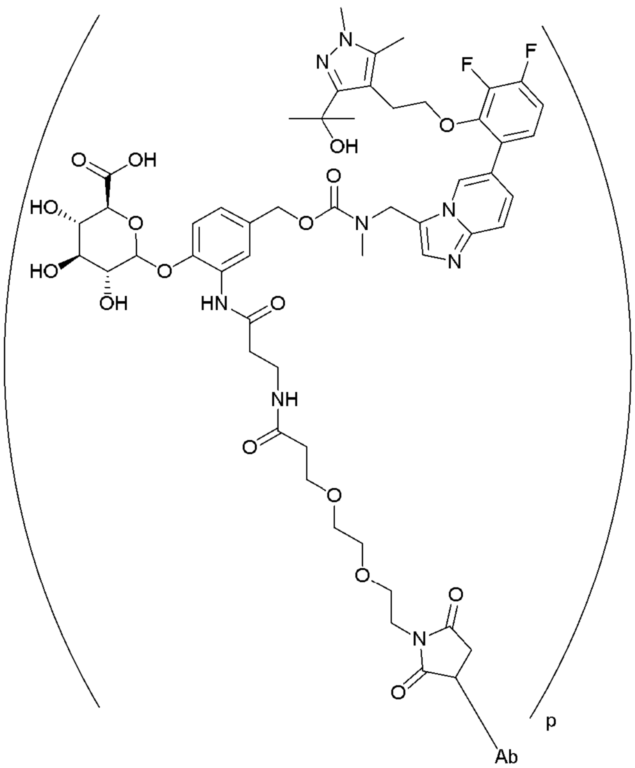

,

wherein Ab is an antibody as defined herein and p is as defined herein. Suitably, the ADC of the invention, or a salt thereof is bound to the antibody via a sulfhydryl group on the side chain of a cysteine amino acid on the antibody. Suitably, the antibody is trastuzumab or rituximab, especially trastuzumab. Alternatively, the antibody is sacituzumab. Alternatively, the antibody is ifinatamab. Suitably p is between 1 to 10, for example p is between 2 and 6, 4 and 6, 8 and 10, or 6 and 8. Most suitably, p is around e.g. is 5.

In one embodiment, the ADC of the invention comprises the following formula:

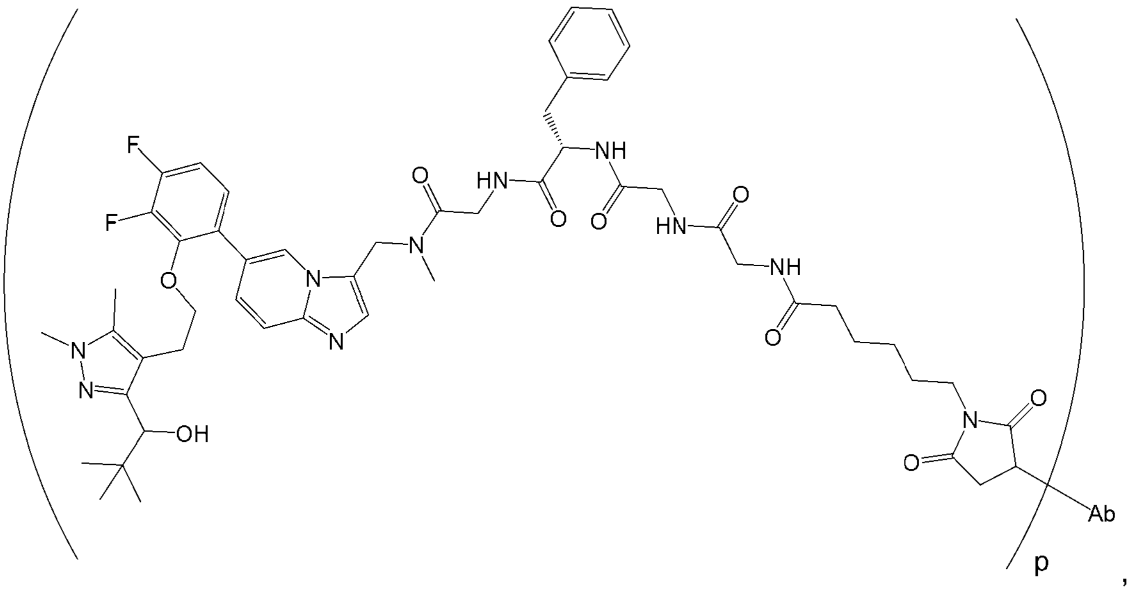

wherein Ab is an antibody as defined herein and p is as defined herein. Suitably, the ADC of the invention, or a salt thereof is bound to the antibody via a sulfhydryl group on the side chain of a cysteine amino acid on the antibody. Suitably, the antibody is trastuzumab or rituximab, especially trastuzumab. Alternatively, the antibody is sacituzumab. Alternatively, the antibody is ifinatamab. Suitably p is between 1 to 10, for example p is between 2 and 6, 4 and 6, 8 and 10, or 6 and 8. Most suitably, p is around e.g. is 5.

In one embodiment, the ADC of the invention comprises the following formula:

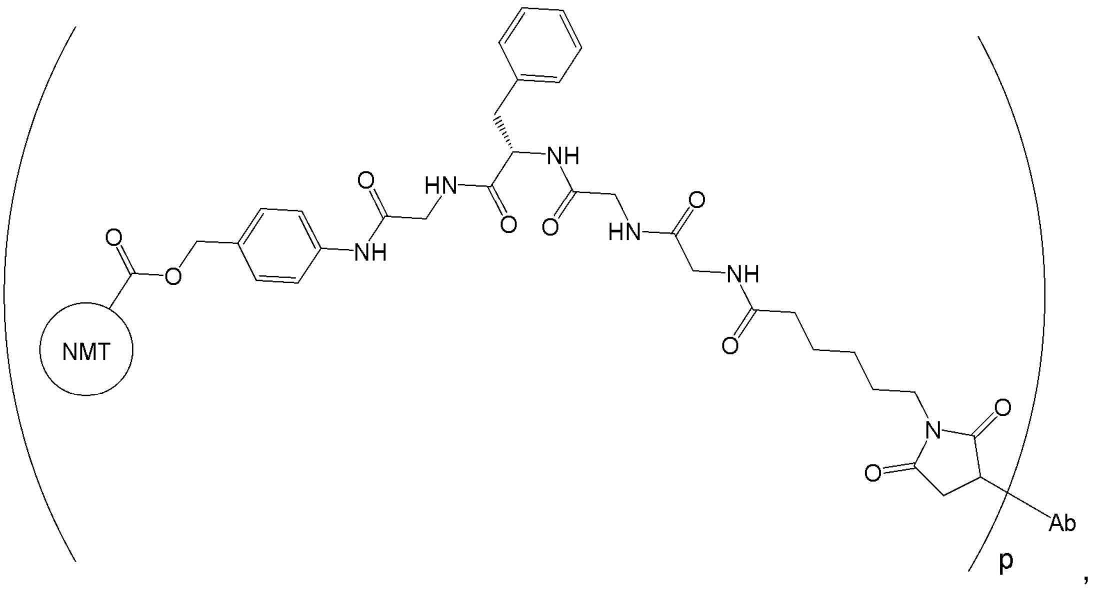

wherein Ab is an antibody as defined herein and p is as defined herein. NMT is a NMT inhibitor. Suitably, the NMT inhibitor is a compound of formula (VI), or a salt thereof. Suitably, the NMT inhibitor is a compound of formula (VIA), or a salt thereof. Suitably, the ADC of the invention, or a salt thereof is bound to the antibody via a sulfhydryl group on the side chain of a cysteine amino acid on the antibody. Suitably, the antibody is trastuzumab or rituximab, especially trastuzumab.

Alternatively, the antibody is sacituzumab. Alternatively, the antibody is ifinatamab. Suitably p is between 1 to 10, for example p is between 2 and 6, 4 and 6, 8 and 10, or 6 and 8. Most suitably, p is around e.g. is 5.

**Drug conjugates**

The ADCs of the invention may be prepared using a drug conjugate or salt and/or solvate thereof which is later covalently bonded to the antibody. Therefore, in one embodiment there is provided a drug conjugate or a salt and/or solvate thereof, which comprises a group capable of forming a covalent bond to a chain terminus (such as a N-terminus) or a functional group on an amino acid side chain of an antibody e.g. a sulfhydryl group.
In one embodiment, the drug conjugate has the formula:

$A_a$-$W_w$-$Y_y$-NMT (formula (X))

wherein:

A is a first stretcher unit which when present comprises a group capable of forming a covalent bond to a chain terminus (such as a N-terminus) or a functional group on an amino acid side chain of an antibody e.g. a sulfhydryl group;
NMT is a NMT inhibitor as defined above; and
a, W, w, Y and y are as defined above,

or a salt and/or solvate thereof.
It will be understood that preferences and embodiments disclosed herein in respect of the ADC of formula (VII) apply equally to the drug conjugate of formula (X).
The NMT inhibitor forms a covalent bond with the second stretcher unit (Y), when present, or when Y is absent, with the amino acid unit or glucuronide unit (W). Suitably, the functional group on the NMT inhibitor is an amino or alcohol, for example an amino. When the NMT inhibitor forms a covalent bond via an amino group, the amino group must have an available hydrogen atom, in order to permit reaction with the corresponding functional group (e.g. carbonyl group) in the linker, specifically in the second stretcher unit (Y), when present, or when Y is absent, with the amino acid unit or glucuronide unit (W), i.e. the amino group cannot be tertiary.
In one embodiment the group capable of forming a covalent bond to a chain terminus (such as a N-terminus) or a functional group on an amino acid side chain of an antibody is a maleimide moiety.
In one embodiment, A is:

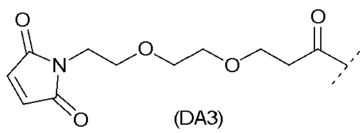
(DA3)
,

wherein 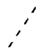 denotes the point of attachment to W.
In one embodiment, the drug conjugate is a compound of formula (DC-1):

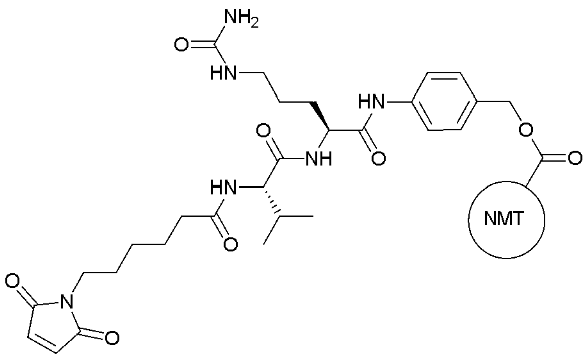
(DC-1),

or a salt and/or solvate thereof, wherein

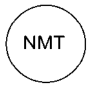

is a NMT inhibitor. Suitably, the NMT inhibitor is a compound of formula (VI), or a salt thereof. Suitably, the NMT inhibitor is a compound of formula (VIA), or a salt thereof.

The phrase "

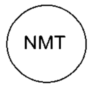

is a NMT inhibitor" as used herein would be understood by the skilled person to be the moiety which remains after an NMT inhibitor, such as an NMT inhibitor comprising a suitable functional group for attachment to a linker, such as an amino group (which comprises a hydrogen atom) or an alcohol (-OH), reacts with a suitable functional group on the linker, for example a carbonyl group, thus forming the linker-NMT inhibitor covalent bond.

When the NMT inhibitor is a compound of formula (VI),

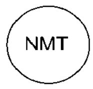

in any one of the embodiments disclosed herein means:

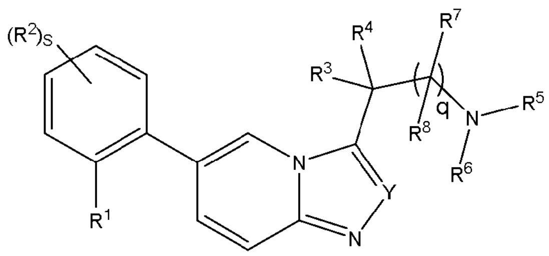

(VI)

wherein all variables are as defined above;
wherein one group of $R_5$ and $R_6$ is absent such that the N atom may form a covalent bond with the linker.

Similarly, when the NMT inhibitor is a compound of formula (VIA),

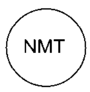

in any one of the embodiments disclosed herein means:

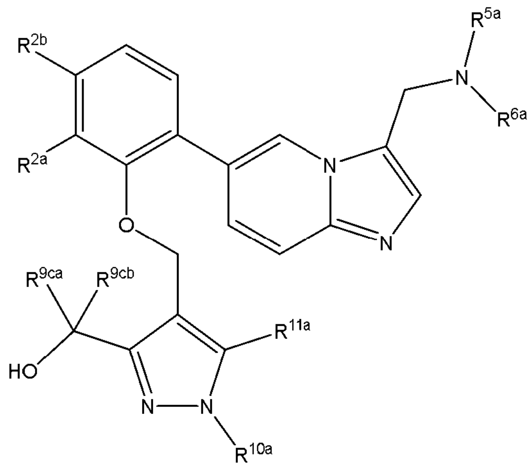

(VIA),

wherein all variables are as defined above;
wherein one group of $R_5$ and $R_6$ is absent such that the N atom may form a covalent bond with the linker.

In one embodiment the drug conjugate has the formula (DC-2):

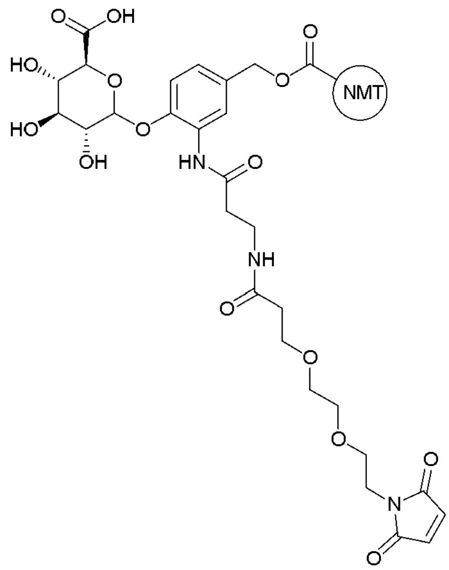

(DC-2),

or a salt and/or solvate thereof, wherein

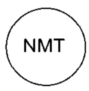

is a NMT inhibitor. Suitably, the NMT inhibitor is a compound of formula (VI), or a salt thereof. Suitably, the NMT inhibitor is a compound of formula (VIA), or a salt thereof.

In one embodiment, the drug conjugate is a compound of formula (DC-5):

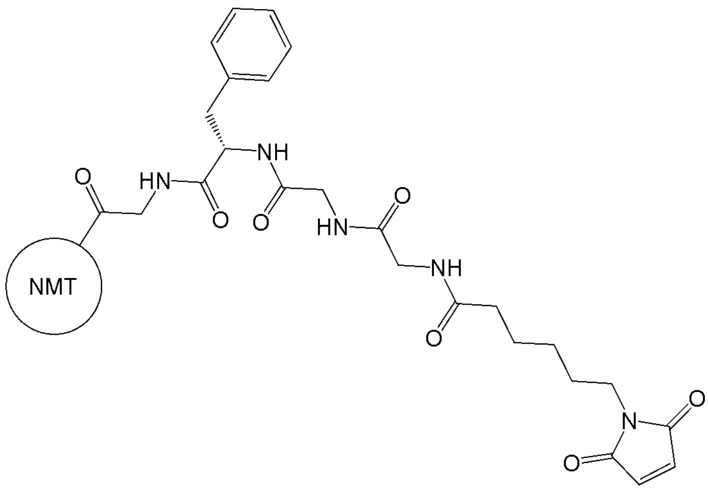

or a salt and/or solvate thereof, wherein

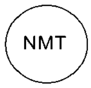

is a NMT inhibitor. Suitably, the NMT inhibitor is a compound of formula (VI), or a salt thereof. Suitably, the NMT inhibitor is a compound of formula (VIA), or a salt thereof.

In one embodiment, the drug conjugate is a compound of formula (DC-6):

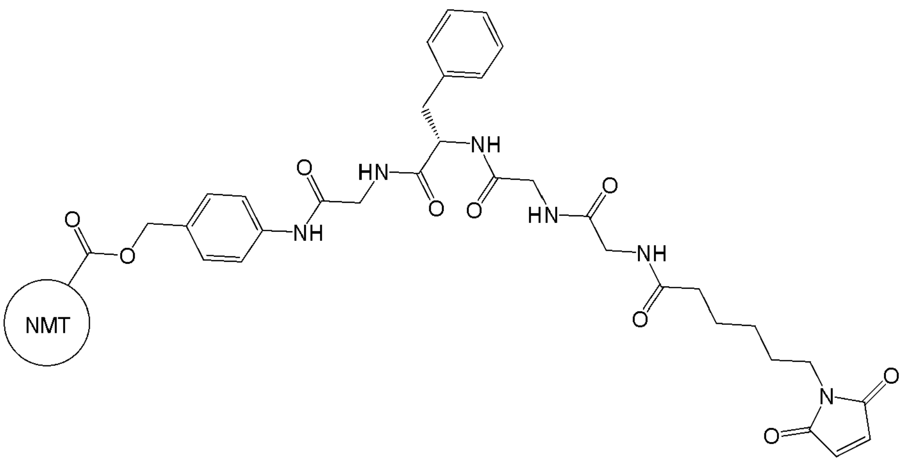

or a salt and/or solvate thereof, wherein

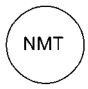

is a NMT inhibitor. Suitably, the NMT inhibitor is a compound of formula (VI), or a salt thereof. Suitably, the NMT inhibitor is a compound of formula (VIA), or a salt thereof.

In one embodiment, the drug conjugate is (1S,2R,3S,4R,5R)-5-(4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3yl]methyl}(methyl)carbamoyl)oxy] methyl}-2-[3-(3-{2-[2-(2,5-dioxo-2,5-dihydro-1H-pyrrol-1-yl)ethoxy]ethoxy}propanamido)propanamido]phenoxy)-3,4-dihydroxy-2-methylcyclohexane-1-carboxylic acid:

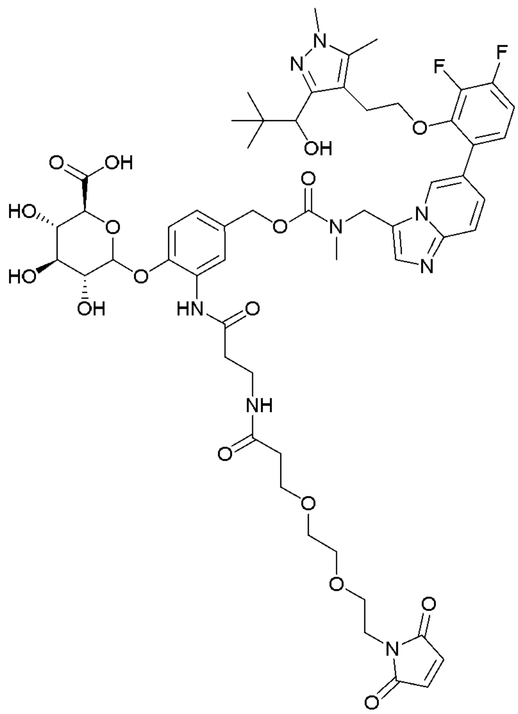

,

or a salt and/or solvate thereof.

In one embodiment, the drug conjugate is {4-[(2S)-5-(carbamoylamino)-2-[(2S)-2-[6-(2,5-dioxo-2,5-dihydro-1H-pyrrol-1-yl)hexanamido]-3-methylbutanamido]pentanamido]phenyl}methyl N-{[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl}-N-methylcarbamate:

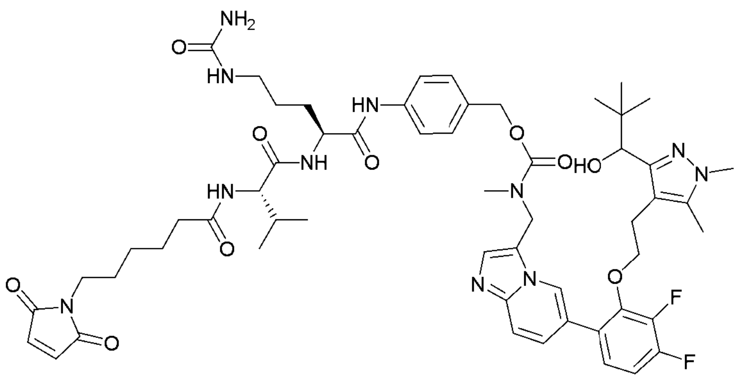

,

or a salt and/or solvate thereof.

In one embodiment, the drug conjugate is (1S,2R,3S,4R,5R)-5-(4-{[({[6-(3,4-difluoro-2-{2-[3-(2-hydroxypropan-2-yl)-1,5-dimethyl-1H-pyrazol-4-yl]ethoxy}phenyl)imidazo[1,2-a]pyridin-3-yl]methyl}(methyl)carbamoyl)oxy]methyl}-2-[3-(3-{2-[2-(2,5-dioxo-2,5-dihydro-1H-pyrrol-1-yl)ethoxy]ethoxy}propanamido)propanamido]phenoxy)-3,4-dihydroxy-2-methylcyclohexane-1-carboxylic acid:

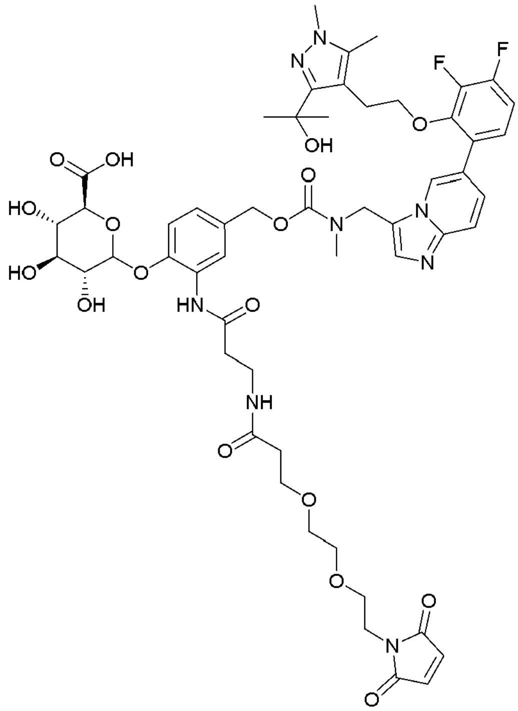

,

or a salt and/or solvate thereof.

In one embodiment, the drug conjugate is N-[({[(1-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl}(methyl)carbamoyl)methyl]carbamoyl}-2-phenylethyl)carbamoyl]methyl}carbamoyl)methyl]-6-(2,5-dioxo-2,5-dihydro-1H-pyrrol-1-yl)hexanamide:

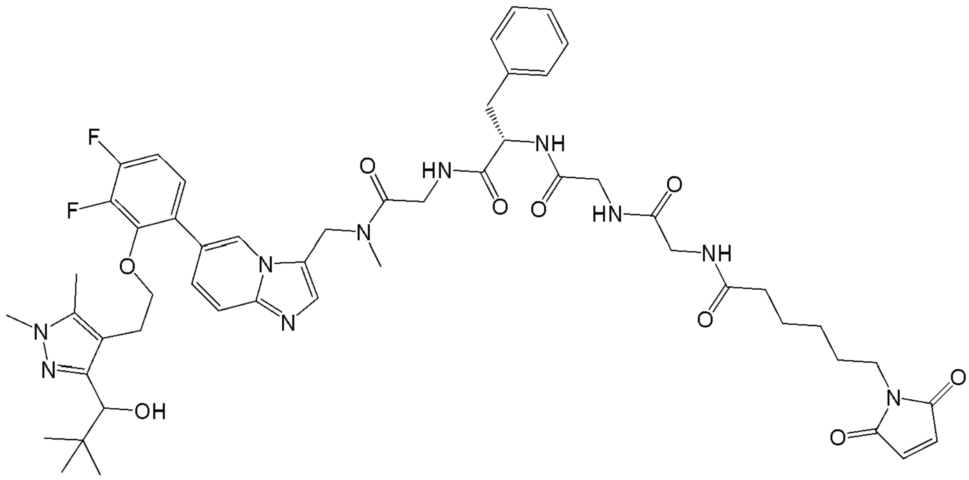

,

or a salt and/or solvate thereof.

The drug conjugate may be provided in the form of a salt. The drug conjugate is provided in the form of a solvate. The drug conjugate may be provided in the form of a solvate of a salt. Suitably, the drug conjugate is provided.

The invention also provides intermediate compounds which are useful in the preparation of drug conjugates.

In one embodiment there is provided a compound of formula (ADC-I):

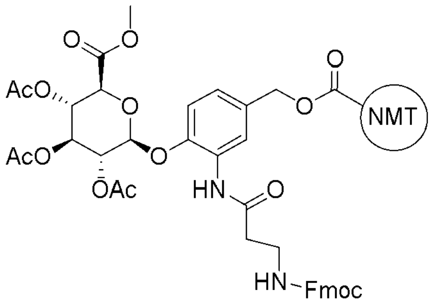

(ADC-I),

or a salt and/or solvate thereof, wherein NMT is a NMT inhibitor. Suitably, the NMT inhibitor is a compound of formula (VI), or a salt thereof. Suitably, the NMT inhibitor is a compound of formula (VIA), or a salt thereof. Suitably the NMT inhibitor is 1-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}-2,2-dimethylpropan-1-ol, or a salt thereof.

In one embodiment there is provided a compound of formula (ADC-II):

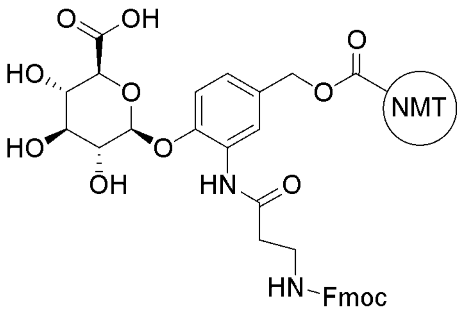

(ADC-II),

or a salt and/or solvate thereof, wherein NMT is a NMT inhibitor. Suitably, the NMT inhibitor is a compound of formula (VI), or a salt thereof. Suitably, the NMT inhibitor is a compound of formula (VIA), or a salt thereof. Suitably the NMT inhibitor is 1-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}-2,2-dimethylpropan-1-ol, or a salt thereof.

In one embodiment the invention provides a compound selected from the group consisting of:

{4-[(2S)-5-(carbamoylamino)-2-[(2S)-2-[6-(2,5-dioxo-2,5-dihydro-1H-pyrrol-1-yl)hexanamido]-3-methylbutanamido]pentanamido]phenyl}methyl 4-nitrophenyl carbonate;
(1S,2R,3S,4R,5R)-5-[2-(3-{[(9H-fluoren-9-yloxy) carbonyl]amino}propanamido)-4-({[(4-nitrophenoxy)carbonyl]oxy}methyl)phenoxy]-2,3,4-trihydroxycyclohexane-1-carboxylic acid; (1S,2R,3S,4R,5R)-5-(4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl}(methyl)carbamoyl)oxy]methyl}-2-(3-{[(9H-fluoren-9-yloxy) carbonyl]amino}propanamido)phenoxy)-2,3,4-trihydroxycyclohexane-1-carboxylic acid; and
(1S,2R,3S,4R,5R)-5-[2-(3-aminopropanamido)-4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl}(methyl)carbamoyl)oxy]methyl}phenoxy]-2,3,4-trihydroxycyclohexane-1-carboxylic acid,
or a salt and/or solvate of any one thereof.

It will be appreciated that for use in medicine the salts of the ADCs of the invention should be pharmaceutically acceptable. Non-pharmaceutically acceptable salts of the ADCs of the invention may be of use in other contexts such as during preparation of the ADCs of the invention. Suitable pharmaceutically acceptable salts will be apparent to those skilled in the art. Pharmaceutically acceptable salts include those described by Berge et al. (1977). Such pharmaceutically acceptable salts include acid and base addition salts. Pharmaceutically acceptable acid additional salts may be formed with inorganic acids e.g. hydrochloric, hydrobromic, sulphuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates, may be used, for example in the isolation of the ADCs of the invention and are included within the scope of this invention.

Certain of the ADCs of the invention may form acid addition salts with one or more equivalents of the acid. The present invention includes within its scope all possible stoichiometric and non-stoichiometric forms.

It is to be understood that the present invention encompasses all isomers of the ADCs of the invention, including all geometric, tautomeric and optical forms, and mixtures thereof (e.g. racemic mixtures). Where additional chiral centres are present in the ADCs of the invention, the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The present disclosure includes all isotopic forms of the ADC of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exist as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or >99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of an ADC of the invention may thus contain one or more artificial or uncommon isotopes such as deuterium ($^{2}H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{17}Cl$), fluorine-18 ($^{18}F$) iodine-123 ($^{123}I$), iodine-125 ($^{125}I$) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection. Unnatural variant isotopic forms which incorporate deuterium i.e. $^{2}H$ or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

In one embodiment, an ADC of the invention, or a salt thereof is provided in a natural isotopic form.

In one embodiment, an ADC of the invention, or a salt thereof is provided in an unnatural variant isotopic form. In a specific embodiment, the unnatural variant isotopic form is a form in which deuterium (i.e. $^{2}H$ or D) is incorporated where hydrogen is specified in the chemical structure in one or more atoms of an ADC of the invention, or a salt thereof. In one embodiment, the atoms of an ADC of the invention, or a salt thereof are in an isotopic form which is not radioactive. In one embodiment, one or more atoms of an ADC of the invention, or a salt thereof are in an isotopic form which is radioactive. Suitably radioactive isotopes are stable isotopes. Suitably the unnatural variant isotopic form is a pharmaceutically acceptable form.

In one embodiment, an ADC of the invention, or a salt thereof is provided whereby a single atom of the compound exists in an unnatural variant isotopic form. In another embodiment, an ADC of the invention, or a salt thereof is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the Examples. Since the ADCs of the invention are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

In general, the ADCs of the invention may be made according to the organic synthesis techniques known to those skilled in this field, as well as by the representative methods set forth below, those in the Examples and modifications thereof.

## Uses of the ADCs of the Invention

The terms "prophylaxis" or "prevention" as used herein to mean the provision in advance, and as such may

involve preventing symptoms of a disease or disorder in a subject or preventing recurrence of symptoms of a disease or disorder in an afflicted subject and is not limited to complete prevention of an affliction.

The terms "treatment" or "treating" as used herein includes the control, mitigation, reduction, or modulation of the disease state or its symptoms.

**Inhibition of human NMT**

Inhibition of human NMT has been suggested as a target for treating or preventing various diseases or disorders, as described above. The present invention provides ADCs which comprise human NMT inhibitors. The term "human NMT inhibitor" as used herein is intended to cover any moiety which binds to human NMT. Human NMT is suitably HsNMT1. The inhibitor may act as a competitive inhibitor, or a partial competitive inhibitor. The inhibitor may bind to human NMT at the myr-CoA binding pocket or at the peptide binding pocket (or inhibit human NMT through another mechanism). As the ADCs of the present invention or pharmaceutically acceptable salts thereof comprise NMT inhibitors which are human NMT inhibitors, it is expected that after intracellular release of the NMT inhibitor from the ADCs of the inventon, the NMT inhibitor suitably binds and inhibits human NMT through the peptide binding pocket.

Consequently, the ADCs of the present invention or pharmaceutically acceptable salts thereof are expected to be useful in the treatment or prevention of diseases or disorders associated with human NMT activity or are expected to be useful in the treatment or prevention of a disease or disorder by targeting human NMT activity, for example, hyperproliferative diseases such as cancer. Accordingly, the present invention provides an ADC of the invention, or a pharmaceutically acceptable salt thereof for use as a medicament.

There is also provided an ADC of the invention, or a pharmaceutically acceptable salt thereof for use in the treatment or prevention of a disease or disorder in which inhibition of human NMT provides a therapeutic or prophylactic effect. In one embodiment there is provided an ADC of the invention, or a pharmaceutically acceptable salt thereof for use in the treatment of a disease or disorder in which inhibition of human NMT provides a therapeutic effect. In one embodiment there is provided an ADC of the invention, or a pharmaceutically acceptable salt thereof for use in the prevention of a disease or disorder in which inhibition of human NMT provides a prophylactic effect.

The invention also provides a method for the treatment or prevention of a disease or disorder in a subject in which inhibition of human NMT provides a therapeutic or prophylactic effect in a subject (e.g. a mammal, for example a human), which comprises administering to the subject a therapeutically effective amount of an ADC of the invention, or a pharmaceutically acceptable salt thereof and a pharmaceutically acceptable carrier. The invention also provides a method for the treatment of a disease or disorder in a subject in which inhibition of human NMT provides a therapeutic effect in a subject (e.g. a mammal, for example a human), which comprises administering to the subject a therapeutically effective amount of an ADC of the invention, or a pharmaceutically acceptable salt thereof and a pharmaceutically acceptable carrier. The invention also provides a method for the prevention of a disease or disorder in a subject in which inhibition of human NMT provides a prophylactic effect in a subject (e.g. a mammal, for example a human), which comprises administering to the subject a therapeutically effective amount of an ADC of the invention, or a pharmaceutically acceptable salt thereof and a pharmaceutically acceptable carrier.

The invention also provides the use of an ADC of the invention, or a pharmaceutically acceptable salt thereof, for the manufacture of a medicament for the treatment or prevention of a disease or disorder in which inhibition of human NMT provides a therapeutic or prophylactic effect. The invention also provides the use of an ADC of the invention, or a pharmaceutically acceptable salt thereof, for the manufacture of a medicament for the treatment of a disease or disorder in which inhibition of human NMT provides a therapeutic effect. The invention also provides the use of an ADC of the invention, or a pharmaceutically acceptable salt thereof, for the manufacture of a medicament for the prevention of a disease or disorder in which inhibition of human NMT provides a prophylactic effect.

**Hyperproliferative disorders**

As the ADCs of the invention have cytotoxic activity, the ADCs of the invention or pharmaceutically acceptable salts thereof are expected to be useful in the treatment or prevention of a hyperproliferative disorder.

Therefore, the invention provides an ADC of the invention or a pharmaceutically acceptable salt thereof for use in the treatment or prevention of a hyperproliferative disorder. In one especially suitable embodiment, the ADCs of the invention, or pharmaceutically acceptable salts thereof are for use in the treatment of a hyperproliferative disorder.

In one embodiment, the invention provides the use of an ADC of the invention, or a pharmaceutically acceptable salt thereof for the manufacture of a medicament for the treatment or prevention of a hyperproliferative disorder. In one especially suitable embodiment, the invention provides the use of an ADC of the invention or a pharmaceutically acceptable salt thereof for the manufacture of a medicament for the treatment of a hyperproliferative disorder.

In one embodiment, the invention provides a method of treating or preventing a hyperproliferative disorder in a subject, said method comprising administering a therapeutically effective amount of an ADC of the invention, or a

pharmaceutically acceptable salt thereof. In one especially suitable embodiment, the invention provides a method of treating a hyperproliferative disorder in a subject, said method comprising administering a therapeutically effective amount of an ADC of the invention, or a pharmaceutically acceptable salt thereof.

In one embodiment, the hyperproliferative disorder is a cancer.

In one embodiment, the cancer is a haematologic malignancy selected from the group consisting of lymphoma (for example B-cell lymphoma, and in particular a lymphoma selected from the group consisting high grade mantle zone lymphoma, follicular lymphoma, plasmablastic lymphoma, diffuse large B-cell lymphoma and Burkitt's lymphoma), myeloma (for example multiple myeloma), leukaemia (for example a leukaemia selected from the group consisting chronic lymphocytic leukaemia, AML and B-acute lymphocytic leukaemia), and melanoma (for example a melanoma selected from the group consisint of superficial spreading melanoma, nodular melanoma, lentigo maligna melanoma, amelanotic melanoma, and acral lentiginous melanoma).

The cancer may additionally, or alternatively, be a solid tumour selected from the group consisting of brain, lung, breast (e.g. triple negative breast cancer or a breast invasive carcinoma), prostate, ovary, colorectal (e.g. colon), gallbladder, kidney and liver cancer. For example, the cancer may be ovarian serous cystadenocarcinoma, esophageal carcinoma, lung squamous cell carcinoma, lung adenocarcinoma, bladder urothelial carcinoma, uterine carcinosarcoma, stomach adenocarcinoma, breast invasive carcinoma or liver hepatocellular carcinoma. In one suitable embodiment, the cancer is breast cancer, for example triple negative breast cancer or a breast invasive carcinoma. In one suitable embodiment, the cancer is brain, breast, prostate, colon, gallbladder or kidney cancer. In certain embodiments, the cancer is breast, colon or gallbladder cancer.

The cancer may also additionally, or alternatively, be a blastoma, and in particular a neuroblastoma, for example a retinoblastoma, a glioblastoma, a small cell lung carcinoma or an astrocytoma.

In an especially suitable embodiment, the cancer may be selected from the group consisting of a haematologic malignancy (such as a lymphoma, and in particular a B-cell lymphoma (e.g. high grade mantle zone lymphoma, follicular lymphoma, plasmablastic lymphoma, diffuse large B-cell lymphoma and Burkitt's lymphoma), a myeloma (e.g multiple myeloma) or a leukaemia (e.g. chronic lymphocytic leukaemia, AML and B-acute lymphocytic leukaemia)), a solid-tumour (such as brain, lung, breast (e.g. triple negative breast cancer or a breast invasive carcinoma), prostate, ovary, colorectal (e.g. colon), gallbladder, kidney or liver cancer, or a neuroblastoma (for example a retinoblastoma, a glioblastoma, a small cell lung carcinoma or an astrocytoma)), and a melanoma (such as superficial spreading melanoma, nodular melanoma, lentigo maligna melanoma, amelanotic melanoma, or acral lentiginous melanoma).

In a suitable embodiment, the cancer may be selected from the group consisting of diffuse large B-cell lymphoma, Burkitt's lymphoma, multiple myeloma, neuroblastoma, AML, and B-acute lymphocytic leukaemia. In a suitable embodiment, the cancer may be selected from the group consisting of diffuse large B-cell lymphoma, Burkitt's lymphoma, neuroblastoma, AML, B-acute lymphocytic leukaemia and breast cancer. In a suitable embodiment, the cancer may be selected from the group consisting of diffuse large B-cell lymphoma, neuroblastoma, B-acute lymphocytic leukaemia and triple negative breast cancer. In a suitable embodiment, the cancer may be selected from the group consisting of diffuse large B-cell lymphoma, Burkitt's lymphoma, multiple myeloma, neuroblastoma, AML, B-acute lymphocytic leukaemia and triple negative breast cancer. In a suitable embodiment, the cancer may be selected from the group consisting of multiple myeloma, neuroblastoma, AML, B-acute lymphocytic leukaemia and triple negative breast cancer. In a suitable embodiment, the cancer may be selected from the group consisting of multiple myeloma, neuroblastoma and triple negative breast cancer.

In one suitable embodiment, the cancer expresses HER2 protein i.e. a cancer containing cells having HER2 protein on their cell surface. The HER2 protein is overexpressed in a variety of tumours and can be evaluated using a method generally carried out in the art, such as immunohistochemical staining (IHC) for evaluating overexpression of the HER2 protein, or fluorescence in situ hybridisation (FISH) for evaluating amplification of the HER2 gene.

Suitably, the cancer expressing HER2 protein is selected from the group consisting of lung cancer, urothelial cancer, colorectal cancer, prostate cancer, ovarian cancer, pancreatic cancer, breast cancer, bladder cancer, gastric cancer (e.g. stomach adenocarcinoma), gastrointestinal stromal tumor, uterine cervix cancer, esophageal cancer, squamous cell carcinoma, peritoneal cancer, liver cancer, hepatocellular cancer, colon cancer, rectal cancer, colorectal cancer, endometrial cancer, uterine cancer, salivary gland cancer, kidney cancer, vulval cancer, thyroid cancer, and penis cancer, such as breast cancer.

In one embodiment, the cancer is HER2 positive breast cancer.

In one suitable embodiment the cancer expresses CD20. Expression of CD20 by a cancer cell be evaluated using a method generally carried out in the art, such as immunohistochemical staining (IHC). Suitably, the cancer expressing CD20 is a lymphoma such as a high grade mantle zone lymphoma, follicular lymphoma, plasmablastic lymphoma, diffuse large B-cell lymphoma or Burkitt's lymphoma), a myeloma (such as multiple myeloma) or a leukaemia such as chronic lymphocytic leukaemia, acute myeloid leukemia (AML) or B-acute lymphocytic leukaemia.

In one suitable embodiment, the cancer is a *MYC* addicted cancer as described in WO2020/128475, the entire contents of which are incorporated by reference for the purpose of defining the *MYC* addicted cancer.

## Combination therapies

Whilst an ADC of the invention or a pharmaceutically acceptable salt thereof may be used as the sole active ingredient in a medicament, it is also possible for an ADC of the invention, or a pharmaceutically acceptable salt thereof to be used in combination with one or more further therapeutic agents. Accordingly, the present invention also provides an ADC of the invention, or a pharmaceutically acceptable salt thereof together with a further therapeutic agent. The further therapeutic ingredient may be for simultaneous, sequential or separate administration. The invention also provides a kit of parts comprising: (a) a first pharmaceutical composition comprising an ADC of the invention, or a pharmaceutically acceptable salt thereof and a pharmaceutically acceptable carrier; and (b) a second pharmaceutical composition comprising a further therapeutic agent, and a pharmaceutically acceptable carrier. Such further therapeutic agents may be an ADC of the invention, or a pharmaceutically acceptable salt thereof.

The ADCs of the invention, or pharmaceutically acceptable salts thereof can be used in combination with one or more further therapeutic agents useful for the treatment or prevention of hyperproliferative disorders such as cancer or another disease or disorder in which inhibition of human NMT provides a therapeutic or prophylactic effect. The individual components of such combinations can be administered separately at different times during the course of therapy or concurrently in divided or single combination forms. The present invention is therefore to be understood as embracing all such regimes of simultaneous or alternating treatment and the term "administering" is to be interpreted accordingly. It will be understood that the scope of combinations of the ADCs of the invention, or pharmaceutically acceptable salts thereof with other therapeutic agents useful for treating or prevention of a disease or disorder in which inhibition of human NMT provides a therapeutic or prophylactic effect includes in principle any combination with any pharmaceutical composition useful for treating or prevention of a disease or disorder in which inhibition of human NMT provides a therapeutic or prophylactic effect.

A further therapeutic agent, when employed in combination with an ADC of the invention, or a pharmaceutically acceptable salt thereof may be used, for example, in those amounts indicated in the Physicians' Desk Reference (PDR) for that agent, or as otherwise determined by one of ordinary skill in the art. Where an ADC of the invention, or a pharmaceutically acceptable salt thereof is utilized in combination with one or more further therapeutic agent(s), either concurrently or sequentially, the following combination ratios and dosage ranges are suitable: when combined with a further therapeutic agent, an ADC of the invention, or a pharmaceutically acceptable salt thereof may for example be employed in a weight ratio to the further therapeutic agent within the range from about 10:1 to about 1:10.

In one embodiment, where an ADC of the invention, or a pharmaceutically acceptable salt thereof is for the treatment or prevention of cancer, the ADC of the invention or a pharmaceutically acceptable salt thereof may be utilized in combination with one or more further therapeutic agent(s), either concurrently or sequentially, for the treatment or prevention of cancer. More suitably, where an ADC of the invention or a pharmaceutically acceptable salt thereof is for the treatment of cancer, the ADC of the invention or a pharmaceutically acceptable salt thereof may be utilized in combination with one or more further therapeutic agent(s), either concurrently or sequentially, for the treatment of cancer.

Suitable, but non-limiting, examples of other therapeutic agents which may be administered in combination with the NMT inhibitor include one or more other chemotherapeutic agents.

Suitably, the chemotherapeutic agent is selected from the group consisting of fluorouracil (5-FU), pertuzumab, paclitaxel, carboplatin, cisplatin, gemcitabine, capecitabine, irinotecan (CPT-11), paclitaxel, docetaxel, pemetrexed, sorafenib, vinblastin, vinorelbine, everolims, tanespimycin, bevacizumab, oxaliplatin, lapatinib, ado-trastuzumab emtansine (TDM1), or drugs described in International Publication No. WO 2003/038043, LH-RH analogues (such as leuprorelin or goserelin), estramustine phosphate, estrogen antagonists (such as tamoxifen or raloxifene), and aromatase inhibitors (such as anastrozole, letrozole or exemestane).

Such conjoint treatment may be achieved by way of the simultaneous, sequential or separate dosing of the ADC of the invention, or a pharmaceutically acceptable salt thereof, and the one or more other therapeutic agents of the treatment. Such combination products may employ the ADCs of the invention, or pharmaceutically acceptable salts thereof, within any suitable dosage range, such as, for example, the dosage range described herein, and the other pharmaceutically-active agent may be within its approved dosage range.

## Doses and Formulations

It will be appreciated by one of skill in the art that appropriate dosages of the ADC of the invention or a pharmaceutically acceptable salt thereof, and compositions comprising the ADC of the invention or a pharmaceutically acceptable salt thereof, can vary from patient to patient. Determining the optimal dosage will generally involve the balancing of the level of therapeutic benefit against any risk or deleterious side effects. The selected dosage level will depend on a variety of factors including, but not limited to, the activity of the particular ADC, the route of administration, the time of administration, the rate of excretion of the ADC, the duration of the treatment, other drugs, ADCs, and/or materials used in combination, the severity of the condition, and the species, sex, age, weight, condition, general health, and prior

medical history of the patient. The amount of ADC and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the ADC is in the range of about 100 ng to about 25 mg (more typically about 1 μg to about 10 mg) per kilogram body weight of the subject per day. Where the ADC is a salt, an ester, an amide, a prodrug, or the like, the amount administered is calculated on the basis of the parent ADC and so the actual weight to be used is increased proportionately.

In one embodiment, the ADC of the invention or a pharmaceutically acceptable salt thereof is administered to a human patient according to the following dosage regime: about 100 mg, 3 times daily. In one embodiment, the ADC of the invention or a pharmaceutically acceptable salt thereof is administered to a human patient according to the following dosage regime: about 150 mg, 2 times daily. In one embodiment, the ADC of the invention or a pharmaceutically acceptable salt thereof is administered to a human patient according to the following dosage regime: about 200 mg, 2 times daily. In one embodiment, the ADC of the invention or a pharmaceutically acceptable salt thereof is administered to a human patient according to the following dosage regime: about 50 or about 75 mg, 3 or 4 times daily. In one embodiment, the the ADC of the invention or a pharmaceutically acceptable salt thereof is administered to a human patient according to the following dosage regime: about 100 or about 125 mg, 2 times daily.

The dosage amounts described above may apply to the ADC of the invention or a pharmaceutically acceptable salt thereof (including the NMT inhibitor and the linker to the antibody) or to the effective amount of NMT inhibitor provided, for example the amount of NMT inhibitor that is releasable after cleavage of the linker.

For the prevention or treatment of disease, the appropriate dosage of an ADC of the invention will depend on the type of disease to be treated, as defined above, the severity and course of the disease, whether the molecule is administered for preventive or therapeutic purposes, previous therapy, the patient's clinical history and response to the antibody, and the discretion of the attending physician. The ADC of the invention or a pharmaceutically acceptable salt thereof is suitably administered to the patient at one time or over a series of treatments. Depending on the type and severity of the disease, about 1 μg/kg to 15 mg/kg (e.g. 0.1-20 mg/kg) of an ADC of the invention or a pharmaceutically acceptable salt thereof is an initial candidate dosage for administration to the patient, whether, for example, by one or more separate administrations, or by continuous infusion. A typical daily dosage might range from about 1 μg/kg to 100 mg/kg or more, depending on the factors mentioned above. An exemplary dosage of an ADC of the invention or a pharmaceutically acceptable salt thereof to be administered to a patient is in the range of about 0.1 to about 10 mg/kg of patient weight. For repeated administrations over several days or longer, depending on the condition, the treatment is sustained until a desired suppression of disease symptoms occurs. An exemplary dosing regimen comprises a course of administering an initial loading dose of about 4 mg/kg, followed by additional doses every week, two weeks, or three weeks of an ADC of the invention or a pharmaceutically acceptable salt thereof. Other dosage regimens may be useful. The progress of this therapy is easily monitored by conventional techniques and assays.

While it is possible for the active ingredient to be administered alone, it is preferable for it to be present in a pharmaceutical formulation or composition. Accordingly, the invention provides a pharmaceutical formulation or composition comprising an ADC of the invention, or a pharmaceutically acceptable salt thereof, and a pharmaceutically acceptable diluent, excipient or carrier (collectively referred to herein as "carrier" materials). Pharmaceutical compositions of the invention may take the form of a pharmaceutical formulation.

Therefore, in one embodiment the invention provides a pharmaceutical composition comprising an ADC of the invention, or a pharmaceutically acceptable salt thereof and a pharmaceutically acceptable carrier.

In one embodiment, there is provided a pharmaceutical composition comprising an ADC of the invention, or a pharmaceutically acceptable salt (e.g. pharmaceutically acceptable salt) thereof, for use in the treatment or prophylaxis of a disease or disorder as described herein. In one embodiment, there is provided a pharmaceutical composition comprising an ADC of the invention, or a pharmaceutically acceptable salt (e.g. pharmaceutically acceptable salt) thereof, for use in the treatment of a disease or disorder as described herein. In one embodiment, there is provided a pharmaceutical composition comprising an ADC of the invention, or a pharmaceutically acceptable salt (e.g. pharmaceutically acceptable salt) thereof, for use in the prophylaxis of a disease or disorder as described herein.

In a further embodiment, there is provided a method for the treatment or prophylaxis of a disease or disorder as described herein, which comprises administering to a subject in need thereof an effective amount of a pharmaceutical composition comprising an ADC of the invention, or a pharmaceutically acceptable salt (e.g. pharmaceutically acceptable salt) thereof. In a further embodiment, there is provided a method for the treatment of a disease or disorder as described herein, which comprises administering to a subject in need thereof an effective amount of a pharmaceutical composition

comprising an ADC of the invention, or a pharmaceutically acceptable salt (e.g. pharmaceutically acceptable salt) thereof. In a further embodiment, there is provided a method for the prophylaxis of a disease or disorder as described herein, which comprises administering to a subject in need thereof an effective amount of a pharmaceutical composition comprising an ADC of the invention, or a pharmaceutically acceptable salt (e.g. pharmaceutically acceptable salt) thereof. Pharmaceutical compositions of the invention may take the form of a pharmaceutical formulation as described below.

The invention also provides the use of a pharmaceutical composition comprising an ADC of the invention, or a pharmaceutically acceptable salt thereof (e.g. pharmaceutically acceptable salt) thereof, in the manufacture of a medicament for the treatment or prophylaxis of a disease or disorder as described herein. The invention also provides the use of a pharmaceutical composition comprising an ADC of the invention, or a pharmaceutically acceptable salt thereof (e.g. pharmaceutically acceptable salt) thereof, in the manufacture of a medicament for the treatment of a disease or disorder as described herein. The invention also provides the use of a pharmaceutical composition comprising an ADC of the invention, or a pharmaceutically acceptable salt thereof (e.g. pharmaceutically acceptable salt) thereof, in the manufacture of a medicament for the prophylaxis of a disease or disorder as described herein.

Suitably the disease or disorder is a hyperproliferative disorder, such as cancer. In one suitable embodiment the cancer is a cancer expressing HER2 protein.

The ADC of the invention, or a pharmaceutically acceptable salt thereof may be adminstereed by intravenous, cutaneous, intramuscular or subcutaneous injection or infusion. The active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's Injection, Lactated Ringer's Injection, preservatives, stabilisers, buffers, antioxidants and/or other additives may be included, as required.

In one embodiment, the pharmaceutical composition further comprises other active agents, for example, other therapeutic or prophylactic agents.

Suitable carriers, diluents, excipients, etc. can be found in standard pharmaceutical texts. See, for example, Handbook of Pharmaceutical Additives, 2nd Edition (eds. M. Ash and I. Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients. 2nd edition, 1994.

Another aspect of the present invention pertains to methods of making a pharmaceutical composition comprising admixing at least one [11C]-radiolabelled ADC of the invention or a pharmaceutically acceptable salt thereof, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the active compound.

The term "pharmaceutically acceptable" as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

The formulations may be prepared by any methods well known in the art of pharmacy. Such methods include the step of bringing into association the ADC of the invention or a pharmaceutically acceptable salt thereof with a carrier which constitutes one or more accessory ingredients. In general, the formulations are prepared by uniformly and intimately bringing into association the ADC of the invention or a pharmaceutically acceptable salt thereof with carriers (e.g., liquid carriers, finely divided solid carrier, etc.), and then shaping the product, if necessary.

Formulations suitable for parenteral administration (e.g., by injection or infusion), include aqueous or non-aqueous, isotonic, pyrogen-free, sterile liquids (e.g., solutions, suspensions), in which the active ingredient is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include sodium chloride injection, Ringer's Solution, or Lactated Ringer's Injection. Typically, the concentration of the ADC of the invention or a pharmaceutically acceptable salt thereof in the liquid is from about 1 ng/ml to about 10 μg/ml, for example from about 10 ng/ml to about 1 μg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

The formulation may be prepared to provide for rapid or slow release; immediate, delayed, timed, or sustained release; or a combination thereof.

In some embodiments the ADCs of the invention or pharmaceutically acceptable salts thereof may be

administered orally. Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

In one suitable embodiment the administration is made by infusion or injection, particularly injection bolus injection.

Further embodiments of the invention are as follows:

1. An antibody drug conjugate (ADC) comprising a NMT inhibitor conjugated to an antibody *via* a linker, or a salt thereof.

2. The salt of an ADC according to embodiment 1.

3. The salt according to embodiment 1 or 2, which is a pharmaceutically acceptable salt.

4. The ADC according to embodiment 1.

5. The ADC or salt thereof according to any one of embodiments 1 to 4, wherein the NMT inhibitor is a compound of formula (I):

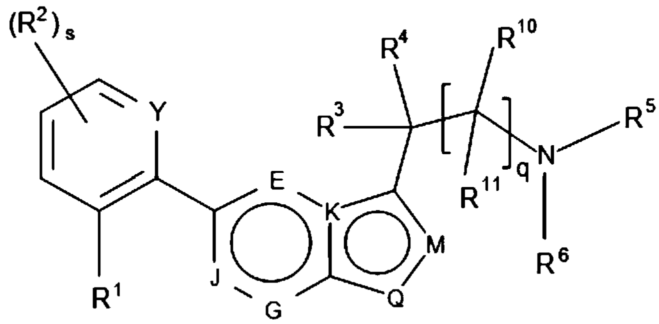

(I)

wherein:

Y is selected from the group consisting of -CH-, -C($R^2$)- and -N-;
$R^1$ is a group of formula -X-L-A;
X represents -O-;
L represents -$(CH_2)_m$-;
m is 1, 2 or 3;
A is a 6-10-membered aromatic carbocycle or a 5-10-membered aromatic heterocycle, said aromatic carbocycle or heterocycle being optionally substituted with 1, 2, or 3 substituents each independently selected from the group consisting of -F, -Cl, -Br, -$OCH_3$, -$OCF_3$, -CN, -$C_{1-6}$alkyl optionally substituted by up to 3 halogen, hydroxyl, or -$OC_{1-4}$alkyl groups, -S(O)$C_{1-4}$alkyl, -S$(O)_2C_{1-4}$alkyl, -C(O)N$(R^9)_2$, -C(O)N($R^{13}$)$C_{1-4}$alkylO$C_{1-4}$alkyl , -C(O)N($C_{1-4}$alkylO$C_{1-4}$alkyl$)_2$, -CH-$_2$C(O)N$(R^9)_2$, -$CH_2$C(O)N($R^{13}$)$C_{1-4}$alkylO$C_{1-4}$alkyl , -$CH_2$C(O)N($C_{1-4}$alkylO-$C_{1-4}$alkyl$)_2$, -S$(O)_2$NH$C_{1-4}$alkyl, -S$(O)_2$N($C_{1-4}$alkyl$)_2$, -NH$C_{1-4}$alkyl, -N($C_{1-4}$alkyl$)_2$, -NHC(O)$C_{1-4}$alkyl,-NHC(O)$CF_3$, - NHS$(O)_2C_{1-4}$alkyl, $CH_2$N$(R^{13})_2$, $CH_2$N($R^{13}$)C(O)$C_{1-4}$alkyl, $CH_2$N($R^{13}$)S$(O)_2C_{1-4}$alkyl, -$CH_2$S$(O)_2C_{1-4}$alkyl, and $CO_2$H;
s is 0, 1, 2, or 3;
each $R^2$ is independently selected from the group consisting of -F, -Cl, -Br, -$OCH_3$, -$OCF_3$, -CN, - $C_{1-4}$alkyl optionally substituted by up to 3 halogen or hydroxyl groups, -S(O)$C_{1-4}$alkyl, -S$(O)_2C_{1-4}$alkyl, -S$(O)_2$NH$C_{1-4}$alkyl, -S$(O)_2$N($C_{1-4}$alkyl$)_2$, -NH$C_{1-4}$alkyl, -N($C_{1-4}$alkyl$)_2$, -NHC(O)$C_{1-4}$alkyl, - NHC(O)$CF_3$, and -NHS$(O)_2C_{1-4}$alkyl;
q is 0 or 1;
$R^3$ is hydrogen or methyl; $R^4$ is hydrogen or methyl;
$R^5$ is hydrogen; $R^6$ is hydrogen or $C_{1-6}$alkyl optionally substituted by up to 3 -F, -Cl, -Br, -OH, - $OCH_3$, -$OCF_3$ or -CN groups;
when present $R^{10}$ is hydrogen or methyl;
when present $R^{11}$ is hydrogen or methyl;

or the $R^3$ group and the $R^5$ group and the intervening atoms form a 3 to 7 membered non-aromatic heterocycle composed of the intervening atoms and bond, or the intervening atoms and $-(CHR^a)_r-$; or the $R^{10}$ group and the $R^5$ group and the intervening atoms form a 3 to 7 membered non-aromatic heterocycle composed of the intervening atoms and $-(CHR^a)_r-$;
r is 1, 2, 3, 4 or 5; $R^a$ is hydrogen or methyl;
each $R^7$ is independently selected from the group consisting of hydrogen, halogen, $C_{1-4}$alkoxy, and $C_{1-4}$alkyl optionally substituted with 1, 2 or 3 halogens; and
$R^8$ is selected from the group selected from hydrogen and $C_{1-4}$alkyl;
each $R^9$ is independently selected from the group consisting of hydrogen and $C_{1-4}$alkyl, or two $R^9$ groups and the N they are bonded to form a 4 to 7 membered non-aromatic heterocycle, the heterocycle optionally comprising 1 or 2 further heteroatoms selected from N, O and S;
each $R^{13}$ is independently selected from the group consisting of hydrogen and $C_{1-4}$alkyl; and wherein:
i) E, J and G are each $C(R^7)$, K is carbon, Q is $N(R^8)$, and M is nitrogen;
ii) E, J and G are each $C(R^7)$, and K, Q and M are each nitrogen; or
iii) E, J, G and M are each $C(R^7)$, and K and Q are each nitrogen,
or a salt thereof.

6. The ADC or salt thereof according to embodiment 5 wherein the NMT inhibitor is a compound of formula (IA^^):

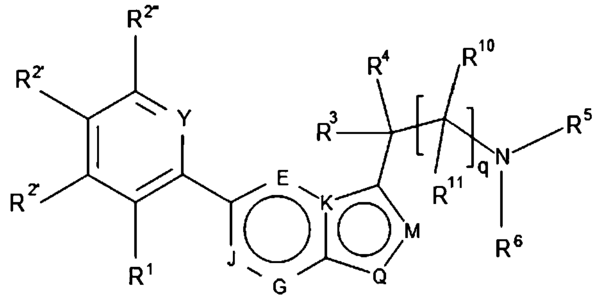

(IA^^)

wherein:

$R^1$ is a group of formula -X-L-A;
A is 4-pyrazolyl, said pyrazolyl being optionally substituted with up to 3 substituent groups selected from methyl and $-C(O)N(CH_3)_2$;
X is -O-;
L is $-(CH_2)_m-$;
m is 2;
$R^{2'}$ is selected from the group consisting of fluorine or chlorine (preferably fluorine);
$R^{2''}$ is selected from the group consisting of hydrogen, fluorine or chlorine;
q is 0;
$R^3$ is hydrogen or methyl;
$R^4$ is hydrogen or methyl;
$R^5$ is hydrogen;
$R^6$ is hydrogen or methyl; or
the $R^3$ group and the $R^6$ group and the intervening atoms form a 3 to 7 membered non-aromatic heterocycle composed of the intervening atoms and bonds;
E, J, G, K, Q and M are:

i) E, J and G are each CH, K is carbon, Q is $N(R^8)$, M is nitrogen; and $R^8$ is hydrogen or methyl; or
ii) E, J, G and M are each CH, and K and Q are each nitrogen;

with the proviso that A is substituted with no more than one $-C(O)N(CH_3)_2$ group,
or a salt thereof.

7. The ADC or salt thereof according to any one of embodiments 1 to 6, wherein the NMT inhibitor is a compound of formula (II):

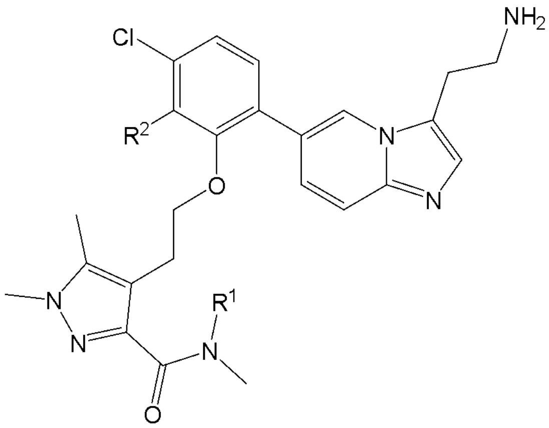

(II),

wherein:

$R^1$ is H or $-CH_3$; and
$R^2$ is H or F,
or a salt thereof.

8. The ADC or salt thereof according to embodiment 7, wherein the NMT inhibitor is 4-(2-{2-[3-(2-aminoethyl)imidazo [1,2-a]pyridyl-6-yl]-5-chlorophenoxy}ethyl)-N,N,1,5-tetramethyl-1H-pyrazole-3-carboxamide:

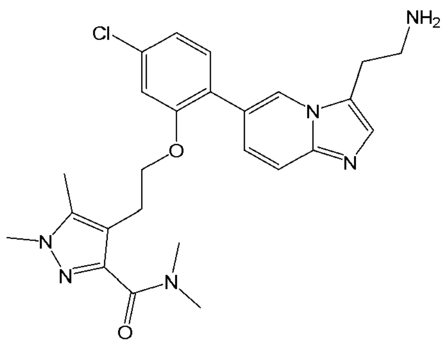

,

or a salt thereof.

9. The ADC or salt thereof according to any one of embodiments 1 to 4, wherein the NMT inhibitor is a compound of formula (III) or (IV):

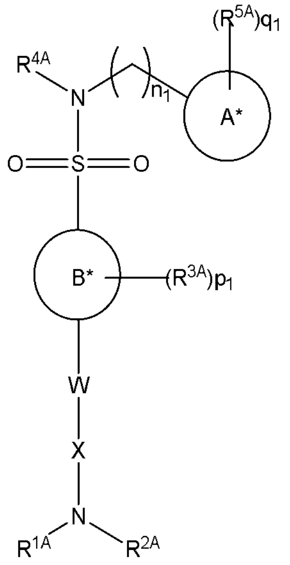

(III); or

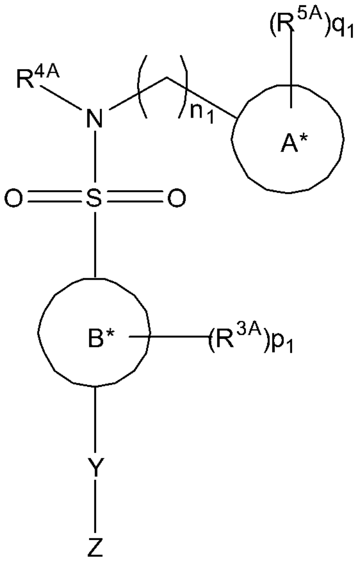

(IV)

wherein:

$n_1$ is 0, 1, 2, 3, 4, 5 or 6;
Ring A*, is an optionally substituted nitrogen containing aryl group wherein each substituable carbon or nitrogen in Ring A* is optionally and independently substituted by one or more $R^{5A}$ and wherein if Ring A* contains an -NH- moiety that nitrogen may be optionally substituted by $C_{1-6}$alkyl (e.g. methyl); and wherein $R^{4A}$ and Ring A* together with the atoms to which they are attached may form a cyclic group,
Ring B* is an optionally substituted aryl or heteroaryl group wherein each substitutable carbon or heteroatom in Ring B* is optionally and independently substituted by one or more $R^{3A}$;
W and X, one of which may be absent, are independently selected from $R^{11A}$, hydrocarbyl (e.g. $C_{1-8}$ alkyl, alkenyl, alkynyl, or haloalkyl) optionally substituted with $R^{11A}$, and $-(CH_2)_{k1}$-heterocyclyl optionally substituted with $R^{12A}$;
$k_1$ is 0, 1, 2, 3, 4, 5 or 6;
$R^{1A}$ is hydrogen;
$R^{2a}$, $R^{3A}$, $R^{4A}$ and $R^{5A}$ are independently selected from hydrogen, $R^{12A}$, hydrocarbyl (e.g. $C_{1-6}$ alkyl, alkenyl, alkynyl, or haloalkyl) optionally substituted with $R^{12A}$, and a $-(CH_2)_{L1}$- heterocyclyl optionally substituted with one or more $R^{12A}$; wherein $R^{2A}$ taken together with W or X may form a heterocycle optionally substituted with one or more $R^{12A}$; and wherein one or more of $R^{3A}$ and $R^{5A}$ taken together with the atoms to which they are attached may form a carbocycle, for example heterocyclyl, optionally substituted with $R^{12A}$; $L_1$ is 0,1, 2, 3, 4, 5 or 6;
wherein:

each $R^{11A}$ and $R^{12A}$ is independently selected from halogen, trifluoromethyl, cyano, thio, nitro, oxo, $=NR^{13A}$, $-OR^{13A}$, $-SR^{13A}$, $-C(O)R^{13A}$, $-C(O)OR^{13A}$, $-OC(O)R^{13A}$, $-NR^{13A}COR^{14A}$,$-NR^{13A}CON(R^{13A})_2$, $-NR^{13a}$-$COR^{14a}$, $-NR^{13a}CO_2R^{14A}$, $-S(O)R^{13A}$, $-S(O)_2R^{13A}$, $-SON(R^{13A})_2$, - $NR^{13A}S(O)_2R^{14A}$; $-CSR^{13A}$, $-N(R^{13A)}$ $R^{14A}$, $-C(O)N(R^{13A})R^{14A}$, $-SO_2N(R^{13A})R^{14A}$ and $R^{15A}$;
$R^{13A}$ and $R^{14A}$ are each independently selected from hydrogen or $R^{15A}$;
$R^{15A}$ is selected from hydrocarbyl (e.g. $C_{1-6}$alkyl, alkenyl, alkynyl, or haloalkyl), carbocyclyl and - $(CH_2)_{m1}$-heterocyclyl, and each $R^{15A}$ is optionally and independently substituted with one or more of halogen, cyano, amino, hydroxy, $C_{1-6}$alkyl or cycloalkyl and $C_{1-6}$alkoxy;
$m_1$ is 0, 1, 2, 3, 4, 5 or 6;
$p_1$ is 0,1, 2, 3 or 4; the values of $R^{4A}$ may be the same or different; and
$q_1$ is 0, 1, 2, 3 or 4; wherein the values of $R^{5A}$ may be the same or different;
Y and Z, one or both of which may be absent, are independently selected from hydrogen, $R^{16A}$, hydrocarbyl (e.g.$C_{1-6}$alkyl, alkenyl, alkynyl.or haloalkyl) optionally substituted with $R^{16A}$, and - $(CH_2)_{r1}$-heterocyclyl optionally substituted with $R^{16A}$, wherein each $R^{16A}$ is independently selected from halogen, trifluoromethyl, cyano, thio, nitro, oxo, $=NR^{17A}$, $-OR^{17A}$, $-SR^{17A}$, $-C(O)R^{17A}$, - $C(O)OR^{17A}$, $-OC(O)R^{17A}$, - $NR^{17A}COR^{18A}$, $-NR^{17A}CON(R^{18A})_2$, $-NR^{17A}COR^{18A}$, $-NR^{17A}CO_2R^{18A}$, - $S(O)R^{17A}$, $-S(O)_2R^{17A}$, - $SON(R^{17A})_2$, $-NR^{17A}$-$S(O)_2R^{18A}$; $-CSR^{17A}$, $-N(R^{17A})R^{18A}$, $-C(O)N(R^{17A})R^{18A}$,$-SO_2N(R^{17A})R^{18A}$ and $R^{19A}$; $r_1$ is 0, 1,2, 3, 4, 5 or 6;

wherein:
$R^{17A}$ and $R^{18A}$ are each independently selected from hydrogen or $R^{19A}$;
$R^{19A}$ is selected from hydrocarbyl (e.g.$C_{1-6}$alkyl, alkenyl, alkynyl, or haloalkyl), carbocyclyl and - $(CH2)_{s1}$-heterocyclyl, and each $R^{19A}$ is optionally and independently substituted with one or more of halogen, cyano, amino, hydroxy, $C_{1-6}$alkyl and $C_{1-6}$alkoxy; and
$s_1$ is 0, 1, 2, 3, 4, 5 or 6,
or a salt thereof.

10. The ADC or salt thereof according to embodiment 9, wherein the NMT inhibitor is a compound of formula (IIIa):

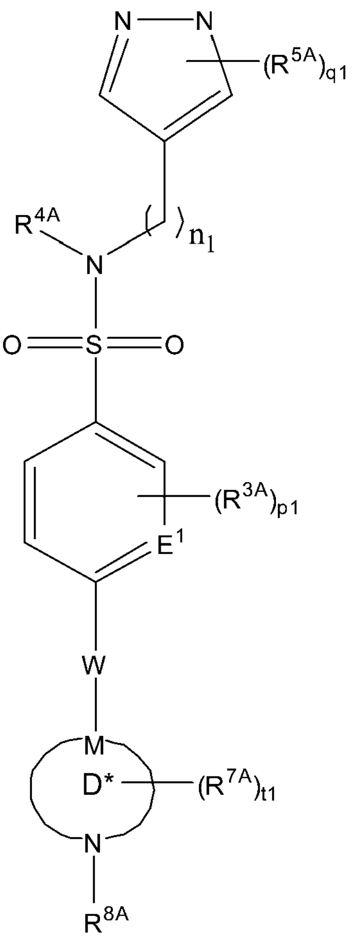

(IIIa)

wherein:

$n_1$ is 0 or 1;
$E^1$ is C;
W is a (1-4C)hydrocarbyl, an aryl (e.g. phenyl) or heteroaryl group (e.g. pyridinyl);
M is selected from C and N;
$R^{3A}$, $R^{4A}$ and $R^{5A}$ are independently selected from hydrogen, $R^{12A}$, and (1-3C)hydrocarbyl optionally substituted with $R^{12A}$;
$R^{12A}$ is independently selected from halogen, trifluoromethyl, cyano, thio, nitro, oxo, $-OR^{13A}$, $-SR^{13A}$, $-C(O)R^{13A}$, $-C(O)OR^{13A}$, $-OC(O)R^{13A}$, $-NR^{13A}COR^{14A}$ and $R^{15A}$;
$R^{13A}$ and $R^{14A}$ are each independently selected from hydrogen or a (1-4C)hydrocarbyl (e.g. methyl);
Ring D* is an optionally substituted nitrogen containing 6 or 7 membered heterocycle, wherein each substitutable carbon or nitrogen in Ring D* is optionally and independently substituted by one or more $R^{7A}$;
$R^{7A}$ is independently selected from hydrogen, (1-4C)hydrocarbyl, halogen, trifluoromethyl, cyano, thio, nitro or oxo;
$R^{8A}$ is hydrogen;
$p_1$ is 0, 1 or 2, wherein the values of $R^{4A}$ may be the same or different;

$q_1$ is 3, wherein the values of $R^{5A}$ may be the same or different; and
$t_1$ is 0, 1 or 2, wherein the values of $R^{7A}$ may be the same or different,
or a salt thereof.

11. The ADC or salt thereof according to embodiment 10, wherein the NMT inhibitor is (2,6-dichloro-4-(2-piperazin-1-yl-pyridin-4-yl)-N-(1,3,5-trimethyl-1H-pyraxol-4-yl)-benzenesulfonamide):

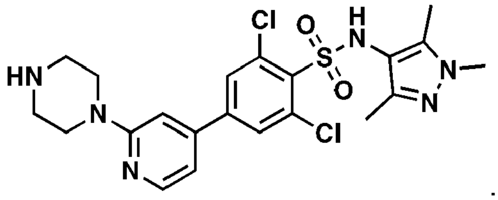

or a salt thereof.

12. The ADC or salt thereof according to embodiment 10, wherein the NMT inhibitor is 2,6-dichloro-N-(5-isobutyl-1,3-dimethyl-1H-pyrazol-4-yl(-4-(2-piperazin-1-yl-pyridin-4-yl)-benzenesulfonamide:

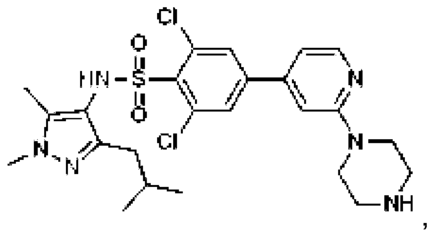

or a salt thereof.

13. The ADC or salt thereof according to any one of embodiments 1 to 4, wherein the NMT inhibitor is a compound of formula (V):

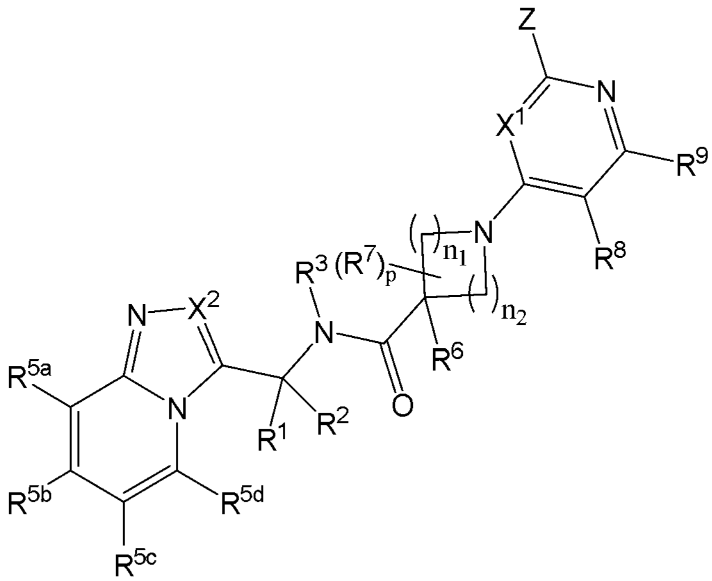

Formula (V)

wherein:

$n_1$ is 1 or 2; $n_2$ is 1 or 2;

$X^1$ is selected from the group consisting of $CR^x$ and N;

when present, $R^x$ is selected from the group consisting of hydrogen, halogen, and -$C_{1-4}$alkyl optionally substituted by 1, 2 or 3 substituents, each substituent being independently selected from the group consisting of halogen, -OH, -$OCH_3$, and -$OCF_3$;

$R^1$ is selected from the group consisting of hydrogen; -$C_{1-4}$alkyl optionally substituted by 1, 2 or 3 substituents, each substituent being independently selected from the group consisting of halogen, -$OCH_3$, and -$OCF_3$; and -$C_{3-6}$cycloalkyl optionally substituted by 1, 2 or 3 substituents, each substituent being independently selected from the group consisting of halogen, -$CH_3$, -$OCH_3$, and -$OCF_3$;

$R^2$ is selected from the group consisting of hydrogen; -$C_{1-4}$alkyl optionally substituted by 1, 2 or 3 substituents, each substituent being independently selected from the group consisting of halogen, -$OCH_3$, and -$OCF_3$; and -$C_{3-6}$cycloalkyl optionally substituted by 1, 2 or 3 substituents, each substituent being independently selected from the group consisting of halogen, -$CH_3$, -$OCH_3$, and -$OCF_3$;

or $R^1$ and $R^2$ are linked such that together with the atom to which they are attached they form a $C_3$-$_6$cycloalkyl group or a 3- to 6-membered non-aromatic heterocyclyl group comprising 1 heteroatom selected from the group consisting of O and N, wherein said $C_3$-$_6$cycloalkyl group or 3- to 6- membered non-aromatic heterocyclyl group is optionally substituted by 1 or 2 substituents, each substituent being independently selected from the group consisting of halogen, -OH, -$CH_3$, -$OCH_3$, and -$OCF_3$;

$R^3$ is selected from the group consisting of hydrogen; -$C_{1-4}$alkyl optionally substituted by 1, 2 or 3 substituents, each substituent being independently selected from the group consisting of halogen, -OH, -$OCH_3$, and -$OCF_3$; and -$C_{3-6-}$cycloalkyl optionally substituted by 1, 2 or 3 substituents, each substituent being independently selected from the group consisting of halogen, -OH, -$CH_3$, -$OCH_3$, and -$OCF_3$;

or $R^1$ and $R^3$ are linked such that together with the atoms to which they are attached they form a 3- to 6-membered non-aromatic heterocyclyl group comprising 1 N heteroatom, wherein said 3-to 6-membered non-aromatic heterocyclyl group is optionally substituted by 1 or 2 substituents, each substituent being independently selected from the group consisting of halogen, -$CH_3$, -OH, -$OCH_3$, and -$OCF_3$;

$X^2$ is selected from the group consisting of $CR^4$ and N;

when present, $R^4$ is selected from the group consisting of hydrogen; halogen; -$C_{1-4}$alkyl optionally substituted by 1, 2 or 3 substituents, each substituent being independently selected from the group consisting of halogen, -OH, -$OCH_3$, -$OCF_3$, and -$NR^aR^b$;

$R^{5a}$ and $R^{5d}$ are independently selected from the group consisting of hydrogen; halogen; methyl optionally substituted by 1, 2 or 3 substituents, each substituent being independently selected from the group consisting of halogen, -OH, -$OCH_3$, and -$OCF_3$; and methoxy optionally substituted by 1, 2 or 3 substituents, each substituent being independently selected from the group consisting of halogen, -OH, -$OCH_3$, and -$OCF_3$;

$R^{5b}$ and $R^{5c}$ are independently selected from the group consisting of hydrogen; halogen; -$C_{1-4}$alkyl optionally substituted by 1, 2 or 3 substituents, each substituent being independently selected from the group consisting of halogen, -OH, -$OCH_3$, and -$OCF_3$; -O-$C_{1-4}$alkyl optionally substituted by 1, 2 or 3 substituents, each substituent being independently selected from the group consisting of halogen, -OH, -$OCH_3$, and -$OCF_3$; and $C_{3-6}$cycloalkyl optionally substituted by 1, 2 or 3 substituents, each substituent being independently selected from the group consisting of halogen, -$CH_3$, -OH, -$OCH_3$, and -$OCF_3$;

or $R^{5b}$ and $R^{5c}$ are linked such that together with the atoms to which they are attached they form a 6-membered aryl group or a 5- or 6-membered aromatic heterocyclyl group comprising 1 or 2 heteroatoms selected from the group consisting of S, O and N, wherein said 6-membered aryl group or 5- or 6-membered aromatic heterocyclyl group is optionally substituted by 1 or 2 substituents, each substituent being independently selected from the group consisting of halogen, -OH, -$OCH_3$, and -$OCF_3$;

$R^6$ is selected from the group consisting of hydrogen and methyl;

when present, each $R^7$ is -$C_{1-4}$alkyl optionally substituted by 1, 2 or 3 substituents, each substituent being independently selected from the group consisting of halogen, -OH, -$OCH_3$, and -$OCF_3$;

$R^8$ is selected from the group consisting of hydrogen; halogen; -OH; -CN; -$C_{1-4}$alkyl optionally substituted by 1, 2 or 3 substituents, each substituent being independently selected from the group consisting of halogen, -OH, -CN, and methoxy optionally substituted by 1, 2 or 3 halogen; -$C_{3-6}$cycloalkyl optionally substituted by 1, 2 or 3 substituents, each substituent being independently selected from the group consisting of halogen, -$CH_3$, -OH, -CN, and methoxy optionally substituted by 1, 2 or 3 halogen; -$C_{1-4}$alkenyl optionally substituted by 1, 2 or 3 substituents, each substituent being independently selected from the group consisting of halogen, -OH, -CN, and methoxy optionally substituted by 1, 2 or 3 halogen; and -O-$C_{1-4}$alkyl optionally substituted by 1, 2 or 3 substituents, each substituent being independently selected from the group consisting of halogen, -OH, CN and methoxy optionally substituted by 1, 2 or 3 halogen;

$R^9$ is selected from the group consisting of hydrogen, and -$C_{1-4}$alkyl optionally substituted by 1, 2 or 3

substituents, each substituent being independently selected from the group consisting of halogen, -OH, -$OCH_3$, and -$OCF_3$; or

$R^8$ and $R^9$ are linked such that together with the atoms to which they are attached they form a 6-membered aryl group, a $C_{5-6}$cycloalkyl group, or a 5- or 6- membered aromatic heterocyclyl group comprising 1 or 2 heteroatoms selected from N, O and S, and wherein said 6-membered aryl group, $C_{5-6}$cycloalkyl group or 5- to 6- membered aromatic heterocyclyl group is optionally substituted by 1, 2 or 3 substituents, each substituent being independently selected from the group consisting of halogen; -OH; -CN; -$C_{1-4}$alkyl optionally substituted by 1, 2 or 3 substituents, each substituent being independently selected from the group consisting of halogen, -OH, and methoxy optionally substituted by 1, 2 or 3 halogen; and -O-$C_{1-4}$alkyl optionally substituted by 1, 2 or 3 substituents, each substituent being independently selected from the group consisting of - halogen, -OH, and methoxy optionally substituted by 1, 2 or 3 halogen;

p is 0, 1, or 2;

Z is a 5- to 13-membered non-aromatic heterocyclyl group comprising 1, 2 or 3 heteroatoms selected from N, O and S, wherein at least one of the heteroatoms is N, and wherein said 5- to 13-membered non-aromatic heterocyclyl group is optionally substituted with 1, 2, 3 or 4 substituents, each substituent being independently selected from the group consisting of halogen; -$C_{1-6}$alkyl optionally substituted by 1, 2 or 3 substituents, each substituent being independently selected from the group consisting of halogen, -OH, and -$OC_{1-3}$alkyl optionally substituted by 1, 2 or 3 halogen; -O-$C_{1-6}$alkyl optionally substituted by 1, 2 or 3 substituents, each substituent being independently selected from the group consisting of halogen, -OH, and -O-$C_{1-3}$alkyl optionally substituted by 1, 2 or 3 halogen; $NR^cR^d$; and $C_{3-6}$cycloalkyl optionally substituted by 1, 2 or 3 substituents, each substituent being independently selected from the group consisting of halogen, -OH, and -O-$C_{1-3}$alkyl optionally substituted by 1, 2 or 3 halogen; or when two substituents are on adjacent ring positions they may be linked such that together with the atoms to which they are attached they form a $C_{3-6}$cycloalkyl group or a 4- to 6-membered non-aromatic heterocyclyl group comprising 1 heteroatom selected from the group consisting of O and N, wherein said $C_{3-6}$cycloalkyl group or 4- to 6-membered non-aromatic heterocyclyl group is optionally substituted by 1 or 2 substituents, each substituent being independently selected from the group consisting of halogen; -$C_{1-6}$alkyl optionally substituted by 1, 2 or 3 substituents, each substituent being independently selected from the group consisting of halogen, -OH, and -$OC_{1-3}$alkyl optionally substituted by 1, 2 or 3 halogen; and -O-$C_{1-6}$alkyl optionally substituted by 1, 2 or 3 substituents, each substituent being independently selected from the group consisting of halogen, -OH, and - O-$C_{1-3}$alkyl optionally substituted by 1, 2 or 3 halogen;

$R^c$ is hydrogen;

$R^d$ is selected from the group consisting of hydrogen; -$C_{1-6}$alkyl optionally substituted by 1, 2 or 3 substituents, each substituent being independently selected from the group consisting of halogen, -$OCH_3$, and -$OCF_3$; and $C_{3-6}$cycloalkyl optionally substituted by 1, 2 or 3 substituents, each substituent being independently selected from the group consisting of halogen, -$CH_3$, -OH, -$OCH_3$, and -$OCF_3$;

or Z is -$NR^{10}R^{11}$, wherein

$R^{10}$ is hydrogen; and

$R^{11}$ is a 5- to 10-membered non-aromatic heterocyclyl group comprising 1, 2 or 3 heteroatoms selected from N, O and S, wherein at least one of the heteroatoms is N, and wherein said 5- to 10-membered non-aromatic heterocyclyl group is optionally substituted with 1, 2, 3 or 4 substituents independently selected from the group consisting of halogen; -OH; -$C_{1-6}$alkyl optionally substituted by 1, 2 or 3 substituents, each substituent being independently selected from the group consisting of halogen, -OH, and -$OC_{1-3}$alkyl optionally substituted by 1, 2 or 3 halogen; and -O-$C_{1-6}$alkyl optionally substituted by 1, 2 or 3 substituents, each substituent being independently selected from the group consisting of halogen, -OH, and -O-$C_{1-3}$alkyl optionally substituted by 1, 2 or 3 halogen; and

when present, each $R^a$ and $R^b$ are independently selected from the group consisting of hydrogen and -$C_{1-4}$alkyl, or a salt thereof.

14. The ADC or salt thereof according to embodiment 13, wherein the NMT inhibitor is a compound of a compound of formula (Vq):

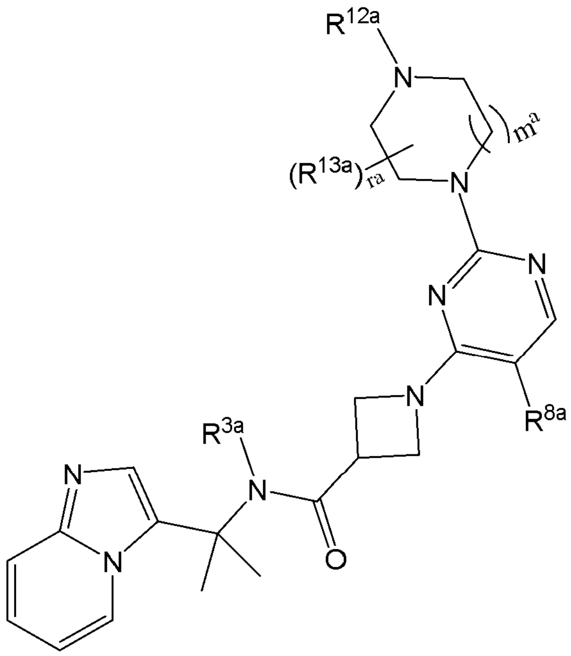

(Vq)

wherein:

$R^{3a}$ is H or $C_{1-4}$alkyl optionally substituted by 1, 2 or 3 substituents, each substituent being independently selected from the group consisting of halogen, $-OCH_3$, and $-OCF_3$;
$R^{8a}$ is halogen or $C_{1-4}$alkyl optionally substituted by 1, 2 or 3 substituents, each substituent being independently selected from the group consisting of halogen, $-OCH_3$, and $-OCF_3$;
ra is 0, 1 or 2;
$m^a$ is 1 or 2;
$R^{12a}$ is hydrogen; and
when present each $R^{13a}$ is independently selected from the group consisting of $C_{1-4}$alkyl optionally substituted by 1, 2 or 3 substituents, each substituent being independently selected from the group consisting of halogen, $-OCH_3$, and $-OCF_3$; and $C_{3-6}$cycloalkyl optionally substituted by 1, 2 or 3 substituents, each substituent being independently selected from the group consisting of halogen, $-CH_3$, $-OCH_3$, and $-OCF_3$; or
when ra is 2 and two $R^{13a}$ groups are on adjacent ring positions, said two $R^{13a}$ are linked such that together with the atoms to which they are attached they form a $C_{3-6}$cycloalkyl group optionally substituted by 1 or 2 substituents, each substituent being independently selected from the group consisting of halogen; $-C_{1-6}$alkyl optionally substituted by 1, 2 or 3 substituents, each substituent being independently selected from the group consisting of halogen, -OH, and $-OC_{1-3}$alkyl optionally substituted by 1, 2 or 3 halogen,
or a salt thereof.

15. The ADC or salt thereof according to embodiment 13 or 14, wherein the NMT inhibitor is (S)-1-(5-chloro-2-(2-methylpiperazin-1-yl)pyrimidin-4-yl)-N-(2-(imidazo[1,2-a]pyridine-3-yl)propan-2-yl)azetidine-3-carboxamide:

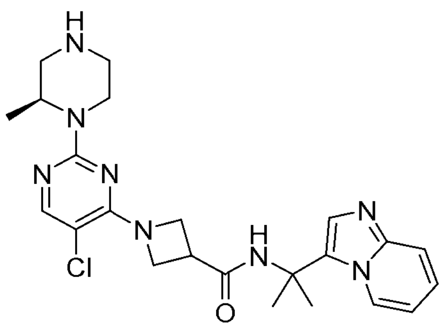

,

or a salt thereof.

16. The ADC or salt thereof according to any one of embodiments 1 to 4, wherein the NMT inhibitor is a compound of formula (VI):

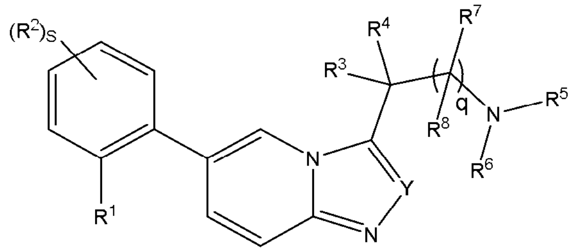

(VI)

wherein:

$R^1$ is a group of formula O-L-A;

L is $-(CHR^{12})_m-$;

each $R^{12}$ is independently H or $C_{1-4}$alkyl;
m is 1, 2 or 3;

A is:

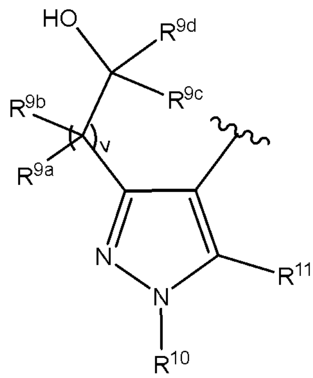

v is 0, 1 or 2;
$R^{9a}$ is H, $C_{1-4}$alkyl or $C_{1-4}$haloalkyl;
$R^{9b}$ is H, $C_{1-4}$alkyl or $C_{1-4}$haloalkyl;
$R^{9c}$ is $C_{1-4}$alkyl or $C_{1-4}$haloalkyl;
$R^{9d}$ is H, $C_{1-4}$alkyl or $C_{1-4}$haloalkyl;
$R^{10}$ is H, $C_{1-4}$alkyl or $C_{1-4}$haloalkyl;
$R^{11}$ is H, halo, CN, $C_{1-4}$alkyl, $C_{1-4}$haloalkyl, $C_{1-4}$alkoxy or $C_{1-4}$haloalkoxy;

s is 0, 1, 2 or 3;
each $R^2$ is independently F, Cl, Br, $C_{1-4}$alkyl optionally substituted by up to 3 halogen groups, $OCH_3$ or $OCF_3$;
Y is CH or $C_{1-4}$alkyl;
$R^3$ is H or $C_{1-4}$alkyl;
$R^4$ is H or $C_{1-4}$alkyl;
$R^5$ is H;
$R^6$ is H or $C_{1-4}$alkyl;
q is 0 or 1;
$R^7$ is H or methyl;
$R^8$ is H or methyl;
or $R^3$ and $R^6$ and the intervening atoms form a 3 to 7 membered non-aromatic heterocycle composed of the

intervening atoms and bond, or the intervening atoms and $-(CHR^a)_r-$; or the $R^7$ group and the $R^6$ group and the intervening atoms form a 3 to 7 membered non-aromatic heterocycle composed of the intervening atoms and $-(CHR^a)_r-$;
r is 1, 2, 3, 4 or 5; and
$R^a$ is hydrogen or methyl,

or a salt thereof.

17. The ADC or salt thereof according to embodiment 16, wherein the NMT inhibitor is a compound of formula (VIA):

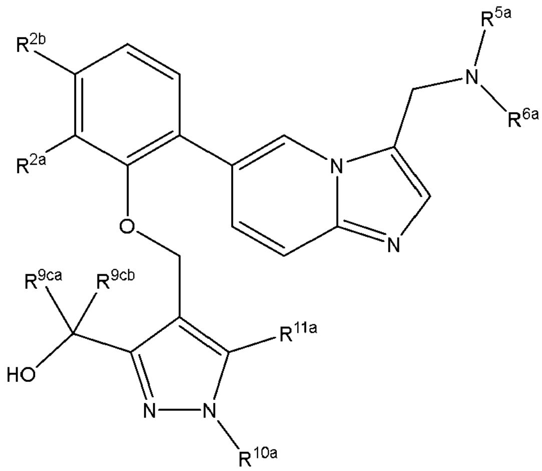

(VIA),

wherein:

$R^{2a}$ is H or F;
$R^{2b}$ is F;
$R^{5a}$ is H;
$R^{6a}$ is H or methyl;
$R^{9ca}$ is methyl, iso-propyl or tert-butyl;
$R^{9cb}$ is H or methyl;
$R^{10a}$ is methyl; and
$R^{11a}$ is methyl;
provided that when $R^{2a}$ is H, $R^{9cb}$ is H,

or a salt thereof.

18. The ADC or salt thereof according to embodiment 16 or 17, wherein the NMT inhibitor is 1-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}-2,2-dimethylpropan-1-ol:

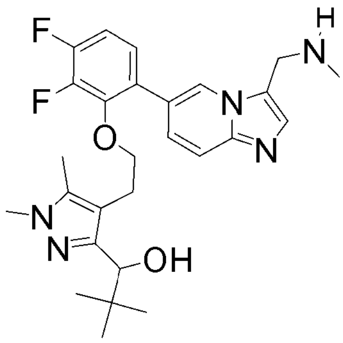

,

or a salt thereof.

19. The ADC or salt thereof according to embodiment 16 or 17, which is 2-{4-[2-(2,3-difluoro-6-{3-[(methylamino)methyl]imidazo[1,2-a]pyridin-6-yl}phenoxy)ethyl]-1,5-dimethyl-1H-pyrazol-3-yl}propan-2-ol:

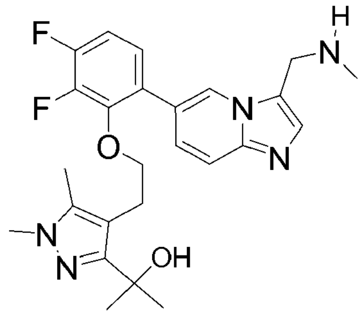

,

or a salt thereof.

20. The ADC or salt thereof according to any one of embodiments 1 to 19, wherein the linker is a cleavable linker.

21. The ADC or salt thereof according to embodiment 20, wherein the linker is cleavable by an enzyme.

22. The ADC or salt thereof according to any one of embodiments 1 to 21, wherein the linker has the following formula (VII):

$$-A_a-W_w-Y_y- \qquad \text{(formula (VII))}$$

wherein:

A is a first stretcher unit which when present forms a covalent bond with a chain terminus (such as a N-terminus) or a functional group of an amino acid side chain of the antibody;
a is 0 or 1;
each W is independently an amino acid unit or a glucuronide unit which when A and/or Y are absent forms a covalent bond with a chain terminus (such as a N-terminus) or a functional group of an amino acid side chain of the antibody and/or with a functional group of the NMT inhibitor respectively;
when W is an amino acid, w is 1 to 12;
when W is a glucuronide unit, w is 1 or 2;
Y is a second stretcher unit which when present forms a covalent bond with a functional group of the NMT inhibitor; and
y is 0 or 1.

23. The ADC or salt thereof according to embodiment 22, wherein A has the formula (A1):

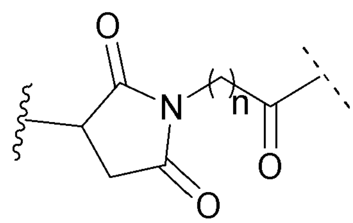

(A1)

,

wherein:

n is 1 to 6;
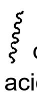 denotes the point of attachment to a chain terminus (such as a N-terminus) or a functional group of an amino acid side chain of the antibody; and

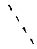 denotes the point of attachment to W.

24. The ADC or salt thereof according to embodiment 23, wherein A is

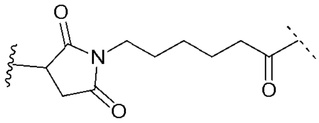

(A2)

.

25. The ADC or salt thereof according to embodiment 22, wherein A is

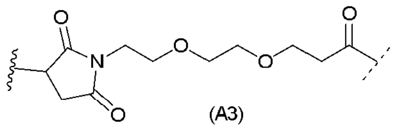

(A3)

.

26. The ADC or salt thereof according to any one of embodiments 22 to 25, wherein each W is an amino acid.

27. The ADC or salt thereof according to embodiment 26, wherein each W has the formula (WI):

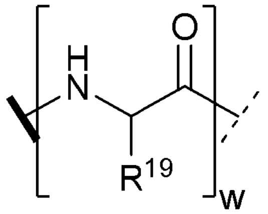

(WI)

wherein w is as previously defined; and

$R^{19}$ is H, methyl, iso-propyl, iso-butyl, sec-butyl, benzyl, p-hydroxybenzyl, $CH_2OH$, CH(OH)Me, $CH_2CH_2Sme$, $CH_2C(O)Sme$, $CH_2C(O)NH_2$, $CH_2C(O)OH$, $CH_2CH_2C(O)NH_2$, $CH_2CH_2C(O)OH$, $(CH_2)_3NHC(=NH)NH_2$, $(CH_2)_3NH_2$, $(CH_2)_3NHC(O)Me$, $(CH_2)_3NHCHO$, $(CH_2)_4NHC(=NH)NH_2$, $(CH_2)_4NH_2$, $(CH_2)_4NHC(O)Me$, $(CH_2)_4NHCHO$, $(CH_2)_3NHC(O)NH_2$, $(CH_2)_4NHC(O)NH_2$, $CH_2CH_2CH(OH)CH_2NH_2$, 2-pyridylmethyl,3-pyridylmethyl, 4-pyridylmethyl, phenyl,

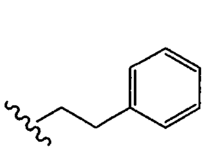 , 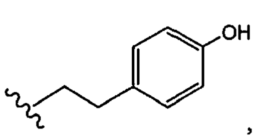 , 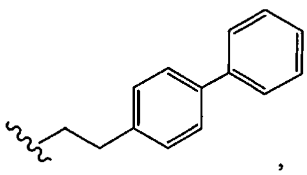 ,

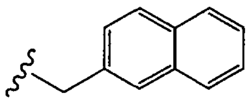 , 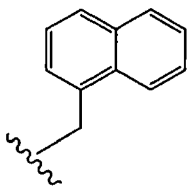 , 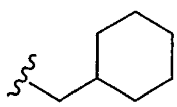 ,

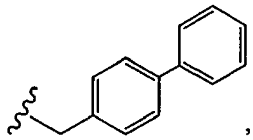, 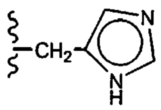, or 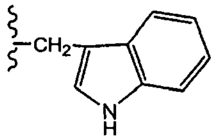.
;

wherein 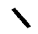 denotes the point of attachment to A (when present) or to a chain terminus (such as a N-terminus) or a functional group of an amino acid side chain of the antibody; and

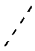 denotes the point of attachment to Y (when present) or to a functional group of the NMT inhibitor.

28. The ADC or salt thereof according to embodiment 27, wherein at least one $R^{19}$ is *iso*-propyl.

29. The ADC or salt thereof according to embodiment 27, wherein at least one $R^{19}$ is $(CH_2)_3NHC(=NH)NH_2$.

30. The ADC or salt thereof according to any one of embodiments 22 to 29, wherein w is 2.

31. The ADC or salt thereof according to any one of embodiments 22 to 30, wherein (W)w has the formula (WII):

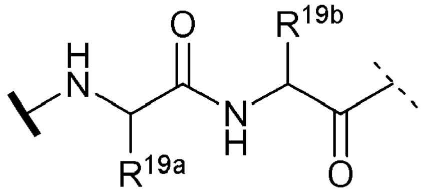
(WII),

wherein:

$R^{19a}$ is H, methyl, iso-propyl, iso-butyl, sec-butyl, benzyl, p-hydroxybenzyl, $CH_2OH$, CH(OH)Me, 2-pyridylmethyl,3-pyridylmethyl, 4-pyridylmethyl, phenyl,

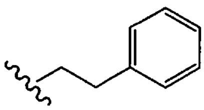, 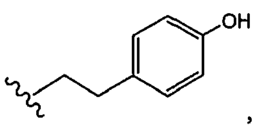, 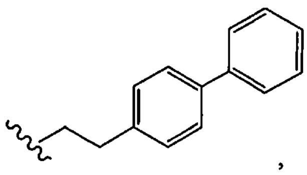,

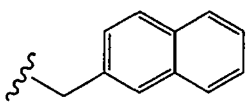, 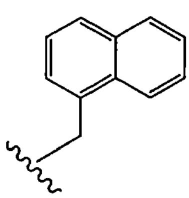, 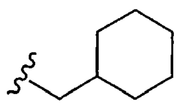,

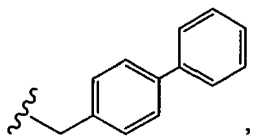, 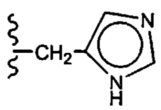, or 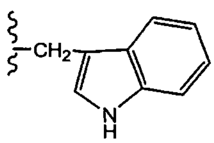.
;

and
$R^{19b}$ is $CH_2CH_2Sme$, $CH_2C(O)Sme$, $CH_2C(O)NH_2$, $CH_2C(O)OH$, $CH_2CH_2C(O)NH_2$, $CH_2CH_2C(O)OH$, $(CH_2)_3NHC(=NH)NH_2$, $(CH_2)_3NH_2$, $(CH_2)_3NHC(O)Me$, $(CH_2)_3NHCHO$, $(CH_2)_4NHC(=NH)NH_2$, $(CH_2)_4NH_2$, $(CH_2)_4NHC(O)Me$, $(CH_2)_4NHCHO$, $(CH_2)_3NHC(O)NH_2$, $(CH_2)_4NHC(O)NH_2$ or $CH_2CH_2CH(OH)CH_2NH_2$.

32. The ADC or salt thereof according to embodiment 31, wherein $R^{19a}$ is iso-propyl.

33. The ADC or salt thereof according to embodiment 31 or 32, wherein $R^{19b}$ is $(CH_2)_3NHC(=NH)NH_2$.

34. The ADC or salt thereof according to embodiment 22, wherein each W is a glucuronide unit.

35. The ADC or salt thereof according to embodiment 34, wherein (W)w has the formula (WIII):

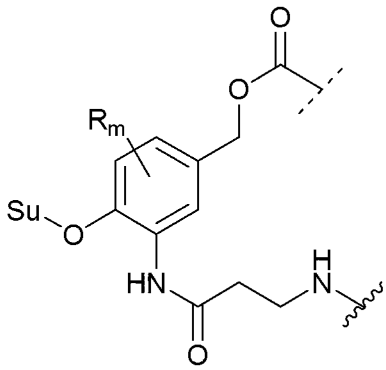

(WIII),

wherein:

R is H, halo, CN or $NO_2$;
m is 0, 1, 2 or 3;
Su is a sugar moiety;

wherein 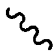 denotes the point of attachment to A (when present) or to a chain terminus (such as a N-terminus) or a functional group of an amino acid side chain of the antibody; and

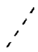 denotes the point of attachment to Y (when present) or to a functional group of the NMT inhibitor.

36. The ADC or salt thereof according to embodiment 35, wherein m is 0.

37. The ADC or salt thereof according to embodiment 35 or 36, wherein Su has the following formula:

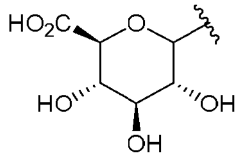

.

38. The ADC or salt thereof according to any one of embodiments 34 to 37, wherein y is 0.

39. The ADC or salt thereof according to any one of embodiments 22 to 33, wherein y is 1 and Y has the formula (Y1):

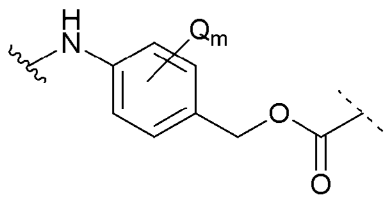

(Y1),

wherein:

each Q is independently halo, $NO_2$, CN, $C_{1-6}$alkyl, $C_{1-6}$haloalkyl, $C_{1-6}$alkoxy or $C_{1-6}$haloalkoxy; m is 0 to 4;

wherein 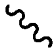 denotes the point of attachment to W; and

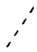 denotes the point of attachment to a functional group of the NMT inhibitor.

40. The ADC or salt thereof according to embodiment 39, wherein m is 0.

41. The ADC or salt thereof according to embodiment 22, wherein the linker has the formula (LI):

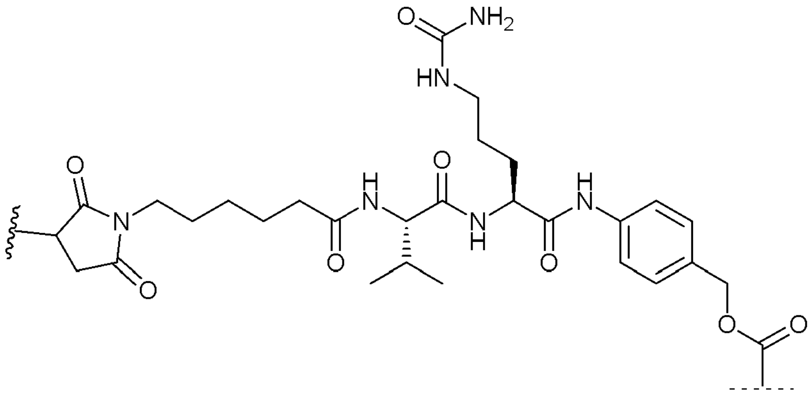 (LI),

wherein 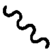 denotes the point of attachment to a chain terminus (such as a N-terminus) or a functional group on an amino acid side chain of the antibody; and

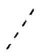 denotes the point of attachment to a functional group of the NMT inhibitor.

42. The ADC according to embodiment 22, wherein the linker has the formula (LII):

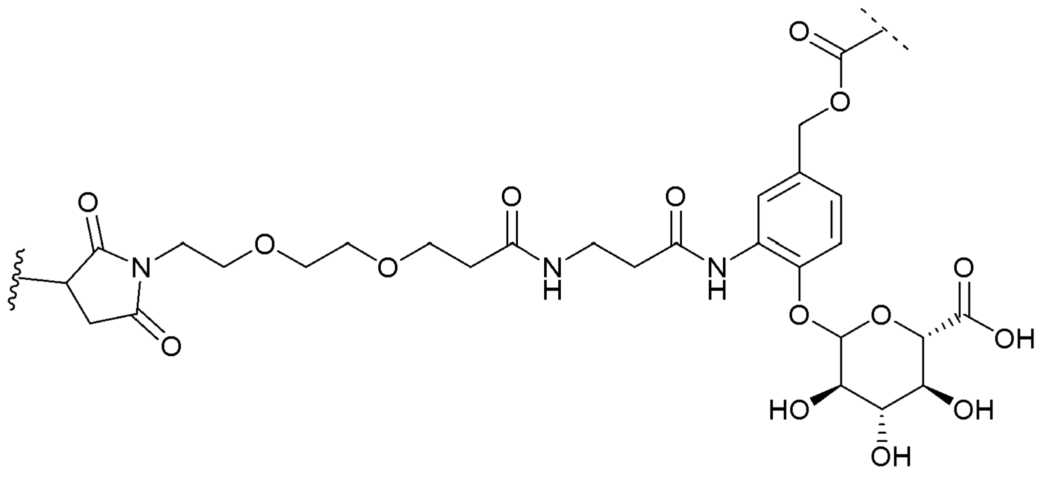 ,

wherein 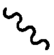 denotes the point of attachment to a chain terminus (such as a N-terminus) or a functional group on an amino acid side chain of the antibody; and

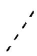 denotes the point of attachment to a functional group of the NMT inhibitor.

43. The ADC according to embodiment 20 or 21, wherein the linker has the formula (LIII):

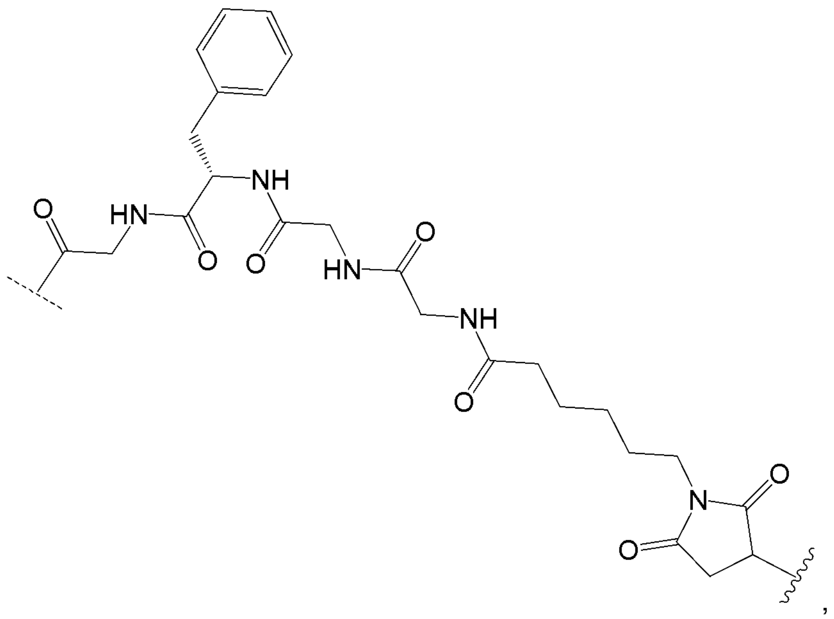

wherein 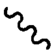 denotes the point of attachment to a chain terminus (such as a N-terminus) or a functional group on an amino acid side chain of the antibody; and

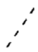 denotes the point of attachment to a functional group of the NMT inhibitor.

44. The ADC according to embodiment 20 or 21, wherein the linker has the formula (LIV):

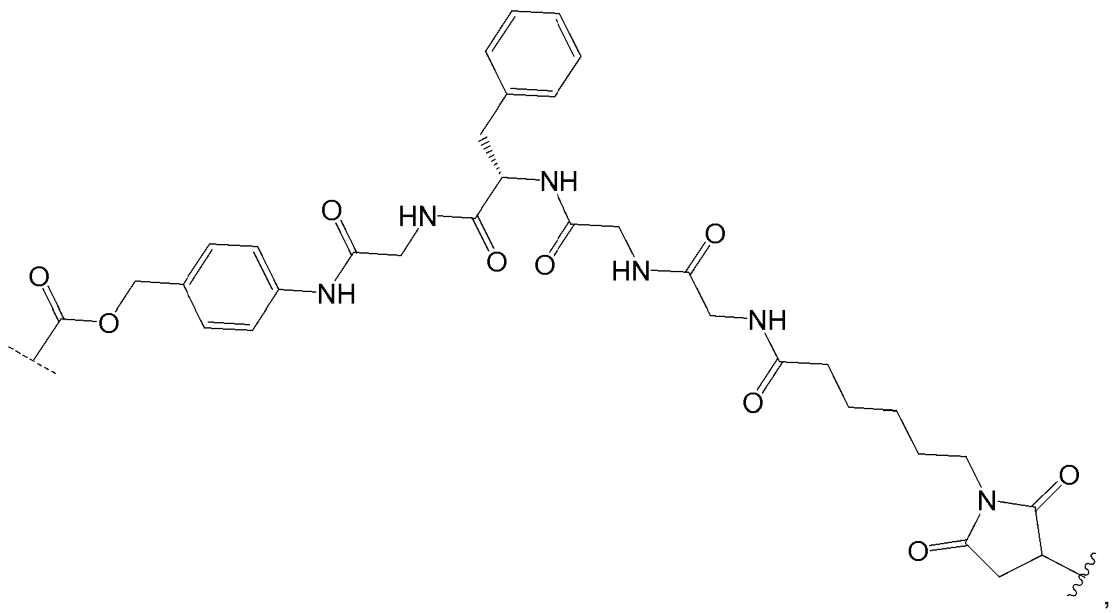

wherein 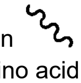 denotes the point of attachment to a chain terminus (such as a N-terminus) or a functional group on an amino acid side chain of the antibody; and

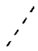 denotes the point of attachment to a functional group of the NMT inhibitor.

45. The ADC or salt thereof according to any one of embodiments 22 to 44, wherein the functional group on the amino acid side chain of the antibody is a sulfhydryl.

46. The ADC or salt thereof according to any one of embodiments 22 to 44, wherein the functional group on the NMT inhibitor is an amino or alcohol, for example an amino.

47. The ADC or salt thereof according to any one of embodiments 1 to 46, wherein the antibody is a humanised, chimeric, human antibody or an antibody fragment.

48. The ADC or salt thereof according to embodiment 47, wherein the antibody binds to HER2.

49. The ADC or salt thereof according to embodiment 48, wherein the antibody is trastuzumab, pertuzumab, margetuximab, ertumaxomab, MM-111, HER2Bi-aATCs, MCLA-128, ZW25, MDX-210, ado-trastuzumab or fam-trastuzumab.

50. The ADC or salt thereof according to embodiment 49, wherein the antibody is trastuzumab.

51. The ADC or salt thereof according to embodiment 47, wherein the antibody binds to CD20.

52. The ADC or salt thereof according to embodiment 51, wherein the antibody is rituximab.

53. The ADC or salt thereof according to embodiment 47, wherein the antibody binds to Trop-2.

54. The ADC or salt thereof according to embodiment 53, wherein the antibody is sacituzumab.

55. The ADC or salt thereof according to embodiment 47, wherein the antibody binds to B7-H3.

56. The ADC or salt thereof according to embodiment 55, wherein the antibody is ifinatamab.

57. The ADC of salt thereof according to any one of embodiments 1 to 56, wherein the drug loading (p) of NMT inhibitor to antibody is between 1 to 10 NMT inhibitor(s) to antibody, such as between 2 and 6, 4 and 6, 8 and 10, or 6 and 8 NMT inhibitors to antibody.

58. The ADC or salt thereof according to any one of embodiments 1 to 57, wherein the ADC or salt thereof comprises the following formula:

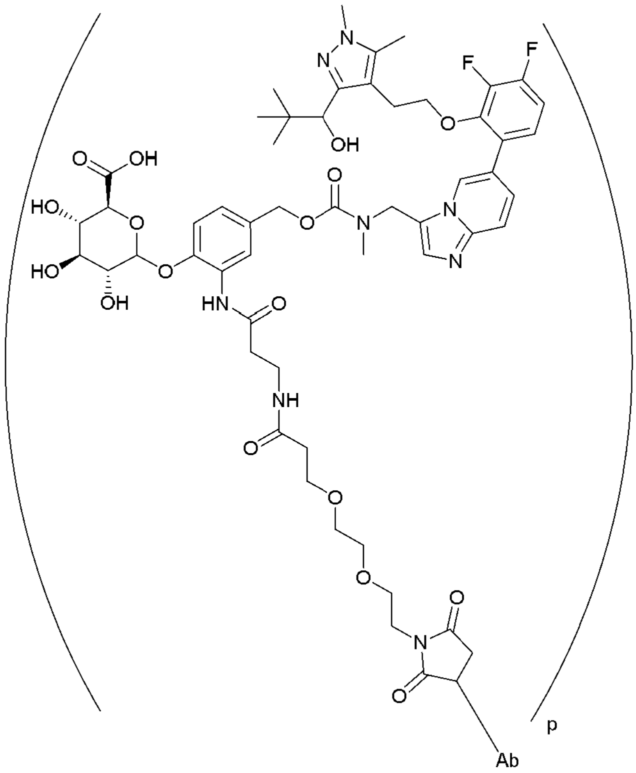

,

or a salt thereof, wherein Ab is an antibody as defined in any one of embodiments 1 to 56, such as the antibody is trastuzumab, and p is the drug loading of NMT inhibitor as defined in embodiment 57.

59. The ADC or salt thereof according to any one of embodiments 1 to 57, wherein the ADC or salt thereof comprises the following formula:

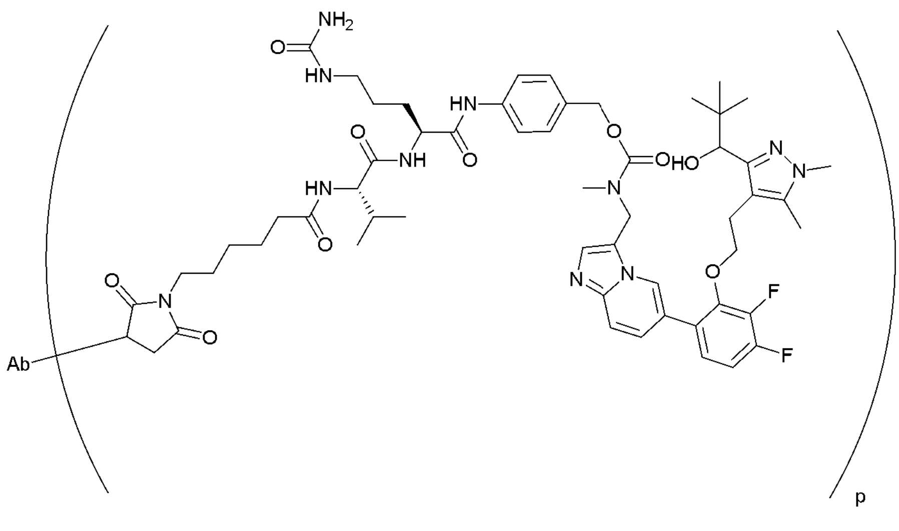

or a salt thereof, wherein Ab is an antibody as defined in any one of embodiments 1 to 56, such as the antibody is trastuzumab, and p is the drug loading of NMT inhibitor as defined in embodiment 57.

60. The ADC or salt thereof according to any one of embodiments 1 to 57, wherein the ADC or salt thereof comprises the following formula:

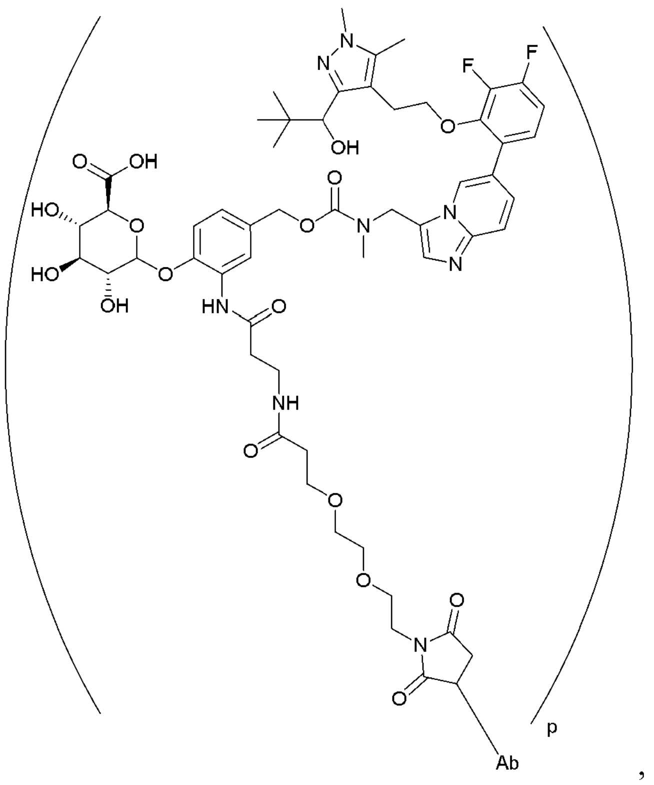

or a salt thereof, wherein Ab is sacituzumab, and p is the drug loading of NMT inhibitor as defined in embodiment 57.

61. The ADC or salt thereof according to any one of embodiments 1 to 57, wherein the ADC or salt thereof comprises the following formula:

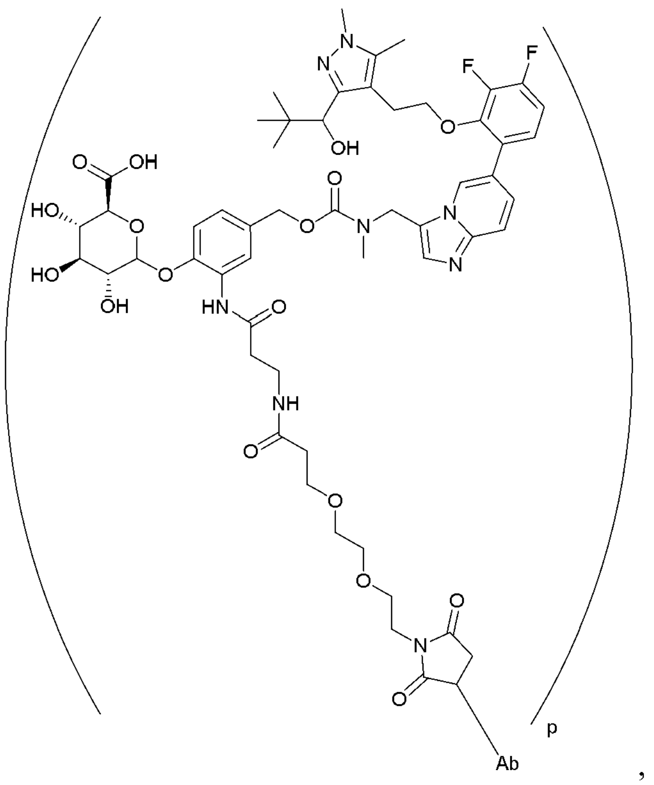

or a salt thereof, wherein Ab is ifinatamab, and p is the drug loading of NMT inhibitor as defined in embodiment 57.

62. The ADC or salt thereof according to any one of embodiments 1 to 57, wherein the ADC or salt thereof comprises the following formula:

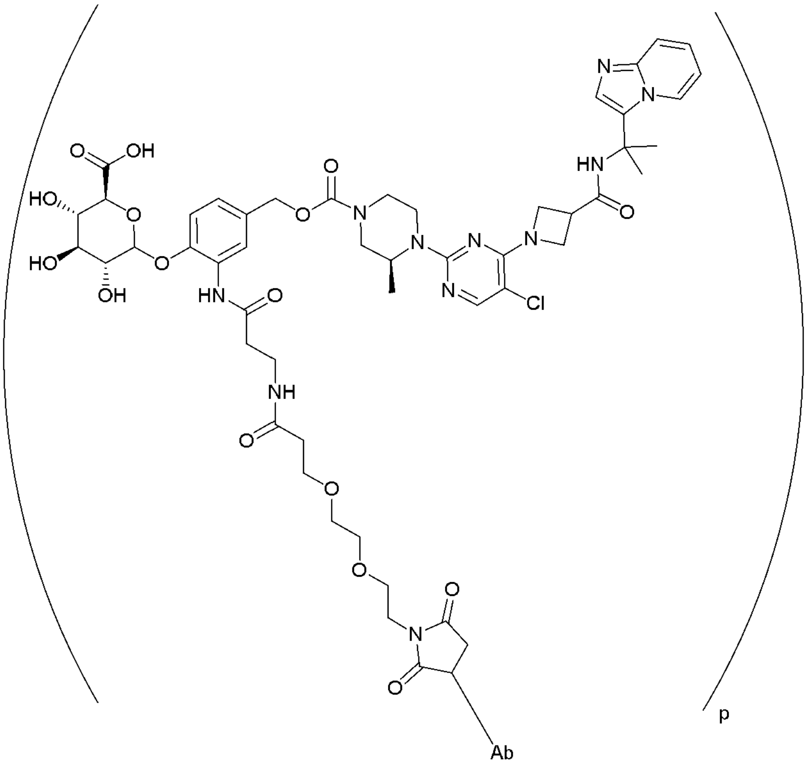

,

wherein Ab is an antibody as defined in any one of embodiments 1 to 56, such as the antibody is trastuzumab, and p is the drug loading of NMT inhibitor as defined in embodiment 57.

63. The ADC or salt thereof according to any one of embodiments 1 to 57, wherein the ADC or salt thereof comprises the following formula:

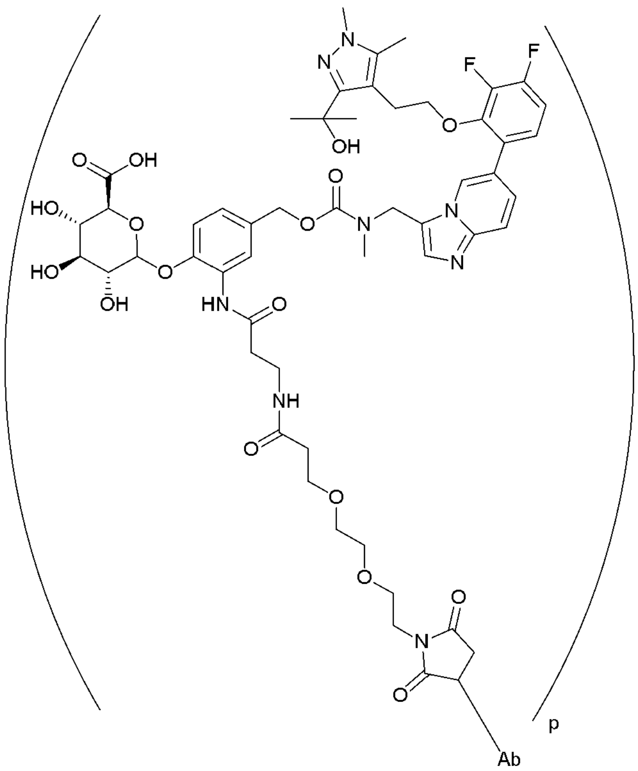

wherein Ab is an antibody as defined in any one of embodiments 1 to 56, such as the antibody is trastuzumab, and p is the drug loading of NMT inhibitor as defined in embodiment 57.

64. The ADC or salt thereof according to any one of embodiments 1 to 57, wherein the ADC or salt thereof comprises the following formula:

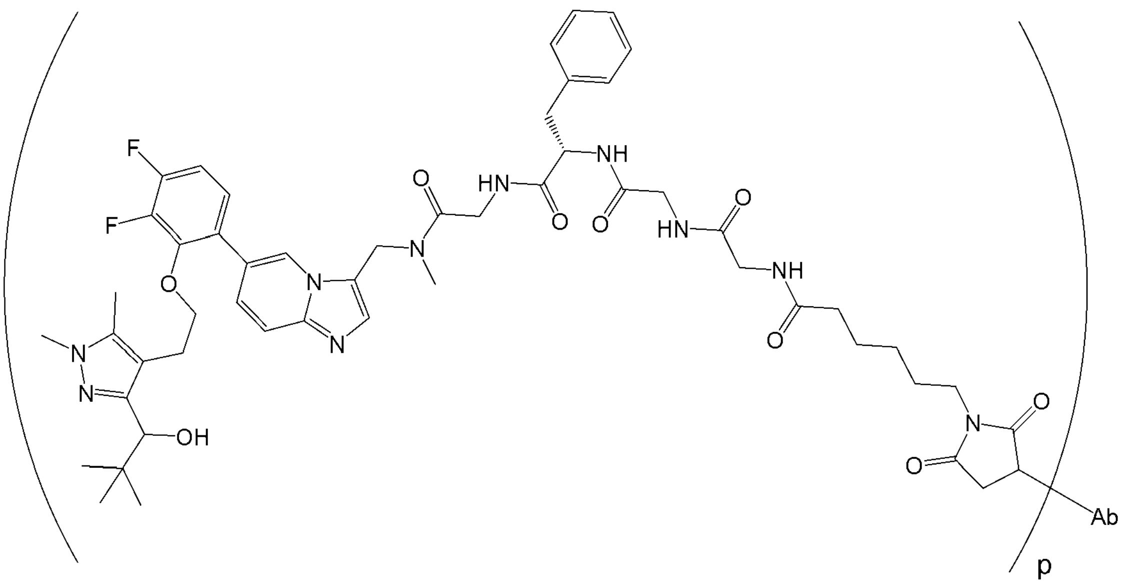

wherein Ab is an antibody as defined in any one of embodiments 1 to 56, such as the antibody is trastuzumab, and p is the drug loading of NMT inhibitor as defined in embodiment 57.

65. The ADC or salt thereof according to any one of embodiments 1 to 57, wherein the ADC or salt thereof comprises the following formula:

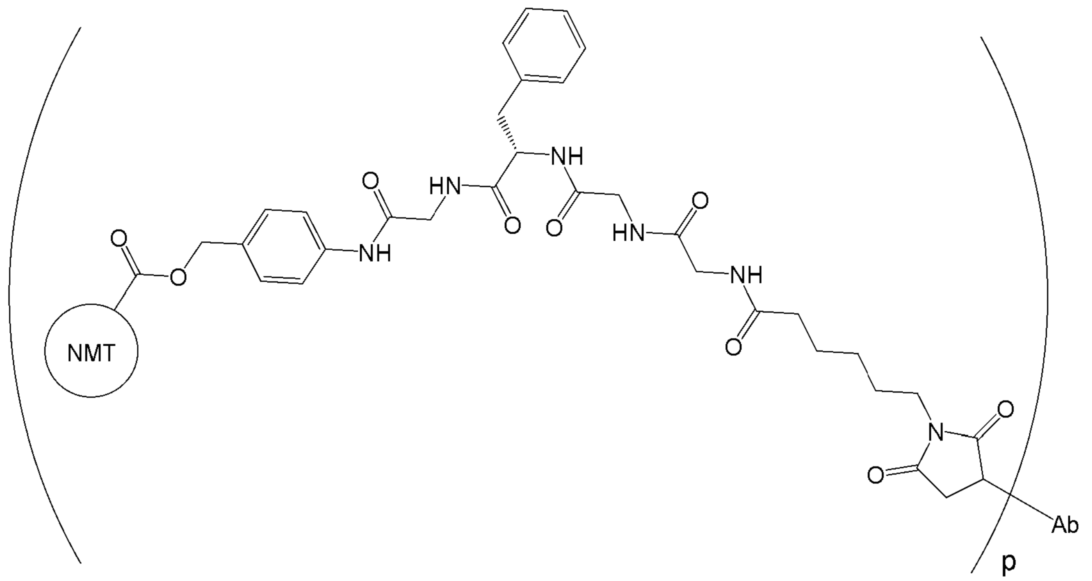

wherein Ab is an antibody as defined in any one of embodiments 1 to 56, such as the antibody is trastuzumab, and p is the drug loading of NMT inhibitor as defined in embodiment 57.

66. The ADC of salt thereof according to any one of embodiments 58 to 65, wherein the ADC or salt thereof is bound to the antibody via a sulfhydryl group on the side chain of an amino acid on the antibody.

67. A pharmaceutical composition comprising the ADC or a pharmaceutically acceptable salt thereof according to any one of embodiments 3 to 66.

68. The ADC or pharmaceutically acceptable salt thereof according to any one of embodiments 3 to 66, for use as a medicament.

69. The ADC or pharmaceutically acceptable salt thereof according to embodiment 68, for use in the prevention or treatment of a disease or disorder in which inhibition of N-myristoyl transferase provides a therapeutic or prophylactic effect.

70. Use of an ADC or pharmaceutically acceptable salt thereof according to any one of embodiments 3 to 66, in manufacture of a medicament for the prevention or treatment of a disease or disorder in which inhibition of N-myristoyl transferase provides a therapeutic or prophylactic effect.

71. A method of preventing or treating a disease or disorder in a subject in which inhibition of N-myristoyl transferase provides a therapeutic or prophylactic effect, said method comprising administering a therapeutically effective amount of an ADC or pharmaceutically acceptable salt thereof according to any one of embodiments 3 to 66.

72. The ADC or pharmaceutically acceptable salt thereof for use according to embodiment 69, the use according to embodiment 70 or the method according to embodiment 71, wherein the disease or disorder is a hyperproliferative disorder, and wherein the hyperproliferative disorder is cancer.

73. The ADC or pharmaceutically acceptable salt thereof for use, use or method according to embodiment 72 wherein the cancer is colorectal cancer, gallbladder carcinoma, brain tumor, lymphoma (such as B-cell lymphoma or disuse large B-cell lymphoma), leukemia (such as AML) or neuroblastoma.

74. The ADC or pharmaceutically acceptable salt thereof for use, use or method according to embodiment 72, wherein the cancer is a haematologic malignancy (such as a lymphoma, and in particular a B-cell lymphoma (for example high grade mantle zone lymphoma, follicular lymphoma, plasmablastic lymphoma, diffuse large B-cell lymphoma and Burkitt's lymphoma), a myeloma (such as multiple myeloma) or a leukaemia (such as chronic lymphocytic leukaemia, AML and B-acute lymphocytic leukaemia)) or a solid-tumor (such as brain, lung, breast, prostate, ovary, colorectal, gallbladder, kidney or liver cancer, or a blastoma (for example a neuroblastoma, a retinoblastoma or a glioblastoma)).

75. The ADC or pharmaceutically acceptable salt thereof for use, use or method according to embodiment 72, wherein the cancer expresses HER2 protein, such as selected from the group consisting of lung cancer, urothelial cancer, colorectal cancer, prostate cancer, ovarian cancer, pancreatic cancer, breast cancer, bladder cancer, gastric cancer, gastrointestinal stromal tumor, uterine cervix cancer, esophageal cancer, squamous cell carcinoma, peritoneal cancer, liver cancer, hepatocellular cancer, colon cancer, rectal cancer, colorectal cancer, endometrial cancer, uterine cancer, salivary gland cancer, kidney cancer, vulval cancer, thyroid cancer, and penis cancer.

76. A drug conjugate or salt and/or solvate thereof comprising a NMT inhibitor and a linker, wherein the linker comprises a group capable of forming a covalent bond to a chain terminus (such as a N-terminus) or a functional group on an amino acid side chain of an antibody e.g. a sulfhydryl group.

77. The drug conjugate, salt and/or solvate thereof according to embodiment 76, which has the formula:

$$A_a\text{-}W_w\text{-}Y_y\text{-NMT} \qquad \text{(formula (X))}$$

wherein:

A is a first stretcher unit which when present comprises a group capable of forming a covalent bond to a chain terminus (such as a N-terminus) or a functional group on an amino acid side chain of an antibody e.g. a sulfhydryl group;
NMT is a NMT inhibitor as defined in any one of embodiments 1 to 19; and
a, W, w, Y and y are as defined in any one of embodiments 22 to 59,

or a salt and/or solvate thereof.

78. The drug conjugate, salt and/or solvate thereof according to embodiment 77, wherein A is

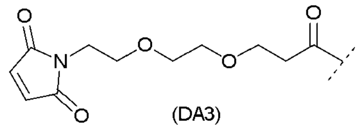

(DA3) ,

wherein 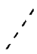 denotes the point of attachment to W.

79. The drug conjugate, salt and/or solvate thereof according to any one of embodiments 76 to 78, wherein the drug conjugate has the formula (DC-2):

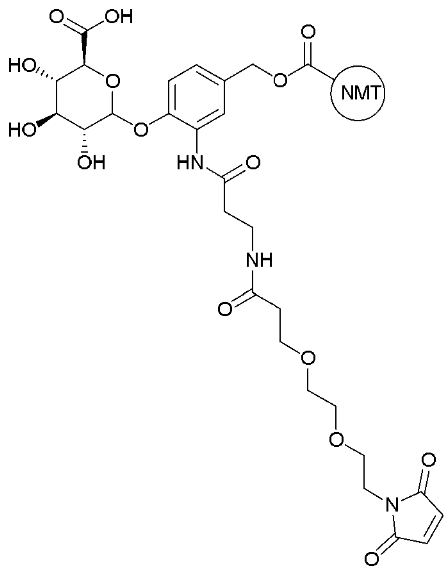

(DC-2),

or a salt and/or solvate thereof, wherein NMT is a NMT inhibitor as defined in any one of embodiments 1 to 19, particularly a NMT inhibitor as defined in any one of embodiments 16 to 19.

80. The drug conjugate, salt and/or solvate thereof according to any one of embodiments 76 to 79, which is (1S,2R,3S,4R,5R)-5-(4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3yl]methyl}(methyl)carbamoyl)oxy]methyl}-2-[3-(3-{2-[2-(2,5-dioxo-2,5-dihydro-1H-pyrrol-1-yl)ethoxy]ethoxy}propanamido)propanamido]phenoxy)-3,4-dihydroxy-2-methylcyclohexane-1-carboxylic acid:

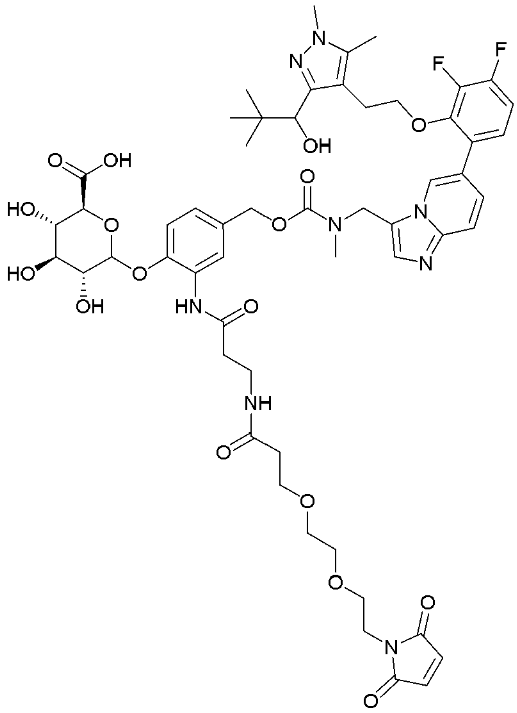

,

or a salt and/or solvate thereof.

81. The drug conjugate, salt and/or solvate thereof according to any one of embodiments 76 to 79, which is:

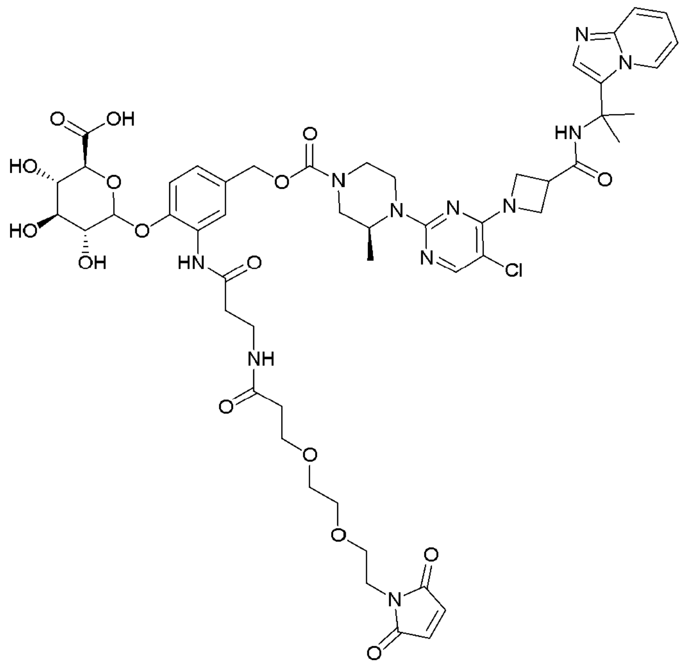

or a salt and/or solvate thereof.

82. The drug conjugate, salt and/or solvate thereof according to any one of embodiments 76 to 77, wherein the drug conjugate has the formula (DC-1):

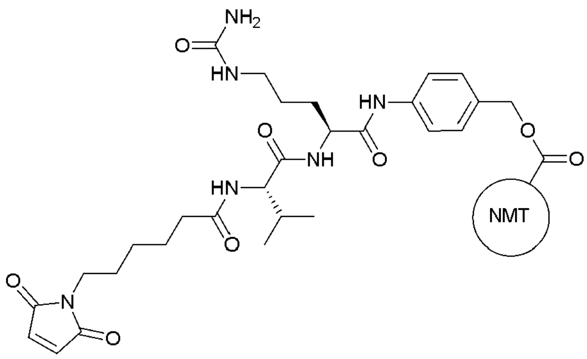

(DC-1),

or a salt and/or solvate thereof, wherein NMT is a NMT inhibitor as defined in any one of embodiments 1 to 19, particularly a NMT inhibitor as defined in any one of embodiments 16 to 19.

83. The drug conjugate, salt and/or solvate thereof according to embodiment 76, 77 or 82, which is {4-[(2S)-5-(carbamoylamino)-2-[(2S)-2-[6-(2,5-dioxo-2,5-dihydro-1H-pyrrol-1-yl)hexanamido]-3-methylbutanamido] pentanamido]phenyl}methyl N-{[6-(3,4-difluoro-2-{2-[3-(1-hydroxy-2,2-dimethylpropyl)-1,5-dimethyl-1H-pyrazol-4-yl]ethoxy} phenyl)imidazo[1,2-a]pyridin-3-yl]methyl}-N-methylcarbamate:

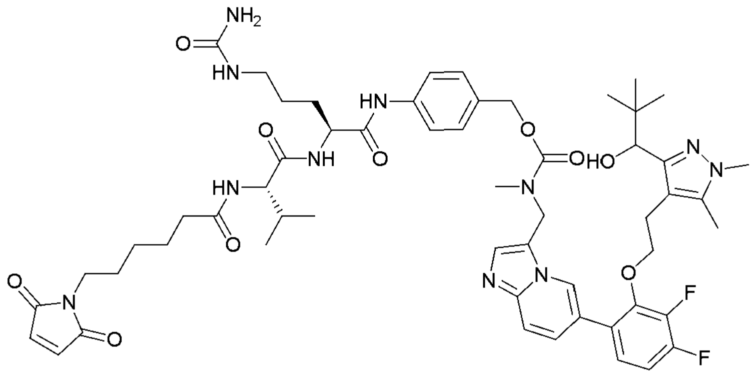

,

or a salt and/or solvate thereof.

84. The drug conjugate, salt and/or solvate thereof according to any one of embodiments 76 to 79, which is:

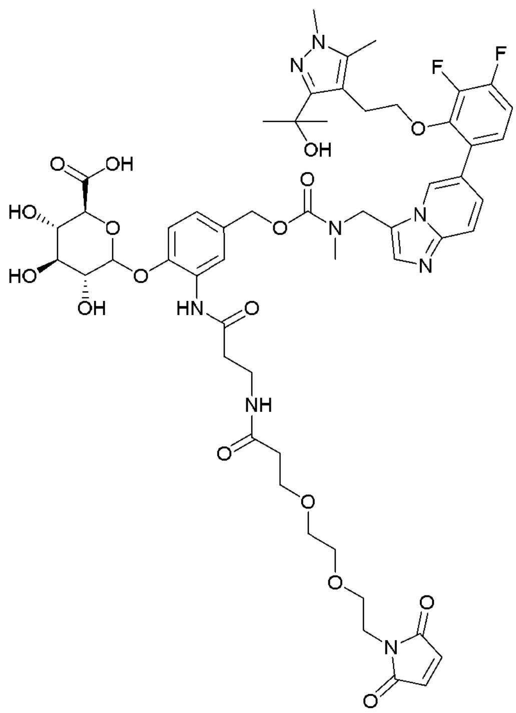

or a salt and/or solvate thereof.

85. The drug conjugate, salt and/or solvate thereof according to any one of embodiments 76 to 77, wherein the drug conjugate has the formula (DC-5):

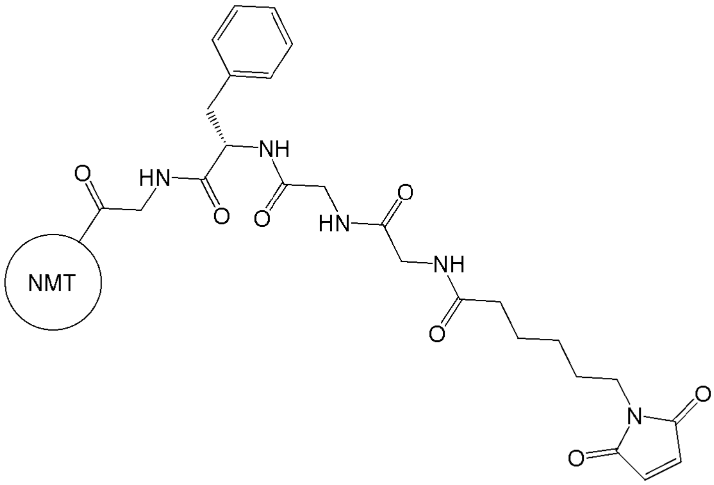

(DC-5),

or a salt and/or solvate thereof, wherein NMT is a NMT inhibitor as defined in any one of embodiments 1 to 19, particularly a NMT inhibitor as defined in any one of embodiments 16 to 19.

86. The drug conjugate, salt and/or solvate thereof according to embodiment 85, which is:

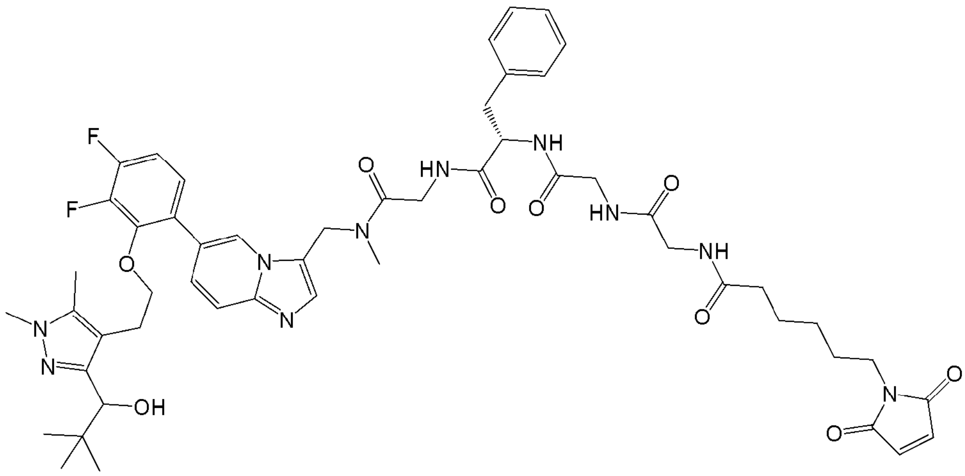

or a salt and/or solvate thereof.

87. The drug conjugate, salt and/or solvate thereof according to any one of embodiments 76 to 77, wherein the drug conjugate has the formula (DC-6):

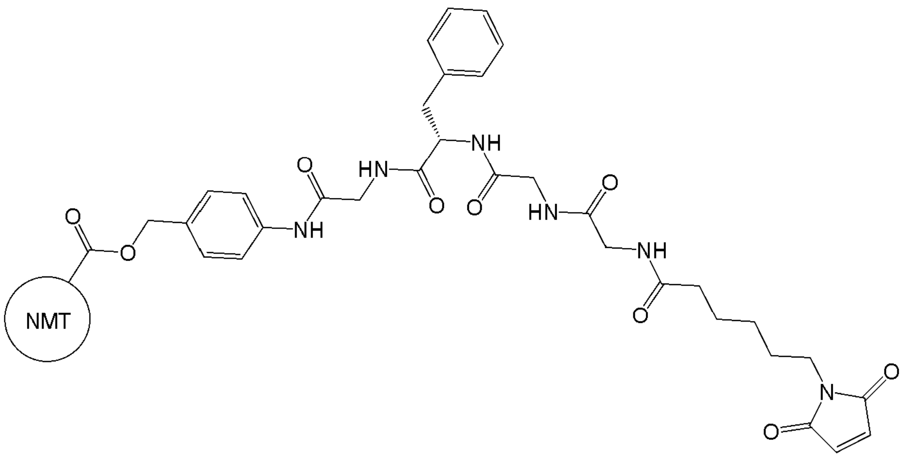

(DC-6),

or a salt and/or solvate thereof, wherein NMT is a NMT inhibitor as defined in any one of embodiments 1 to 19, particularly a NMT inhibitor as defined in any one of embodiments 16 to 19.

88. The salt of a drug conjugate according to any one of embodiments 76 to 87.

89. The solvate of a drug conjugate according to any one of embodiments 76 to 87.

90. The solvate of a salt of a drug conjugate according to any one of embodiments 76 to 87.

91. The drug conjugate according to any one of embodiments 76 to 87.

92. A compound selected from the group consisting of:

- a compound of formula (ADC-I):

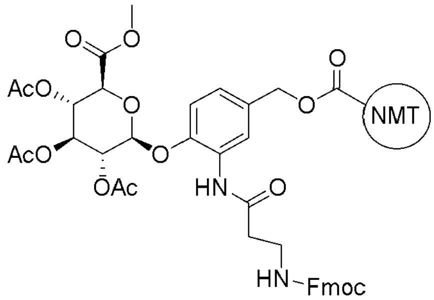

(ADC-I);

and

- a compound of formula (ADC-II):

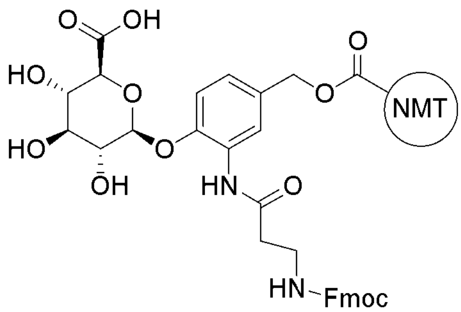

(ADC-II),

wherein **NMT** is a **NMT** inhibitor as defined in any one of embodiments 1 to 19, particularly a **NMT** inhibitor as defined in any one of embodiments 16 to 19,
or a salt and/or solvate of any one thereof.

The ADCs of the invention or pharmaceutically acceptable salts thereof are expected to display one of more of the following advantageous properties:

- *in vivo* cytotoxic activity e.g. as demonstrated in the mouse xenograft model of Biological Example 2; and
- *in vivo* tolerability e.g. as demonstrated in the mouse xenograft model of Biological Examples 2, 3, 10, 11, 12, and 13, and the monkey model of Biological Example 9;
- *in vitro* cytotoxicity against various cancer cell lines e.g. as shown in Biological Examples 4, 6, 7 and 14;
- improved efficacy against gastric cancer organoids compared with known controls e.g. as shown in Biological Example 8; and
- improved bystander effect as shown in Biological Example 6.

Said properties are expected to make the ADCs of the invention or pharmaceutically acceptable salts thereof

suitable for use in the treatment or prevention (e.g. treatment) of hyperproliferative disorders such as cancer or other diseases or disorders in which inhibition of human **NMT** provides a therapeutic or prophylactic effect.

## Examples

### Abbreviations

| | |
|---|---|
| ALT | alanine transaminase |
| AST | aspartate transaminase |
| ALP | alkaline phosphatase |
| GGT | gamma-glutamyl transferase |
| CK | creatine kinase |
| LDH | lactate dehydrogenase |
| TP | total protein |
| ALB | Albumin |
| GLO | Globulin |
| A/G | Albumin/Globulin ratio |
| TBIL | total bilirubin |
| BU | blood urea nitrogen |
| CRE | creatinine |
| BUN/C | Blood urea nitrogen/creatinine ratio |
| GLU | Glucose |
| CHO | cholesterol |
| TG | triglycerides |
| Na | sodium |
| K | potassium |
| Cl | chloride |
| Ca | calcium |
| P | phosphate |
| WBC | white blood cells |
| ABNEUT | neutrophils |
| ABLYMP | lymphocytes |
| ABMONO | monocytes |
| ABBASO | basophils |
| ABEOS | eosinophils |
| PLT | platelets |
| MPV | mean platelet volume |
| RBC | red blood cell |
| HCT | haematocrit |
| HGB | haemoglobin |
| MCV | mean corpuscular volume |
| MCH | mean corpuscular hemoglobin |
| MCHC | Mean Corpuscular Haemoglobin Concentration |
| ABRETIC | reticulocytes |
| QW | once a week |

### Synthesis of Example Compounds

#### General Experimental Details

*LC-MS*

Compounds requiring purification under basic conditions were typically purified on an LC-MS system equipped with a YMC Actus Triart C18 5μm (20 x 250mm) column or Gemini NX 5μm C18 (100 x 30mm) column, using a gradient elution of acetonitrile in water containing 20mM Ammonium bicarbonate (10-45% over 30min then 95% acetonitrile for 2

minutes).

*HPLC*

The purity of certain compounds was determined by TyeEclipse Extend or XDB 5μm C18 (150 x 4.6mm), Xbridge 5μm C18 (100 x 4.6mm), Zorbax Extend 5μm C18 (150 x 4.6mm), or Shimadzu L Column 2 ODS 5μm C18 (150x4.6mm) column using gradient elution of acetonitrile in water containing 10mM ammonium acetate over 15 mins (HPLC B) 17 mins (B1) and 18 mins (B3).

The purity of certain compounds was determined by analytical HPLC using a Poroshell 120 2.7μm EC18 (100 x 4.6mm), Luna Omega Polar 3μm C18 (100 x 4.6mm), Xbridge 5μm C18 (150 x 4.6mm) or Sunfire 5μm C18 (100 x 4.6mm) using gradient elution of acetonitrile in water containing 0.05% trifluoroacetic acid over 12 mins (HPLC A), 14 mins (A1) or 17 mins (A2) and 16 mins (A4).

The purity of certain compounds was determined by analytical HPLC using a Gemini NX 3 μm C18 (100 x 4.6mm) column using gradient elution of acetonitrile in water containing 0.05% formic acid over 16 mins (A6).

*NMR*

$^{1}H$ NMR and $^{13}C$ spectra were recorded on 400 MHz and 101 MHz respectively instruments at room temperature unless specified otherwise were referenced to residual solvent signals. Data are presented as follows: chemical shift in ppm, integration, multiplicity (br = broad, s = singlet, d = doublet, t = triplet, q = quartet, p = pentet, m = multiplet) and coupling constants in Hz.

ADC Testing Methods

*SEC-HPLC*

Column: TOSOH TSKgel G3000SWXL 7.8mm x 30cm 5μm particle (MERCK808541) combined with a security guard column (MERCK 822858) with a GFC3000 4x3mm cartridge (Phenomenex); Buffer: 0.2M Phosphate 0.25M KCl 10% IPA; Gradient: Isocratic @0.5ml/min at 25°C. Sample load was approximately 10μg with monomer and concentration determined from 214nm signal. Monomer reported based on peak integration and [ADC] mg/mL based on a calibration curve of antibody.

*RP-HPLC for residual NMT inhibitor*

Column: Kinetex® 2.6 μm C8 100 Å, LC Column 50 x 4.6 mm,(Phenomex 00B-4497-E0); Mobile Phase A 0.05% TFA in water; Mobile Phase B 0.05% TFA in CAN; Gradient at 60°C at 2ml/min:

| **TIME** | **%B** |
|---|---|
| **0.00** | 5 |
| **8.00** | 95 |
| **8.10** | 100 |
| **9.00** | 100 |
| **9.10** | 5 |
| **10.00** | 5 |

50μl sample (ADC or PBS/PS20 matrix) +2μl 5M NaCl +150μl cold MeOH (from -20C freezer). Incubate at -20°C for 30minutes. Centrifuge at 21,000g at 4°C for 30minutes. 125μl of supernatant was extracted and mixed with 125μl WFI. 100μl of this was injected onto the Kinetex column. Data was analysed at 214nm and the residual NMT inhibitor in the sample estimated from an external calibration curve of the relevant NMT inhibitor-linker. The result is expressed as the percentage free relative to free and bound using the ADC concentration and calculated DAR to determine the amount of bound NMT inhibitor.

*HIC-HPLC for Average DAR (drug antibody ratio) calculations*

This method can be used as an alternative to PLRP-HPLC methods for determining average DAR.

**Column:** TOSOH Butyl-NPR 4.6 mm x 3.5 cm, 2.5 µm particle size (Merck 822855); Mobile phase A: 1.5 M (NH4) 2SO4, 25mM NaPi, pH 6.95 ± 0.05; Mobile phase B: 25 mM NaH2PO4 pH 6.95 ± 0.05 + 25 % IPA; Gradient at 25°C 0.8ml/min:

| Time | % B |
|---|---|
| 0 | 0 |
| 12 | 100 |
| 12.1 | 0 |
| 18 | 0 |

Load cartridge. 10µg and report result / analysed at 214nm.

*RP-HPLC for Average DAR calculations*

Column - PLRP-S 2.1mm x 5cm, 5µm (Agilent PL1912-1502); Mobile Phase A: 0.1% TFA in Water; Mobile Phase B: 0.1 % TFA in Acetonitrile; Gradient at 80°C, 1mL/min:

| Time | % B |
|---|---|
| 0 | 22.5 |
| 2 | 22.5 |
| 21.5 | 49.5 |
| 22.5 | 90.0 |
| 26.5 | 90.0 |
| 27.5 | 22.5 |
| 32.0 | 22.5 |

~10ug of sample (ADC) + 5µl 0.1M DTT made up to 50µL with 0.5M Tris, pH 8.0 incubated at 37°C for 15 minutes. Sample then diluted 1:1 (+50µL) with 49% Water, 49% Acetonitrile, 2% Formic Acid. 20uL of this solution then injected onto the RP-HPLC column. Data was analysed at 214nm and average DAR calculated.

*Endotoxin kinetic chromogenic assay*

Endotoxin was determined by kinetic chromogenic LAL assay using an Endosafe PTS endotoxin system. The ADCs were diluted 10-fold in LAL reagent water. All samples were analysed on 0.01 - 1 EU/mL cartridges. The EU/mL value was converted to EU/mg by dividing by the ADC [P] mg/mL.

**Preparation of NMT inhibitor 1: 1-(4-(2-(2,3-difluoro-6-(3-((methylamino)methyl)imidazo[1,2-a]pyridin-6-yl)phenoxy)ethyl)-1,5-dimethyl-1H-pyrazol-3-yl)-2,2-dimethylpropan-1-ol**

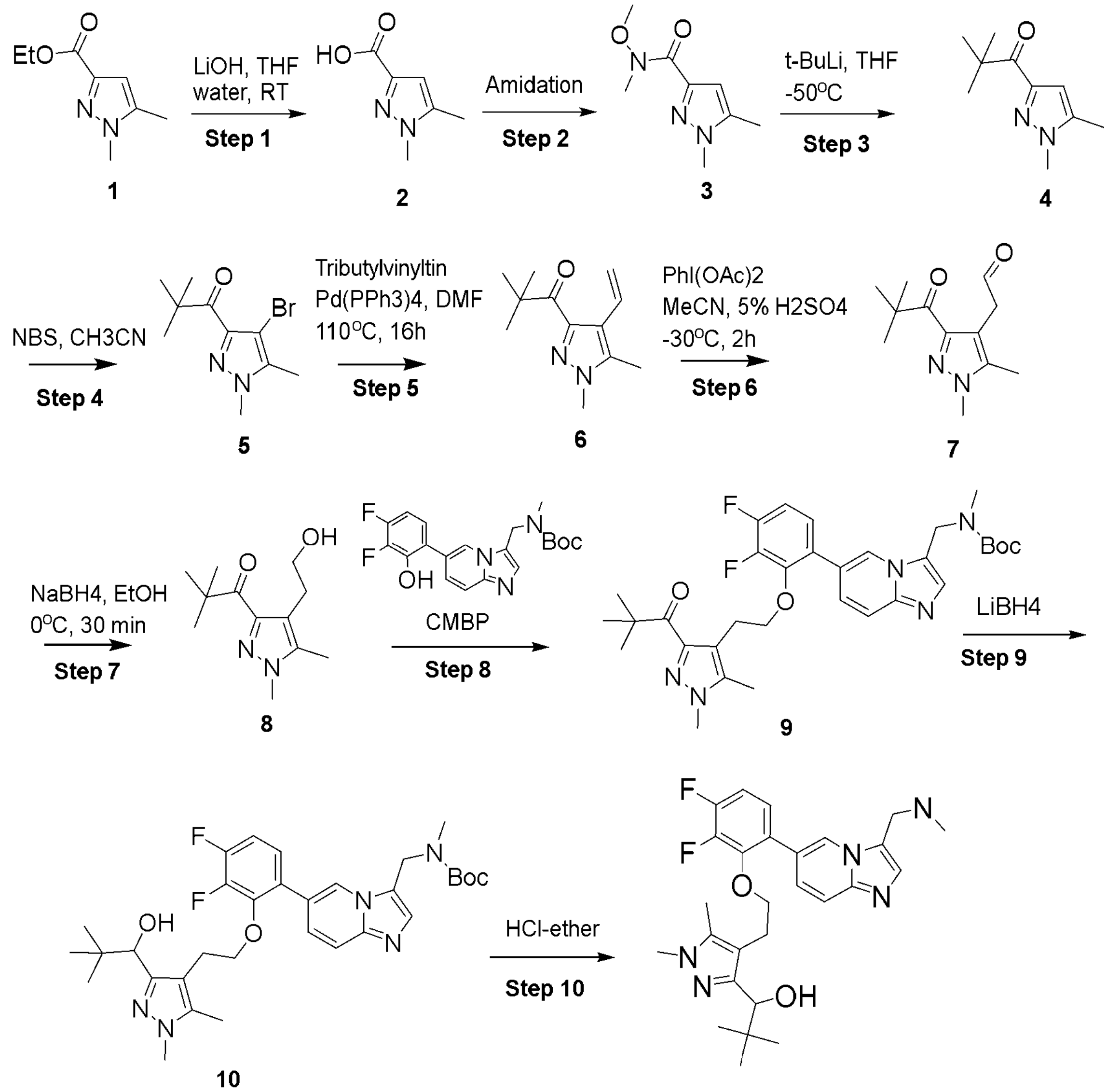


**Step 1** - **Intermediate (2): 1,5-dimethyl-1H-pyrazole-3-carboxylic acid**

**Procedure:** To a solution of ethyl 1,5-dimethyl-1H-pyrazole-3-carboxylate (intermediate (1)) (20.0 g, 118.984 mmol) in THF:water (4:1) (280 ml, 70 ml) were added ethanol (0.4 ml) and LiOH.H2O (9.985 g, 237.968 mmol) at room temperature. The resulting mixture was stirred at room temperature for 16 hrs. TLC/LCMS showed complete consumption of SM. The reaction mixture was acidified with 3N HCl solution (pH~2) at 0°C and extracted with ethyl acetate. The organic layer was dried over anhydrous sodium sulphate and concentrated to get 1,5-dimethyl-1H-pyrazole-3-carboxylic acid (2) as light-yellow solid (16 g, 99%). $^1$H NMR (400 MHz, DMSO) d 12.42 (s, 1H), 6.45 (s, 1H), 3.77 (s, 3H), 2.25 (s, 3H).

**Step 2** - **Intermediate (3): N-methoxy-N,1,5-trimethyl-1H-pyrazole-3-carboxamide**

**Procedure:** To a stirred solution of 1,5-dimethyl-1H-pyrazole-3-carboxylic acid (intermediate (2)) (16.6 g, 118.571 mmol) in tetrahydrofuran (350.0 ml) was added N,O-Dimethylhydroxylamine hydrochloride (17.34 g, 177.857 mmol). Triethylamine (82.633 ml, 592.857 mmol), 1-(3-Dimethylaminopropyl)-3-ethylcarbodiimide hydrochloride (34.095 g, 177.857 mmol) and 1-Hydroxybenzotriazole (24.032 g, 177.857 mmol) were added and the reaction mixture was stirred at RT for 16h. TLC was checked which showed formation of product. The reaction was washed with sodium bicarbonate solution and extracted with ethyl acetate. The organic layer was washed with water, brine, dried over anhydrous sodium sulphate and concentrated under reduced pressure. The crude was purified by combiflash using 5% MeOH in DCM to get N-methoxy-N,1,5-trimethyl-1H-pyrazole-3-carboxamide (3) as light-yellow solid (15.0 g, 69.05%). $^1$H NMR (400 MHz, DMSO) $\delta$ 6.41 (s, 1H), 3.76 (s, 3H), 3.67 (s, 3H), 3.32 (s, 3H), 2.26 (s, 3H); LCMS (NH4Oac:CAN): M+H=184, Rt= 2.17 min in 5 mins run.

**Step 3 - Intermediate (4): 1-(1,5-dimethyl-1H-pyrazol-3-yl)-2,2-dimethylpropan-1-one**

**Procedure:** A stirred solution of N-methoxy-N,1,5-trimethyl-1H-pyrazole-3-carboxamide (intermediate (3)) (15.0 g, 81.922 mmol) in tetrahydrofuran (150.0 ml) was cooled to -50°C and t-butyllithium (1.7M in pentane) (96.379 ml, 163.844 mmol) was added at -50°C. Then the reaction mixture was stirred at -50°C for 2 hrs. TLC was checked which showed formation of product and the reaction mixture was quenched with sat. NH4Cl solution. The reaction mixture was diluted with ethyl acetate and washed with water, brine solution. Organic layer was separated and dried over anhydrous sodium sulphate and concentrated under reduced pressure. The crude was purified by combiflash chromatography using 5% MeOH in DCM to afford 1-(1,5-dimethyl-1H-pyrazol-3-yl)-2,2-dimethylpropan-1-one (4) as light yellow solid (6.0 g, 40.63%). $^1$H NMR (400 MHz, DMSO) δ 6.45 (s, 1H), 3.79 (s, 3H), 2.25 (s, 3H), 1.31 (s, 9H); LCMS (HCOOH:CAN): M+H=181, Rt= 1.86 min in 3 mins run.

**Step 4 - Intermediate (5): 1-(4-bromo-1,5-dimethyl-1H-pyrazol-3-yl)-2,2-dimethylpropan-1-one**

**Procedure:** To a solution of 1-(1,5-dimethyl-1H-pyrazol-3-yl)-2,2-dimethylpropan-1-one (intermediate (4)) (6.0 g, 33.309 mmol) in Acetonitrile (100.0 ml) was added N-bromosuccinimide (6.191 g, 34.975 mmol) portion wise at ice cold condition. The resulting reaction mixture was stirred at RT for 16 hrs. TLC and LCMS was checked which showed formation of the product. The reaction mixture was then diluted with ethyl acetate and washed with Sat.NaHCO3 solution, water and brine solution. The organic layer was dried over anhydrous sodium sulphate and concentrated under vacuum to afford 1-(4-bromo-1,5-dimethyl-1H-pyrazol-3-yl)-2,2-dimethylpropan-1-one (5) as yellow solid compound (8.0 g, 92.68%) which was used for next step without purification. $^1$H NMR (400 MHz, DMSO) δ 3.86 (s, 3H), 2.25 (s, 3H), 1.30 (s, 9H); LCMS (NH4Oac:CAN): M+H=259, Rt= 3.59 min in 5 mins run.

**Step 5** - **Intermediate (6): 1-(1,5-dimethyl-4-vinyl-1H-pyrazol-3-yl)-2,2-dimethylpropan-1-one**

**Procedure:** To a solution of 1-(1,5-dimethyl-4-vinyl-1H-pyrazol-3-yl)-2,2-dimethylpropan-1-one (intermediate (5)) (7.0 g, 27.129 mmol) in dry N,N-dimethylformamide (100.0 ml) was added Tributylvinyltin (17.2 ml, 54.257 mmol) at room temperature. Then argon was purged through the reaction mixture for 15 min and Pd(PPh3)4 (3.133 g, 2.713 mmol) was added. The reaction mixture was stirred at 110°C for 16 hrs. TLC was checked which showed starting material was consumed and formation of the desired product. The reaction mixture was then diluted with ethyl acetate and washed with Potassium Fluoride solution the precipitate was filtered through cintre and washed with water and brine, dried over sodium sulphate and concentrated. The crude was purified by column chromatography (100-200) in 10% Ethyl acetate-Hexane to get 1-(1,5-dimethyl-4-vinyl-1H-pyrazol-3-yl)-2,2-dimethylpropan-1-one (6) (5.0 g, 89.35%). $^1$H NMR (400 MHz, DMSO) δ 6.96-6.89 (m, 1H), 5.29-5.20 (m, 2H), 3.82 (s, 3H), 2.31 (s, 3H), 1.30 (s, 9H); LCMS (HCOOH:CAN): M+H=207, Rt= 2.19 min in 3 mins run.

**Step 6** - **Intermediate (7): 2-(1,5-dimethyl-3-pivaloyl-1H-pyrazol-4-yl)acetaldehyde**

**Procedure:** To a solution of 1-(1,5-dimethyl-4-vinyl-1H-pyrazol-3-yl)-2,2-dimethylpropan-1-one (intermediate (6)) (4.1 g, 19.903 mmol) and (diacetoxyiodo)benzene (6.729 g, 20.898 mmol) in acetonitrile (60.0 ml) was added 5% sulfuric acid (3.525 ml) dropwise at -30°C. The mixture was stirred at -30°C for 1 hour. Upon the completion of the reaction, the residue was treated with ethyl acetate and washed with saturated sodium bicarbonate solution, water and brine solution. The aqueous layer was back-extracted with ethyl acetate and the combined organic layers were dried over anhydrous sodium sulphate, filtered and concentrated in vacuum to get 2-(1,5-dimethyl-3-pivaloyl-1H-pyrazol-4-yl) acetaldehyde (7) (2.7 g, 61.03%). This fraction was then used for next step without purification. $^1$H NMR (400 MHz, DMSO) δ 9.49 (s, 1H), 3.82 (s, 3H), 3.65 (s, 2H), 2.17 (s, 3H), 1.30 (s, 9H); LCMS (NH4Oac:CAN): M+H=223, Rt= 1.86 min in 3 mins run.

**Step 7** - **Intermediate (8): 1-(4-(2-hydroxyethyl)-1,5-dimethyl-1H-pyrazol-3-yl)-2,2-dimethylpropan-1-one**

**Procedure:** To a solution of 2-(1,5-dimethyl-3-pivaloyl-1H-pyrazol-4-yl)acetaldehyde (intermediate (7)) (2.7 g, 12.162 mmol) in ethanol (60.0 ml), sodium borohydride (0.460 g, 12.162 mmol) was added portion wise at ice cold condition. The reaction mixture was stirred at 0°C for 30 min. Upon the completion of the reaction, the mixture was quenched with sodium bicarbonate solution and diluted with ethyl acetate, washed with water, brine concentrated in vacuum to get crude. This batch was purified by combi flash using 2% MeOH in DCM to give 1-(4-(2-hydroxyethyl)-1,5-dimethyl-1H-pyrazol-3-yl)-2,2-dimethylpropan-1-one (8) (2.1 g, 76.98%) as a colorless oil. $^1$H NMR (400 MHz, DMSO) δ 4.46 (t, 1H), 3.77 (s, 3H) 3.36 (t, 2H), 2.69 (t, 2H), 2.17 (s, 3H), 1.30 (s, 9H); LCMS (HCOOH:CAN): M+H=225, Rt= 1.81 min in 3 mins run.

**Step 8 - Intermediate (9): tert-butyl ((6-(2-(2-(1,5-dimethyl-3-pivaloyl-1H-pyrazol-4-yl)ethoxy)-3,4-difluorophenyl)imidazo[1,2-a]pyridin-3-yl)methyl)(methyl)carbamate**

**Procedure:** To a stirred solution of 1-(4-(2-hydroxyethyl)-1,5-dimethyl-1H-pyrazol-3-yl)-2,2-dimethylpropan-1-one (intermediate (8)) (2.3 g, 10.268 mmol) and tert-butyl ((6-(3,4-difluoro-2-hydroxyphenyl)imidazo[1,2-a]pyridyl-3-yl) methyl)(methyl)carbamate (3.994 g, 10.268 mmol) in Toluene (40.0 ml) was added CMBP (5.382 ml, 20.536 mmol) at room temperature and the reaction mixture was stirred at 110°C for 16 hrs. TLC and LCMS were showed formation of product and the reaction mixture was diluted with ethyl acetate and washed with water, brine, dried over anhydrous sodium sulphate and concentrated under reduced pressure. The Crude was purified by combiflash using 5% MeOH-DCM to tert-butyl ((6-(2-(2-(1,5-dimethyl-3-pivaloyl-1H-pyrazol-4-yl)ethoxy)-3,4-difluorophenyl)imidazo[1,2-a]pyridin-3-yl)methyl)(methyl)carbamate (9) as a brown sticky gum (3.0 g, 49.05%). $^1$H NMR (400 MHz, DMSO) δ 8.50-8.42 (brs, 1H), 7.62 (s, 1H), 7.53 (d, 1H), 7.30-7.15 (m, 3H), 4.76 (s, 2H), 3.92 (t, 2H), 3.67 (s, 3H), 2.81 (t, 2H), 2.67 (s, 3H), 1.86 (s, 3H), 1.32 (s, 9H), 1.19 (s, 9H); LCMS (HCOOH:CAN): M+H=596, Rt= 1.75 min in 5 mins run.

**Step 9 - Intermediate (10): tert-butyl ((6-(3,4-difluoro-2-(2-(3-(1-hydroxy-2,2-dimethylpropyl)-1,5-dimethyl-1H-pyrazol-4-yl)ethoxy)phenyl)imidazo[1,2-a]pyridin-3-yl)methyl)(methyl)carbamate**

**Procedure:** To a solution of tert-butyl ((6-(2-(2-(1,5-dimethyl-3-pivaloyl-1H-pyrazol-4-yl)ethoxy)-3,4-difluorophenyl)imidazo[1,2-a]pyridyl-3-yl)methyl)(methyl)carbamate (intermediate (9)) (2.5 g, 4.202 mmol) in methanol (25.0 ml) was added lithium borohydride (0.458 g, 21.008 mmol) . The mixture was stirred at ambient temperature for 5 h. Upon the completion of the reaction, solvent was evaporated, diluted with DCM and washed with sodium bicarbonate solution, water, brine. Organic layer was dried over sodium sulphate and concentrated to get crude product. Crude product was purified by prep TLC using 5% MeOH in DCM to afford tert-butyl ((6-(3,4-difluoro-2-(2-(3-(1-hydroxy-2,2-dimethylpropyl)-1,5-dimethyl-1H-pyrazol-4-yl)ethoxy)phenyl)imidazo[1,2-a]pyridin-3-yl)methyl)(methyl)carbamate (10) (1.9 g, 75.66%). $^1$H NMR (400 MHz, DMSO) δ 8.55-8.45 (brs, 1H), 7.63 (s, 1H), 7.60 (d, 1H), 7.38 (d, 1H), 7.35-7.25 (brs, 2H), 4.78 (s, 2H), 4.60 (d, 1H), 4.07 (d, 1H), 3.92-3.88 (m, 1H), 3.84-3.78 (m, 1H), 3.52 (s, 3H), 2.80-2.70 (m, 1H), 2.68 (s, 3H), 2.60-2.52 (brs, 1H), 1.78 (s, 3H), 1.34 (s, 9H), 0.74 (s, 9H); LCMS (NH4Oac:CAN): M+H=598, Rt= 3.75 min in 5 mins run.

**Step 10 - NMT inhibitor 1: 1-(4-(2-(2,3-difluoro-6-(3-((methylamino)methyl)imidazo[1,2-a]pyridin-6-yl)phenoxy) ethyl)-1,5-dimethyl-1H-pyrazol-3-yl)-2,2-dimethylpropan-1-ol**

**Procedure:** To a solution of tert-butyl ((6-(3,4-difluoro-2-(2-(3-(1-hydroxy-2,2-dimethylpropyl)-1,5-dimethyl-1H-pyrazol-4-yl)ethoxy)phenyl)imidazo[1,2-a]pyridin-3-yl)methyl)(methyl)carbamate (10) (1.2 g, 2.009 mmol) in diethyl ether (10.0 ml) was added 2M HCl in diethyl ethyl ether (40.0 ml) at 0°C. Reaction mixture was stirred at rt for 3h. TLC and LCMS showed starting material was consumed. Reaction mixture was evaporated under reduce pressure to get crude. Crude was triturated with diethyl ether and lyophilized to get 1-(4-(2-(2,3-difluoro-6-(3-((methylamino)methyl)imidazo[1,2-a] pyridin-6-yl)phenoxy)ethyl)-1,5-dimethyl-1H-pyrazol-3-yl)-2,2-dimethylpropan-1-ol as light yellow solid (HCl salt) (1.04 g, 96.93 mmol, 49%). $^1$H NMR (400 MHz, DMSO) δ 10.02-9.96 (brs, 2H), 9.26 (s, 1H), 8.44 (s, 1H) 8.08 (d, 1H), 7.98 (d, 1H), 7.64 (t, 1H), 7.46-7.40 (m, 1H), 4.75 (s, 3H), 4.16 (s, 1H), 3.97 (t, 2H), 3.71 (s, 3H), 2.87-2.80 (m, 1H), 2.72-2.65 (m, 1H), 2.60 (s, 3H), 2.09 (s, 3H), 0.76 (s, 9H); LCMS (HCOOH:CAN): M+H=498, Rt= 2.54 min in 5 mins run; HPLC RT(B3) 8.739 min.

The following compounds were made by using analogous methods to those described for NMT inhibitor 1:

Table 1: NMT inhibitors

| NMT inhibitor | Chemical Name | Structure | Characterising data |
|---|---|---|---|
| 2 | 1-{4-[2-(5-fluoro-2-{3-[(methylamino)methyl] imidazo[1,2-a]pyridin-6-yl}phenoxy)ethyl]-1,5-dimethyl-1H-pyrazol-3-yl}ethan-1-ol | 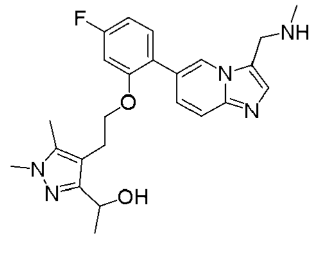 | $^1$H NMR (d6-DMSO, 400 MHz) δ 9.54 (s, 2H), 9.16 (s, 1H), 8.37 (s, 1H), 8.11 (d, 1H), 7.98 (d, 1H), 7.69 (t, 1H), 7.15 (d, 1H), 6.99 (t, 1H), 4.73 (t, 2H), 4.68-4.62 (m, 2H), 4.13 (t, 3H), 3.61 (s, 3H), 2.87-2.82 (m, 2H), 2.64 (t, 2H), 2.01 (s, 3H), 1.29 (d, 3H). |

(continued)

| NMT inhibi- tor | Chemical Name | Structure | Characterising data |
|---|---|---|---|
| 3 | 1-(4-(2-(2-(3-((dimethylamino) meth yl)imidazo[1,2-a]pyridin-6-yl)-5-fluorophenoxy)ethyl)-1,5-dimethyl-1H-pyrazol-3-yl)ethan-1-ol | 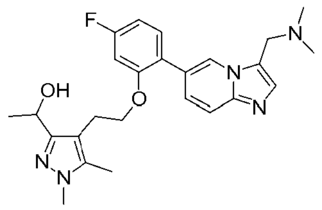 | $^1$H NMR (d6-DMSO, 400 MHz) δ 8.48 (s, 1H), 7.56 (d, 1H), 7.50 (s, 1H), 7.42 (t, 1H), 7.32 (d, 1H), 7.03 (d, 1H), 6.88 (t, 1H), 4.84 (d, 1H), 4.66 (t, 1H), 4.08 (t, 2H), 3.72 (s, 2H), 3.54 (s, 3H), 2.89-2.79 (m, 2H), 2.14 (s, 6H), 1.87 (s , 3H), 1.29 (d, 3H). |
| 4 | (isomer 1) 1-(4-(2-(2-(3-((dimethylamino)meth yl)imidazo[1,2-a]pyridin-6-yl)-5-fluorophenoxy) ethyl)-1,5-dimethyl-1H-pyrazol-3-yl)ethan-1-ol | 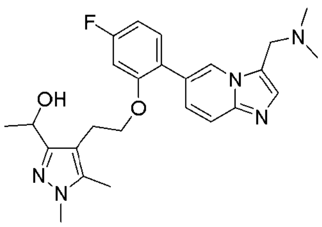 | $^1$H NMR (d6-DMSO, 400 MHz) δ 9.54 (s, 2H), 9.16 (s, 1H), 8.37 (s, 1H), 8.11 (d, 1H), 7.98 (d, 1H), 7.69 (t, 1H), 7.15 (d, 1H), 6.99 (t, 1H), 4.73 (t, 2H), 4.68-4.62 (m, 2H), 4.13 (t, 3H), 3.61 (s, 3H), 2.87-2.82 (m, 2H), 2.64 (t, 2H), 2.01 (s, 3H), 1.29 (d, 3H). |
| 5 | (isomer 2) 1-(4-(2-(2-(3-((dimethylamino)meth yl)imidazo[1,2-a]pyridin-6-yl)-5-fluorophenoxy) ethyl)-1,5-dimethyl-1H-pyrazol-3-yl)ethan-1-ol | 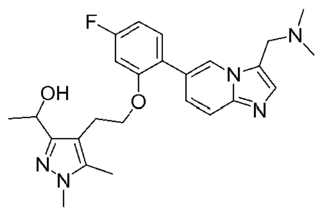 | |
| 6 | 1-{4-[2-(2,3-difluoro-6-{3-[(methylamino)methyl] imidazo[1,2-a]pyridin-6-yl}phenoxy)ethyl]-1,5-dimethyl-1H-pyrazol-3-yl}ethan-1-ol | 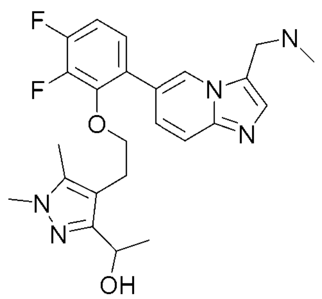 | $^1$H NMR (d6-DMSO, 400 MHz) δ 9.95-9.80 (brs, 2H), 9.22 (s, 1H), 8.42 (s, 1H), 8.00 (d, 1H), 7.92 (d, 1H), 7.62 (t, 1H), 7.44-7.37 (m, 1H), 4.72 (s, 2H), 4.57-4.51 (m, 2H), 4.03 (t, 2H), 3.60 (s, 3H), 2.75-2.66 (m, 2H), 2.61 (s, 3H), 2.00 (s, 3H), 1.19 (d, 3H). |
| 7 | 1-(4-(2-(6-(3-((dimethylamino) methyl)imidazo[1,2-a]pyridin-6-yl)-2,3-difluorophenoxy)ethyl)-1,5-dimethyl-1H-pyrazol-3-yl)ethan-1-ol | 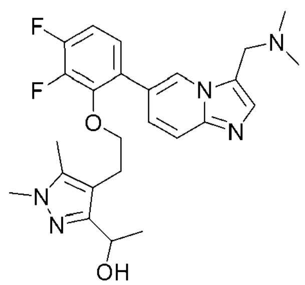 | $^1$H NMR (400 MHz, DMSO-*$d_6$*) d 8.49 (s, 1H), 7.56 (d, 1H), 7.52 (s, 1H), 7.37-7.25 (m, 3H), 4.77-4.67 (m, 1H), 4.61-4.51 (m, 1H), 3.95 (t, 2H), 3.73 (s, 2H), 3.51 (s, 3H), 2.73 (t, 2H), 2.14 (s, 6H), 1.79 (s, 3H), 1.21 (d, 3H). |
| 8 | 1-(4-(2-(2-(3-(2-aminoethyl)imidazo[1, 2-a]pyridin-6-yl)-5-fluorophenoxy)ethyl)-1,5-dimethyl-1H-pyrazol-3-yl)ethan-1-ol | 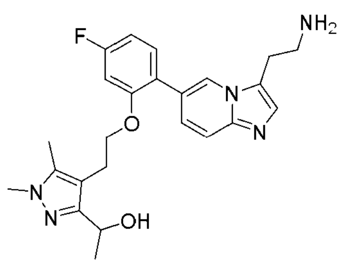 | $^1$H NMR (d6-DMSO, 400 MHz) δ 8.95 (s, 1H), 8.19 (brs, 3H), 8.15 (s, 1H), 8.07 (d, 1H), 8.00 (d, 1H), 7.55 (t, 1H), 7.14 (d, 1H), 6.96 (t, 1H), 4.66 (s, 1H), 4.13 (t, 2H), 3.62 (s, 3H), 3.44 (t, 2H), 3.19 (s, 2H), 2.88-2.81 (m, 2H), 2.02 (s, 3H), 1.28 (d, 3H). |

(continued)

| NMT inhibitor | Chemical Name | Structure | Characterising data |
|---|---|---|---|
| 9 | 1-(4-(2-(5-fluoro-2-(3-((methylamino)methyl) imidazo[1,2-a]pyridin-6-yl)phenoxy)ethyl)-1,5-dimethyl-1H-pyrazol-3-yl)-2,2-dimethylpropan-1-ol | 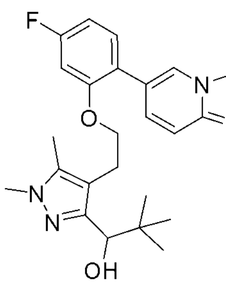 | $^1$H NMR (400 MHz, DMSO-$d_6$) d 10.06-9.65 (m, 2H), 9.20 (s, 1H), 8.42 (s, 1H), 8.17 (d, 1H), 8.01 (d, 1H), 7.75 (t, 1H), 7.17 (d, 1H), 6.99 (t, 1H), 4.88 (s, 2H), 4.26 (s, 1H), 4.18-4.04 (m, 2H), 3.69 (s, 3H), 3.01-2.91 (m, 1H), 2.83-2.72 (m, 1H), 2.62 (s, 3H), 2.07 (s, 3H), 0.86 (s, 9H). |
| 10 | (isomer 1) 1-(4-(2-(5-fluoro-2-(3-((methylamino)methyl) imidazo[1,2-a]pyridin-6-yl)phenoxy) ethyl)-1,5-dimethyl-1H-pyrazol-3-yl)-2,2-dimethylpropan-1-ol | 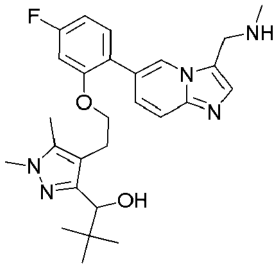 | $^1$H NMR (400 MHz, DMSO-$d_6$) d 9.79 (bs, 2H), 9.20 (s, 1H), 8.41 (s, 1H), 8.17 (d, 1H), 8.01 (d, 1H), 7.74 (t, 1H), 7.19-7.14 (m, 1H), 7.02-6.94 (m, 1H), 4.74 (bs, 2H), 4.25 (s, 1H), 4.18-4.07 (m, 2H), 3.68 (s, 3H), 2.99-2.89 (m, 1H), 2.81-2.71 (m, 1H), 2.62 (t, 3H), 2.06 (s, 3H), 0.85 (s, 9H). |
| 11 | (isomer 2) 1-(4-(2-(5-fluoro-2-(3-((methylamino)methyl) imidazo[1,2-a]pyridin-6-yl)phenoxy) ethyl)-1,5-dimethyl-1H-pyrazol-3-yl)-2,2-dimethylpropan-1-ol | 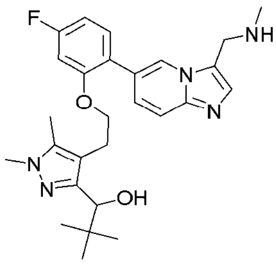 | |
| 12 | 1-(4-(2-(2-(3-((dimethylamino) meth yl)imidazo[1,2-a]pyridin-6-yl)-5-fluorophenoxy)ethyl)-1,5-dimethyl-1H-pyrazol-3-yl)-2,2-dimethylpropan-1-ol | 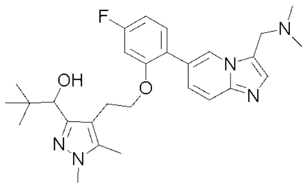 | $^1$H NMR (400 MHz, DMSO-$d_6$) d 8.50 (s, 1H), 7.57 (d, 1H), 7.50 (s, 1H), 7.43 (t, 1H), 7.34 (d, 1H), 7.05 (d, 1H), 6.88 (t, 1H), 4.85 (d, 1H), 4.22 (d, 1H), 4.13-3.98 (m, 2H), 3.73 (s, 2H), 3.56 (s, 3H), 3.01-2.89 (m, 1H), 2.77-2.67 (m, 1H), 2.15 (s, 6H), 1.90 (s, 3H), 0.87 (s, 9H). |
| 13 | (isomer 1): 1-(4-(2-(2,3-difluoro-6-(3-((methylamino)methyl) imidazo[1,2-a]pyridin-6-yl)phenoxy) ethyl)-1,5-dimethyl-1H-pyrazol-3-yl)-2,2-dimethylpropan-1-ol | 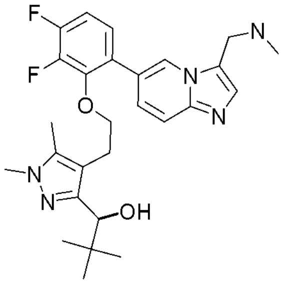 | $^1$H NMR (400 MHz, DMSO-$d_6$) d 9.83 (bs, 2H), 9.23 (s, 1H), 8.42 (s, 1H), 8.07 (d, 1H), 7.97 (d, 1H), 7.62 (t, 1H), 7.47-7.38 (m, 1H), 4.74 (s, 2H), 4.11 (s, 1H), 3.97 (t, 2H), 3.63 (s, 3H), 2.88-2.78 (m, 1H), |

(continued)

| NMT inhibitor | Chemical Name | Structure | Characterising data |
|---|---|---|---|
| 14 | (isomer 2): 1-(4-(2-(2,3-difluoro-6-(3-((methylamino)methyl) imidazo[1,2-a]pyridin-6-yl)phenoxy) ethyl)-1,5-dimethyl-1H-pyrazol-3-yl)-2,2-dimethylpropan-1-ol | 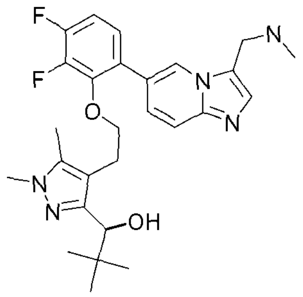 | 2.73-2.52 (m, 4H), 2.04 (s, 3H), 0.76 (s, 9H). |
| 15 | (isomer 1) 1-(4-(2-(6-(3-((dimethylamino)meth yl)imidazo[1,2-a]pyridin-6-yl)-2,3-difluorophenoxy) ethyl) -1,5-dimethyl-1H-pyrazol-3-yl)-2,2-dimethylpropan-1-ol | 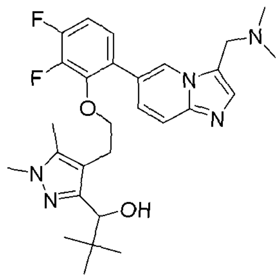 | $^1$H NMR (400 MHz, DMSO-*$d_6$)* d 8.53 (s, 1H), 7.58 (s, 1H), 7.52 (s, 1H), 7.38 (d, 1H), 7.32-7.24 (m, 2H), 4.635 (d, 1H), 4.08 (d, 1H), 3.99-3.91 (m, 1H), 3.88-3.82 (m, 1H), 3.74 (s, 2H), 3.53 (s, 3H), 2.88-2.75 (m, 1H), 2.69-2.59 (m, 1H), 2.14 (s, 6H), 1.80 |
| 16 | (isomer 2) 1-(4-(2-(6-(3-((dimethylamino)meth yl)imidazo[1,2-a]pyridin-6-yl)-2,3-difluorophenoxy) ethyl)-1,5-dimethyl-1H-pyrazol-3-yl)-2,2-dimethylpropan-1-ol | 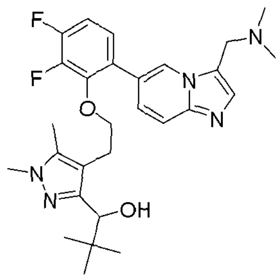 | (s, 3H), 0.76 (s, 9H). |
| 17 | 1-(4-(2-(6-(3-(aminomethyl)imidazo [1,2-a]pyridin-6-yl)-2,3-difluorophenoxy)ethyl) -1,5-dimethyl-1H-pyrazol-3-yl)-2,2-dimethylpropan-1-ol | 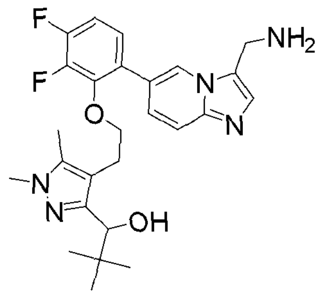 | $^1$H NMR (d6-DMSO, 400 MHz) δ 9.18 (s, 1H), 8.95-8.77 (m, 3H), 8.35 (s, 1H), 8.08 (d, 1H), 7.97 (d, 1H), 7.58 (t, 1H), 7.48-7.35 (m, 1H), 4.77 (s, 2H), 4.12 (s, 1H), 4.03-3.94 (m, 2H), 3.63 (s, 3H), 2.91-2.78 (m, 1H), 2.72-2.61 (m, 1H), 2.03 (s, 3H), 0.77(s, 9H). |
| 18 | 2-(4-(2-(5-fluoro-2-(3-((methylamino)methyl) imidazo[1,2-a]pyridin-6-yl)phenoxy)ethyl)-1,5-dimethyl-1H-pyrazol-3-yl)propan-2-ol | 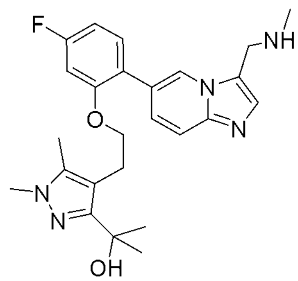 | $^1$H NMR (d6-DMSO, 400 MHz) δ 8.51 (s, 1H), 7.54 (d, 1H), 7.47 (s, 1H), 7.43 (t, 1H), 7.32 (d, 1H) 7.04 (d, 1H), 6.87 (t, 1H), 4.80 (s, 1H), 4.11 (t, 2H), 3.98 (s, 2H), 3.54 (s, 3H), 2.94 (t, 2H), 2.25 (s, 3H), 1.87 (s, 3H), 1.40 (s, 6H). |

(continued)

| NMT inhibitor | Chemical Name | Structure | Characterising data |
|---|---|---|---|
| 19 | 2-(4-(2-(2-(3-((dimethylamino) meth yl)imidazo[1,2-a]pyridin-6-yl)-5-fluorophenoxy)ethyl)-1,5-dimethyl-1H-pyrazol-3-yl)propan-2-ol | 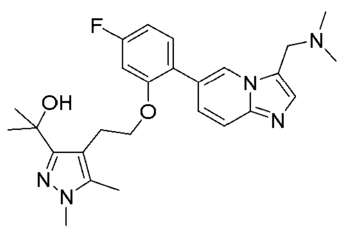 | $^1$H NMR (d6-DMSO, 400 MHz) δ 8.49 (s, 1H), 7.56 (d, 1H), 7.50 (s, 1H), 7.42 (t, 1H), 7.33 (d, 1H), 7.04 (d, 1H), 6.88 (t, 1H), 4.80 (s, 1H), 4.10 (t, 2H), 3.73 (s, 2H), 3.53 (s, 3H), 2.94 (t, 2H), 2.15 (s, 6H), 1.87 (s, 3H), 1.39 (s, 6H). |
| 20 | 2-[4-(2-{6-[3-(aminomethyl)imidazo [1,2-a]pyridin-6-yl]-2,3-difluorophenoxy}ethyl) -1,5-dimethyl-1H-pyrazol-3-yl]propan-2-ol | 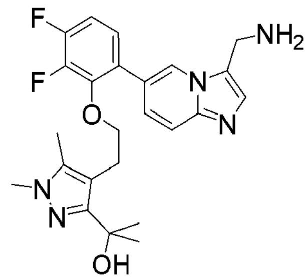 | $^1$H NMR (d6-DMSO, 400 MHz) δ 8.54 (s, 1H), 7.55 (d,1H), 7.47 (s, 1H) 7.31 (m, 3H), 4.66 (brds, 1H) 4.07 (s, 2H), 3.95 (t, 2H), 2.83 (m, 2H), 2.40 (brds,1H), 1.81 (s, 3H), 1.28(s, 6H). |
| 21 | 2-(4-(2-(2,3-difluoro-6-(3-((methylamino)methyl) imidazo[1,2-a]pyridin-6-yl)phenoxy)ethyl)-1,5-dimethyl-1H-pyrazol-3-yl)propan-2-ol | 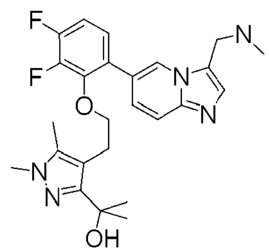 | $^1$H NMR (d6-DMSO, 400 MHz) δ 8.52 (s, 1H), 7.55 (d, 1H), 7.49 (s, 1H), 7.35-7.25 (m, 3H), 4.64 (s, 1H), 3.99 (s, 2H), 3.95 (d, 2H), 3.49 (s, 3H), 2.83 (t, 2H), 2.24 (s, 3H), 1.79 (s, 3H), 1.28 (s, 6H). |
| 22 | 2-{4-[2-(2-{3-[(ethylamino)methyl]imidazo[1,2-a]pyridin-6-yl}-5-fluorophenoxy)ethyl]-1,5-dimethyl-1H-pyrazol-3-yl}propan-2-ol | 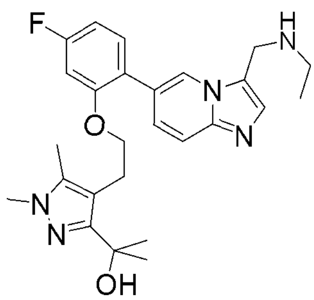 | $^1$H NMR (d6-DMSO, 400 MHz) δ 8.51 (s, 1H), 7.54 (d, 1H), 7.48 (s, 1H), 7.43 (t, 1H), 7.32 (d, 1H) 7.05 (d, 1H), 6.878 (t, 1H), 4.80 (s, 1H), 4.12 (t, 2H), 4.07 (s, 2H), 3.54 (s, 3H), 2.94 (t, 2H), 2.55 (q,2H) 2.26(s, 3H), 1.87 (s , 3H), 1.40 (s, 6H), 1.01(t, 3H). |
| 23 | 2-(4-(2-(2-(3-(2-aminoethyl)imidazo[1, 2-a]pyridin-6-yl)-5-fluorophenoxy)ethyl)-1,5-dimethyl-1H-pyrazol-3-yl)propan-2-ol | 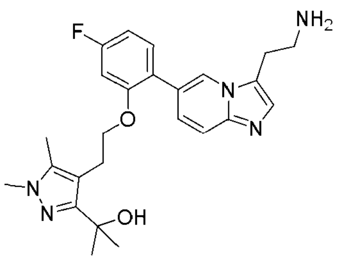 | $^1$H NMR (d6-DMSO, 400 MHz) δ 8.41 (s, 1H), 7.52 (d, 1H), 7.44-7.41 (m, 2H), 7.28 (d, 1H), 7.06 (d, 1H), 6.86 (t, 1H), 4.89-4.85 (brs, 1H), 4.11 (t, 2H), 3.54 (s, 3H), 2.97-2.87 (m, 6H), 1.90 (s, 3H), 1.40 (s, 6H). |

(continued)

| NMT inhibitor | Chemical Name | Structure | Characterising data |
|---|---|---|---|
| 24 | 1-(4-(2-(2-(3-((dimethylamino) meth yl)imidazo[1,2-a]pyridin-6-yl)-5-fluorophenoxy)ethyl)-1,5-dimethyl-1H-pyrazol-3-yl)-2-methylpropan-1-ol | 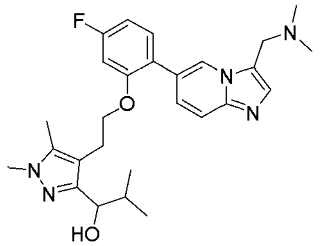 | $^1$H NMR (d6-DMSO, 400 MHz) δ 8.46 (s, 1H), 7.53 (d, 1H), 7.47 (s, 1H), 7.40 (t, 1H), 7.30 (d, 1H), 7.01 (d, 1H), 6.85 (t, 1H), 4.82 (d, 1H), 4.11-3.94 (m, 3H), 3.70 (s, 2H), 3.53 (s, 3H), 2.88-2.71 (m, 2H), 2.12 (s, 6H), 1.98-1.81 (m, 4H), 0.91 (d, 3H), 0.63 (d, 3H). |
| 25 | 1-{4-[2-(2,3-difluoro-6-{3-[(methylamino)methyl] imidazo[1,2-a]pyridin-6-yl}phenoxy)ethyl]-1,5-dimethyl-1H-pyrazol-3-yl}-2-methylpropan-1-ol | 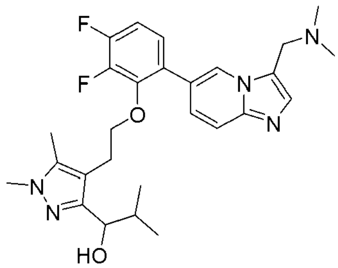 | $^1$H NMR (d6-DMSO, 400 MHz) δ 8.55 (s, 1H), 7.56 (d, 1H), 7.51(s, 1H), 7.31(m, 3H), 4.72 (s, 1H), 4.03(s,2H), 3.94 (m,2H), 3.88 (m, 1H), 3.53 (s, 3H) , 2.72 (t, 2H), 2.26 (s, 3H), 0.84 (d, 3H), 0.52 (d, 3H). |

NMT inhibitors 1 to 25 may be prepared according to methods disclosed in EP patent application no. 22194959.7, the entire contents of which are incorporated by reference for the purpose of describing the synthesis and activity of NMT inhibitors.

**Synthesis of Drug Conjugate** 1: {4-[(2S)-5-(carbamoylamino)-2-[(2S)-2-[6-(2,5-dioxo-2,5-dihydro-1H-pyrrol-1-yl)hexanamido]-3-methylbutanamido]pentanamido]phenyl}methyl N-{[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl}-N-methylcarbamate:

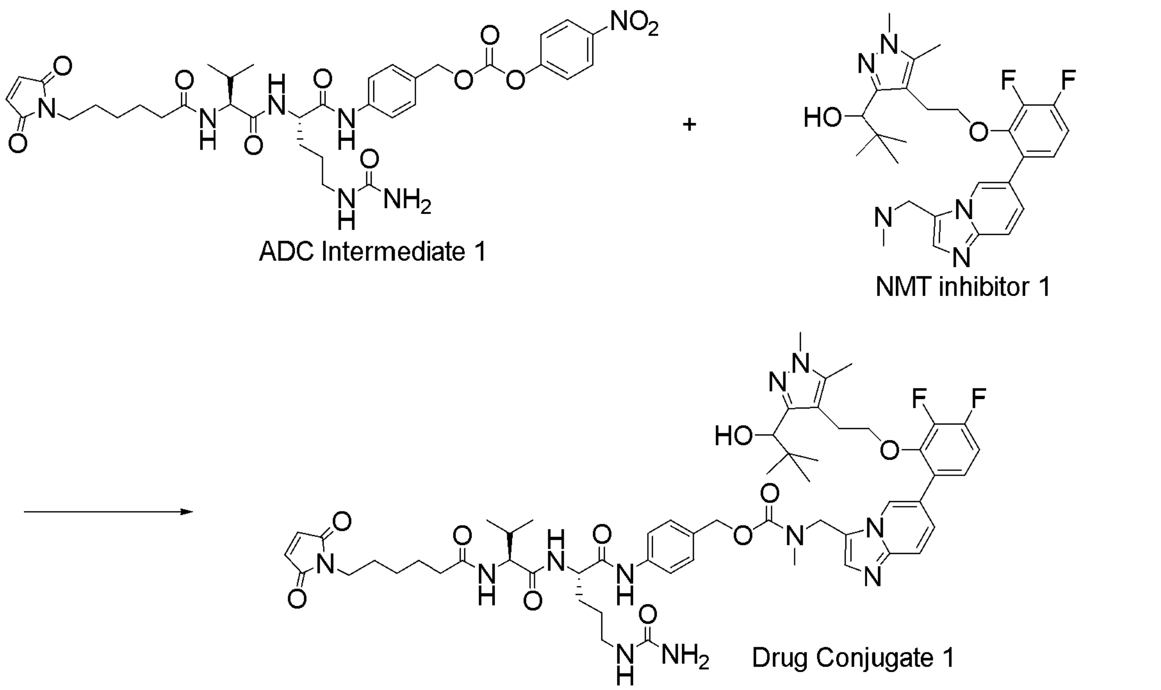

To a solution of {4-[(2S)-5-(carbamoylamino)-2-[(2S)-2-[6-(2,5-dioxo-2,5-dihydro-1H-pyrrol-1-yl)hexanamido]-3-methylbutanamido]pentanamido]phenyl}methyl 4-nitrophenyl carbonate hydrate (ADC Intermediate 1, prepared from commercially available (N-[(1S)-1-{[(1S)-4-(carbamoylamino)-1-{[4-(hydroxymethyl)phenyl]carbamoyl}butyl]carbamoyl}-2-methylpropyl]-6-(2,5-dioxo-2,5-dihydro-1H-pyrrol-1-yl)hexanamide using standard carbonate formation conditions as described in Synlett, 2009, No. 18, pp 3050-3051, 12 mg, 16 mmol) in anhydrous DMF (2 mL) was added NMT inhibitor 1 (8 mg, 16 mmol), followed by DIEA (8.4 mL) and HOAt (1 mg), and the reaction was stirred at room temperature (22 °C). After 16 h, the mixture was purified directly by RP-HPLC to give {4-[(2S)-5-(carbamoylamino)-2-[(2S)-2-[6-(2,5-dioxo-2,5-dihydro-1H-pyrrol-1-yl)hexanamido]-3-methylbutanamido]pentanamido]phenyl}methyl N-{[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl] methyl}-N-methylcarbamate (Drug Conjugate 1) as a white solid after lyophilization (10.5 mg). Drug conjugate 1 may be used to prepare an ADC by reaction with an antibody using methods known to the skilled person and described herein, such as those described for ADC Example 1 or ADC Example 2.

**Synthesis of Drug Conjugate 2:** (1S,2R,3S,4R,5R)-5-(4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3yl]methyl}(methyl)carbamoyl)oxy] methyl}-2-[3-(3-{2-[2-(2,5-dioxo-2,5-dihydro-1H-pyrrol-1-yl)ethoxy]ethoxy}propanamido)propanamido]phenoxy)-3,4-dihydroxy-2-methylcyclohexane-1-carboxylic acid (as hydrate)

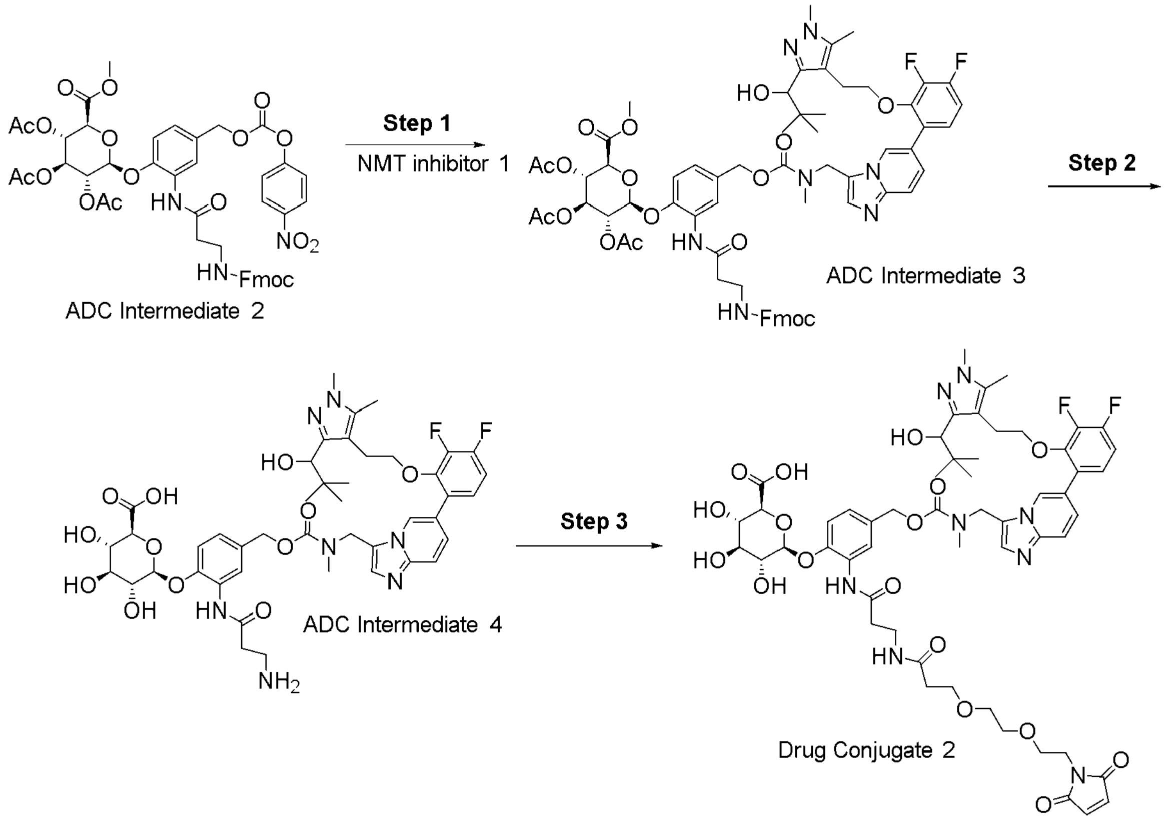


ADC Intermediate 2

ADC Intermediate 3

ADC Intermediate 4

Drug Conjugate 2

**Step** 1: To a solution of (1S,2R,3S,4R,5R)-5-[2-(3-{[(9H-fluoren-9-yl oxy)carbonyl]amino}propanamido)-4-({[(4-nitrophenoxy)carbonyl]oxy}methyl)phenoxy]-2,3,4-trihydroxycyclohexane-1-carboxylic acid (ADC Intermediate 2, 100 mg, 0.11 mmol) (Bioconjugate Chem., 2006, 17, 831-840) in anhydrous DMF (2 mL) was added NMT inhibitor 1 (50 mg), followed by DIEA (40 mL) and HOAt (3 mg), and the reaction was stirred at room temperature (22 °C). After 16 h, the mixture was purified directly by RP-HPLC to give (1S,2R,3S,4R,5R)-5-(4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl}(methyl)carbamoyl)oxy] methyl}-2-(3-{[(9H-fluoren-9-yloxy)carbonyl]amino}propanamido)phenoxy)-2,3,4-trihydroxycyclohexane-1-carboxylic acid (ADC Intermediate 3) as a white solid after lyophilization (107 mg).

**Step 2:** (1S,2R,3S,4R,5R)-5-(4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl} (methyl)carbamoyl)oxy]methyl}-2-(3-{[(9H-fluoren-9-yloxy)carbonyl]amino}propanamido) phenoxy)-2,3,4-trihydroxycyclohexane-1-carboxylic acid (ADC Intermediate 3,

105 mg) was dissolved in acetonitrile/water (6/4, v/v, 4 mL), and NaOH (1N, aq., 0.5 mL) was added dropwise at room temperature. The mixture was stirred at room temperature for 8 h. HCl (4 N in dioxane, 0.1 mL) was added, and the mixture was purified by RP-HPLC to give (1S,2R,3S,4R,5R)-5-[2-(3-aminopropanamido)-4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl}(methyl)carbamoyl)oxy] methyl}phenoxy]-2,3,4-trihydroxycyclohexane-1-carboxylic acid (ADC Intermediate 4) as a white solid (TFA salt, 42 mg) after lyophilization.

**Step 3:** To a solution of (1S,2R,3S,4R,5R)-5-[2-(3-aminopropanamido)-4-{[({[6-(3,4-difluoro-2-{2-[3-(1-hydroxy-2,2-dimethylpropyl)-1,5-dimethyl-1H-pyrazol-4-yl]ethoxy}phenyl)imidazo[1,2-a]pyridin-3-yl]methyl}(methyl)carbamoyl)oxy]methyl}phenoxy]-2,3,4-trihydroxycyclohexane-1-carboxylic acid (ADC Intermediate 4, 40 mg) in acetonitrile/-water (6/4, v/v, 2 mL) was added Mal-PEG2-OSu (15 mg), followed by DIEA (14 mL). The reaction mixture was stirred at room temperature for 1 h, and purified directly by RP-HPLC to give (1S,2R,3S,4R,5R)-5-(4-{[({[6-(3,4-difluoro-2-{2-[3- (1-hydroxy-2,2-dimethylpropyl)-1,5-dimethyl-1H-pyrazol-4-yl] ethoxy}phenyl)imidazo[1,2-a]pyridin-3-yl]methyl}(methyl) carbamoyl)oxy]methyl}-2-(3-{[(9H-fluoren-9-yloxy)carbonyl]amino}propanamido)phenoxy)-2,3,4-trihydroxycyclohexane-1-carboxylic acid hydrate (Drug Conjugate 2) as a white solid after lyophilization (36 mg).

**Synthesis of Drug Conjugate 3**

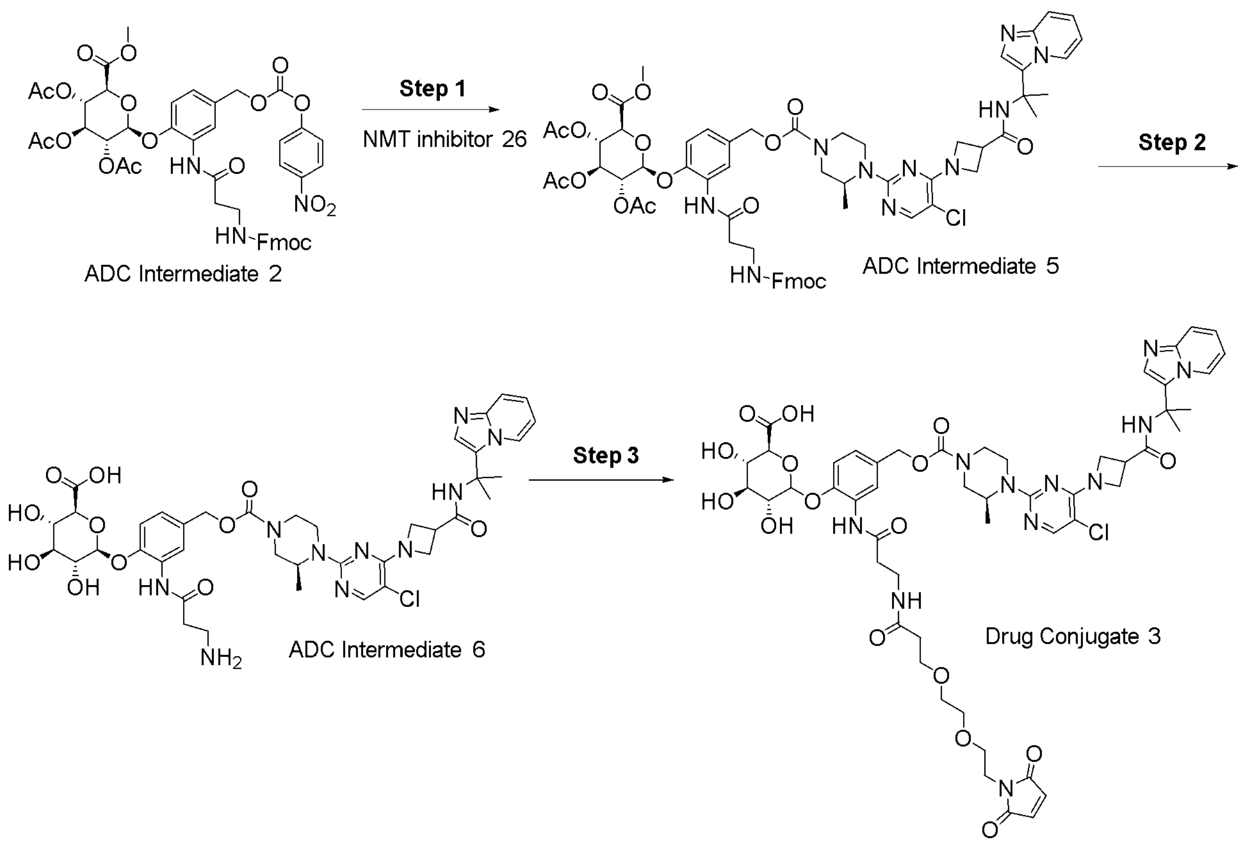


ADC Intermediate 2

ADC Intermediate 5

ADC Intermediate 6

Drug Conjugate 3

**Step 1:** To a solution of ADC Intermediate 2 (Bioconjugate Chem., 2006, 17, 831-840) in anhydrous DMF was added NMT inhibitor 26, followed by DIEA and HOAt, and the reaction was stirred at room temperature (22 °C). After 16 h, the mixture was purified directly by RP-HPLC to give ADC Intermediate 5 as a white solid after lyophilization.

**Step 2:** ADC Intermediate 5 was dissolved in acetonitrile/water (6/4, v/v), and NaOH (1N, aq.) was added dropwise at room temperature. The mixture was stirred at room temperature for 8 h. HCl (4 N in dioxane) was added, and the mixture was purified by RP-HPLC to give (ADC Intermediate 6) as a white solid after lyophilization.

**Step 3:** To a solution of ADC Intermediate 6 in acetonitrile/water (6/4, v/v) was added Mal-PEG2-OSu, followed by DIEA. The reaction mixture was stirred at room temperature for 1 h, and purified directly by RP-HPLC to give Drug Conjugate 3 as a white solid after lyophilization.

**Preparation of ADC Example 1 - trastuzumab-NMT inhibitor ADC (DAR 5)**

Trastuzumab was purchased and reconstituted to yield a 25mg/mL solution. 5% v/v of 500mM Tris, 25mM EDTA, pH 8.5 was added to adjust the pH prior to reduction and conjugation. 2.5 molar equivalents of TCEP (tris(2-carboxyethyl) phosphine) relative to antibody was added from a 10mM stock in water and the antibody left to reduce for 90 minutes. 8 molar equivalents of Drug Conjugate 2 was added from a 10mM stock in DMA (dimethylacetamide) and the reduced antibody allowed to conjugate for 60 minutes. 8 molar equivalents of NAC (N-acetylcysteine) was added from a 10mM stock in water to quench unreacted Drug Conjugate 2 and allowed to react for 20 minutes. The conjugate was purified by preparative SEC (size exclusion chromatography) using a Superdex 200PG column equilibrated in PBS. The protein containing fractions were pooled and terminally filtered through a suitably sized 0.2μm PES filter (chromatography direct / FIL-S-PES-022-13-100-S) under grade A laminar flow. The final product was sampled for QC testing - monomer and [ADC] mg/ml by SEC-HPLC, average DAR by PLRP, Residual NMT inhibitor 1 by RP-HPLC, and endotoxin by Endosafe kinetic chromogenic.

**Preparation of ADC Example 2 - Rituximab-NMT inhibitor ADC (DAR 5)**

Rituximab was purchased as a 10mg/mL solution. 5% v/v of 500mM Tris, 25mM EDTA, pH 8.5 was added to adjust the pH prior to reduction and conjugation. 2.7 molar equivalents of TCEP (tris(2-carboxyethyl)phosphine) relative to antibody was added from a 10mM stock in water and the antibody left to reduce for 120 minutes. 8 molar equivalents of Drug Conjugate 2 were added from a 10mM stock in DMA (dimethylacetamide) and the reduced antibody allowed to conjugate for 60 minutes. 8 molar equivalents of NAC (N-acetylcysteine) were added from a 10mM stock in water to quench unreacted Drug Conjugate 2 and allowed to react for 20 minutes. The conjugate was purified by preparative SEC (size exclusion chromatography) using a Superdex 200PG column equilibrated in 30 mM Histidine, 0.25 M Sucrose 100mM NaCl. The protein containing fractions were pooled and terminally filtered through a suitably sized 0.2μm PES filter (chromatography direct / FIL-S-PES-022-13-100-S) under grade A laminar flow. The final product was sampled for QC testing - monomer and [ADC] mg/ml by SEC-HPLC, average DAR by PLRP, Residual NMT inhibitor 1 by RP-HPLC, and endotoxin by Endosafe kinetic chromogenic assay.

ADC Example 3 was prepared using the same method as described for ADC Example 1 except sacituzumab was used as the antibody instead of trastuzumab.

ADC Example 4 was prepared using the same method as described for ADC Example 1 except ifinatamab was used as the antibody instead of trastuzumab.

ADC Example 5 was prepared using the same method as described for ADC Example 1 except Drug Conjugate 3 was used instead of Drug Conjugate 2.

**Preparation of ADC Example 6**

ADC Example 6 was prepared using Drug Conjugate 4. Drug Conjugate 4 was prepared using the same method as Drug Conugate 2 except that NMT inhibitor 21 was used instead of NMT inhibitor 1:

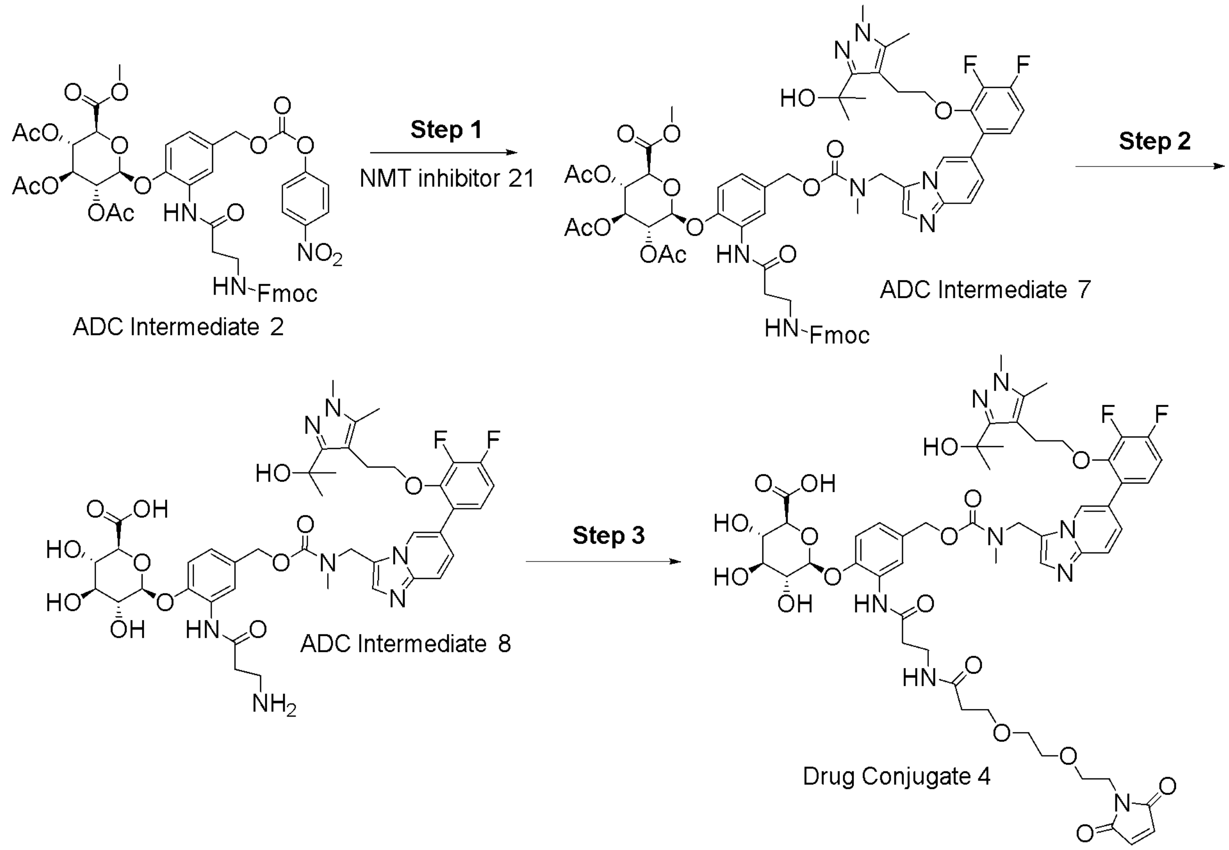


ADC Intermediate 2

ADC Intermediate 7

ADC Intermediate 8

Drug Conjugate 4

wherein Steps 1 to 3 are as described for Drug Conjugate 2.

Herceptin (Trastuzumab) was purchased and reconstituted to yield a 25.6mg/mL solution. 5% v/v of 500mM Tris, 25mM EDTA, pH 8.5 was added to adjust the pH prior to reduction and conjugation. 2.55 molar equivalents of TCEP (tris(2-carboxyethyl)phosphine) relative to antibody was added from a 5mM stock in water and the antibody was left to reduce for 120 minutes. The reduced mAb was diluted 1/3 with PBS prior to conjugation. 8 molar equivalents of Drug Conjugate 4 was added from a 10mM stock in DMA (dimethylacetamide) and the reduced antibody was allowed to conjugate for 90 minutes. 8 molar equivalents of NAC (N-acetylcysteine) was added from a 100mM stock in water to quench unreacted Drug Conjugate 4 and allowed to react for 20 minutes. The conjugate was buffer exchanged into PBS using G25 Resin (NAP25 columns) and then dosed with activated carbon at a 1mg Carbon: 1mg ADC ratio and allowed to incubate overnight on a roller mixer at 10rpm at room temperature. The conjugate was then spun down at 4000xG for 15 minutes to pellet the carbon and the supernatant (ADC) was removed and filtered through a 0.2uM PES filter. The conjugate was then further purified and concentrated via diafiltration using Amicon15 devices where 6x Diafiltration Volumes (DV's) of PBS, pH 7.4 were used in the buffer exchange. The conjugate was then terminally filtered through a 13mm 0.2μm PES filter (chromatography direct / FIL-S-PES-022-13-100-S) under grade A laminar flow and then formulated to 0.02% PS80. The final product was sampled for QC testing - monomer and [ADC] mg/ml by SEC HPLC, average DAR by PLRP, Residual toxin by RP-HPLC, and endotoxin by Endosafe.

**Preparation of ADC Example 7**

ADC Example 7 was prepared using the same method as described for ADC Example 1, except Drug Conjugate 5 was used wherein the linker used was GGFG.

Structure of Drug Conjugate 5:

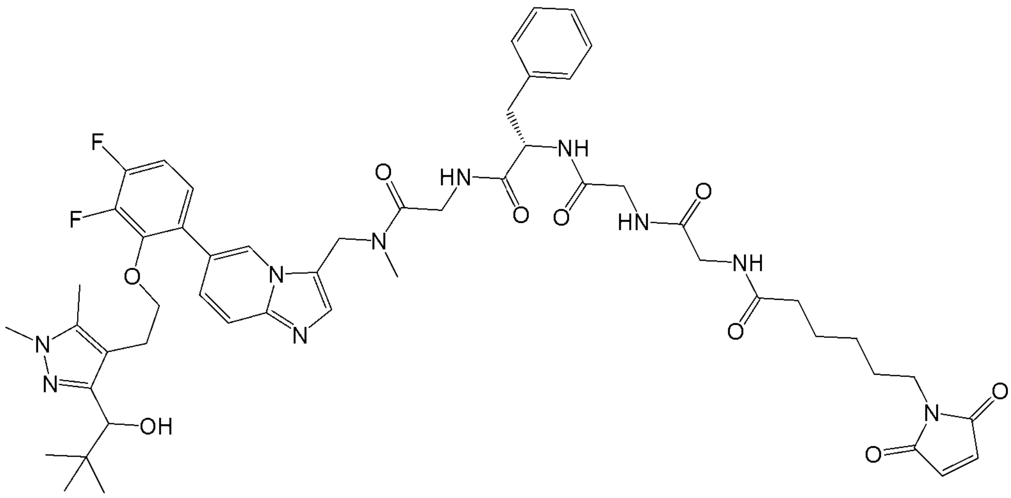

which may be prepared using the same method as Drug Conjugate 2.

**Preparation of ADC Example 8**

Herceptin (trastuzumab) was purchased and reconstituted to yield a 25.6mg/mL solution. 5% v/v of 500mM Tris, 25mM EDTA, pH 8.5 was added to adjust the pH prior to reduction and conjugation. 2.55 molar equivalents of TCEP (tris(2-carboxyethyl)phosphine) relative to antibody was added from a 5mM stock in water and the antibody was left to reduce for 120 minutes. The reduced mAb was diluted 1/3 with PBS prior to conjugation. 8 molar equivalents of Drug Conjugate 1 was added from a 10mM stock in DMA (dimethylacetamide) and the reduced antibody was allowed to conjugate for 90 minutes. 8 molar equivalents of NAC (N-acetylcysteine) was added from a 100mM stock in water to quench unreacted Drug Conjugate 1 and allowed to react for 20 minutes. The conjugate was buffer exchanged into PBS using G25 Resin (NAP25 columns) and then dosed with activated carbon at a 1mg Carbon: 1mg ADC ratio and allowed to incubate overnight on a roller mixer at 10rpm at room temperature. The conjugate was then spun down at 4000xG for 15 minutes to pellet the carbon and the supernatant (ADC) was removed and filtered through a 0.2uM PES filter. The conjugate was then further purified and concentrated via diafiltration using Amicon15 devices where 6x Diafiltration Volumes (DV's) of PBS, pH 7.4were used in the buffer exchange. The conjugate was then terminally filtered through a 13mm 0.2μm PES filter (chromatography direct / FIL-S-PES-022-13-100-S) under grade A laminar flow and then formulated to 0.02% PS80. The final product was sampled for QC testing - monomer and [ADC] mg/ml by SEC HPLC, average DAR by HIC, Residual toxin by RP-HPLC, and endotoxin by Endosafe.

**Biological Example 1: HsNMT1 and SU-DHL-10 assays**

**HsNMT1 $IC_{50}$**

The $IC_{50}$ values for certain NMT inhibitors were measured using a sensitive fluorescence-based assay based on detection of CoA by 7-diethylamino-3-(4-maleimido-phenyl)-4-methylcoumarin, as described in Goncalves, V., et al., Analytical Biochemistry, 2012, 421, 342-344 and Goncalves, V., et al., J. Med. Chem, 2012, 55, 3578.

**Cell cytotoxicity in SU-DHL-10 cell line**

Certain NMT inhibitors were tested in the SU-DHL-10 cell line (human B cell lymphoma). Compounds which show efficacy in this assay are expected to be useful as agents for treating or preventing hyperproliferative disorders such as cancer.

Cells were seeded in 96-well microplates and treated with compounds or cisplatin (as a positive control) at 9 increasing concentrations in technical triplicate. $IC_{50}$ of the tested compound and cisplatin were determined following 72h treatment in each cell line.

1. On day 1, 90 μL of various cell suspensions with the number of cells ranging from 5000-8000 cells/well were seeded into wells of a 96-well plate (Corning). The number of cells to be seeded was previously determined.
2. All 96-wells plates with cells were placed in an incubator overnight at 37°C with 5% $CO_2$.
3. On day 2, cells were observed under a microscope ensuring the cells treated with vehicle control were in good

condition.

4. A dilution series of the test compounds and cisplatin were prepared at 10X the final concentrations required. 10 μL/well of 10X compound solution was added to the corresponding plates. The final volume was 100 μL /well for all the plates. Final DMSO concentration was 0.1%.
5. On day 5 (after 72 hours of incubation), 50 μL of CTG reagent was added to each well.
6. Contents were mixed for 5 minutes on an orbital shaker to facilitate cell lysis.
7. The plate was allowed to incubate at room temperature for 10 minutes to stabilize luminescent signals.

Luminescence was recorded using an EnVision Multi Label Reader. Data analysis was performed using GraphPad Prism 8.0.

To calculate $IC_{50}$, a concentration-response curve was generated using a nonlinear regression model with a sigmoidal concentration response. The formula for calculating the % of viable cells was shown below, and the $IC_{50}$s are automatically generated by GraphPad Prism 8.0.

$$\%\ viable\ cells = \left(\frac{LumTest\ article\ - LumMedium\ control}{LumNone\ treated - LumMedium\ control}\right) \times 100\%$$

(LumTest article = luminescence in test article treated well; LumNone treated = luminescence in vehicle treated well; LumMedium control = luminescence in a well containing only medium and no cells; LumNone treated-LumMedium control was set as 100%).

**Results**

The results of the HsNMT1 and SU-DHL-10 experiments described above are shown in Table 2 below:

Table 2: Results of Biological Example 1

| **NMT inhibitor** | **HsNMT1 $IC_{50}$ (nM)** | **SU-DHL-10 $IC_{50}$ (nM)** |
|---|---|---|
| 1 | 2.1 | 0.6 |
| 2 | 2.2 | 15.3 |
| 3 | - | - |
| 4 | - | 9.4 |
| 5 | - | 12 |
| 6 | 2.2 | 5.9 |
| 7 | - | 5.0 |
| 8 | 9 | - |
| 9 | - | 0.7 |
| 10 | - | 0.6 |
| 11 | - | 2.0 |
| 12 | - | 0.9 |
| 13 | - | 0.7 |
| 14 | - | 0.4 |
| 15 | - | 1.2 |
| 16 | - | 0.2 |
| 17 | 2.0 | 1.0 |
| 18 | 1.9 | 5.3 |
| 19 | - | - |
| 20 | 2.0 | 12.0 |
| 21 | - | 0.4 |

(continued)

| NMT inhibitor | HsNMT1 $IC_{50}$ (nM) | SU-DHL-10 $IC_{50}$ (nM) |
|---|---|---|
| 22 | 3.0 | 9 |
| 23 | - | - |
| 24 | - | 0.7 |
| 25 | 2.0 | 1.0 |

The results of Biological Example 1 demonstrate that the tested NMT inhibitor compounds are inhibitors of HsNMT1 and show potent *in vitro* cytotoxic activity.

**Biological Example 2: Orthotopic Breast Cancer Xenograft Model**

NOD/SCID mice were implanted with estrogen pellets (17β-estradiol, 60 day release, 0.36mg) subcutaneously in the right flank one day before the tumour inoculation. On day -8 each mouse was then inoculated in the right mammary fat pad with $1\times10^7$ viable BT474 breast cancer cells resuspended in 0.2 mL of Phosphate Buffered Saline mixed with matrigel (1:1). Mice were assigned to treatment groups when tumour volumes averaged 149.78 $mm^3$ on study day 0. Dosing commenced the following day, and all animals were dosed intravenously with trastuzumab, NMT inhibitor 1 or ADC Example 1. The study was terminated on Study Day 35. Mice were dosed once per week for four weeks with either vehicle alone (Group 1), 2.5 mg/Kg trastuzumab (Group 2), 5mg/Kg trastuzumab (Group 3), 2.5mg/Kg ADC Example 1 (Group 4) or 5mg/Kg ADC Example 1 (Group 5) or 2mg/Kg NMT inhibitor 1 dosed for 2 days followed by a 5 day holiday (Group 6). Each group was comprised of 10 mice. Tumour volumes in mice were measured three times per week and tumour volume was calculated using the formula 0.5 ($LxW^2$). The mean tumour volumes (+SEM) for each study group at each measurement are shown in Figures 1 and 2 plotted as Last Observation Carried Forward. Statistical analyses were carried out on tumour readings for Groups 1, 2, 3, 4 and 5 up to Study Day 23 (after this point >50% of the animals within one of the study groups were lost; Group 2) using two-way ANOVA, or a Mixed-Effects model was fitted when values were missing from groups (PRISM GraphPad Software Inc.). Statistical analysis of Group 6 was conducted until day 13 (at this point the study was terminated due to the significant loss of observed body weight). Using a ROUT outlier analysis in GraphPad Prism, one mouse in Group 5 was identified as an outlier across all timepoints (at the 5% confidence level) and has therefore been excluded from analysis.

ΔTGI% = ((mean(C)-mean (C0))-(mean(T)-mean(T0)))/(mean(C)-mean(C0))x100% where T is the mean tumour volume of the treated group on the measurement day and T0 is the mean tumour volume on Study Day 0. C is the mean tumour volume of the control Group 1 mice on the measurement day and C0 is the mean tumour volume on Study Day 0.

During the study, body weights were measured three times weekly for all animals. Animals were given Diet Gel for the entire duration on the study. The mean body weights for each group during the dosing phase are presented in Figures 3 and 4.

Results

The results of treatment with trastuzumab alone or ADC Example 1 on tumour size are depicted as percentage tumour growth inhibition in Table 3, and as tumour volume ($mm^3$) in Figures 1 and 2. Figures 1 and 2 also depict the tumour volume of mice treated with NMT inhibitor 1 alone, but this study was terminated early due to significant loss of body weight observed in the treatment group.

Table 3: Tumour growth inhibition

| Group | Treatment | Tumor growth inhibition % on day 23 |
|---|---|---|
| 1 | Vehicle | - |
| 2 | trastuzumab 2.5 mg/kg | 3.6% |
| 3 | trastuzumab 5mg/kg | 46% |
| 4 | ADC Example 1 2.5mg/kg | 55% |
| 5 | ADC Example 1 5mg/kg | 109% |

Mice treated with ADC Example 1, 2.5 mg/kg (Group 4) had a significantly reduced tumour volume compared treated to animals receiving trastuzumab, 2.5 mg/kg (Group 2; p<0.0001) (Figure 1). Furthermore, animals treated with ADC Example 1, 5.0 mg/kg (Group 5) had a significantly reduced tumour volume compared to animals treated with trastuzumab, 5 mg/kg (Group 3; p<0.0001) (Figure 2). Mice treated with ADC Example 1, 5.0 mg/kg (Group 5) had a significantly reduced tumour volume compared to animals treated with ADC Example 1, 2.5 mg/kg (Group 4; p<0.0001) (see Table 3). Mice treated with ADC Example 1, 5.0 mg/kg (Group 5) had a significantly reduced tumour volume compared to mice treated with 2.0 mg/Kg of NMT inhibitor 1. Mice treated with NMT inhibitor 1, 2.0 mg/kg (Group 6) reduced tumour size (see Figures 1 and 2) but due to the significant loss in observed body weight in this study group, the study was stopped at day 13. ADC Example 1 dosed at 2.5 mg/kg was approximately equieffective at that time point compared to NMT inhibitor 1 (Group 4, see Figure 1) although ADC Example 1 dosed at 5.0 mg/kg was more effective (Figure 2). The dose of NMT inhibitor 1 delivered when administered as ADC Example 1 dosed at 2.5 mg/kg is approximately 100 fold lower than NMT inhibitor 1 dosed alone, meaning that ADC Example 1 is approximately 100 fold more potent *in vivo* than NMT inhibitor 1.

The effect of treatment with trastuzumab alone, NMT inhibitor 1 alone or ADC Example 1 on the body weights of mice are shown in Figures 3 and 4. Mice treated with trastuzumab or ADC Example 1 did not differ significantly from the vehicle control, whilst body weights of mice in Group 6 (NMT inhibitor 1) decreased significantly (Figures 3 and 4) before this study was terminated early due to the significant loss in observed body weight.

**Biological Example 3: Gastric Cancer Xenograft Model**

The objective of this study was to evaluate preclinically the in vivo therapeutic efficacy of an antibody-drug conjugate (ADC Example 1) in the treatment of subcutaneous NCI-N87 human gastric xenograft model in female BALB/c nude mice.

In this study, 143 mice were inoculated subcutaneously in the right flank region with $1\times10^7$ viable NCI-N87 tumour cells resuspended in 0.1 mL of PBS mixed with Matrigel (1:1) for tumour development. 102 mice were assigned to 9 treatment groups on study day 0 when tumour volumes averaged 168.08 $mm^3$. Dosing commenced the following day, and animals were dosed intravenously with vehicle control, trastuzumab, ADC Example 1, trastuzumab deruxtecan and an isotype control antibody conjugated to NMT inhibitor 1 (Isotype control). The study was terminated on study Day 28. Mice were dosed once per week for two weeks with either vehicle control (Group 1), 2.5 mg/Kg trastuzumab (Group 2), 5mg/Kg trastuzumab (Group 3), 2.5mg/Kg ADC Example 1 (Group 4), 5mg/Kg ADC Example 1 (Group 5), 2.5mg/Kg trastuzumab deruxtecan (Group 6), 5mg/Kg trastuzumab deruxtecan (Group 7) or 5mg/Kg isotype control antibody (Group 8).

Table 4: Summary of dosing regimen

| GROUP | TREATMENT | Dose mg/Kg (mpk) | Schedule | TGI study day 28 |
|---|---|---|---|---|
| 1 | Vehicle | - | QW x 2 weeks | |
| 2 | trastuzumab | 2.5 | QW x 2 weeks | 123.08 |
| 3 | trastuzumab | 5 | QW x 2 weeks | 167.70 |
| 4 | ADC Example 1 | 2.5 | QW x 2 weeks | 153.55 |
| 5 | ADC Example 1 | 5 | QW x 2 weeks | 224.01 |
| 6 | trastuzumab-deruxtecan | 2.5 | QW x 2 weeks | 71.66 |
| 7 | trastuzumab-deruxtecan | 5 | QW x 2 weeks | 140.71 |
| 8 | Isotype control-ADC | 5 | QW x 2 weeks | - |

Tumour volumes in mice were measured three times per week and tumour volume was calculated using the formula 0.5 (LxW2). The mean tumour volumes (+SEM) for each study group at each measurement are shown in Figures 5 and 6.

$\Delta$TGI% = ((mean(C)-mean (C0))-(mean(T)-mean(T0)))/(mean(C)-mean(C0))x100% where T is the mean tumour volume of the treated group on the measurement day and T0 is the mean tumour volume on Study Day 0. C is the mean tumour volume of the control Group 1 mice on the measurement day and C0 is the mean tumour volume on Study Day 0.

During the study, body weights were measured three times weekly for all animals. Animals were given Diet Gel for the entire duration on the study. The mean body weights for each group during the dosing phase are presented in Figures 7A (2.5mpk study) and 7B (5mpk study).

**Results**

Significant body weight loss (>10%) was observed for 3/10 animals in Group 1 (vehicle control); one animal in Group 2 (2.5mg/Kg trastuzumab) and one animal in Group 3 (5mg/Kg trastuzumab); all mice regained body weight by the next measurement. No significant body weight loss was observed in any of the other groups.

There was a significant decrease ($p<0.0001$) in the tumour volumes of mice treated with trastuzumab, ADC Example 1 and trastuzumab deruxtecan at all concentrations (2.5mg/kg or 5mg/kg), when compared to vehicle alone (Group 1). Isotype control ADC (Group 8; $p=0.7935$) showed no significant difference compared to vehicle alone (Group 1).

Mice treated with trastuzumab 5mg/kg (Group 3; $p<0.0001$) and ADC Example 1 5mg/kg (Group 5; $p<0.0001$), showed a significant decrease in tumour volume compared to those treated with trastuzumab 2.5mg/kg (Group 2). Mice treated with trastuzumab deruxtecan 2.5mg/kg (Group 6; $p<0.0001$) and Isotype control-ADC (Group 8; $p<0.0001$) showed significantly higher tumour volumes compared to Group 2 (trastuzumab 2.5mg/kg). There was no significant difference between ADC Example 1 2.5mg/kg (Group 4; $p=0.8757$) and trastuzumab deruxtecan 5mg/kg (Group 7; $p=0.9965$) compared to trastuzumab 2.5mg/kg (Group 2).

Mice treated with ADC Example 1 5mg/kg (Group 5; $p<0.0001$) showed a significant decrease in tumour volume compared to trastuzumab 5mg/kg (Group 3). There was no significant difference between Group 3 (trastuzumab 5mg/Kg) and Group 4 (ADC Example 1 2.5mg/kg). Otherwise all other groups (Groups 6-8) had significantly higher tumour volume compared to Group 3 (trastuzumab 5mg/kg).

Mice treated with ADC Example 1 5mg/kg (Group 5; $p<0.0001$) showed a significant decrease in tumour volume compared to ADC Example 1 2.5mg/kg (Group 4). There was no significant difference between Group 4 and Group 7 (trastuzumab deruxtecan 5mg/kg $p=0.9932$). Otherwise all other groups (Groups 6 and 8) had significantly higher tumour volume compared to Group 4 (ADC Example 1 2.5mg/kg).

Group 5 mice treated with ADC Example 1 5mg/kg had significantly lower tumour volume compared to all other groups ($p<0.0001$).

Mice treated with trastuzumab deruxtecan 5mg/kg (Group 7 $p<0.0001$) showed a significant decrease in tumour volume compared to trastuzumab deruxtecan 2.5mg/kg (Group 6). Group 8 showed significantly higher tumour volume ($p<0.0001$) compared to Group 6.

Mice treated with Isotype control-ADC (Group 8; $p<0.0001$) had significantly higher tumour volume compared to Group 7 (trastuzumab deruxtecan 5mg/kg).

Increased tumour growth inhibition (TGI) compared to the vehicle group (Group 1) was apparent in all treatment groups other than Groups 8. Treatment with 5.0 mg/kg ADC Example 1 was the most efficacious when the tumour growth inhibition of all treatment groups was compared (Group 5; TGI = 224.01%).

TGI% = ((mean(C)-mean (C0))-(mean(T)-mean(T0)))/(mean(C)-mean(C0))x100% where T is the mean tumour volume of the treated group on the measurement day and T0 is the mean tumour volume on Study Day 0. C is the mean tumour volume of the control Group 1 mice on the measurement day and C0 is the mean tumour volume on Study Day 0.

## Biological Example 4: In vitro assessment of cytotoxicity of trastuzumab conjugated to NMT inhibitor 1 against a HER2 positive breast cancer cell line, BT474

Trastuzumab was conjugated to NMT inhibitor 1 (ADC Example 1) and tested for its cytotoxic activity against a HER2 positive breast cancer cell line, BT474 in an *in vitro* assay. Cells were plated in 96 well plates at 10% confluency (8000 for BT474). The following day cells were treated with ADC Example 1 at 50nM, 3.3nM, and 0.2nM and controls in 250nM Sytox Green containing medium. Plates were imaged every four hours for ten days in the IncuCyte S3. Images for % phase (confluence) and green (Sytox Green) were collected and analysed using the IncuCyte software 2022A. Data are presented as green area normalised to phase area ($\mu m^2$) over time whilst cells are in log phase proliferation.

Figures 8 to 11 show ADC Example 1 effectively induces cell killing of BT474 cells at a concentration of 0.2nM, whereas naked trastuzumab and an isotype control IgG conjugated to NMT inhibitor 1 were ineffective at killing BT474 cells under these conditions. Trastuzumab-deruxtecan also effectively killed BT474 cells but was less efficacious than ADC Example 1 under these conditions. Puromycin was included as a positive cytotoxic control.

## Biological Example 5: In vitro assessment of cytotoxicity of trastuzumab conjugated to NMT inhibitor 1 against a HER2 negative breast cancer cell line, MCF7

Trastuzumab was conjugated to NMT inhibitor 1 (ADC Example 1) and tested for its cytotoxic activity against a HER2 negative breast cancer cell line, MCF7 in an in vitro assay. Cells were plated in 96 well plates at 10% confluency (2000 for MCF7). The following day cells were treated with ADC Example 1 at 50nM, 3.3nM, and 0.2nM and controls in 250nM Sytox Green containing medium. Plates were imaged every four hours for ten days in the IncuCyte S3. Images for

% phase (confluence) and green (Sytox Green) were collected and analysed using the IncuCyte software 2022A. Data are presented as green area normalised to phase area ($\mu m^2$) over time whilst cells are in log phase proliferation.

Figures 12 to 15 shows ADC Example 1, an isotype control IgG conjugated to NMT inhibitor 1, trastuzumab or trastuzumab-deruxtecan were unable to induce cell killing of HER2 negative MCF7 cells at a concentration up to 50nM under these conditions. This was expected since trastuzumab targets HER2 positive cells. Puromycin was included as a positive cytotoxic control.

**Biological Example 6: In vitro "bystander effect" of trastuzumab conjugated to NMT inhibitor 1**

HER2 negative MCF7 cells were stably transduced with pHIV eGFP (Addgene plasmid 21373) and sorted by flow cytometry to obtain a cell population that was 100% GFP positive (Green Fluorescent Protein). After two passages, eGFP positive MCF7 cells were plated in a 1:1 ratio with Wild Type MCF7 cells (1000 cells each) or with HER2 positive BT474 cells in 96 well plates. Cells were treated with ADC Example 1, Reference Example 1 (trastuzumab-Monomethyl auristatin E (MMAE)), Isotype control (defined above), trastuzumab deruxtecan or naked trastuzumab at 12.5nM, 3.13nM, 0.78nM, 0.2nM and 0.05nM and controls in culture medium (50% MCF7 and 50% BT474 cell culture medium). Cells were imaged every four hours for ten days in the IncuCyte S3. Images for % green (eGFP+MCF7) were collected and analysed using the IncuCyte software 2022A. Data are presented as % green area ($\mu m^2$) as a measure of eGFP+ MCF7 confluence on Day 9.

Figure 16 shows eGFP+MCF7 cells when co-cultured with wild type MCF7 undergo no significant cytotoxicity when incubated with ADC Example 1 for 9 days, nor with any control or reference standards. This was expected since trastuzumab targets HER2 positive cells. However, when eGFP+MCF7 cells were co-cultured with HER2 positive BT474 cells (Figure 17), there is significant loss of GFP signal corresponding to bystander killing of the eGFP+MCF7 cells. Of the 3 ADCs tested (ADC Example 1, Reference Example 1, trastuzumab deruxtecan), ADC Example 1 was the most efficacious at inducing bystander killing, ADC Reference Example 1 was the second most effective and trastuzumab-deruxtecan was the least effective. Naked trastuzumab and an isotype control IgG conjugated to NMT inhibitor 1 were used as controls and neither induced any bystander killing.

**Biological Example 7: In vitro assessment of cytotoxicity of antibodies conjugated to NMT inhibitor 1**

NMT inhibitor 1 was conjugated as described to trastuzumab (ADC Example 1) and sacituzumab (ADC Example 3). NMT inhibitor 26 was conjugated to trastuzumab (ADC Example 5). ADC Examples 1, 3 and 5 were tested for their cytotoxic activity against a panel of cell lines including BT474, JIMT 1, NCI N87, NCI H292, IM95-m, ZR-75-30 and NCI H2170 (Figures 18 to 24).

NMT inhibitor 1 was conjugated as described to ifinatamab (ADC Example 4) and was tested for its cytotoxic activity against a panel of prostate cancer cell lines including, LNCaP, C42 and VCaP (Figures 25 to 27). Cells were plated at set initial densities in 96 well flat bottom plates in the appropriate growth media. 24 hours later, dosing solutions were added at serial dilutions of each test compound spanning a concentration range of either 10 nM to 0.001 nM for NMT inhibitor 1, or 50 nM to 0.005 nM for the various ADCs. Cell proliferation was measured after 144 hours exposure, by CellTiter-Glo 2.0 Solution Cell Viability Assay. The percentage inhibition of viability was calculated in relation to the DMSO vehicle control at endpoint (after blank correction, time zero correction, and log transformation of compound concentrations) using the equation:

% Viability = (luminescence treated cells day6 - blank) - (luminescence day0 - blank) / (mean of the luminescence vehicle treated cells day6 - blank) - (luminescence day0 - blank)

**Results**

Table 5 shows the inhibition of cell viability quantified as above and defined as the Absolute IC50 - the concentration of an inhibitor where the response is reduced by half or the Relative IC50 - the concentration required to bring the curve down to point halfway between the top and bottom plateaus of the curve. The full concentration response curves for each cell line are shown in Figures 18 to 27 (mean +/-SEM).

Table 5: Inhibition of cell viability

| Cell line | ADC Example | Absolute IC50 (nM) | Relative IC50 (nM) |
|---|---|---|---|
| BT474 | Example 1 | 0.172 | 0.255 |
| BT474 | Example 5 | 2.712 | 1.334 |
| JIMT 1 | Example 1 | 52.07 | 4.625 |

(continued)

| Cell line | ADC Example | Absolute IC50 (nM) | Relative IC50 (nM) |
|---|---|---|---|
| JIMT 1 | Example 3 | 0.486 | 0.247 |
| NCI N87 | Example 1 | 0.791 | 0.475 |
| NCI N87 | Example 3 | 3.999 | 0.799 |
| NCI H292 | Example 3 | 0.544 | 0.269 |
| IM95-m | Example 3 | 0.423 | 0.323 |
| ZR-75-30 | Example 1 | <0.2 | 0.253 |
| ZR-75-30 | Example 5 | 0.18 | 0.393 |
| NCI H2170 | Example 1 | 1.581 | 0.946 |
| NCI H2170 | Example 3 | 51.61 | 11.94 |
| LNCaP | Example 4 | 0.154 | 0.147 |
| C42 | Example 4 | 1.345 | 0.343 |
| VCaP | Example 4 | 0.158 | 0.212 |

**Biological Example 8: Evaluation of the effect of test articles as single agents on cell viability of gastric cancer organoid lines using CellTiter-Glo (CTG) luminescent cell viability assay**

**Methods**

Day -1: Patient derived gastric cancer organoids (PDXOs) are sheared to uniform sizes and the required number of organoids are combined 1:1 with 50% Matrigel to produce the right size of the organoids to perform the screen.

Day 0: Organoid seeding

1. Collect organoids from each well by adding 20 µl 100x Dispase solution to each well from a 6-well plate, which contains 2 ml of organoid medium.
2. Put the plates back in the incubator, 37°C for 30 min.
3. Collect organoids from all wells and pipet through a pre-wet 100 µm filter into a 50 ml plastic tube.
4. Once all wells have been filtered over 100 µm filters, filter the flow though over a pre-wet 20 µm filter.
5. Invert the 20 µm filter and recover the organoids in a new 50 ml tube.
6. Collect and resuspend the organoids in the corresponding culture media. Count the organoids to get the concentration.
7. Adjust cell concentration with culture medium to the proper concentration.
8. Add Matrigel to a final concentration of 5% v/v and keep the organoids suspension on ice.
9. Add 40 µL cell suspensions to 384-well plate by Multidrop dispenser with corresponding culture medium at the below seeding density:

| No. | Organoid line | Seeding density |
|---|---|---|
| 1 | GA0429B | 300/well |
| 2 | GA6877B | 120/well |
| 3 | GA6894B | 320/well |
| 4 | GA2434B | 250/well |
| 5 | GA3102B | 250/well |
| 6 | GA0119B | 500/well |
| 7 | GA0091B | 300/well |
| 8 | GA6815B | 400/well |
| 9 | GA0098B | 350/well |

(continued)

| No. | Organoid line | Seeding density |
|---|---|---|
| 10 | GA6833B | 350/well |
| 11 | GA0087B | 300/well |
| 12 | GA2109B | 350/well |
| 13 | GA3055B | 300/well |
| 14 | GA6866B | 300/well |
| 15 | GA0060B | 100/well |
| 16 | GA6891B | 350/well |

10. Place screening plates back into the incubator before adding compounds and day 0 reading. Two duplicate plates were set up. One is for day 0 reading (T0) and the other for reading at the end point.

Day 0: Compounds treatment and Day 0 baseline CTG (cell titre glo) reading

11. Add test articles according to the drug dilution scheme and plate map by Tecan D300e
12. Place screening plates back into the incubator.
13. For day 0 reading plate, add 40 μL of CTG 3D per well and read luminescent signal on Envision plate reader.

Day 6: Endpoint CTG reading.

1. Read assay plates for luminescent CTG signal at the end of the assays: add 40 μL of CTG 3D per well by Multidrop dispenser, mix the contents for 5 min on the plate shaker, and incubate the plates for 30 min at room temperature in dark. Read luminescent signal on Envision plate reader.

The data were displayed graphically using GraphPad Prism. In order to calculate absolute IC50, a concentration-response curve was fitted using nonlinear regression model with a sigmoidal dose response with variable slope. The formula for calculating surviving rate is shown below and the absolute IC50 were calculated according to the dose-response curve generated by GraphPad Prism.

The surviving rate (%) = (Lum Test article-Lum Medium control)/ (Lum Vehicle control-Lum Medium control) ×100%.

Gastric cancer organoids were incubated for 5 or 6 days with a concentration range of (1) the isotype control ADC, (2) ADC Example 1, (3) Staurosporin as a positive control, (4) trastuzumab or (5) trastuzumab-deruxtecan (trastuzumab-DXd). Table 6 shows the IC50 (μM), the maximum inhibition (%) and the area under the curve.

Table 6: Results from organoid study

| No. | Model name | No. | Test_Article | IC50 (μM) | Max_ Inhibition (%) | AUC (area under curve) |
|---|---|---|---|---|---|---|
| **1** | GA0429B | 1 | Isotype Control | 0.0064 | 89.31 | 299.5734 |
| | | 2 | ADC Example 1 | 0.0005 | 96.1 | 190.0932 |
| | | 3 | Staurosporine | 0.0054 | 99.92 | 170.8729 |
| | | 4 | trastuzumab | >0.05 | 7.66 | NA |
| | | 5 | trastuzumab-DXd | >0.05 | 41.08 | 340.3922 |

(continued)

| No. | Model name | No. | Test_Article | IC50 (μM) | Max_ Inhibition (%) | AUC (area under curve) |
|---|---|---|---|---|---|---|
| **2** | GA6877B | 1 | Isotype control | 0.0147 | 85.54 | 348.8528 |
| | | 2 | ADC Example 1 | 0.0008 | 96.86 | 213.6828 |
| | | 3 | Staurosporine | 0.0059 | 99.99 | 175.472 |
| | | 4 | trastuzumab | >0.05 | 40.47 | 328.7294 |
| | | 5 | trastuzumab-DXd | 0.0004 | 80.47 | 196.3687 |
| **3** | GA6894B | 1 | Isotype control | 0.0109 | 99.04 | 339.4386 |
| | | 2 | ADC Example 1 | 0.0044 | 98.02 | 284.3133 |
| | | 3 | Staurosporine | 0.0085 | 100.01 | 230.6577 |
| | | 4 | trastuzumab | >0.05 | 28.1 | 338.0717 |
| | | 5 | trastuzumab-DXd | >0.05 | 44.79 | 320.9681 |
| **4** | GA2434B | 1 | Isotype control | >0.05 | 16.66 | 372.6593 |
| | | 2 | ADC Example 1 | >0.05 | 36.66 | 378.1264 |
| | | 3 | Staurosporine | 0.0239 | 99.86 | 226.6809 |
| | | 4 | trastuzumab | >0.05 | 27.95 | 368.0264 |
| | | 5 | trastuzumab-DXd | >0.05 | 21.96 | 374.6871 |
| **5** | GA3102B | 1 | Isotype control | 0.0446 | 51.88 | 358.0517 |
| | | 2 | ADC Example 1 | 0.0029 | 72.15 | 285.1367 |
| | | 3 | Staurosporine | 0.0106 | 99.71 | 200.7667 |
| | | 4 | trastuzumab | >0.05 | 13.75 | 362.8386 |
| | | 5 | trastuzumab-DXd | 0.0007 | 90.24 | 215.0035 |
| **6** | GA0119B | 1 | Isotype control | >0.05 | 54.4 | 384.4805 |
| | | 2 | ADC Example 1 | 0.0187 | 59.37 | 373.0805 |
| | | 3 | Staurosporine | 0.0089 | 99.89 | 212.2837 |
| | | 4 | trastuzumab | >0.05 | 12.83 | 382.5737 |
| | | 5 | trastuzumab-DXd | >0.05 | 26.83 | 364.6999 |
| **7** | GA0091B | 1 | Isotype control | >0.05 | 17.83 | 425.8 |
| | | 2 | ADC Example 1 | >0.05 | 18.13 | 462.2 |
| | | 3 | Staurosporine | 0.0222 | 99.29 | 199.9 |
| | | 4 | trastuzumab | >0.05 | 17.54 | 402.9 |
| | | 5 | trastuzumab-DXd | >0.05 | -4.07 | 459.8 |
| **8** | GA6815B | 1 | Isotype control | 0.0156 | 71.42 | 371.8204 |
| | | 2 | ADC Example 1 | 0.0007 | 89.66 | 245.5017 |
| | | 3 | Staurosporine | 0.0158 | 99.01 | 194.3428 |
| | | 4 | trastuzumab | 0.0019 | 64.47 | 284.7039 |
| | | 5 | trastuzumab-DXd | 0.0004 | 63.4 | 246.891 |

(continued)

| No. | Model name | No. | Test_Article | IC50 (μM) | Max_ Inhibition (%) | AUC (area under curve) |
|---|---|---|---|---|---|---|
| **9** | GA0098B | 1 | Isotype control | >0.05 | 28.61 | 408.2 |
| | | 2 | ADC Example 1 | >0.05 | 25.23 | 385.3 |
| | | 3 | Staurosporine | 0.0221 | 99.46 | 239.6 |
| | | 4 | trastuzumab | >0.05 | 15.3 | 389.5 |
| | | 5 | trastuzumab-DXd | >0.05 | 8.49 | 399.6 |
| **10** | GA6833B | 1 | Isotype control | 0.0115 | 96.15 | 353.3708 |
| | | 2 | ADC Example 1 | 0.0068 | 93.75 | 305.6923 |
| | | 3 | Staurosporine | 0.0053 | 100.02 | 158.1701 |
| | | 4 | trastuzumab | >0.05 | 32.44 | 311.5796 |
| | | 5 | trastuzumab-DXd | >0.05 | 30.23 | 347.9672 |
| **11** | GA0087B | 1 | Isotype control | 0.0492 | 71.07 | 435.2 |
| | | 2 | ADC Example 1 | 0.0226 | 78.92 | 485.0 |
| | | 3 | Staurosporine | 0.0029 | 98.04 | 113.2 |
| | | 4 | trastuzumab | >0.05 | 35.31 | 447.7 |
| | | 5 | trastuzumab-DXd | >0.05 | 42.94 | 330.3 |
| **12** | GA2109B | 1 | Isotype control | 0.0162 | 89.6 | 334.7632 |
| | | 2 | ADC Example 1 | 0.0093 | 93.34 | 314.0499 |
| | | 3 | Staurosporine | 0.0393 | 99.99 | 233.3136 |
| | | 4 | trastuzumab | >0.05 | 34.63 | 365.1782 |
| | | 5 | trastuzumab-DXd | >0.05 | 18.41 | 376.8581 |
| **13** | GA3055B | 1 | Isotype control | 0.0079 | 93.85 | 297.0282 |
| | | 2 | ADC Example 1 | 0.0046 | 94.46 | 275.49 |
| | | 3 | Staurosporine | 0.0033 | 98.42 | 144.7132 |
| | | 4 | trastuzumab | >0.05 | 43.3 | 343.8818 |
| | | 5 | trastuzumab-DXd | >0.05 | 32.59 | 365.9383 |
| **14** | GA6866B | 1 | Isotype control | 0.0065 | 81.77 | 326.5628 |
| | | 2 | ADC Example 1 | 0.0004 | 88.15 | 189.9801 |
| | | 3 | Staurosporine | 0.0061 | 99.97 | 201.1356 |
| | | 4 | trastuzumab | 0.0073 | 62.62 | 304.9903 |
| | | 5 | trastuzumab-DXd | 0.0004 | 85.3 | 192.9042 |
| **15** | GA0060B | 1 | Isotype control | 0.0127 | 97.1 | 381.1712 |
| | | 2 | ADC Example 1 | 0.0037 | 98.82 | 289.755 |
| | | 3 | Staurosporine | 0.0006 | 99.99 | 85.1 |
| | | 4 | trastuzumab | >0.05 | 34.19 | 348.9219 |
| | | 5 | trastuzumab-DXd | >0.05 | 36.69 | 330.4667 |

(continued)

| No. | Model name | No. | Test_Article | IC50 (μM) | Max_ Inhibition (%) | AUC (area under curve) |
|---|---|---|---|---|---|---|
| 16 | GA6891B | 1 | Isotype control | 0.0173 | 73.24 | 392.3645 |
| | | 2 | ADC Example 1 | 0.0022 | 78.99 | 304.6253 |
| | | 3 | Staurosporine | 0.009 | 99.57 | 174.0868 |
| | | 4 | trastuzumab | >0.05 | -1.19 | 440.8762 |
| | | 5 | trastuzumab-DXd | >0.05 | -4.87 | 442.4096 |

The concentration response curves for 16 gastric cancer organoids results are shown in Figures 28 to 43. The data shows the organoid viability (mean +/-SEM) following treatment with the isotype control conjugated to NMT inhibitor 1, ADC Example 1, trastuzumab-deruxtecan (DXd) and trastuzumab.

The results show that in the majority of the gastric cancer organoids, ADC Example 1 is the most efficacious as shown by its IC50 value compared with the controls. In certain organoids, ADC Example 1 had comparable activity to trastuzumab-DXd as shown by the IC50 values.

**Biological Example 9: A Maximum Tolerated Dose study of ADC Example 1 Following Single Intravenous Injection in Male Cynomolgus Monkeys**

6 conventional non-naïve male Cynomolgus monkeys were used in this MTD study. The service provider laboratory performing the study had obtained a certificate from the Association for Assessment and Accreditation of Laboratory Animal Care International (AAALAC). An animal care and use application for this study was submitted to the Service Provider's Institutional Animal Care and Use Committee (IACUC) for approval. The IACUC reviewed this protocol for its accordance with their TSP IACUC policies and procedures. Monkeys were quarantined for at least 2 weeks at the Vendor before being shipped to the Service Provider's facility. Animals were screened against active infection with tuberculosis, herpes B, simian acquired immunodeficiency virus (SIV), simian retrovirus (SRV), simian T-cell lymphotropic virus (STLV), shigella, and salmonella, and were evaluated for normal hematology and clinical chemistry parameters. The serological detection or fecal analysis was performed for evidence of parasites.

Animals were randomised to 3 groups with two animals per group and were administered the test article, ADC Example 1 via slow intravenous injection (5 minutes) according to Table 7.

Table 7: Dosing regimen for Biological Example 9

| Group | Treatment | Dose Level (mg/kg/day) | No. of Males |
|---|---|---|---|
| 1 | ADC Example 1 | 5 | 2 |
| 2 | | 10 | 2 |
| 3 | | 20 | 2 |

Animals were evaluated during the in life phase according to Table 8.

Table 8: Evaluation protocol for Biological Example 9

| Procedure | Frequency |
|---|---|
| Cageside Observations | Twice daily plus one more time at 2-hour post dose (only on Day 1) |
| Detailed Clinical Observations | Prior to the first dose and daily thereafter for 6 days |
| Body Weight | Prior to the first dose and daily thereafter for 6 days |
| Qualitative Food Consumption | Daily, qualitative |
| Clinical Pathology (Clinical Chemistry, Hematology and Coagulation) | Prior to dosing, Day 1, after dosing on Day 2 and prior to the study termination on Day 7 or Day 11 |

Blood samples were collected from each study animal on the following days; the day prior to dosing (day 1) on

study day 2 and at the termination (day 7 or day 11). Animals were food fasted overnight prior to sample collection for clinical pathology. Blood samples were collected by puncture of the peripheral vessels. Blood samples were processed and analyzed for haematology, clinical biochemistry and coagulation. Each of the 5, 10 and 20 mg/Kg doses were well tolerated by the respective groups and there were no significant changes in haematological or clinical chemistry parameters. In particular, each of the 5 and 10 mg/Kg groups showed no changes. Figures 44a and 44b show representative data from the 20mg/Kg group. Specifically, the mean (+/- SEM) level of haematologial markers is plotted as normalised to baseline (pre-dose) values. No changes are observed at day 7, whilst relatively small, not significant, changes are observed at day 11.

## Biological Example 10: In vivo Tolerability Study of ADC Example 1 in Non-Tumour Bearing Female CD1 Mice

The objective of this study was to evaluate the in vivo tolerability of ADC Example 1 in female CD1 mice. Body weights were measured each day and terminal whole blood and sera collected from every animal at termination to measure complete blood counts and blood biochemistry.

24 mice were randomised into 4 groups and dosing commenced the following day. All animals were dosed intravenously with a single dose either PBS (Group 1: vehicle control) or ADC Example 1 (Groups 2, 3 and 4; 25, 50 or 100mg/kg). The study was terminated on day 7. Daily food intake was measured and a full necropsy was performed on all animals, and 2 naïve animals, at study termination.

Significant body weight loss (>10%) was observed for 3 animals from Group 4 (ADC Example 1 100mg/kg) on Study Day 5 and Group 4 were terminated on day 6 (Figure 45). No significant body weight loss was observed in any of the other groups (Figure 45) and no clinical observations were observed for any animal on study.

Whole blood for complete blood counts (CBC) and sera for blood biochemical testing was collected from all animals on study at termination. Figure 46 shows mean values for AST (aspartate transaminase), ALT (alanine transaminase), ALP (alkaline phosphatase), LDH (lactate dehydrogenase), CK (creatine kinase) and GGT (gamma-glutamyl transferase) in blood of animals at termination. Significant changes were only seen in Group 4 (100mg/Kg). Figure 47 shows mean values of cells in the blood of animals at termination: LYM (lymphocytes), MON (monocytes), NEU (neutrophils), RBC (red blood cells), HGB (haemoglobin) and PLT (platelets). Significant changes were only seen in Group 4 (ADC Example 1 100 mg/Kg).

The conclusion is that ADC Example 1 is well tolerated in mice following a single IV dose up to 50mg/Kg which is 10-fold higher than the efficacious dose observed in Biological Examples 2 and 3.

## Biological Example 11: LNCaP prostate cancer xenograft model.

The objective of the study was to evaluate the efficacy of ADC Example 4 in male NOD SCID mice bearing LNCaP tumours.

A total of 84 male NOD SCID mice aged 5-8 weeks and weighing 25-30 g were used for the study. $1x10^7$ LNCaP tumour cells at 78% viability and approximately 70-80% confluency were implanted subcutaneously onto the flank of male NOD SCID mice. When tumours reached approximately 80-100 $mm^3$, animals were assigned to treatment groups as demonstrated below in Table 9, allocating 10 mice per group with a similar mean and distribution of tumour volumes to each group. Mice were treated with vehicle alone, unconjugated ifinatamab, ifinatamab-deruxtecan (ifinatamab-DXd) or ADC Example 4.

Table 9: Dosing regimen for Biological Example 11

| Group | *n* | Treatment | Dose | Route | Dosing frequency |
|---|---|---|---|---|---|
| **1** | 10 | Vehicle (PBS + 0.02% PS80) | - | IV | Q7D (three total doses) |
| **2** | 10 | ifinatamab | 10 mg/kg | IV | Q7D (three total doses) |
| **3** | 10 | ifinatamab | 5 mg/kg | IV | Q7D (three total doses) |
| **4** | 10 | ifinatamab-DXd | 10 mg/kg | IV | Q7D (three total doses) |
| **5** | 10 | ifinatamab-DXd | 5 mg/kg | IV | Q7D (three total doses) |
| **6** | 10 | ADC Example 4 | 10 mg/kg | IV | Q7D (three total doses) |
| **7** | 10 | ADC Example 4 | 5 mg/kg | IV | Q7D (three total doses) |
| Duration of observation: 35 days.<br>Dosing volume: 5 mL/kg for all IV doses | | | | | |

During the course of the study, no adverse responses to any doses were observed and the mean bodyweight of each group remained within 10% of the pre-treatment level (Figure 49).

Individual cases of bodyweight loss >10% were observed at various points in the study. Three weeks after the first animal entered treatment, DietGel was provided to all mice to ameliorate bodyweight loss. No animals were euthanised early due to the bodyweight loss and these instances of weight loss were likely to be associated with tumour burden.

Animals that received three Q7D doses of ADC Example 4 at 10 mg/kg showed a significantly higher mean bodyweight than those receiving vehicle control treatments on day 28 of the study (One way ANOVA, Dunnett's p=0.0046). At this timepoint, no other treatment groups significantly differed from the vehicle in bodyweight.

Two animals were terminated early due to welfare concerns. The first was euthanized on day 26 of treatment with 10 mg/kg ifinatamab, while the second was euthanized on day 33 of treatment with 5 mg/kg ifinatamab-DXd. Both animals were primarily euthanized due to gasping. Necropsies on each recorded large spontaneous thymic tumours.

Tumours in the vehicle treatment group grew steadily during the study, reaching a mean volume of 752 ± 89.4 $mm^3$ by day 28 of the study.

Treatment with ifinatamab at either 10 mg/kg or 5 mg/kg had no significant effect on LNCaP tumour volume at day 28, and animals receiving this therapy showed a largely similar tumour growth curve to those treated with vehicle alone (Figure 48, Table 10).

Treatment with ifinatamab-DXd at 10 mg/kg significantly reduced the mean volume of LNCaP tumours by day 28 compared to the vehicle control. Animals receiving this therapy largely showed a lower rate of tumour growth than control animals. At a dosage of 5 mg/kg ifinatamab-DXd slowed the growth of LNCaP tumours to a lesser extent (Figure 48, Table 10).

All animals that were dosed with 10 mg/kg ADC Example 4 showed tumour regression within three weeks of starting treatment (Figure 48, Table 10) this therapy produced a significant reduction in mean tumour volume (TV) (Mann-Whitney) from day 7 onwards compared to the vehicle. By day 28, each tumour had regressed to ≤25% of their volume at the start of treatment.

Likewise, animals receiving 5 mg/kg ADC Example 4 showed a significant reduction in tumour volume from days 7 to 28 compared to the control group (Mann-Whitney). By day 28 all but one animal showed a lower tumour volume than recorded at the start of treatment.

Table 10. Tumour volume comparison of treatment groups.

| Adjusted p value calculated by Kruskal-Wallis test and Dunn's multiple comparisons relative to vehicle control. TGI% = ((mean(C)-mean (C0))-(mean(T)-mean(T0)))/(mean(C)-mean(C0))x100% where T is the mean tumour volume of the treated group on the measurement day and T0 is the mean tumour volume on Study Day 0. C is the mean tumour volume of the control Group 1 mice on the measurement day and C0 is the mean tumour volume on Study Day 0. | | | |
|---|---|---|---|
| **Treatment Group** | **Day 28 tumour volume ($mm^3$, mean ±SEM)** | **Adj p value vs. vehicle (TV)** | **Tumour growth inhibition (TGI, %)** |
| **G1:** Vehicle | 752.4 ± 89.4 | - | - |
| **G2:** ifinatamab, 10 mg/kg, IV; Q7D (x3) | 666.56 ± 103.2 | >0.9999 (ns) | 13.0 |
| **G3:** ifinatamab, 5 mg/kg, IV; Q7D (x3) | 551.4 ± 73.1 | >0.9999 (ns) | 30.6 |
| **G4:** ifinatamab-DXd, 10 mg/kg, IV; Q7D (x3) | 259.5 ± 50.3 | 0.0251 (*) | 75.0 |
| **G5:** ifinatamab-DXd, 5 mg/kg, IV; Q7D (x3) | 364.6 ± 51.7 | 0. 1836 (ns) | 58.8 |
| **G6:** ADC Example 4, 10 mg/kg, IV; Q7D (x3) | 10.1 ± 2.3 | <0.0001 (****) | 112.4 |
| **G7:** ADC Example 4, 5 mg/kg, IV; Q7D (x3) | 63.2 ± 16.4 | <0.0001 (****) | 104.4 |

**Biological Example 12: VCaP prostate cancer xenograft model.**

The objective of this study was to evaluate preclinically the *in vivo* therapeutic efficacy of ADC Example 4 in the treatment of subcutaneous VCaP human prostate cancer xenograft model in non-castrated male CB17/SCID mice.

In this study, 144 mice were inoculated subcutaneously in the right front flank region with $1x10^7$ viable VCaP tumour cells resuspended in 0.1 mL of PBS mixed with Matrigel (1:1) for tumour development on Study Day -20. 80 mice were assigned to 8 treatment groups when tumour volumes averaged ~162.16 $mm^3$ on Study Day 0. Dosing commenced the following day and all animals were dosed intravenously with ADC Example 4, ifinatamab-deruxtecan (ifinatamab-Dxd) or unconjugated ifinatamab. All mice received two doses of test agent, on Study Day 1 and Study Day 8. The study was

terminated on Study Day 30.
The 8 groups were assigned as follows:

| | |
|---|---|
| Group 1 | Vehicle control |
| Group 2 | Unconjugated ifinatamab 5mpk |
| Group 3 | Unconjugated ifinatamab 2.5mpk |
| Group 4 | ifinatamab-deruxtecan 5mpk |
| Group 5 | ifinatamab-deruxtecan 2.5mpk |
| Group 6 | ADC Example 4 10mpk |
| Group 7 | ADC Example 4 5mpk |
| Group 8 | ADC Example 4 2.5mpk |

No significant body weight loss was observed in any of the animals on study (Figure 51).

There was a significant decrease ($p<0.0001$) in the tumour volumes of mice treated with ADC Example 4 at all concentrations (Group 8, 2.5mg/kg; Group 7, 5mg/kg and Group 6; 10mg/kg), when compared to vehicle alone (Group 1). Ifinatamab-Dxd 5mg/kg (Group 4; $p$=0.0078) showed a significantly higher tumour volume compared to vehicle alone (Group 1), while ifinatamab-Dxd 2.5mg/kg (Group 5; $p$=0.8127) and unconjugated Infinatamab at both concentrations (Group 2; $p$=*0.1104* and Group 3; $p$=0.6703) showed no significant difference compared to vehicle alone (Group 1), see Figure 50.

Mice treated with ADC Example 4 at all three concentrations (Group 6, 10mg/kg; Group 7, 5mg/kg and Group 8, 2.5mg/kg) showed significant decreases in tumour volume compared to all other treatment groups (Groups 2-5; $p<0.0001$). There was also evidence of a dose response, with the greatest tumour volume reduction in Group 6 (10mg/kg), then Group 7 (5mg/kg), followed by Group 8 (2.5mg/kg); significant differences were observed between each group ($p<0.0001$) , see Figure 50.

There was no significant difference in tumour volume between mice treated with ifinatamab-Dxd 2.5mg/kg (Group 5) and Groups 2, 3 and 4 (unconjugated ifinatamab 5mg/kg, unconjugated ifinatamab 2.5mg/kg, ifinatamab-Dxd 5mg/kg, respectively). Mice treated with ifinatamab-Dxd 5mg/kg (Group 4) had significantly higher tumour volume compared to those treated with unconjugated ifinatamab 5mg/kg (Group 2, p=0.0002) and unconjugated ifinatamab 2.5mg/kg (Group 3, $p$=0.0216), see Figure 50.

Increased tumour growth inhibition (ΔTGI) compared to the vehicle group (Group 1) was apparent in all treatment groups. Treatment with 10mg/kg and 5mg/kg ADC Example 4 were the most efficacious when the tumour growth inhibition of all treatment groups was compared (Group 6; ΔTGI = 114.03% and Group 7; ΔTGI = 114.69%), see Table 11.

Table 11. Dosing regimen for Biological Example 12 and Results

| Group | Treatment Description | Test Article Dose (mg/kg) | Schedule | ΔTGI (Study Day 17) |
|---|---|---|---|---|
| **1** | Vehicle - PBS + 0.02% PS80 pH 7.4 | - | QW x 2 weeks | - |
| **2** | Unconjugated ifinatamab | 5 | QW x 2 weeks | 12.44 |
| **3** | Unconjugated ifinatamab | 2.5 | QW x 2 weeks | 8.47 |
| **4** | ifinatamab-Dxd | 5 | QW x 2 weeks | 2.86 |
| **5** | ifinatamab-Dxd | 2.5 | QW x 2 weeks | 5.24 |
| **6** | ADC Example 4 | 10 | QW x 2 weeks | 114.03 |
| **7** | ADC Example 4 | 5 | QW x 2 weeks | 114.69 |
| **8** | ADC Example 4 | 2.5 | QW x 2 weeks | 108.31 |

**Biological Example 13: JIMT-1 Breast cancer xenograft model.**

The objective of this study was to evaluate preclinically the in vivo therapeutic efficacy of ADC Example 3 in the treatment of subcutaneous JIMT-1 human breast xenograft model in female NOD/SCID mice.

In this study, 128 mice were inoculated subcutaneously in the right front flank region with $5x10^6$ viable JIMT-1 tumour cells resuspended in 0.1 mL of PBS for tumour development on Study Day -15. 80 mice were assigned to 8 treatment groups when tumour volumes averaged ~160.66 $mm^3$ on Study Day 0. Dosing commenced the following day

and all animals were dosed intravenously with ADC Example 3, sacituzumab govitecan or unconjugated sacituzumab. The study was terminated on the Study Day 60. The 8 groups were assigned as follows:

| | |
|---|---|
| Group 1 | Vehicle control |
| Group 2 | Unconjugated sacituzumab 5mpk |
| Group 3 | Unconjugated sacituzumab 2.5mpk |
| Group 4 | sacituzumab govitecan 5mpk (ADC Example 3, 5 mg/kg added on Days 27 and 34) |
| Group 5 | sacituzumab govitecan 2.5mpk (ADC Example 3, 5 mg/kg added on Day 27) |
| Group 6 | ADC Example 3 10mpk |
| Group 7 | ADC Example 3 5mpk |
| Group 8 | ADC Example 3 2.5mpk |

Significant body weight loss (>10%) was observed for one animal in Group 7. No significant body weight loss was observed in any of the other groups on study, see Figures 54 and 55. On Study Day 10, one animal in Group 2 was found dead.

There was a significant decrease ($p<0.0001$) in the tumour volumes of mice treated with ADC Example 3 at all concentrations (Group 8, 2.5mg/kg; Group 7, 5mg/kg and Group 6 10mg/kg) when compared to vehicle alone (Group 1). Sacituzumab govitecan at both concentrations (Group 4, 5mg/kg, $p<0.0001$; Group 5 2.5mg/kg, $p=0.0348$) showed a significant decrease in tumour volume compared to vehicle alone (Group 1). There was a significant decrease in tumour volume with unconjugated sacituzumab in Group 2 (5mg/kg, $p=0.0028$), but there was no significant difference with Group 3 (2.5mg/kg, $p=0.0586$) when compared to vehicle alone (Group 1). Data for 5 mg/kg and 10 mg/kg groups are shown in Figure 52. Data for 2.5 mg/kg groups are shown in Figure 53.

Mice treated with ADC Example 3 at all three concentrations (Group 6, 10mg/kg; Group 7, 5mg/kg and Group 8, 2.5mg/kg) showed significant decreases in tumour volume compared to all other treatment groups (Groups 2-5; $p<0.0001$). There was also evidence of a dose response, with Groups 6 (10mg/kg) and 7 (5mg/kg), showing the greatest tumour volume reduction ($p<0.0001$), compared to Group 8 (2.5mg/kg).

While Group 4 sacituzumab govitecan, 5mg/kg showed a significant decrease in tumour volume compared to unconjugated sacituzumab Group 2 (5mg/kg, $p=0.0097$) and Group 3 (2.5mg/kg, $p=0.0499$), there was no significant difference between Group 5 (sacituzumab govitecan, 2.5mg/kg) and Groups 2 ($p=0.8424$) and 3 ($p=0.9995$). There was also no significant difference between the two concentrations of unconjugated sacituzumab Group 2 (5mg/kg) and Group 3 (2.5mg/kg), $p=0.9837$. However, there was a significant difference between the two concentrations of sacituzumab govitecan, Group 4 (5mg/kg) and Group 5 (2.5mg/kg), $p=0.3802$.

Increased tumour growth inhibition (ΔTGI) compared to the vehicle group (Group 1) was apparent in all treatment groups. Treatment with 10mg/kg and 5mg/kg ADC Example 3 were the most efficacious when the tumour growth inhibition of all treatment groups was compared (Group 6; ΔTGI = 121.55% and Group 7; ΔTGI = 122.04%).

Since treatment with sacituzumab govitecan was only partially efficacious in Group 4, additional doses of ADC Example 3 were administered IV at 5mpk on Study Days 27 and 34. This resulted in a significant reduction in tumour volume compared with vehicle control (see Figure 52). Group 5 also received a dose of ADC Example 3 (IV at 5mpk) on Study Day 27 (see Figure 53) although no significant response was observed.

Table 12. Dosing regimen for Biological Example 13 and Results

| Group | Treatment Description | Test Article Dose (mg/kg) | Schedule | ΔTGI (Study Day 28) |
|---|---|---|---|---|
| 1 | Vehicle (PBS + 0.02% polysorbate 80, pH 7.4) | - | QW x 2 weeks | - |
| 2 | Unconjugated sacituzumab | 5 | QW x 2 weeks | 14.44 |
| 3 | Unconjugated sacituzumab | 2.5 | QW x 2 weeks | 12.11 |
| 4 | sacituzumab govitecan | 5 | QW x 2 weeks | 23.39 |
| 5 | sacituzumab govitecan | 2.5 | QW x 2 weeks | 13.13 |
| 6 | ADC Example 3 | 10 | QW x 2 weeks | 121.55 |
| 7 | ADC Example 3 | 5 | QW x 2 weeks | 122.04 |
| 8 | ADC Example 3 | 2.5 | QW x 2 weeks | 74.76 |

**Biological Example 14: In vitro assessment of cytotoxicity of trastuzumab conjugated to NMT inhibitor 1 against a HER2 positive breast cancer cell line, BT474**

Trastuzumab was conjugated to NMT inhibitor 1 (ADC Example 8) and tested for its cytotoxic activity against a HER2 positive breast cancer cell line, BT474 in an *in vitro* assay. Cells were plated in 96 well plates at 10% confluency (8000 for BT474). The following day cells were treated with ADC Example 8 at 50nM, 3.13nM, and 0.2nM and controls in 250nM Sytox Green containing medium. Plates were imaged every four hours for ten days in the IncuCyte S3. Images for % phase (confluence) and green (Sytox Green) were collected and analysed using the IncuCyte software 2022A. Data are presented as green area normalised to phase area ($\mu m^2$) over time whilst cells are in log phase proliferation.

Figure 56 shows ADC Example 8 effectively induces cell killing of BT474 cells at a concentration of 0.2nM, whereas naked trastuzumab and an isotype control IgG conjugated to NMT inhibitor 1 were ineffective at killing BT474 cells under these conditions (see Figures 9 and 11). Trastuzumab-deruxtecan also effectively killed BT474 cells but was less efficacious than ADC Example 8 under these conditions (see Figure 10). Puromycin was included as a positive cytotoxic control.

**Biological Example 15: In vitro assessment of cytotoxicity of trastuzumab conjugated to NMT inhibitor 1 against a HER2 negative breast cancer cell line, MCF7**

Trastuzumab was conjugated to NMT inhibitor 1 (ADC Example 8) and tested for its cytotoxic activity against a HER2 negative breast cancer cell line, MCF7 in an in vitro assay. Cells were plated in 96 well plates at 10% confluency (2000 for MCF7). The following day cells were treated with ADC Example 8 at 50nM, 3.13nM, and 0.2nM and controls in 250nM Sytox Green containing medium. Plates were imaged every four hours for ten days in the IncuCyte S3. Images for % phase (confluence) and green (Sytox Green) were collected and analysed using the IncuCyte software 2022A. Data are presented as green area normalised to phase area ($\mu m^2$) over time whilst cells are in log phase proliferation.

Figures 13, 14, 15 and 57 show ADC Example 8, an isotype control IgG conjugated to NMT inhibitor 1, trastuzumab or trastuzumab-deruxtecan were unable to induce cell killing of HER2 negative MCF7 cells at a concentration up to 50nM under these conditions. This was expected since trastuzumab targets HER2 positive cells. Puromycin was included as a positive cytotoxic control.

Conclusions

The results of Biological Example 1 demonstrate that the tested NMT inhibitor compounds are inhibitors of HsNMT1 and show potent *in vitro* cytotoxic activity.

The results of Biological Example 2 demonstrate that the tested ADC of the invention demonstrates potent *in vivo* cytotoxic activity and has improved *in vivo* tolerability when compared to NMT inhibitor 1.

The results of Biological Example 3 demonstrate that the tested ADC of the invention, ADC Example 1, demonstrates improved efficacy compared with other treatment groups (see Table 4) in respect of inhibiting tumor growth in a gastric cancer xenograft mouse model, with no significant body weight loss observed.

The results of Biological Example 4 demonstrate that the tested ADC of the invention, ADC Example 1, effectively induces cell killing of HER2 positive breast cancer cell line BT474. The ADC of the invention was more effective at inducing cell killing compared with the other compounds tested: trastuzumab, trastuzumab deruxtecan and Isotype control.

The results of Biological Example 6 show that ADC Example 1 was the most efficacious at inducing bystander killing compared with other ADCs and the controls in eGFP+MCF7 cells which were co-cultured with HER2 positive BT474 cells.

The results of Biological Example 7 show that various ADCs of the invention (specifically ADC Examples 1 and 3-5) have cytotoxic activity against a range of cancer cell lines as measured in a cell viability assay. The various ADCs of the invention (specifically ADC Examples 1 and 3-5) had improved cytotoxicity compared with respective controls as shown by the data in Figures 18 to 27.

The results of Biological Example 8 show that in the majority of the gastric cancer organoids, ADC Example 1 is the most efficacious as shown by its IC50 value compared with the controls. In certain organoids, ADC Example 1 had comparable activity to trastuzumab-DXd as shown by the IC50 values.

The results of Biological Example 9 indicate that ADC Example 1 was well tolerated in a monkey model, even at the highest dose of 20 mg/kg/day, and there were no significant changes in haematological or clinical chemistry parameters.

The results of Biological Example 10 indicate that ADC Example 1 is well tolerated in mice following a single IV dose up to 50mg/Kg which is 10 fold higher than the efficacious dose observed in Biological Examples 2 and 3.

The results of Biological Example **11** indicate that ADC Example 4 is well tolerated in mice at both 5 mg/kg and 10 mg/kg doses in a LNCaP prostate cancer xenograft model. Animals receiving these doses of ADC Example 4 showed a

significant reduction in tumour volume, unlike treatment with ifinatamab alone which had no significant effect on tumour volume. ADC Example 4 also performed better than ifinatamab-DXd at both doses.

The results from Biological Example 12 indicate that ADC Example 4 is well tolerated in mice at 2.5 mg/kg, 5 mg/kg and 10 mg/kg doses in a VCaP human prostate cancer xenograft model. ADC Example 4 reduced tumour volumes more than ifinatamab-Dxd and unconjugated Infinatamab. Indeed using unconjugated Infinatamab showed no significant difference compared to vehicle alone. ADC Example 4 resulted in the most increased tumour growth inhibition compared with the controls, see Table 11.

The results from Biological Example 13 indicate that ADC Example 3 is well tolerated in mice at 2.5 mg/kg, 5 mg/kg and 10 mg/kg doses in a JIMT-1 human breast xenograft model. Mice treated with ADC Example 3 at all three concentrations showed significant decreases in tumour volume compared to all other treatment groups, also shown by the ΔTGI results in Table 12 wherein ADC Example 3 resulted in the most increased tumour growth inhibition.

The results of Biological Example 14 demonstrate that the tested ADC of the invention, ADC Example 8, effectively induces cell killing of HER2 positive breast cancer cell line BT474. The ADC of the invention was more effective at inducing cell killing compared with the other compounds tested: trastuzumab, trastuzumab deruxtecan and Isotype control.

Therefore, overall the ADCs of the invention are expected to be useful as medicaments, particular in the treatment of hyperproliferative disorders such as cancer.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

All patents and patent applications referred to herein are incorporated by reference in their entirety.

## Claims

1. An antibody drug conjugate (ADC) comprising a NMT inhibitor conjugated to an antibody *via* a linker, or a salt thereof, wherein the antibody binds to HER2.

2. The ADC or salt thereof according to claim 1, wherein the linker is a cleavable linker, for example wherein the linker is cleavable by an enzyme.

3. The ADC or salt thereof according to claim 1 or claim 2, wherein the linker has the following formula (VII):

$$-A_a-W_w-Y_y- \quad \text{(formula (VII))}$$

wherein:

A is a first stretcher unit which when present forms a covalent bond with a chain terminus (such as a N-terminus) or a functional group of an amino acid side chain of the antibody;
a is 0 or 1;
each W is independently an amino acid unit or a glucuronide unit which when A and/or Y are absent forms a covalent bond with a chain terminus (such as a N-terminus) or a functional group of an amino acid side chain of the antibody and/or with a functional group of the NMT inhibitor respectively;
when W is an amino acid, w is 1 to 12;
when W is a glucuronide unit, w is 1 or 2;
Y is a second stretcher unit which when present forms a covalent bond with a functional group of the NMT inhibitor; and
y is 0 or 1.

4. The ADC or salt thereof according to claim 3, wherein the functional group on the amino acid side chain of the antibody is an amino or sulfhydryl, for example a sulfhydryl.

5. The ADC or salt thereof according to claim 3 or claim 4, wherein the functional group on the NMT inhibitor is an amino or alcohol, for example an amino.

6. The ADC or salt thereof according to any one of claims 1 to 5, wherein the antibody is a humanised, chimeric, human antibody or an antibody fragment.

**7.** The ADC or salt thereof according to any one of claims 1 to 6, wherein the antibody antibody can bind HER2 with a $K_D$ equal to or less than about $10^{-6}$ M, such as 1 x $10^{-6}$, 1 x $10^{-7}$, 1 x $10^{-8}$, 1 x $10^{-9}$, 1 x $10^{-10}$, 1 x $10^{-11}$, 1 x $10^{-12}$, 1 x $10^{-13}$ or 1 x $10^{-14}$ M.

**8.** The ADC or salt thereof according to any one of claims 1 to 7, wherein the antibody is trastuzumab, pertuzumab, margetuximab, ertumaxomab, MM-111, HER2Bi-aATCs, MCLA-128, ZW25, MDX-210, ado-trastuzumab or fam-trastuzumab,

**9.** The ADC or salt thereof according to claim 8, wherein the antibody is trastuzumab.

**10.** The ADC or salt thereof according to any one of claims 1 to 7, wherein the antibody has the CDRs of trastuzumab, and in particular has the CDRs of the amino acid sequences of SEQ ID NO: 1 and SEQ ID NO: 2.

**11.** The ADC of salt thereof according to any one of claims 1 to 10, wherein the drug loading (p) of NMT inhibitor to antibody is between 1 to 10 NMT inhibitor(s) to antibody, such as between 2 and 6, 4 and 6, 8 and 10, or 6 and 8 NMT inhibitors to antibody.

**12.** A pharmaceutical composition comprising the ADC or a pharmaceutically acceptable salt thereof according to any one of claims 1 to 11.

**13.** The ADC or pharmaceutically acceptable salt thereof according to any one of claims 1 to 11, or pharmaceutical composition according to claim 12, for use as a medicament.

**14.** The ADC or pharmaceutically acceptable salt thereof, or pharmaceutical composition, for use according to claim 13, for use in the treatment of cancer.

**15.** The ADC or pharmaceutically acceptable salt thereof, or pharmaceutical composition, for use according to claim 14, wherein the cancer is breast cancer, bladder cancer, lung cancer, prostate cancer, kidney cancer, esophageal carcinoma, colorectal cancer, gallbladder carcinoma, brain tumor, lymphoma (such as B-cell lymphoma or disuse large B-cell lymphoma), leukemia (such as AML) or neuroblastoma.

**16.** The ADC or pharmaceutically acceptable salt thereof, or pharmaceutical composition, for use according to claim 15, wherein the cancer is breast cancer, prostate cancer, gastric cancer, or lung cancer.

Figure 1

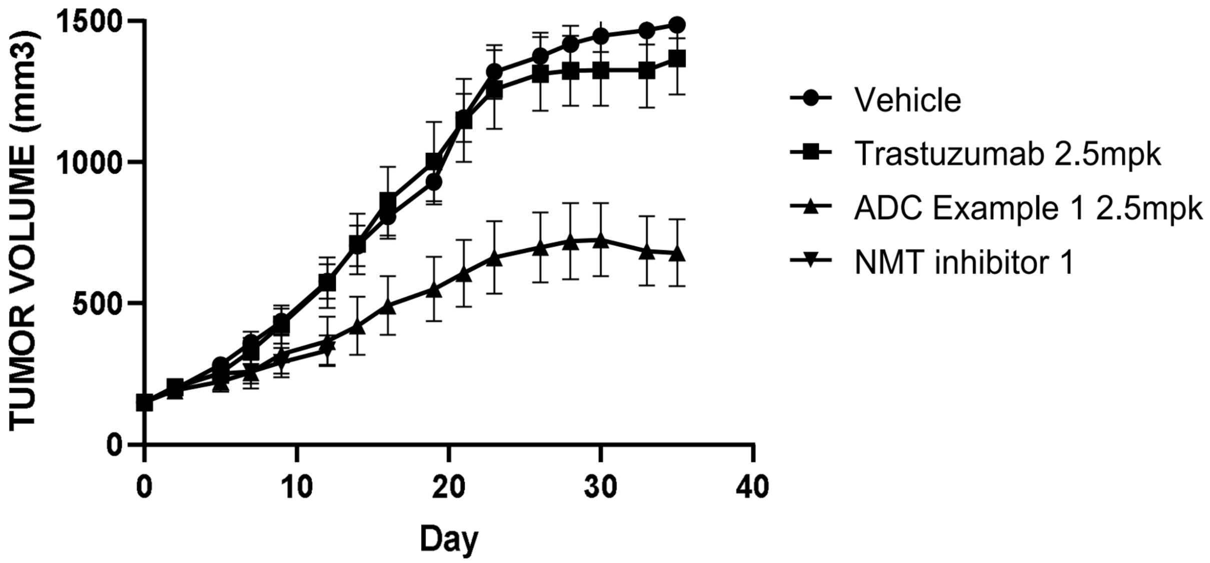


Figure 2

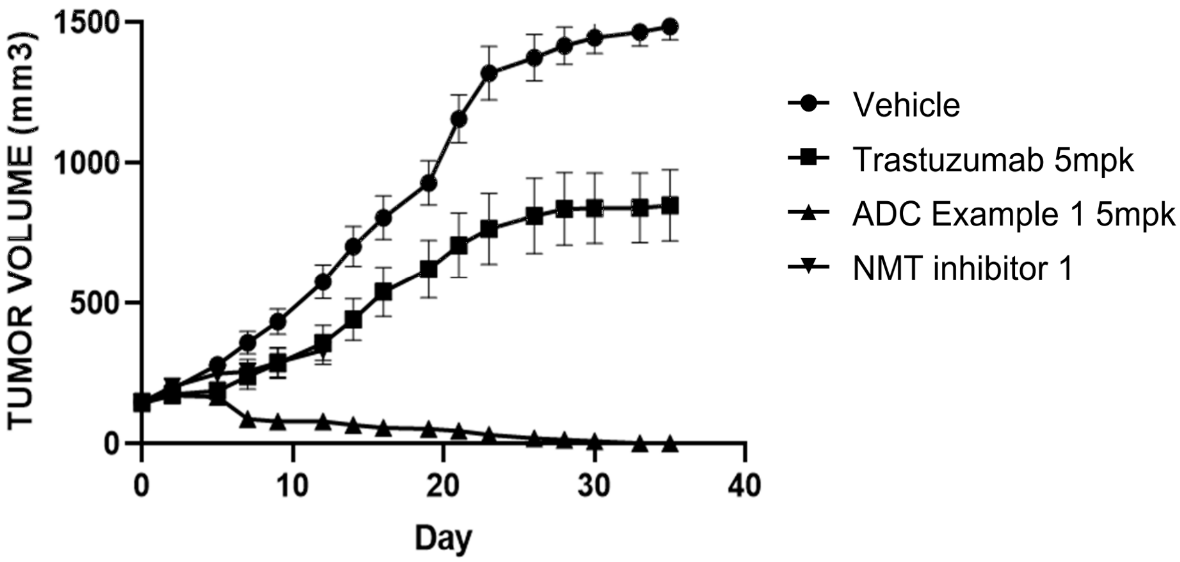

Figure 3

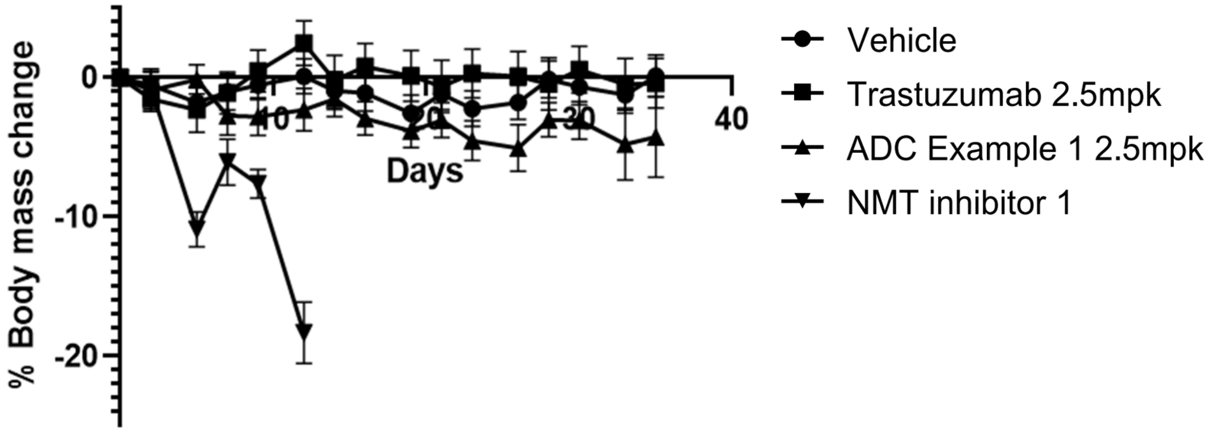


Figure 4

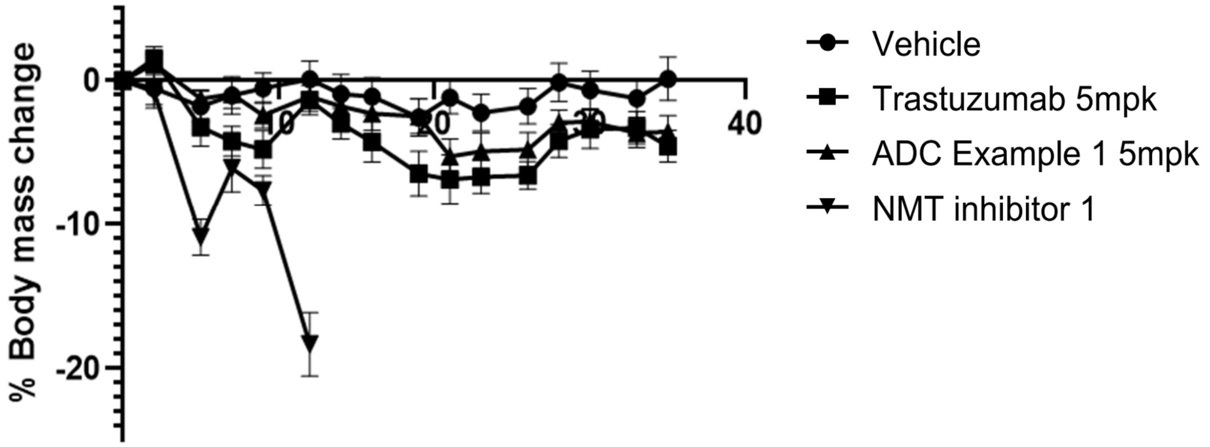

Figure 5

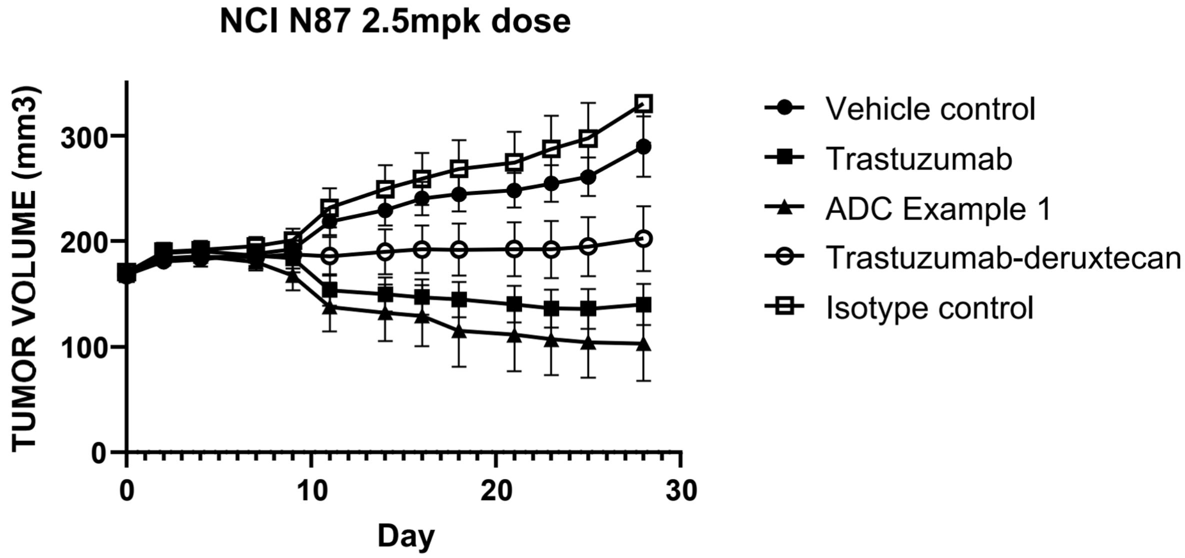


Figure 6

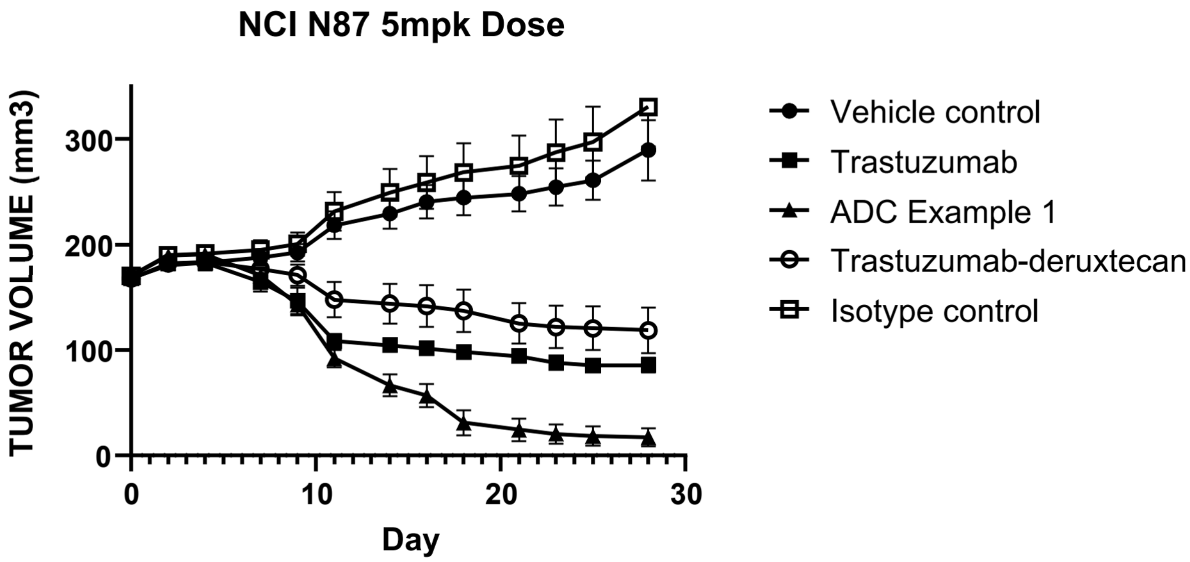

Figure 7A

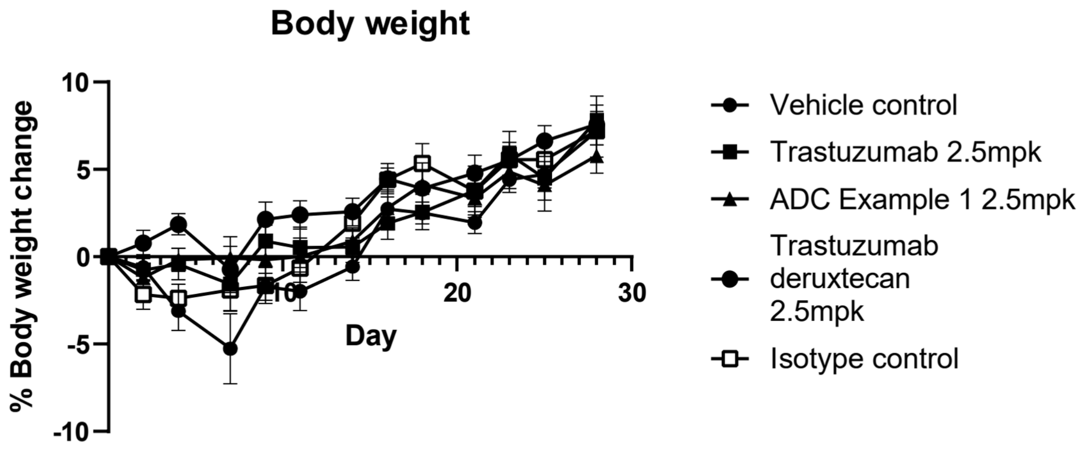


Figure 7B

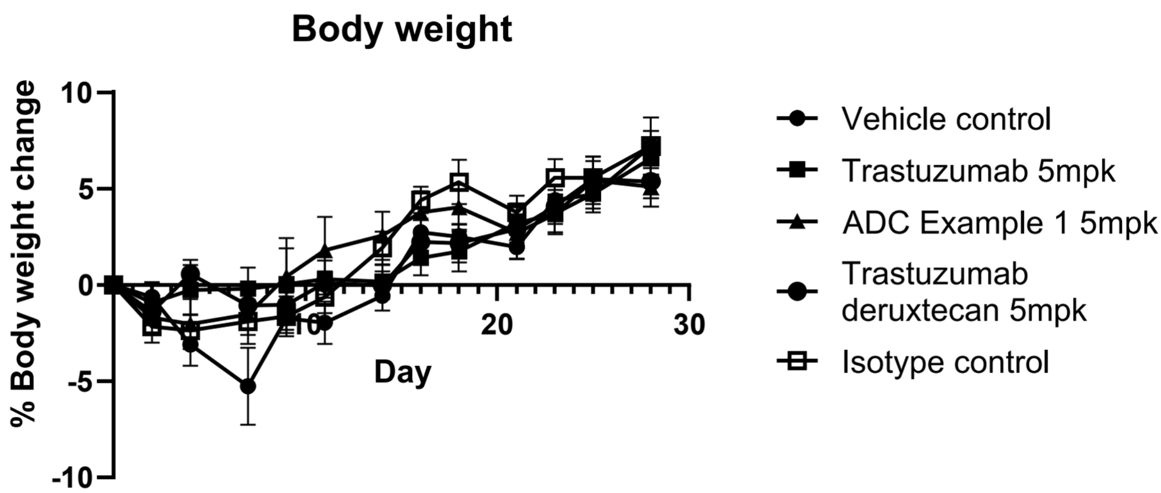


Figure 8

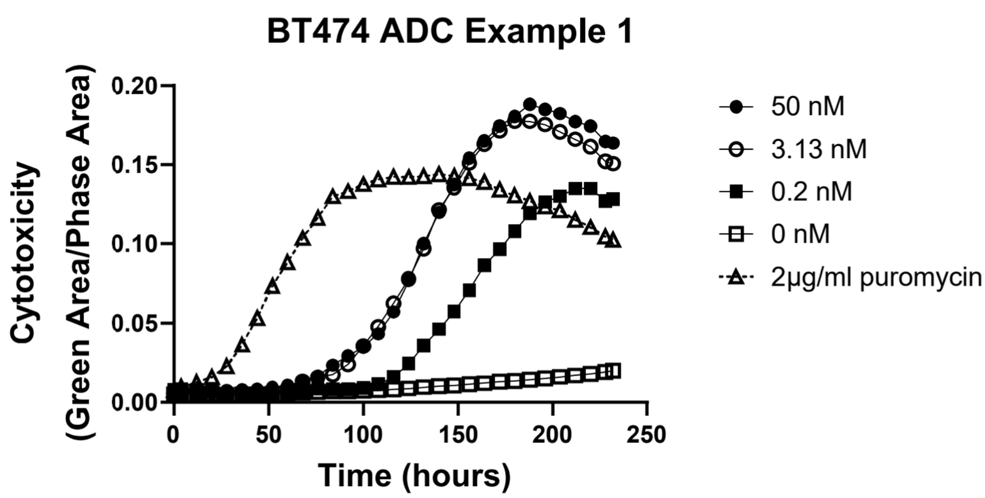

Figure 9

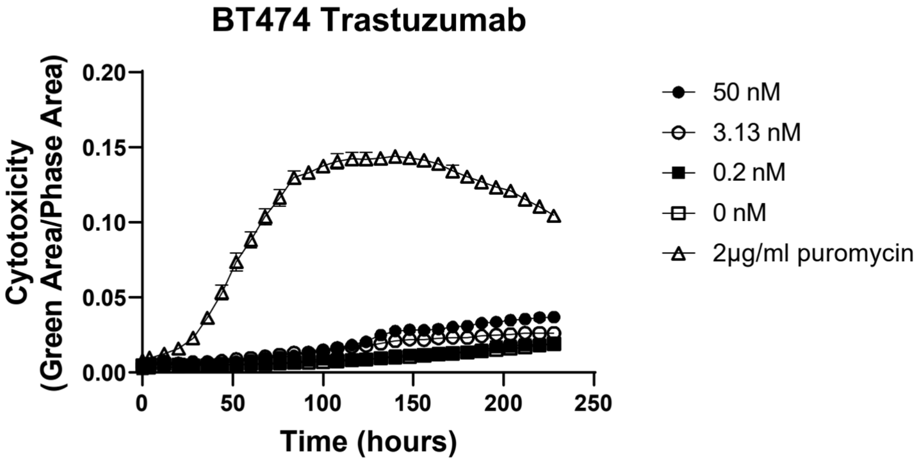


Figure 10

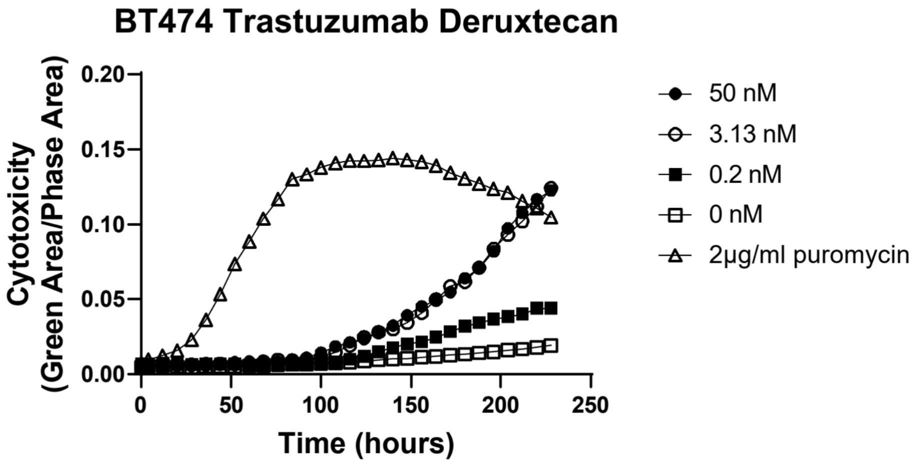


Figure 11

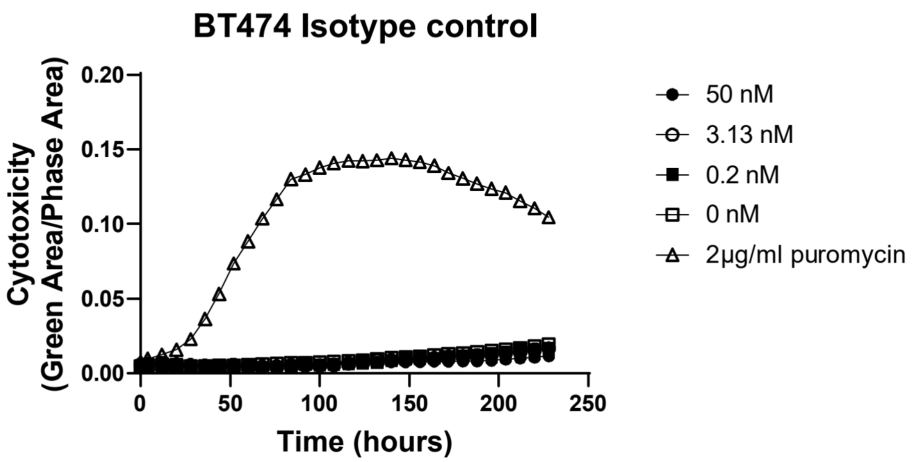

Figure 12

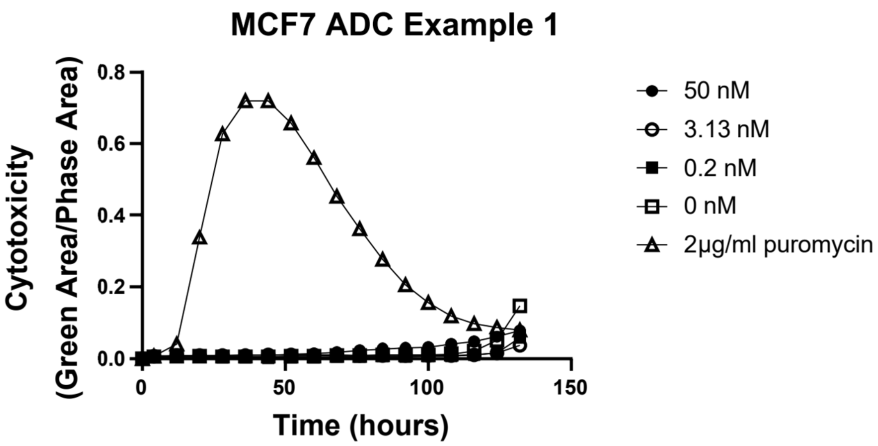


Figure 13

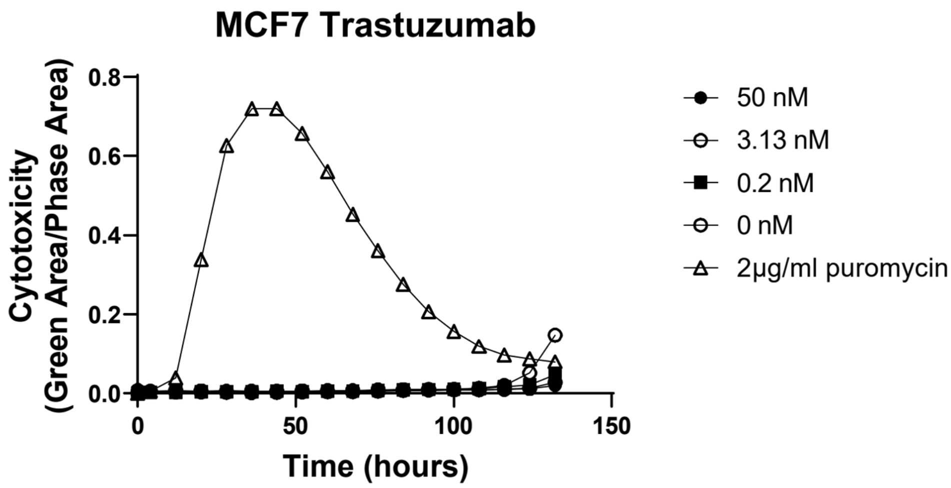


Figure 14

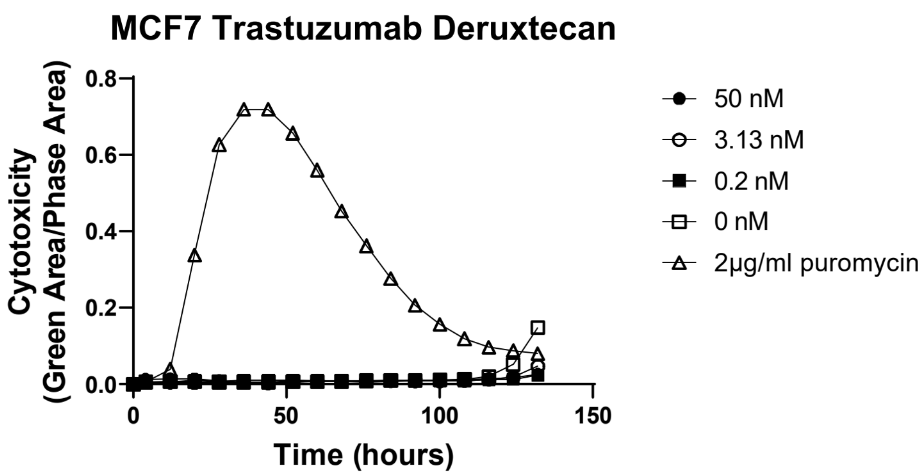

Figure 15

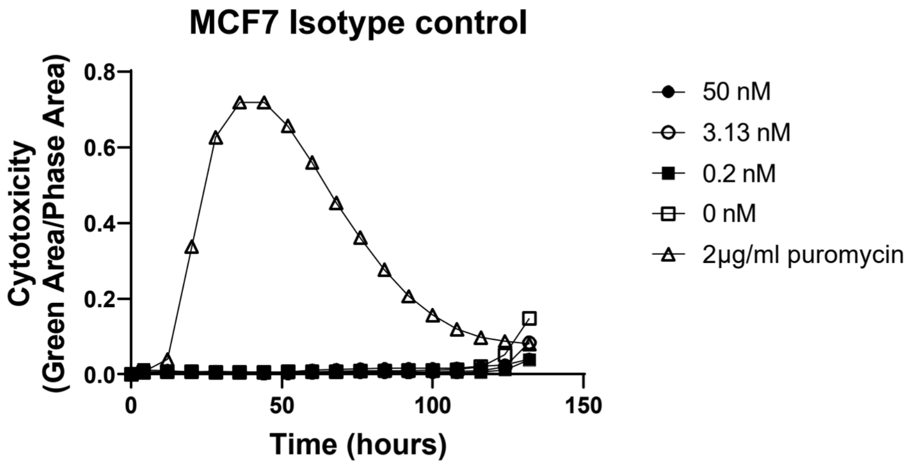


Figure 16

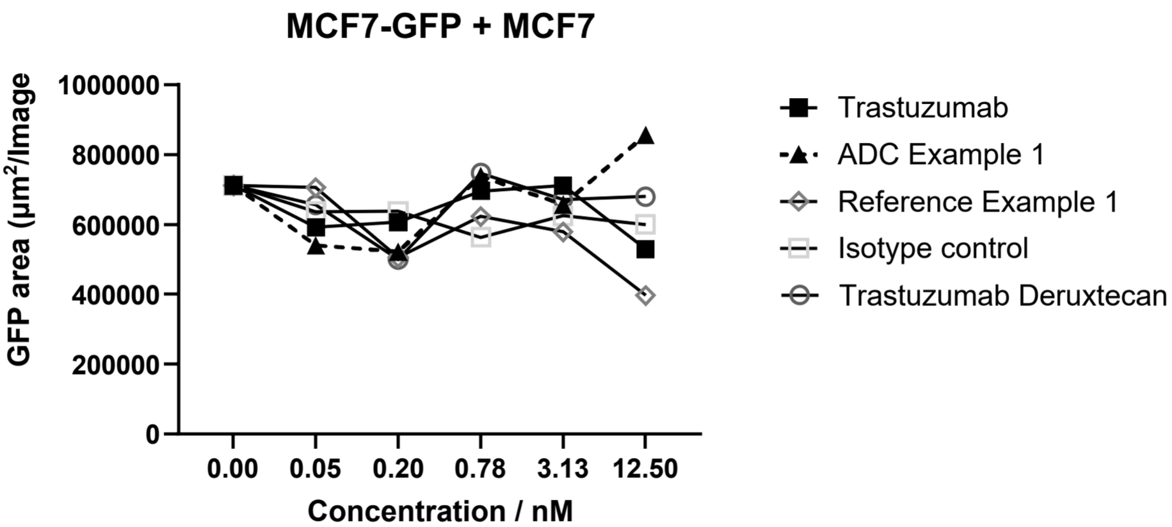


Figure 17

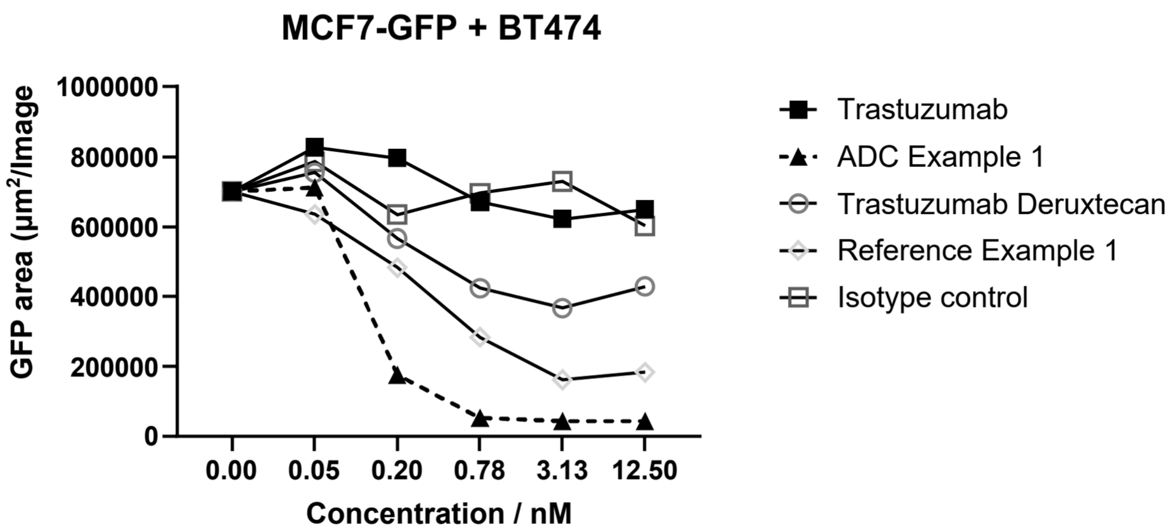

Figure 18

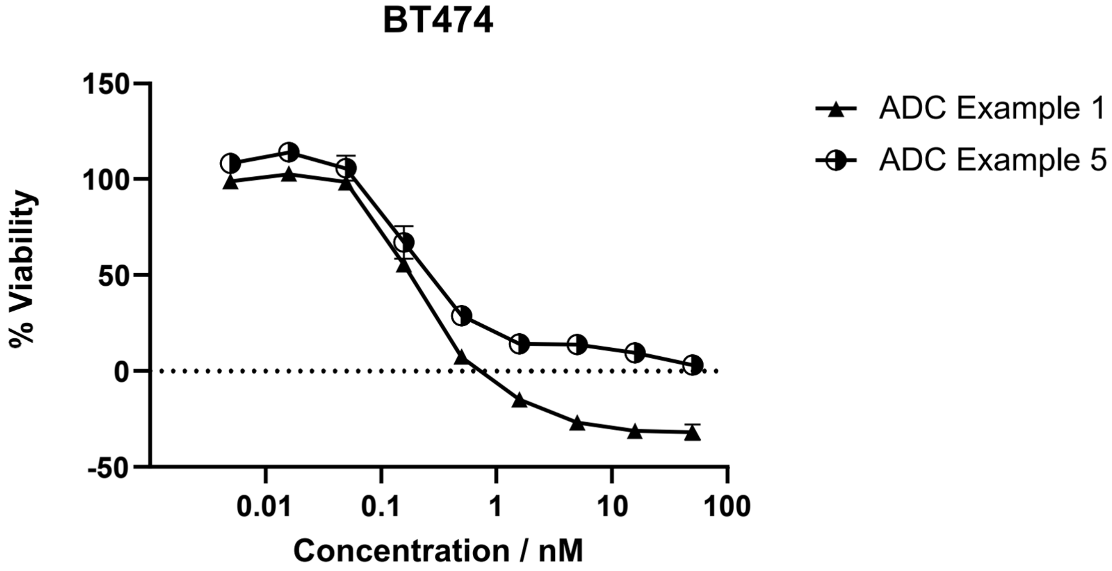


Figure 19

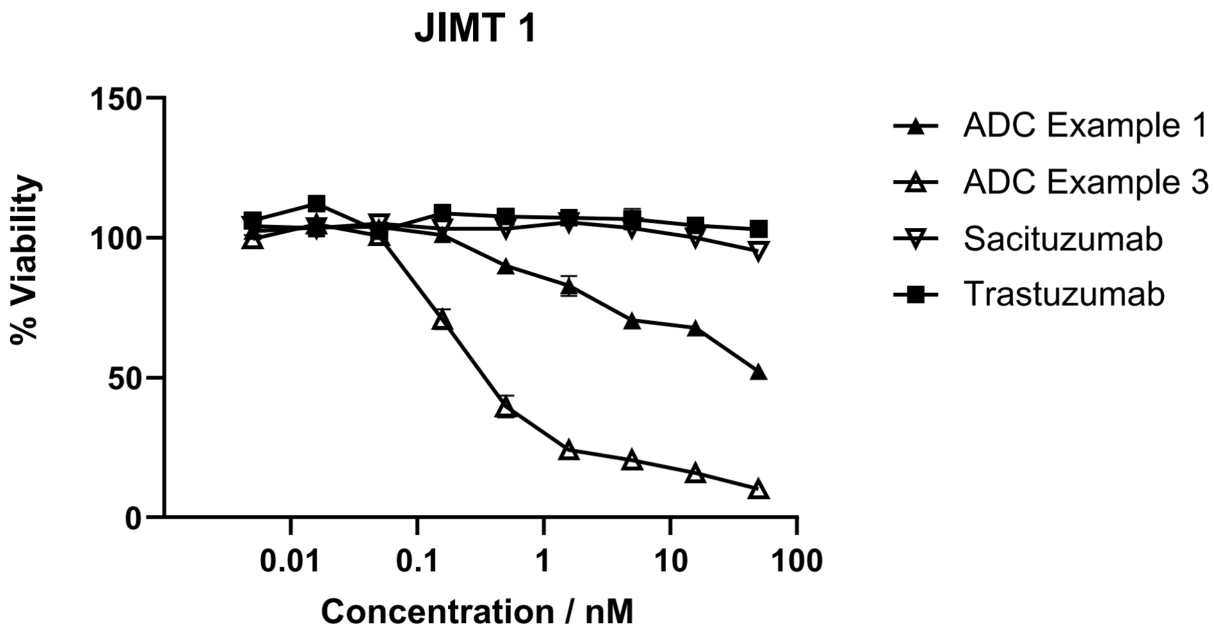

Figure 20

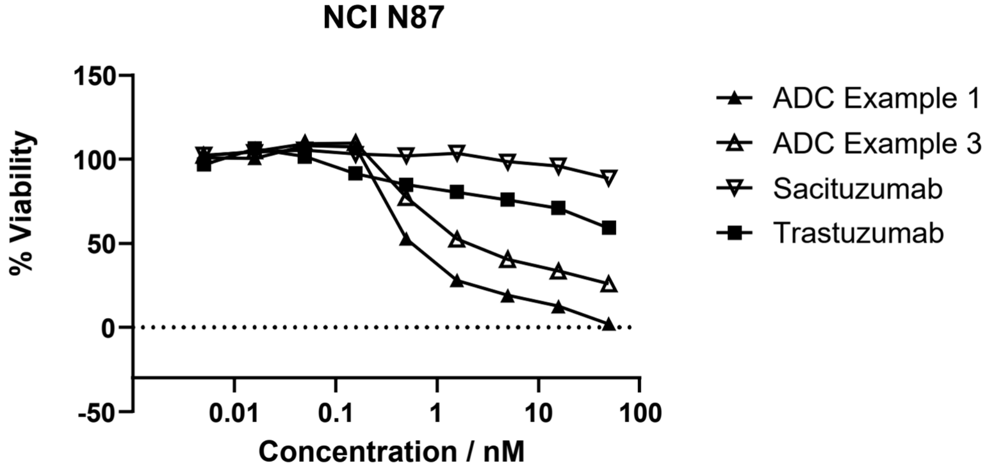


Figure 21

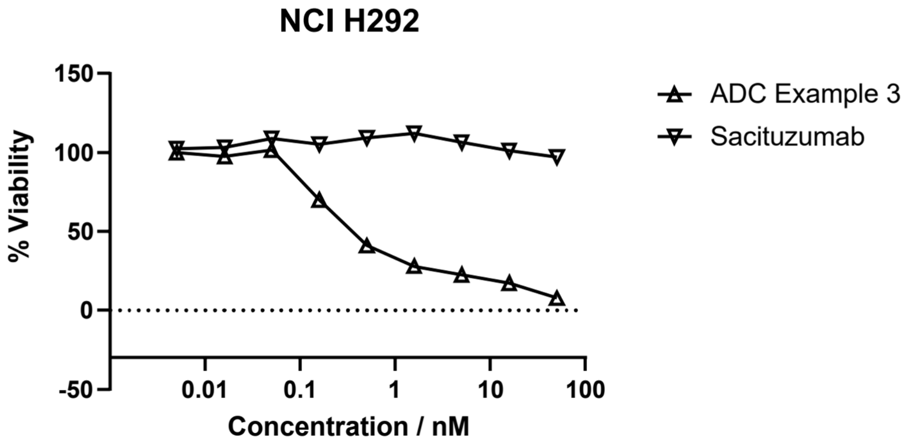


Figure 22

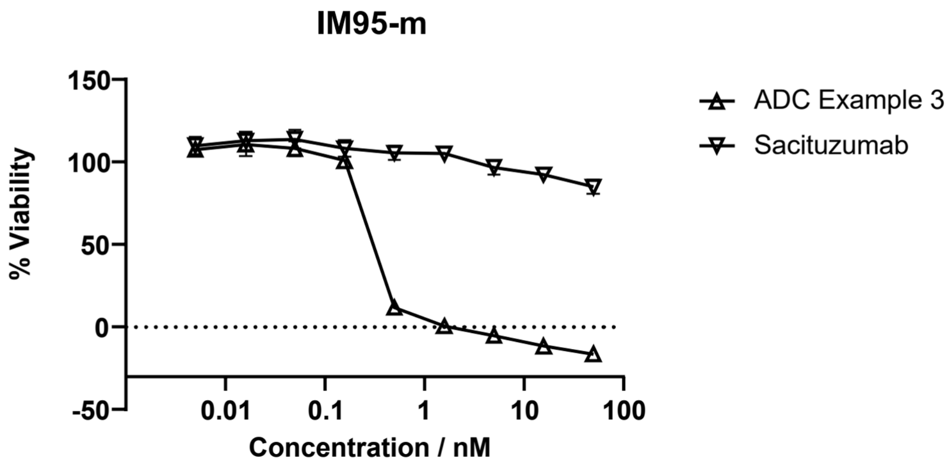

Figure 23

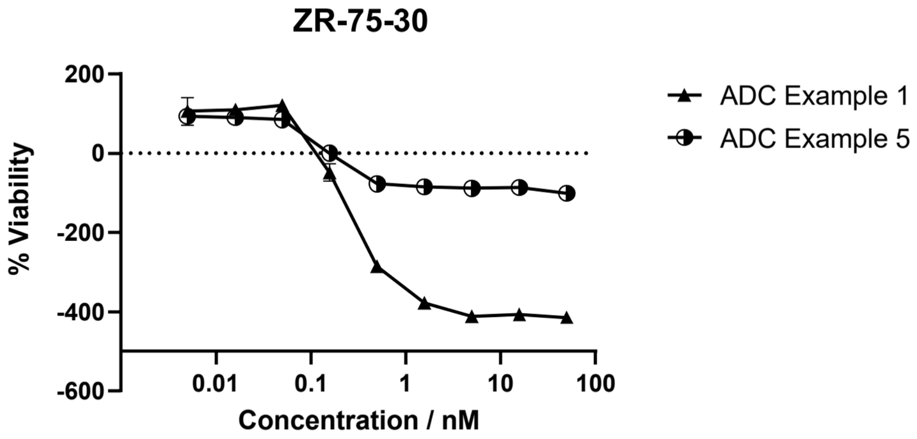


Figure 24

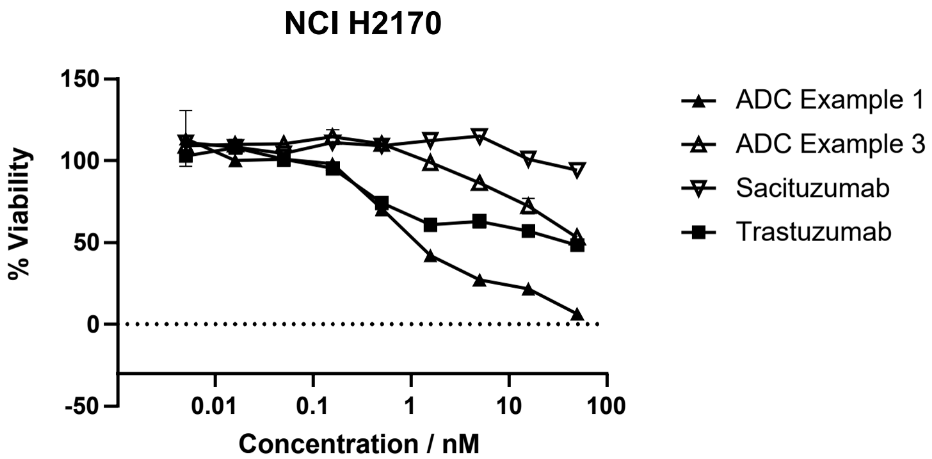


Figure 25

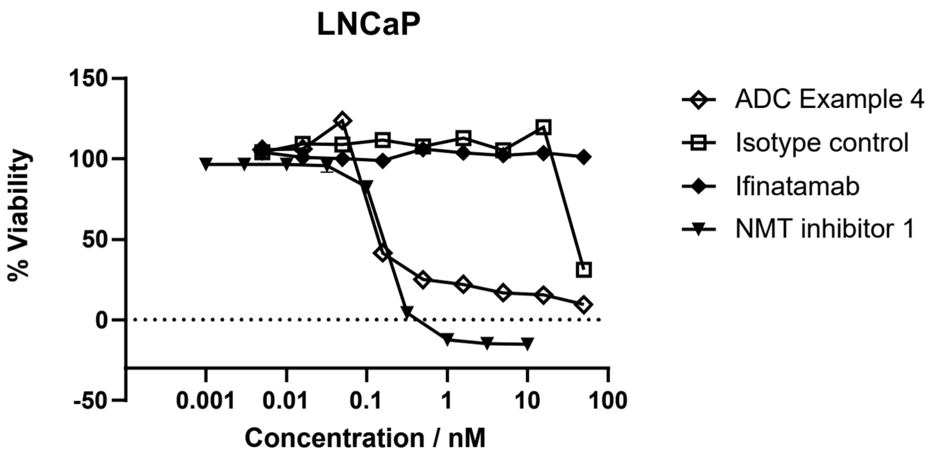

Figure 26

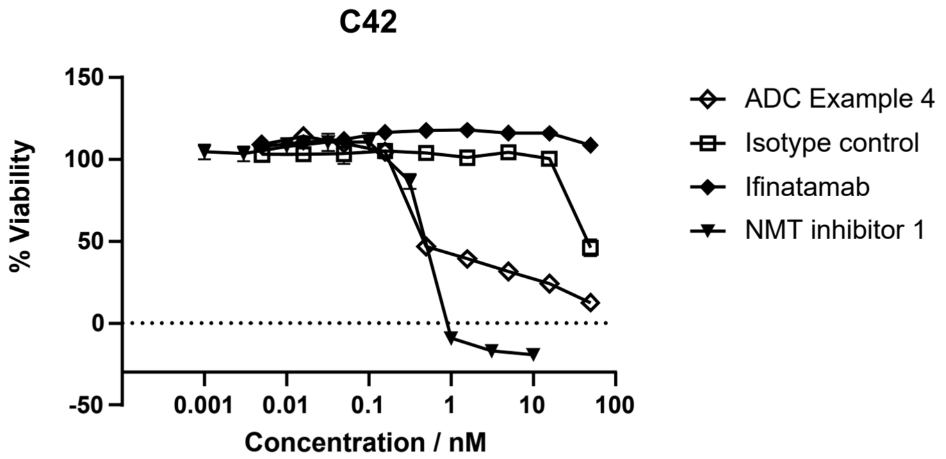


Figure 27

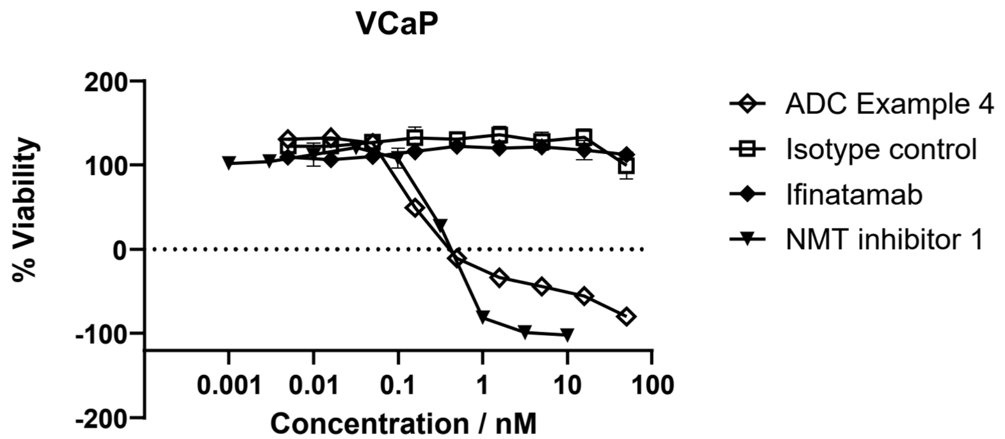


Figure 28

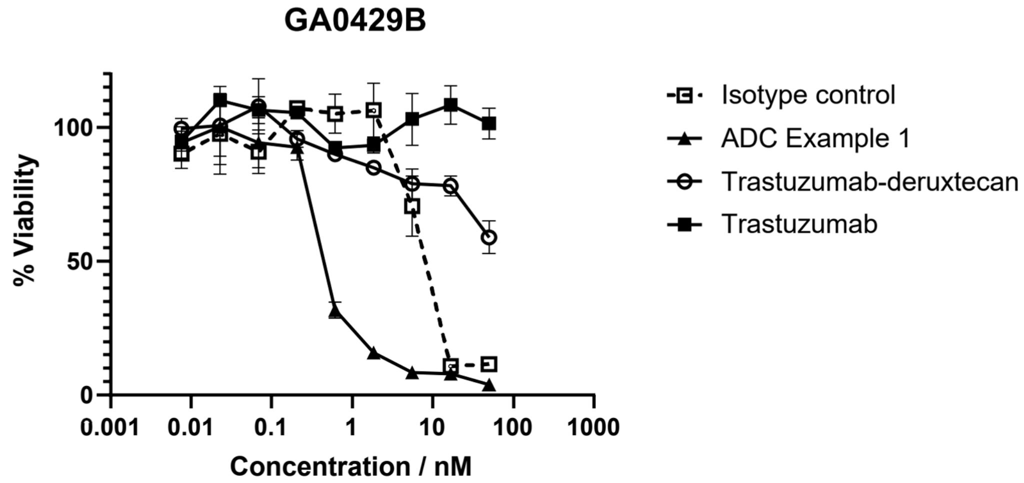

Figure 29

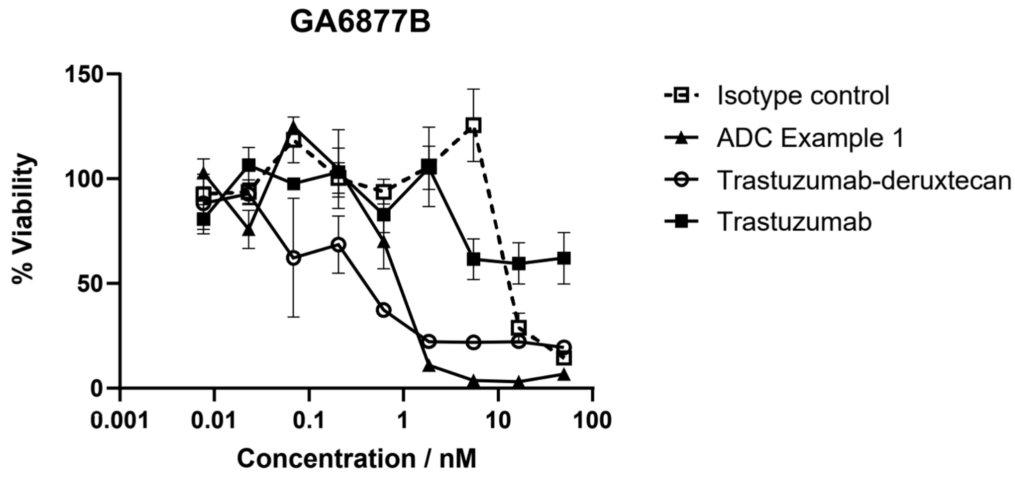


Figure 30

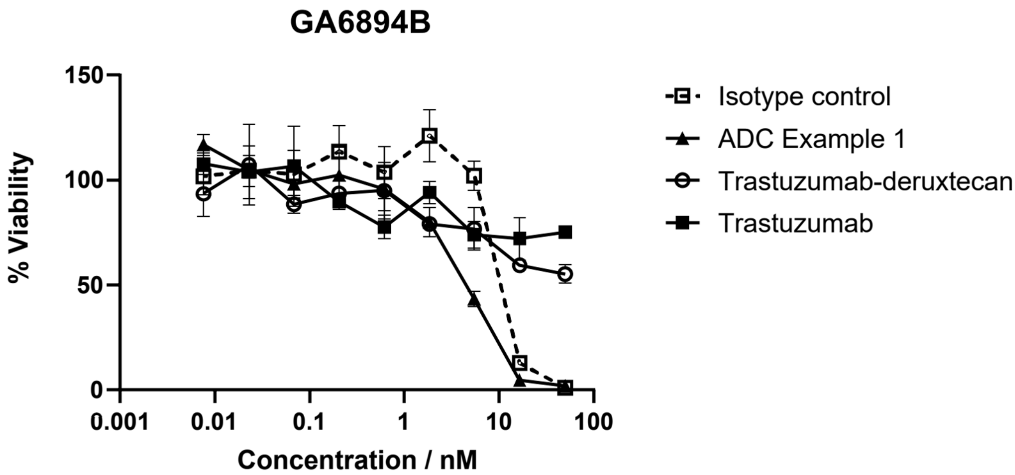


Figure 31

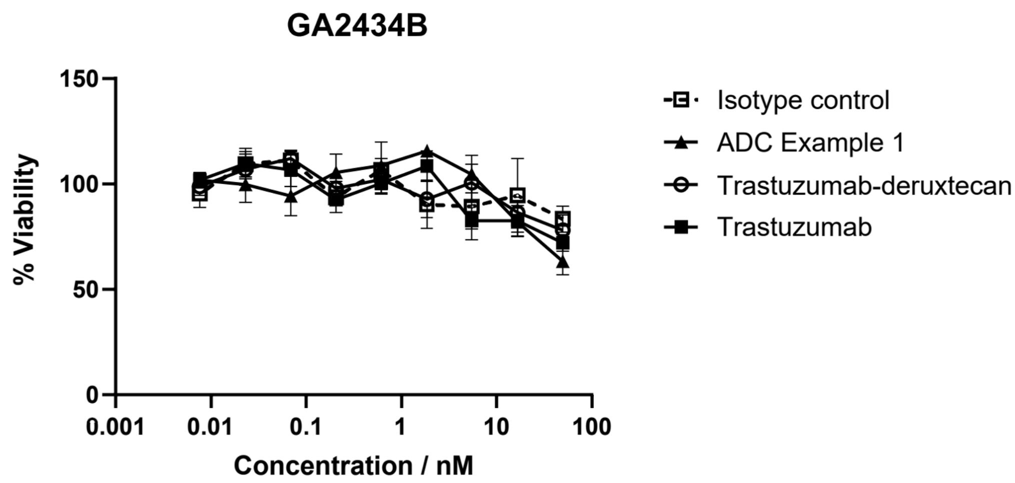

Figure 32

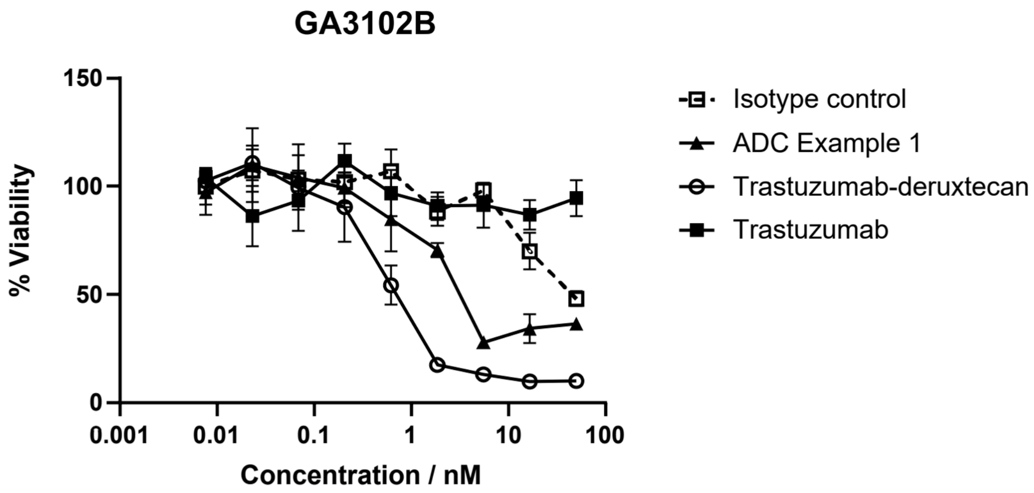


Figure 33

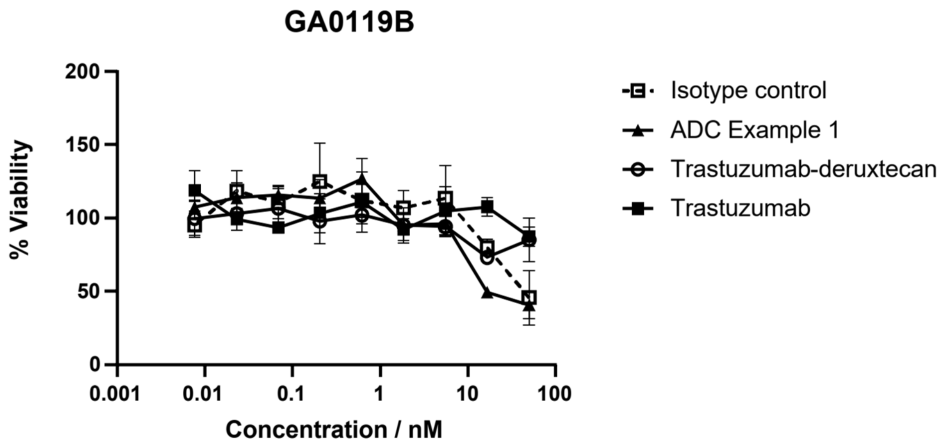


Figure 34

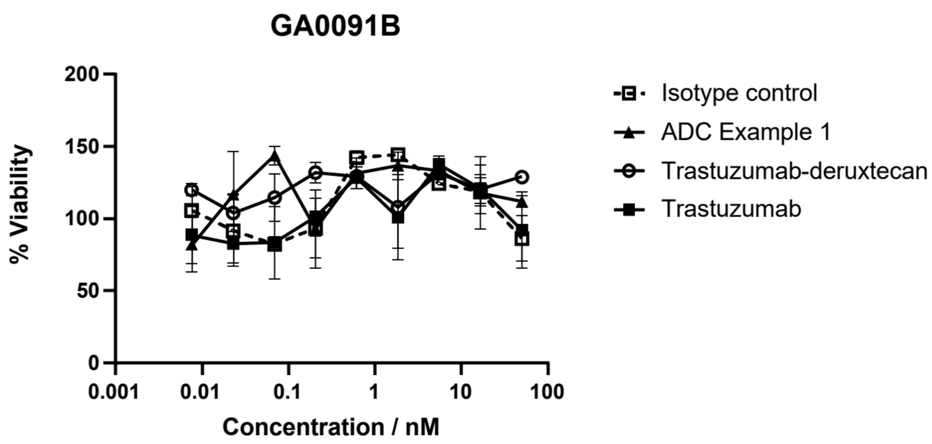

Figure 35

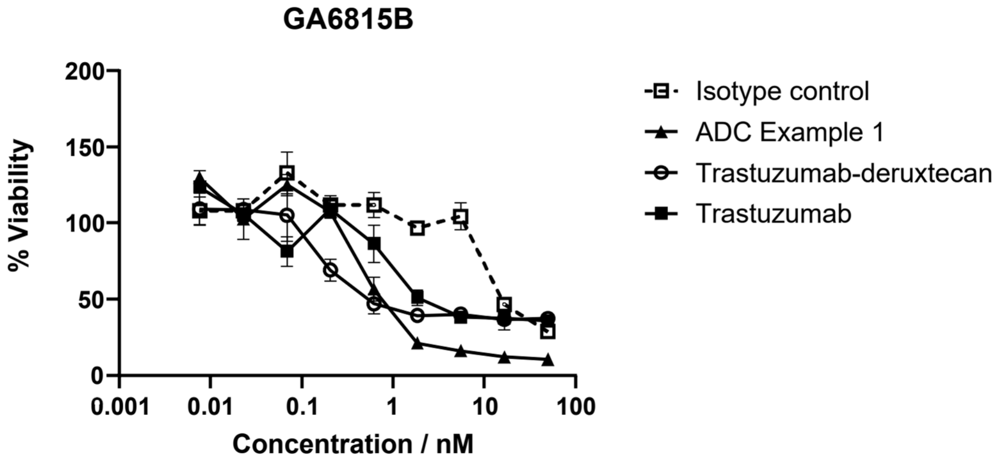


Figure 36

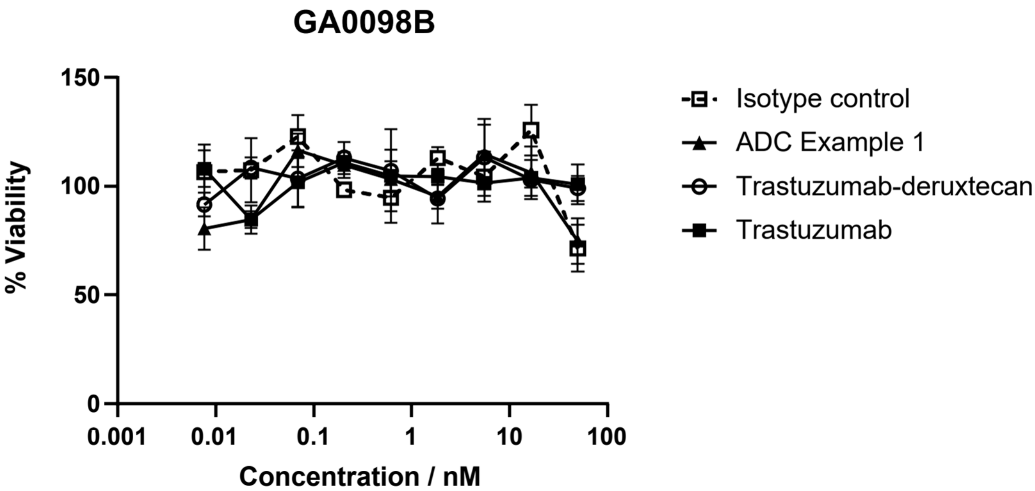


Figure 37

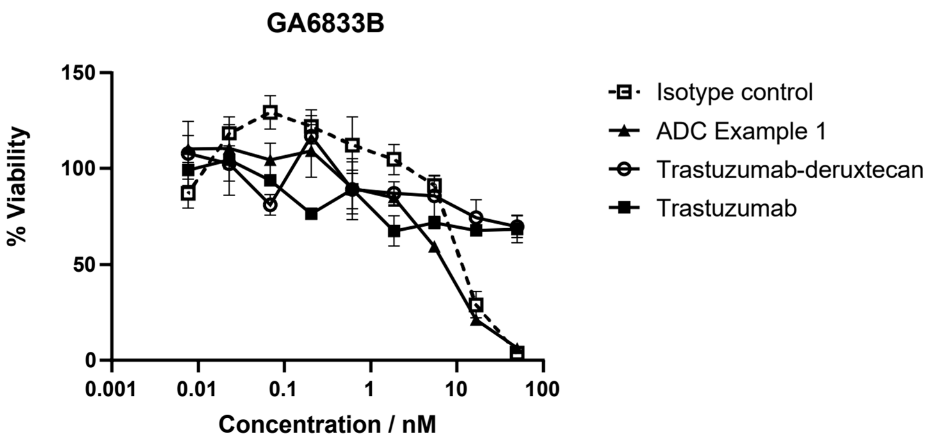

Figure 38

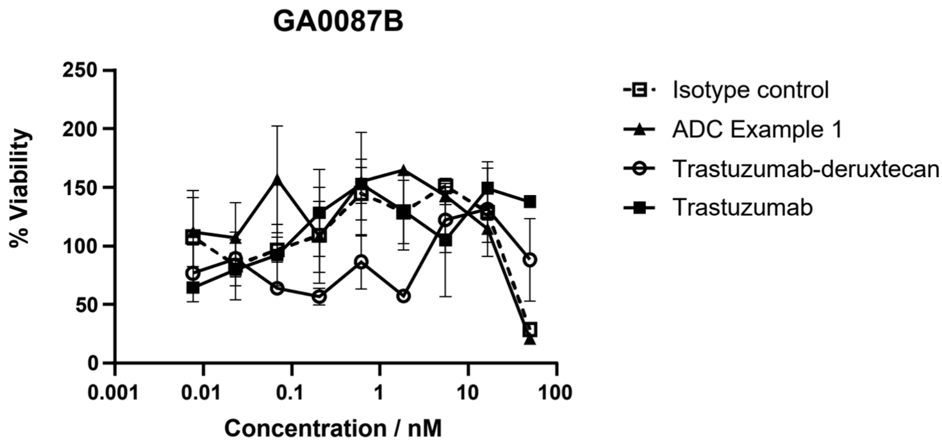


Figure 39

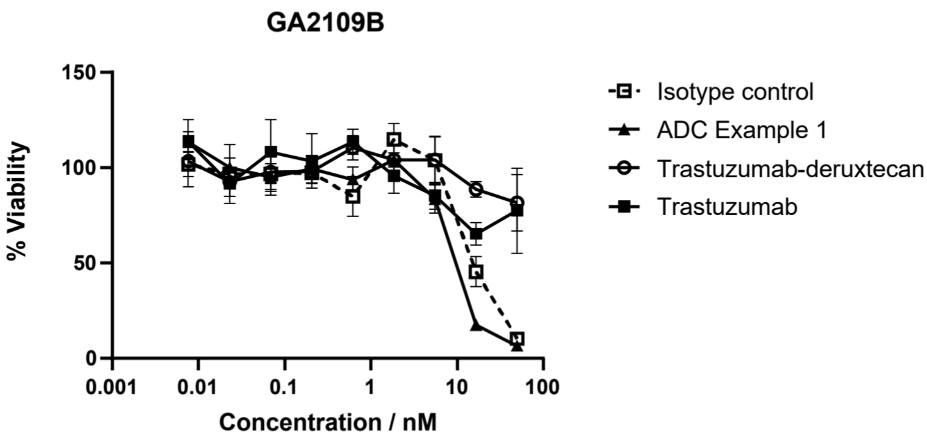


Figure 40

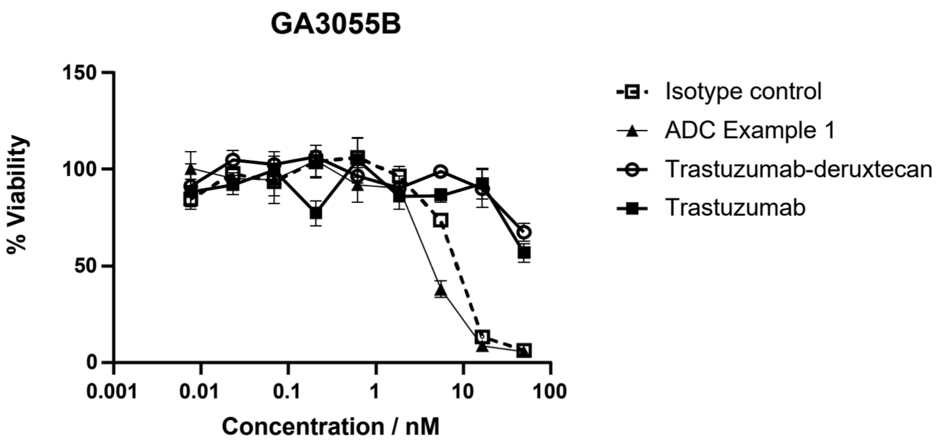

Figure 41

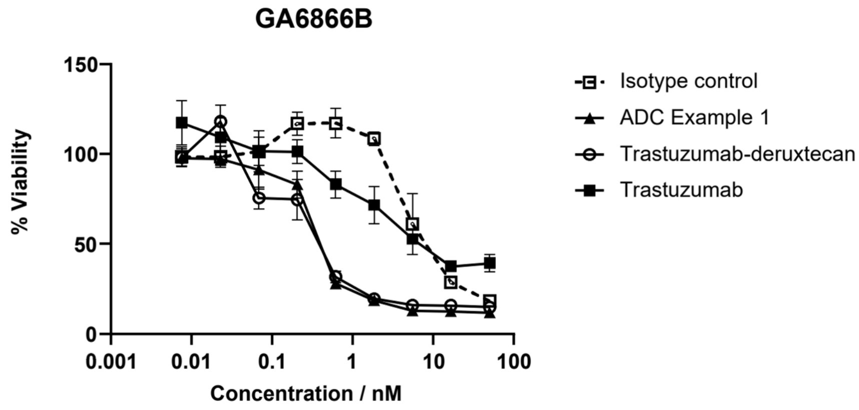


Figure 42

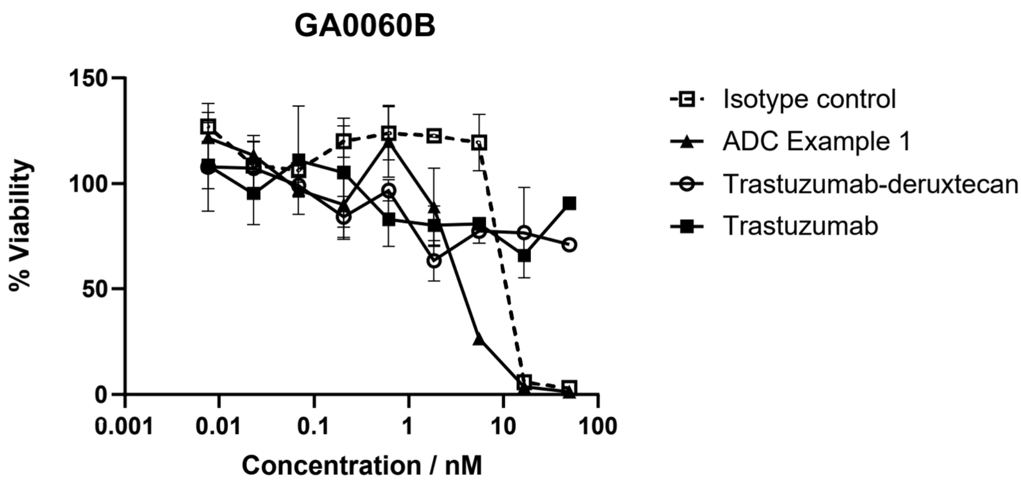


Figure 43

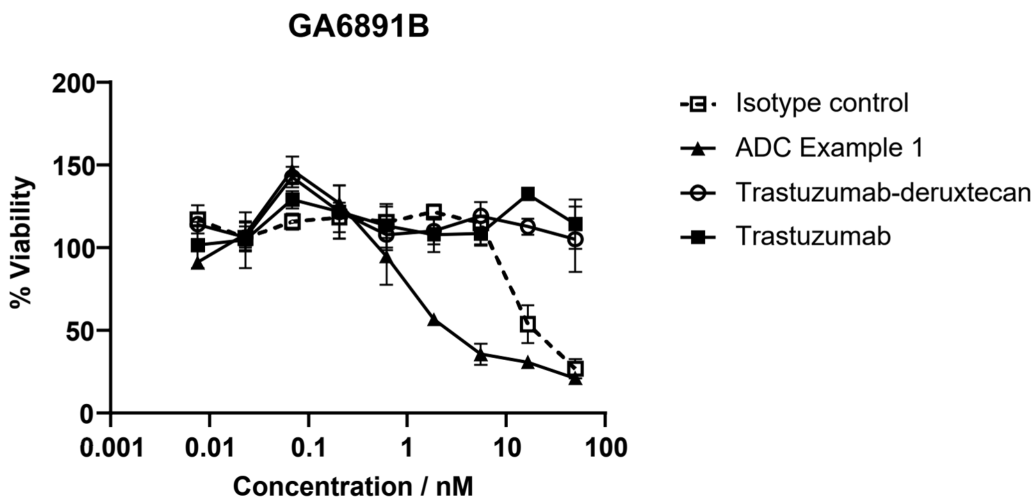

Figure 44a

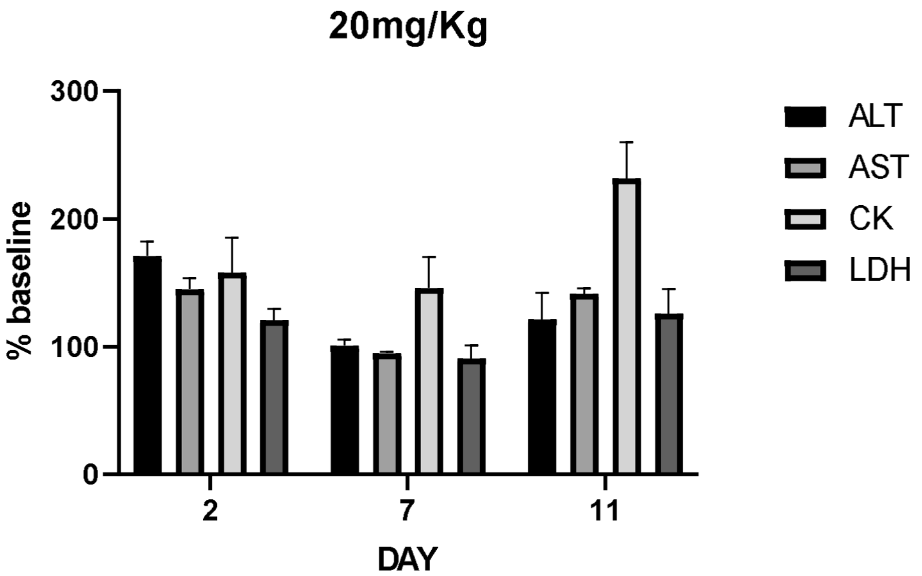


Figure 44b

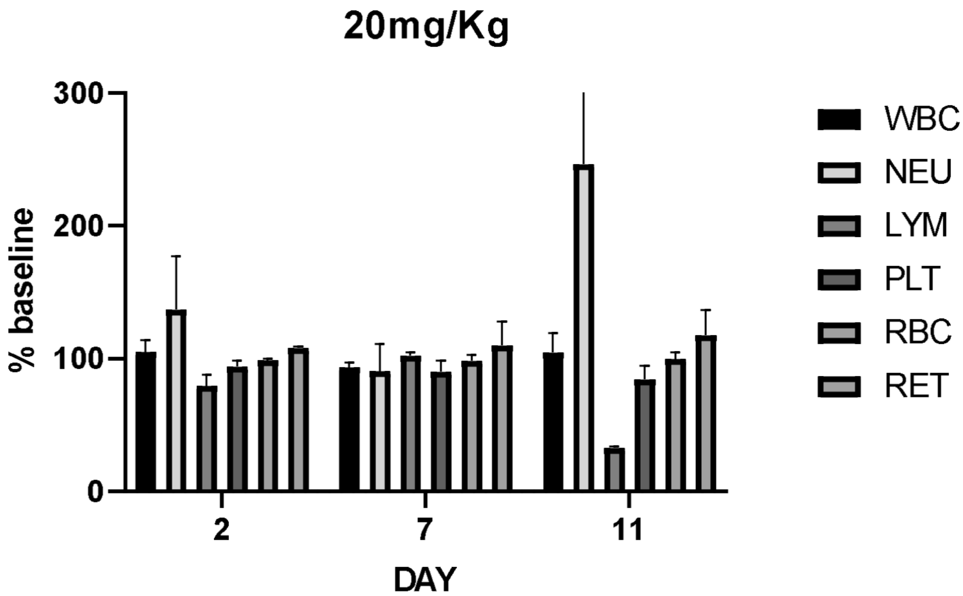

Figure 45

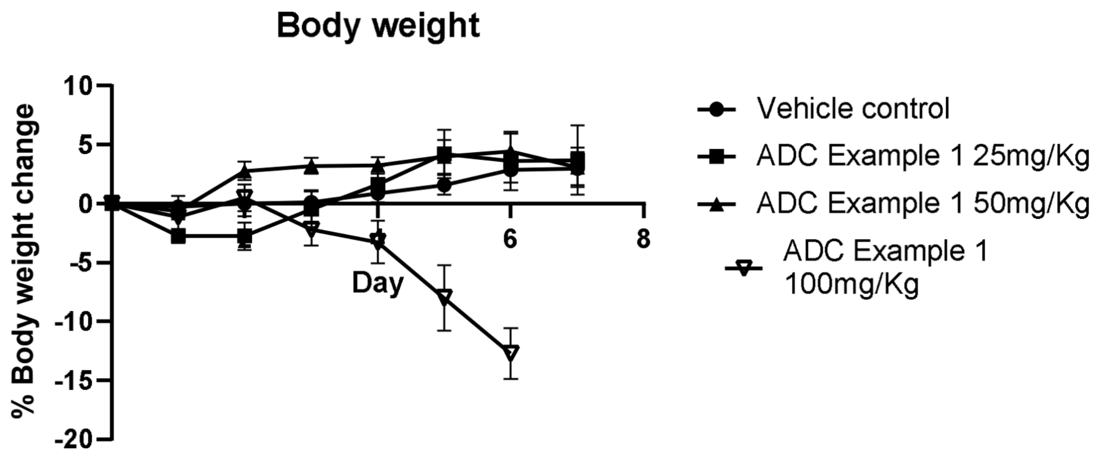


Figure 46

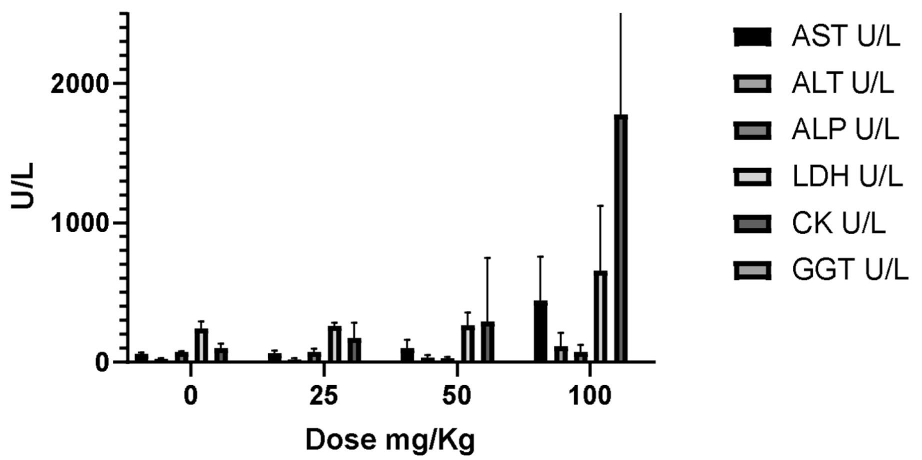


Figure 47

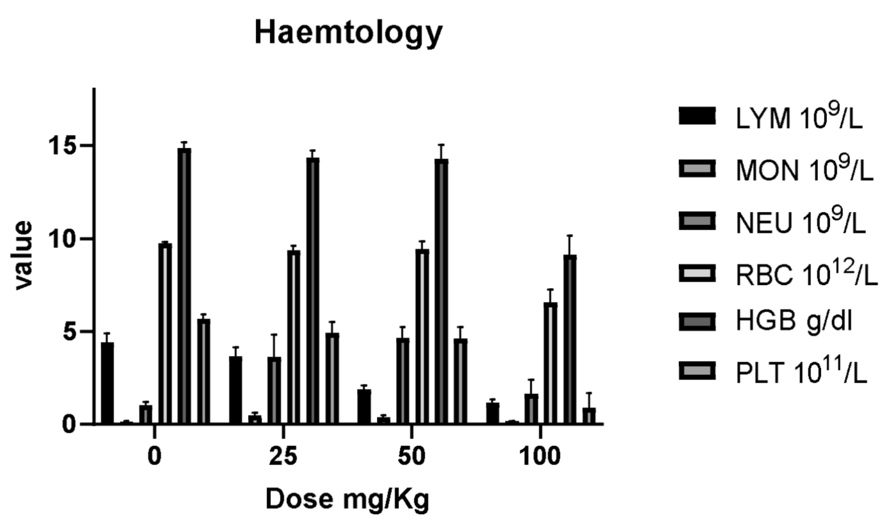

Figure 48

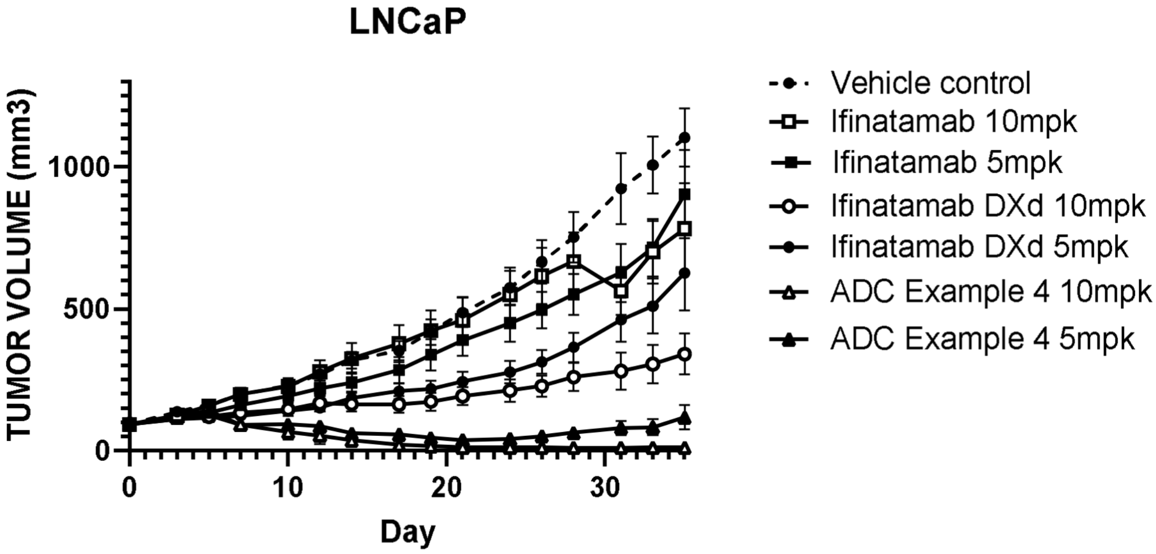


Figure 49

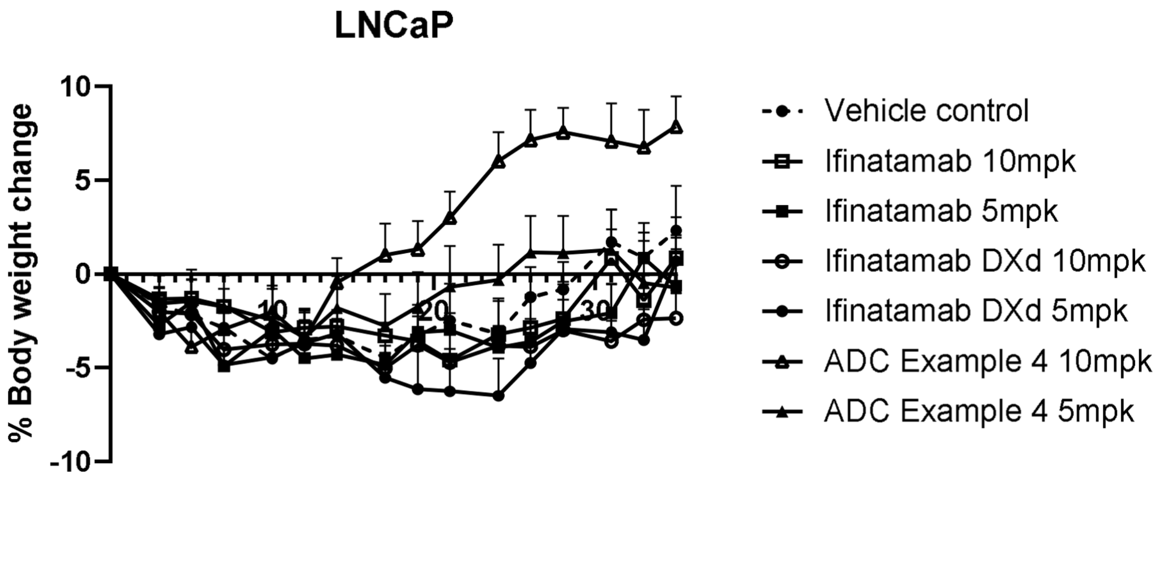

Figure 50

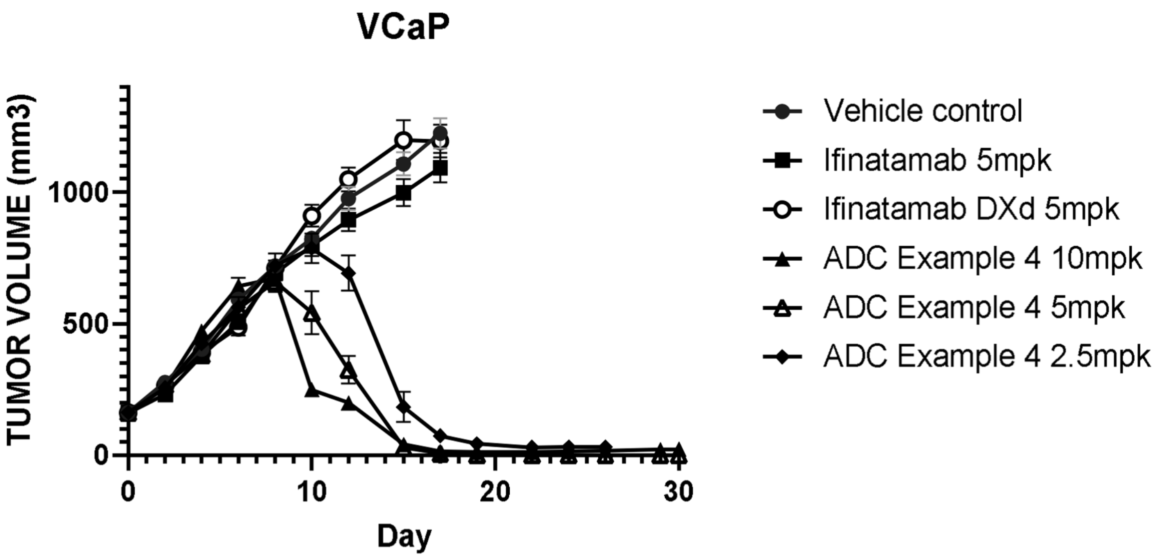


Figure 51

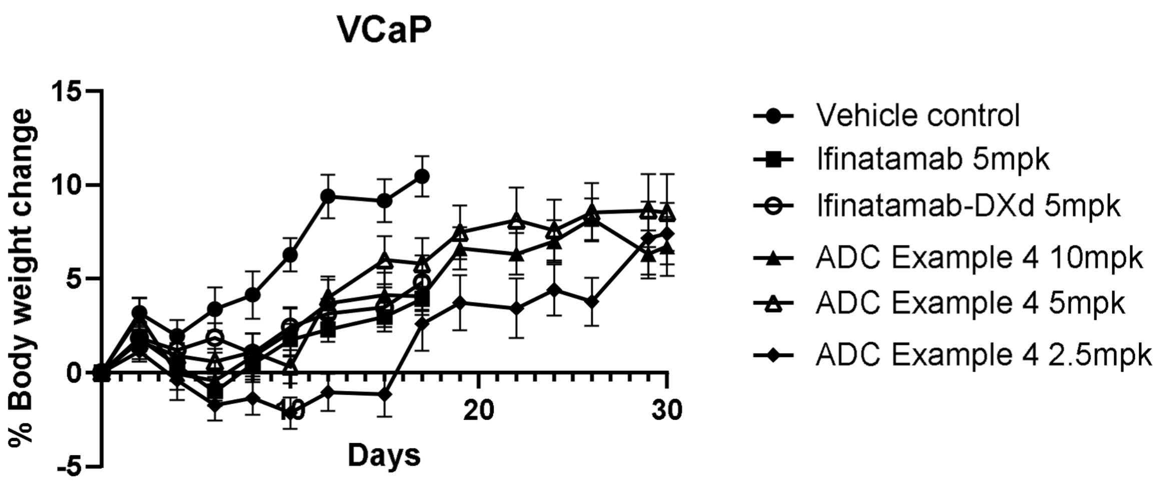

Figure 52

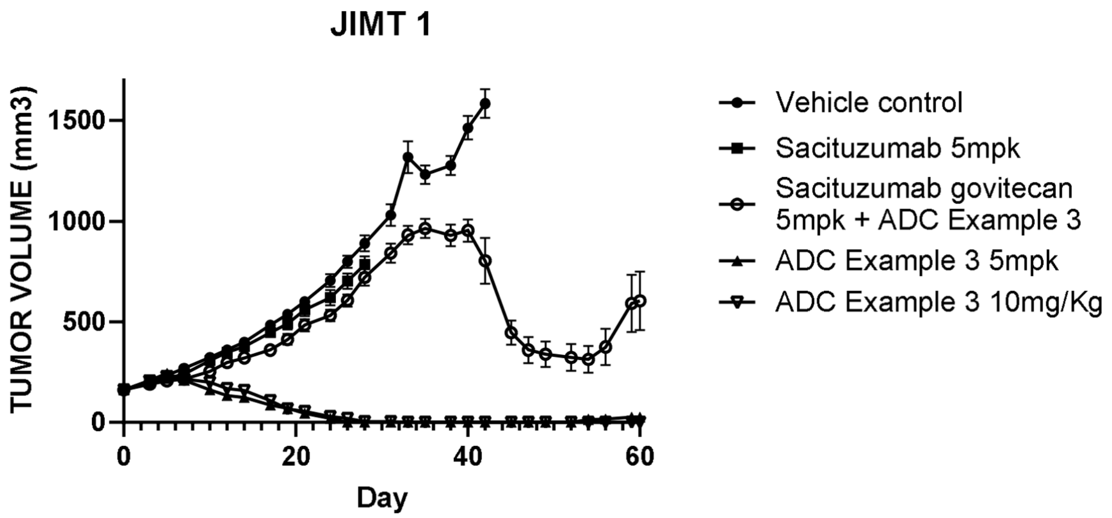


Figure 53

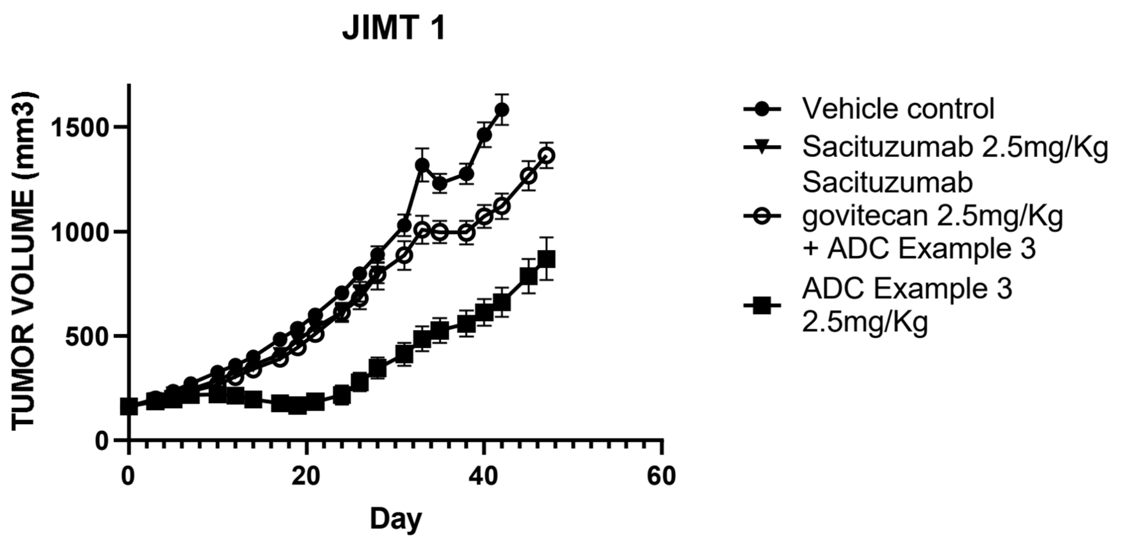


Figure 54

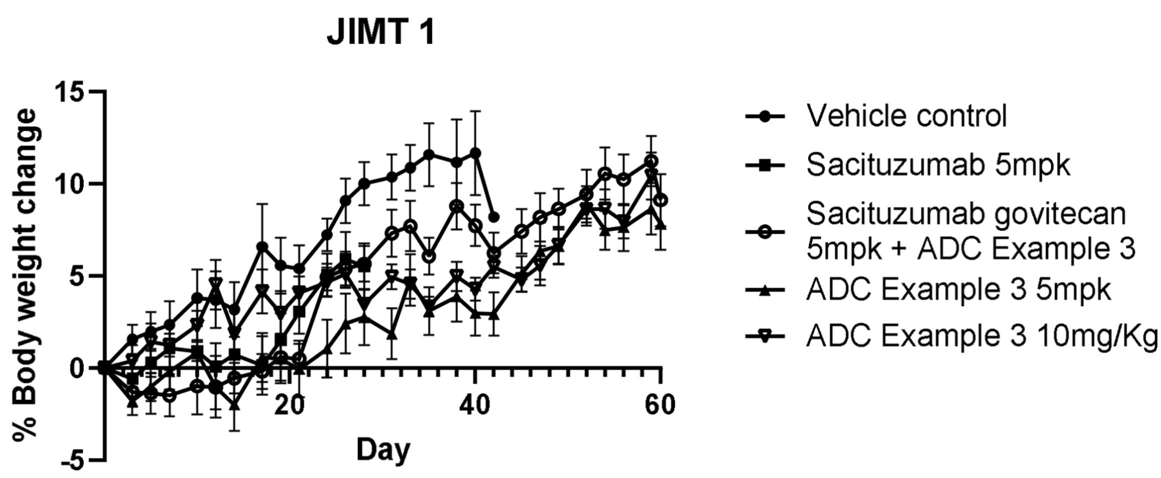

Figure 55

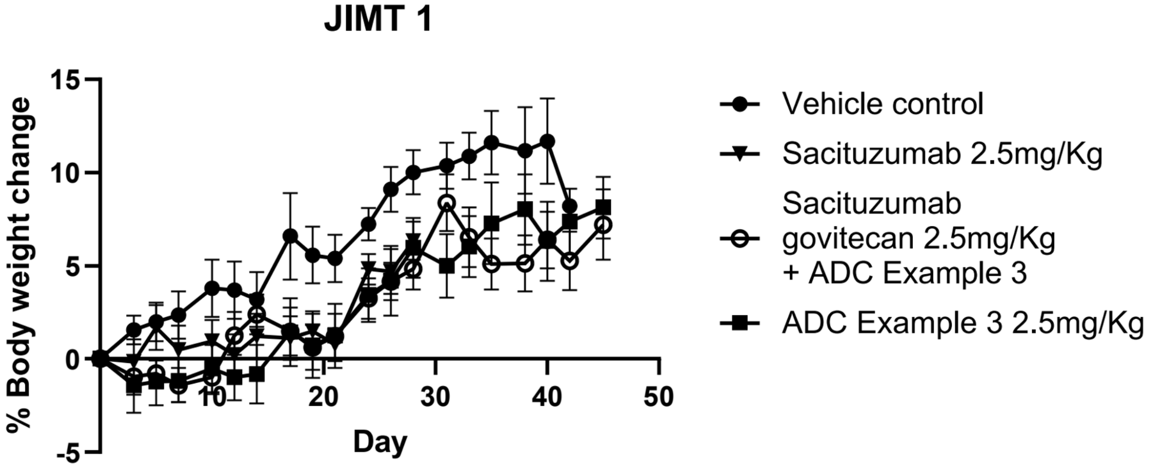


Figure 56

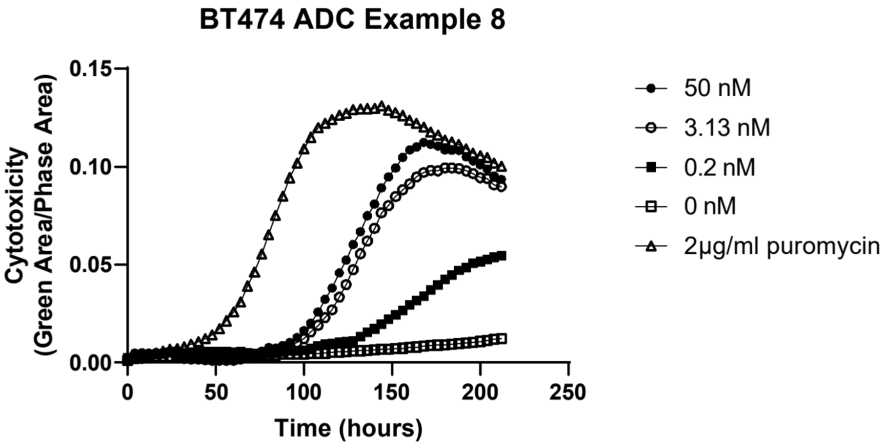


Figure 57

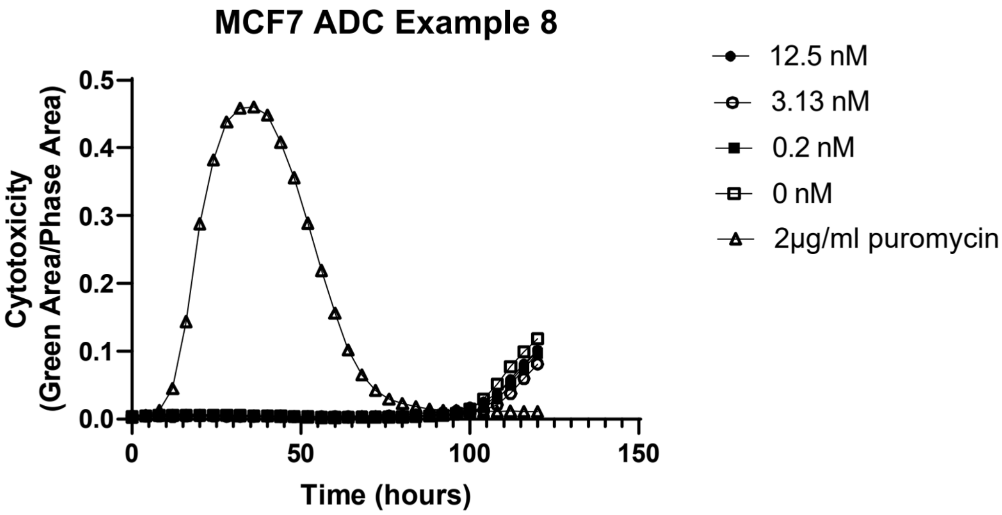

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7521541 B
- US 7723485 B
- WO 2009052249 A
- WO 2017011907 A, Bertiaume LG, Beuachamp E
- WO 0037464 A, Roche
- WO 2010026365 A
- WO 2013083991 A
- WO 2017001812 A
- WO 2020128473 A
- WO 2020128475 A
- WO 2022058745 A
- WO 2022090746 A
- WO 2022082306 A
- EP 22194959
- US 20050256030 A
- US 4816567 A
- US 20080206239 A1
- WO 2005079479 A2
- WO 2003038043 A


### Non-patent literature cited in the description


- **CARTER, P**. *Nature Reviews Immunology*, 2006, vol. 6, 343-357
- **XIE et al.** *Expert. Opin. Biol. Ther.*, 2006, vol. 6 (3), 281-291
- **KOVTUN**. *Cancer Res.*, 2006, vol. 66 (6), 3214-3121
- **LAW**. *Cancer Res.*, 2006, vol. 66 (4), 2328-2337
- **WU et al.** *Nature Biotech.*, 2005, vol. 23 (9), 1137-1145
- **LAMBERT J.** *Current Opin. in Pharmacol.*, 2005, vol. 5, 543-549
- **HAMANN P.** *Expert Opin. Ther. Patents*, 2005, vol. 15 (9), 1087-1103
- **PAYNE, G.** *Cancer Cell*, 2003, vol. 3, 207-212
- **TRAIL et al.** *Cancer Immunol. Immunother.*, 2003, vol. 52, 328-337
- **SYRIGOS ; EPENETOS**. *Anticancer Research*, 1999, vol. 19, 605-614
- **JUNUTULA et al.** *Nature Biotech.*, 2008, vol. 26 (8), 925-932
- **DORNAN et al.** *Blood*, 2009, vol. 114 (13), 2721-2729
- **MCDONAGH**. *Protein Eng. Design & Sei.*, 2006, vol. 19 (7), 299-307
- **DORONINA et al.** *Bioconj. Chem.*, 2006, vol. 17, 114-124
- **ERICKSON et al.** *Cancer Res.*, 2006, vol. 66 (8), 1-8
- **SANDERSON et al.** *Clin. Cancer Res.*, 2005, vol. 11, 843-852
- **JEFFREY et al.** *J. Med. Chem.*, 2005, vol. 48, 1344-1358
- **HAMBLETT et al.** *Clin. Cancer Res.*, 2004, vol. 10, 7063-7070
- **FARAZI, T.A. ; G. WAKSMAN ; J.I. GORDON**. *J. Biol. Chem.*, 2001, vol. 276 (43), 39501-39504
- **RESH MD**. *Biochern. Biophys.Acta*, 1993, vol. 1115, 307-22
- **GOTTLINGER HG ; SODROSKI JG ; HASELTINE WA**. *Proc. Nat. Acad. Sci. USA*, 1989, vol. 86, 5781-85
- **BRYANT ML ; RATNER L**. *Proc. Natl. Acad. Sci. USA*, 1990, vol. 87, 523-27
- **DAVIS MP ; BOTTLEY, G ; BEALES LP ; KILLINGTON, RA ; ROWLANDS DJ ; TUTHILL, TJ**. *Journal of Virology*, 2008, vol. 82, 4169-4174
- **MOUSNIER A ; BELL AS ; SWIEBODA DP ; MORALES-SANFRUTOS J ; PEREZ-DORADO I ; BRANNIGAN JA ; NEWMAN J ; RITZEFELD M ; HUTTON, JA ; GUEDAN A**. *Nature Chemistry*, 2018, vol. 10 (6), 599-606
- **CORBIC RAMLJAK I ; STANGER J ; REAL-HOHN A ; DREIER D ; WIMMER L ; REDLBERGER-FRITZ M ; FISCHL W ; KLINGEL K ; MIHOVILOVIC MD ; BLAAS D**. *PLOS Pathogens*, vol. 14 (8), e1007203
- **HERMANSON, G.T.** Bioconjugate Techniques. Academic Press, 1996, 234-242
- **DORONINA et al.** *Nat. Biotechnol.*, 2003, vol. 21, 778-784
- **HAY et al.** *Bioorg. Med. Chem. Lett.*, 1999, vol. 9, 2237
- **RODRIGUES et al.** *Chemistry Biology*, 1995, vol. 2, 223
- **STORM et al.** *J. Amer. Chem. Soc.*, 1972, vol. 94, 5815
- **AMSBERRY et al.** *J. Org. Chem.*, 1990, vol. 55, 5867

- **KINGSBURY et al.** *J. Med. Chem.*, 1984, vol. 27, 1447
- **JANEWAY, C** ; **TRAVERS, P** ; **WALPORT, M.** ; **SHLOMCHIK**. Immuno Biology. Garland Publishing, 2001
- **KOHLER et al.** *Nature*, 1975, vol. 256, 495
- **CLACKSON et al.** *Nature*, 1991, vol. 352, 624-628
- **MARKS et al.** *J. Mol. Biol.*, 1991, vol. 222, 581-597
- **LONBERG**. *Curr. Opinion*, 2008, vol. 20 (4), 450-459
- **MORRISON et al.** *Proc. Natl. Acad. Sci. USA*, 1984, vol. 81, 6851-6855
- *Nature Biotechnology*, 1994, vol. 12, 899-903
- **ALTUVIA et al.** *J. Mol. Biol.*, 1995, vol. 249, 244-250
- *Molecular Immunology*, 2007, vol. 44, 1986-1998
- *Methods*, 2005, vol. 36, 43-60
- **STEBBING, J.** ; **COPSON,E** ; **O'REILLY, S**. Herceptin (trastuzamab) in advanced breast cancer. *Cancer Treat Rev*, 2000, vol. 26, 287-90
- **MILLER et al.** *Journal of Immunology*, 2003, vol. 170, 4854-4861
- **TRAIL, P. A.** ; **WILLNER, D.** ; **LASCH, S. J** ; **HENDERSON, A. J.** ; **HOFSTEAD, S. J.** ; **CASAZZA, A. M.** ; **FIRESTONE, R. A.** ; **HELLSTROM, I.** ; **HELLSTROM, K. E**. Cure of Xenografited Human Carcinomas by BR96-Doxorubicin Immunoconjugates. *Science*, 1993, vol. 261, 212-215
- **TRAIL, PA** ; **WILLNER, D** ; **KNIPE, J.** ; **HENDERSON, A. J.** ; **LASCH, S. J** ; **ZOECKLER, M. E.** ; **TRAILSMITH, M. D.** ; **DOYLE, T. W** ; **KING, H. D.** ; **CASAZZA, A. M.** Effect of Linker Variation on the Stability, Potency, and Efficacy of Carcinoma-reactive BR64-Doxorubicin Immunoconjugates. *Cancer Research*, 1997, vol. 57, 100-105
- **FRANCISCO, J. A** ; **DONALDSON, K. L.** ; **CHACE, D** ; **SIEGALL, C. B.** ; **WAHL, A. F.** Agonistic properties and in vivo antitumor activity of the anti-CD-40 antibody, SGN-14. *Cancer Res.*, 2000, vol. 60, 3225-3231
- **BOWEN, M. A** ; **OLSEN, K. J.** ; **CHENG, L.** ; **AVILA, D** ; **PODACK, E. R**. Functional effects of CD30 on a large granular lymphoma cell line YT. *J. Immunol.*, 1993, vol. 151, 5896-5906
- **FRANKE, A. E.** ; **SIEVERS, E. L.** ; **SCHEINBERG, D. A.** Cell surface receptor-targeted therapy of acute myeloid leukemia: a review. *Cancer Biother Radiopharm.*, 2000, vol. 15, 459-76
- **MURRAY, J. L**. Monoclonal antibody treatment of solid tumors: a coming of age. *Semin Oncol.*, 2000, vol. 27, 64-70
- **BREITLING, F.** ; **DUBEL, S.** Recombinant Antibodies. John Wiley, and Sons, 1998
- **TONG et al.** *Molecules*, 2021, vol. 26, 5847, https://doi.org/10.3390/molecules26195847
- **COATS et al.** *Clin Cancer Res*, 2019, vol. 25, 5441-8
- **AXUP et al.** *Proc Natl Acad Sci USA.*, 2012, vol. 109 (40), 16101-16116
- Handbook of Pharmaceutical Additives. Synapse Information Resources, Inc., 2001
- Remington's Pharmaceutical Sciences. Lippincott, Williams & Wilkins, 2000
- Handbook of Pharmaceutical Excipients. 1994
- *Bioconjugate Chem*, 2006, vol. 17, 831-840
- **GONCALVES, V. et al.** *Analytical Biochemistry*, 2012, vol. 421, 342-344
- **GONCALVES, V. et al.** *J. Med. Chem*, 2012, vol. 55, 3578